# EUROPEAN PATENT SPECIFICATION

(11) **EP 4 493 195 B1**
(45) Date of publication and mention of the grant of the patent: **12.08.2026**
(21) Application number: 23716132.8
(22) Date of filing: 15.03.2023
(51) Int. Cl.: A61K 31/555, A61K 39/395, A61P 35/00, C07K 16/24, C07K 16/28

(54) **IL-18BP ANTAGONIST ANTIBODIES AND THEIR USE IN MONOTHERAPY AND COMBINATION THERAPY IN THE TREATMENT OF CANCER**
IL-18BP-ANTAGONIST-ANTIKÖRPER UND DEREN VERWENDUNG IN DER MONOTHERAPIE UND KOMBINATIONSTHERAPIE BEI DER BEHANDLUNG VON KREBS
ANTICORPS ANTAGONISTES DE L'IL-18BP ET LEUR UTILISATION EN MONOTHÉRAPIE ET POLYTHÉRAPIE DANS LE TRAITEMENT DU CANCER

(30) Priority: 15.03.2022 US 202263320202 P; 10.06.2022 US 202263351242 P; 06.01.2023 US 202363478898 P
(43) Date of publication of application: 22.01.2025
(73) Proprietor: Compugen Ltd., 5885849 Holon (IL)
(72) Inventor: NIELSON, Nels P., Lebanon, NH 03766 (US); CHIASSON, Alissa M., Lebanon, NH 03766 (US); MENACHEM, Assaf, 4423510 Kfar-Saba (IL); OPHIR, Eran, 4052431 Even Yehuda (IL); LEIDERMAN, Olga, 7557503 Rishon LeZion (IL); FRIDMAN-KFIR, Tal, 6936426 Tel Aviv (IL); GALPERIN, Moran, 6936426 Ashdod (IL); TILLEMAN, Hadas Galon, 4593000 Ramot HaShavim (IL); BLAT, Dan, 4372202 Raanana (IL); COJOCARU, Gad, 4724800 Ramat-HaSharon (IL); TOPORIK, Amir, 3701600 Pardes Hanah Carkur (IL); NOVIK, Amit, 3050000 Binyamina (IL); ERLICH, Ziv, 5363007 Giv'ataim (IL); ALTEBER, Zoya, 7420209 Nes Ziyona (IL); TATIROVSKY, Evgeny, 5254207 Ramat Gan (IL); PERPINIAL, Michal, 6329707 Tel Aviv (IL); SEVER, Iital, 6997712 Tel Aviv (IL); COHEN, Nadav, 7638767 Rehovot (IL)
(74) Representative: Carpmaels & Ransford LLP
(86) International application number: PCT/US2023/064460
(87) International publication number: WO 2023/178192

(56) References cited:
- WO-A1-2016/139297
- WO-A1-99/09063
- ZHOU TING ET AL: "IL-18BP is a secreted immune checkpoint and barrier to IL-18 immunotherapy", NATURE, NATURE PUBLISHING GROUP UK, LONDON, vol. 583, no. 7817, 24 June 2020 (2020-06-24), pages 609 - 614, XP037200074, ISSN: 0028-0836, [retrieved on 20200624], DOI: 10.1038/S41586-020-2422-6
- LEE SIYOUNG ET AL: "Development of Isoform-specific Monoclonal Antibodies Against Human IL-18 Binding Protein", HYBRIDOMA, vol. 29, no. 6, 1 December 2010 (2010-12-01), US, pages 517 - 524, XP093056264, ISSN: 1554-0014, DOI: 10.1089/hyb.2010.0058
- DETRY SAMMY ET AL: "Structural basis of human IL-18 sequestration by the decoy receptor IL-18 binding protein (IL-18BP) in inflammation and tumor immunity", BIORXIV, 10 February 2022 (2022-02-10), XP093055298, Retrieved from the Internet <URL:https://www.biorxiv.org/content/10.1101/2022.02.10.479912v1.full.pdf> [retrieved on 20230619], DOI: 10.1101/2022.02.10.479912
- Z. HE ET AL: "Interleukin-18 binding protein transgenic mice are protected against ischemic acute kidney injury", AJP: RENAL PHYSIOLOGY, vol. 295, no. 5, 20 August 2008 (2008-08-20), US, pages F1414 - F1421, XP055337145, ISSN: 0363-6127, DOI: 10.1152/ajprenal.90288.2008

## Description

### BACKGROUND OF THE INVENTION

Interleukin 18 (IL-18) is a pro-inflammatory cytokine that can stimulate T-cells, NK-cells, and myeloid cells. IL-18 has been proposed as an immunotherapeutic agent for the treatment of cancer, given its ability to stimulate anti-tumor immune cells. However, the clinical efficacy of IL-18 has been limited and as such there is a need for compositions and methods that provide effective IL-18 signaling activity to treat and prevent cancer and other diseases and disorders.

Interleukin 18 Binding Protein (IL18-BP) binds IL18, prevents the binding of IL18 to its receptor, and thus functions as an inhibitor of the proinflammatory cytokine, IL18. IL18-BP inhibits IL18-induced T and NK cell activation and proliferation, and pro-inflammatory cytokine production, resulting in reduced T and NK cell activity and T-helper type 1 immune responses.

It is an object of the invention to provide anti-IL18-BP antibodies for use in disease treatment. The present invention meets this need by providing anti-IL18-BP antibodies (including antigen-binding fragments), in particular anti-IL18-BP antibodies that block IL18-BP, that can be used in treating diseases such as cancer.

Zhou et al. (Nature. 2020 Jul;583(7817):609-614) describes IL-18BP as a secreted immune checkpoint and barrier to IL-18 immunotherapy.

Lee et al. (Hybridoma (Larchmt). 2010 Dec;29(6):517-24) describes development of isoform-specific monoclonal antibodies against human IL-18BP.

Detry et al. (J Biol Chem, 2022 May;298(5):101908) describes the structural basis of human IL-18 sequestration by the decoy receptor IL-18BP in inflammation and tumor immunity.

### BRIEF SUMMARY OF THE INVENTION

The present invention relates to compositions and methods related to anti-IL18-BP antibodies.

The present invention provides an anti-IL18-BP (interleukin-18 binding protein) antibody, wherein the anti-IL18-BP antibody specifically binds human IL18-BP and exhibits a binding affinity or KD equal to or lower than 1pM, as measured by solution equilibrium titration, and wherein said antibody comprises a vhCDR1, a vhCDR2, a vhCDR3, a vlCDR1, a vlCDR2 and a vlCDR3, wherein the vhCDR1, the vhCDR2, the vhCDR3, the vlCDR1, the vlCDR2 and the vlCDR3 are selected from the group consisting of:
i. a vhCDR1 having the amino acid sequence of SEQ ID NO: 155,
   a vhCDR2 having the amino acid sequence of SEQ ID NO: 156,
   a vhCDR3 having the amino acid sequence of SEQ ID NO: 157,
   a vlCDR1 having the amino acid sequence of SEQ ID NO: 160,
   a vlCDR2 having the amino acid sequence of SEQ ID NO: 161, and
   a vlCDR3 having the amino acid sequence of SEQ ID NO: 162;
ii. a vhCDR1 having the amino acid sequence of SEQ ID NO: 75,
   a vhCDR2 having the amino acid sequence of SEQ ID NO: 76,
   a vhCDR3 having the amino acid sequence of SEQ ID NO: 77,
   a vlCDR1 having the amino acid sequence of SEQ ID NO: 80,
   a vlCDR2 having the amino acid sequence of SEQ ID NO: 81, and
   a vlCDR3 having the amino acid sequence of SEQ ID NO: 82;
iii. a vhCDR1 having the amino acid sequence of SEQ ID NO: 85,
   a vhCDR2 having the amino acid sequence of SEQ ID NO: 86,
   a vhCDR3 having the amino acid sequence of SEQ ID NO: 87,
   a vlCDR1 having the amino acid sequence of SEQ ID NO: 90,
   a vlCDR2 having the amino acid sequence of SEQ ID NO: 91, and
   a vlCDR3 having the amino acid sequence of SEQ ID NO: 92;
iv. a vhCDR1 having the amino acid sequence of SEQ ID NO: 105,
   a vhCDR2 having the amino acid sequence of SEQ ID NO: 106,
   a vhCDR3 having the amino acid sequence of SEQ ID NO: 107,
   a vlCDR1 having the amino acid sequence of SEQ ID NO: 110,
   a vlCDR2 having the amino acid sequence of SEQ ID NO: 111, and
   a vlCDR3 having the amino acid sequence of SEQ ID NO: 112; and
v. a vhCDR1 having the amino acid sequence of SEQ ID NO: 195,
   a vhCDR2 having the amino acid sequence of SEQ ID NO: 196,
   a vhCDR3 having the amino acid sequence of SEQ ID NO: 197,
   a vlCDR1 having the amino acid sequence of SEQ ID NO: 200,
   a vlCDR2 having the amino acid sequence of SEQ ID NO: 201, and
      a vlCDR3 having the amino acid sequence of SEQ ID NO: 202.The present invention also provides the anti-IL18-BP antibody of the invention, for use in a method of treatment.

The present invention also provides the anti-IL18-BP antibody of the invention, wherein the antibody activates T cells, NK cells, NKT cells, Dendritic cells, MAIT T cells, γδ T cells, and/or innate lymphoid cells (ILCs), and/or modulates Myeloid cells, and wherein the antibody is for use in a method for the treatment of cancer.

The present invention also provides a composition comprising the anti-IL18-BP antibody of the invention.

The present invention also provides a composition comprising the anti-IL18-BP antibody of the invention for use according to the invention.

In some embodiments, the present invention provides compositions comprising the anti-IL18-BP (interleukin-18 binding protein) antibody of the invention for activating T cells, NK cells, NKT cells, Dendritic cells, MAIT T cells, γδ T cells, and/or innate lymphoid cells (ILCs), and/or modulating Myeloid cells, for use in the treatment of cancer, wherein the antibody antagonizes at least one immune inhibitory effect of IL18-BP, wherein the anti-IL18-BP antibody blocks the IL18 : IL18-BP binding interaction, and wherein the anti-IL18-BP antibody exhibits a binding affinity of lower than 1pM.

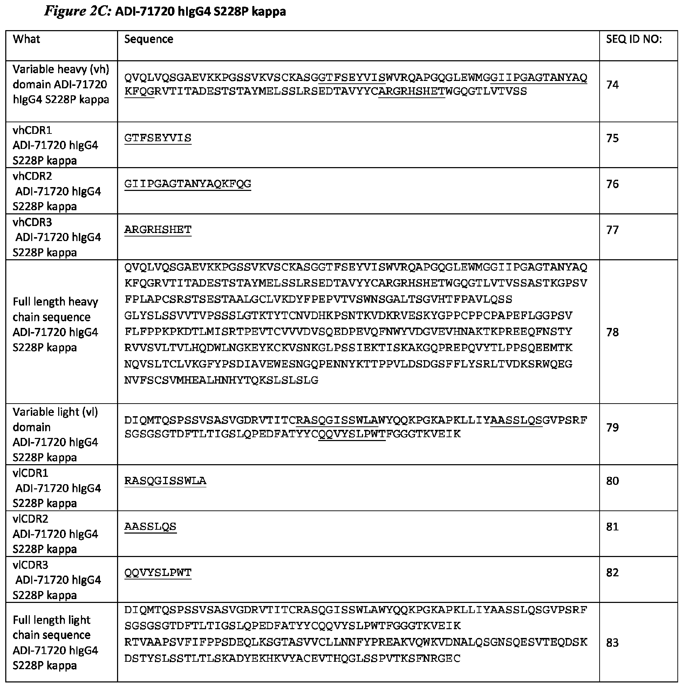

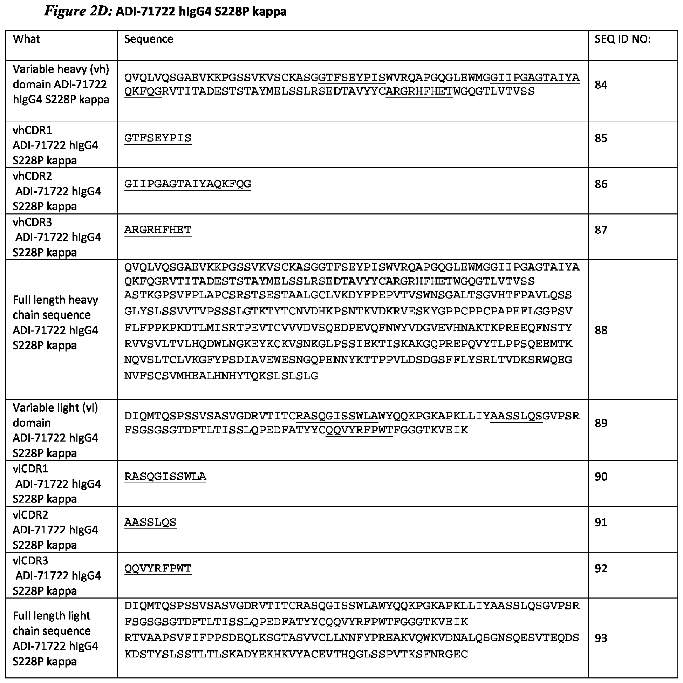

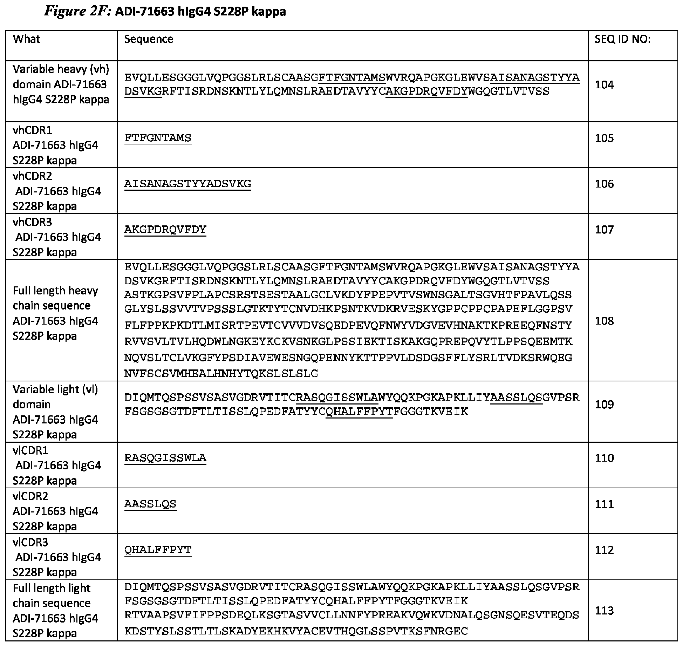

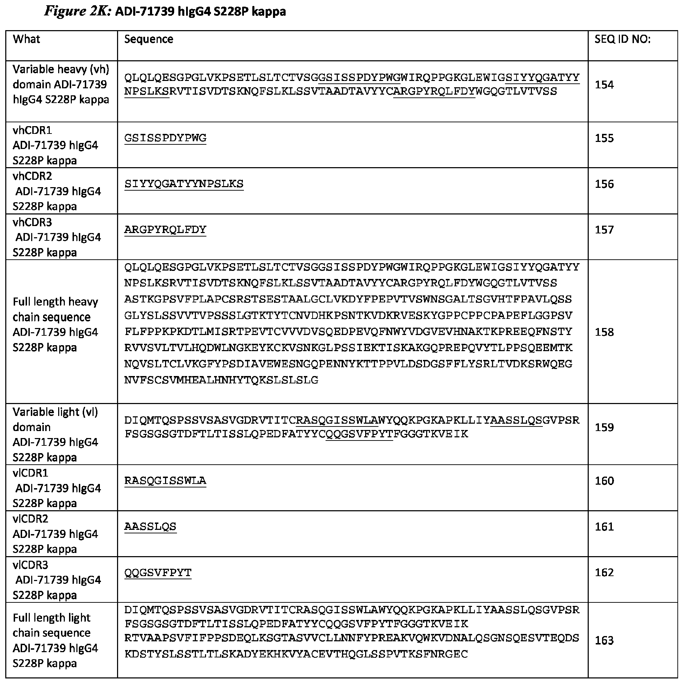

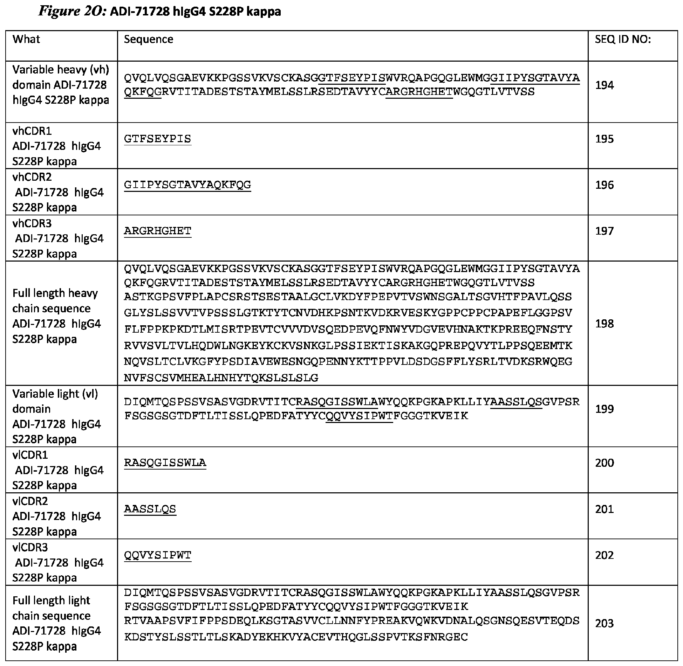

In some embodiments, the composition comprises an antibody, wherein the antibody comprises the heavy chain variable domain and the light chain variable domain of an antibody selected from the group consisting of:
i. the heavy chain variable domain (SEQ ID NO: 74) and the light chain variable domain (SEQ ID NO: 79) of Figure 2C (71720);
ii. the heavy chain variable domain (SEQ ID NO: 84) and the light chain variable domain (SEQ ID NO: 89) of Figure 2D (71722);
iii. the heavy chain variable domain (SEQ ID NO: 104) and the light chain variable domain (SEQ ID NO: 109) of Figure 2F (71663);
iv. the heavy chain variable domain (SEQ ID NO: 154) and the light chain variable domain (SEQ ID NO: 159) of Figure 2K (71739); and
v. the heavy chain variable domain (SEQ ID NO: 194) and the light chain variable domain (SEQ ID NO: 199) of Figure 2O (71728).

In some embodiments, the antibody comprises a CH1-hinge-CH2-CH3 region from human IgG1, IgG2, IgG3, or IgG4, wherein said hinge region optionally comprises mutations.

In some embodiments, the antibody comprises the CH1-hinge-CH2-CH3 region from human IgG4.

In some embodiments, the hinge region comprises mutations.

In some embodiments, the antibody comprises a CL region of human kappa 2 light chain.

In some embodiments, the antibody comprises a CL region of human lambda 2 light chain.

In some embodiments, the anti-IL18-BP antibody comprises:
i) a heavy chain variable domain comprising a sequence exhibiting at least 90%, at least 95%, or at least 98% identity to the heavy chain variable domain from ADI-71663, ADI-71722, ADI-71739, or ADI-71728, wherein each individual vhCDR comprises no substitutions, and
ii) a light chain variable domain comprising a sequence exhibiting at least 90%, at least 95%, or at least 98% identity to the light chain variable domain from ADI-71663, ADI-71722, ADI-71739, or ADI-71728, wherein each individual vlCDR comprises no substitutions.

In some embodiments, the anti IL18-BP antibody comprises:
i) a heavy chain variable domain comprising the vhCDR1, vhCDR2, and vhCDR3 from ADI-71663, ADI-71722, ADI-71739, or ADI-71728, and wherein said heavy chain variable domain comprises a sequence exhibiting at least 90% identity to the heavy chain variable domain from, ADI-71663, ADI-71722, ADI-71739, or ADI-71728, wherein each individual vhCDR comprises no substitutions, and
ii) a light chain variable domain comprising the vlCDR1, vlCDR2, and vlCDR3 from ADI-71663, ADI-71722, ADI-71739, or ADI-71728, and wherein said light chain variable domain comprises a sequence exhibiting at least 90% identity to the light chain variable domain from ADI-71663, ADI-71722, ADI-71739, or ADI-71728, wherein each individual vlCDR comprises no substitutions.

In some embodiments, the antibody comprises the heavy chain variable domain from ADI-71663, ADI-71722, ADI-71739, or ADI-71728 and the light chain variable domain from ADI-71663, ADI-71722, ADI-71739, or ADI-71728.

In some embodiments, the antibody comprises the CH1-hinge-CH2-CH3 region from human IgG4.

In some embodiments, the hinge region comprises mutations.

In some embodiments, the antibody comprises a CL region of human kappa 2 light chain.

In some embodiments, the antibody comprises a CL region of human lambda 2 light chain.

. Any reference to a method of treatment practised on the human or animal body is to be interpreted as substances and compositions for use in such treatments.

The present invention also provides the anti-IL18-BP antibody for use of the invention for use in methods of treating cancer in a patient, comprising administering the anti-IL18-BP antibody, and wherein said cancer is treated.

The present invention also provides the anti-IL18-BP antibody for use of the invention for use in methods of treating cancer in a patient, comprising administering the anti-IL18-BP antibody, wherein said anti-IL18-BP antibody activates T cells, NK cells, NKT cells, Dendritic cells, MAIT T cells, γδ T cells, and/or innate lymphoid cells (ILCs), and/or modulates Myeloid cells, and wherein said cancer is treated.

The present invention also provides the anti-IL18-BP antibody for use of the invention for use in methods of activating T-cells of a patient comprising administering the anti-IL18-BP antibody, and wherein said T-cells are activated.

The present invention also provides the anti-IL18-BP antibody for use of the invention for use in methods of activating NK-cells of a patient comprising administering the anti-IL18-BP antibody, and wherein said NK-cells are activated.

The present invention also provides the anti-IL18-BP antibody for use of the invention for use in methods of activating NKT-cells of a patient comprising administering the anti-IL18-BP antibody, and wherein said NKT-cells are activated.

The present invention also provides the anti-IL18-BP antibody for use of the invention for use in methods of modulating myeloid cells of a patient comprising administering the anti-IL18-BP antibody, and wherein said myeloid cells are modulated.

The present invention also provides the anti-IL18-BP antibody for use of the invention for use in methods of activating dendritic cells of a patient comprising administering the anti-IL18-BP antibody, and wherein said dendritic cells are activated.

The present invention also provides the anti-IL18-BP antibody for use of the invention for use in methods of activating MAIT T cells of a patient comprising administering the anti-IL18-BP antibody, and wherein said MAIT T cells are activated.

The present invention also provides the anti-IL18-BP antibody for use of the invention for use in methods of activating γδ T cells of a patient comprising administering the anti-IL18-BP antibody, and wherein said γδ T cells are activated.

The present invention also provides the anti-IL18-BP antibody for use of the invention for use in methods of activating ILC cells of a patient comprising administering the anti-IL18-BP antibody, and wherein said ILC cells are activated.

The present invention also provides the anti-IL18-BP antibody for use of the invention for use in methods of increasing IL-18 mediated immuno-stimulating activity in the tumor microenvironment (TME), and/or lymph nodes, comprising administering the anti-IL18-BP antibody, wherein said anti-IL18-BP antibody increases IL-18 mediated immuno-stimulating activity in the TME, and/or lymph nodes.

The present invention also provides the anti-IL18-BP antibody for use of the invention for use in methods of restoring IL-18 activity on T cells, NK cells, NKT cells, Myeloid cells, Dendritic cells, MAIT T cells, γδ T cells, and/or innate lymphoid cells (ILCs), comprising administering the anti-IL18-BP antibody, wherein said anti-IL18-BP antibody restores activity on T cells, NK cells, NKT cells, Myeloid cells, Dendritic cells, MAIT T cells, γδ T cells, and/or innate lymphoid cells (ILCs).

In some embodiments, the anti-IL18-BP antibody is administered as a stable liquid pharmaceutical formulation.

In some embodiments, the T-cells are cytotoxic T-cells (CTLs).

In some embodiments, the T-cells are selected from the group consisting of CD4⁺ T-cells and CD8⁺ T-cells.

In some embodiments, the subject for treatment comprises an increase in tumor growth inhibition of at least about 10%, 20%, 30%, 40%, 50%, 60%, 70%, 80%, 90%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000%, as compared to a control or an untreated patient.

In some embodiments, the subject for treatment exhibits a decrease in tumor growth of at least about 10%, 20%, 30%, 40%, 50%, 60%, 70%, 80%, 90%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000%, as compared to a control or an untreated patient.

In some embodiments of the anti-IL18-BP antibody for use of the invention, the NK-cells are CD16+ lymphocytes.

In some embodiments of the anti-IL18-BP antibody for use of the invention, the NK-cells are CD56+ NK cells.

In some embodiments of the anti-IL18-BP antibody for use of the invention, the activation is measured as an increase in expression of one or more activation makers.

In some embodiments of the anti-IL18-BP antibody for use of the invention, the activation markers are selected from the group consisting of CD107a, CD137, CD69, granzyme, and perforin.

In some embodiments of the anti-IL18-BP antibody for use of the invention, the activation is measured as an increase in proliferation of said NK-cells.

In some embodiments of the anti-IL18-BP antibody for use of the invention, the activation is measured as an increase in secretion of one or more cytokines.

In some embodiments of the anti-IL18-BP antibody for use of the invention, the one or more cytokines is selected from the group consisting of IFNy, TNF, GMCSF, MIG (CXCL9), IP-10 (CXCL10) and MCP1 (CCL2).

In some embodiments of the anti-IL18-BP antibody for use of the invention, the activation is measured as an increase in direct killing of target cells.

In some embodiments, the use of the anti-IL18-BP antibody for use of the invention further comprises administering a second antibody.

In some embodiments, the second antibody is an antibody that binds to and/or inhibits a human checkpoint receptor protein.

In some embodiments, the second antibody is selected from the group consisting of an anti-PVRIG antibody, an anti-PD-1 antibody, an anti-PD-L1 antibody, an anti-TIGIT antibody, an anti-CTLA-4 antibody, an anti-PD-L2 antibody, an anti-B7-H3 antibody, an anti B7-H4 antibody, an anti-CEACAM-1 antibody, an anti-PVR antibody, an anti-LAG3 antibody, an anti-CD112 antibody, an anti-CD96 antibody, an anti-TIM3 antibody, an anti-BTLA antibody, an anti-ICOS antibody, an anti-OX40 antibody, or an anti-41BB antibody, an anti-CD27 antibody, or an anti-GITR antibody.

In some embodiments, the PVRIG antibody is selected from the group consisting of CHA.7.518.1.H4(S241P) and CHA.7.538.1.2.H4(S241P).

In some embodiments, the anti-PVRIG antibody comprises: i) a heavy chain variable domain comprising the vhCDR1, vhCDR2, and vhCDR3 from CHA.7.518.1.H4(S241P) (SEQ ID NO:260) and ii) a light chain variable domain comprising the vlCDR1, vlCDR2, and vlCDR3 from CHA.7.518.1.H4(S241P) (SEQ ID NO:265).

In some embodiments, the anti-PVRIG antibody comprises: i) a heavy chain variable domain comprising the vhCDR1, vhCDR2, and vhCDR3 from CHA.7.538.1.2.H4(S241P) (SEQ ID NO:270) and ii) a light chain variable domain comprising the vlCDR1, vlCDR2, and vlCDR3 from CHA.7.538.1.2.H4(S241P) (SEQ ID NO:275).

In some embodiments, the anti-PVRIG antibody comprises: i) a heavy chain variable domain comprising the vhCDR1, vhCDR2, and vhCDR3 from CHA.7.518.4 (SEQ ID NO:1453; Figure 36AG) and ii) a light chain variable domain comprising the vlCDR1, vlCDR2, and vlCDR3 from CHA.7.518.4 (SEQ ID NO:1457; Figure 36AG).

In some embodiments, the anti-PVRIG antibody is selected from the group consisting of GSK4381562/SRF816 (GSK/Surface), NTX2R13(Nectin Therapeutics), an anti-PVRIG antibody as described in WO 2017/041004, an anti-PVRIG antibody antibody as described in WO 2001/008879, an anti-PVRIG antibody as described in WO 2018/017864, and an anti-PVRIG antibody as described in WO 2118/000205.

In some embodiments, the anti-TIGIT antibody is selected from the group consisting of CPA.9.083.H4(S241P) and CPA.9.086.H4(S241P).

In some embodiments, the anti-TIGIT antibody comprises: i) a heavy chain variable domain comprising the vhCDR1, vhCDR2, and vhCDR3 from CPA.9.083.H4(S241P) (SEQ ID NO:350) and ii) a light chain variable domain comprising the vlCDR1, vlCDR2, and vlCDR3 from CPA.9.083.H4(S241P) (SEQ ID NO:355).

In some embodiments, the anti-TIGIT antibody comprises: i) a heavy chain variable domain comprising the vhCDR1, vhCDR2, and vhCDR3 from CPA.9.086.H4(S241P) (SEQ ID NO:360) and ii) a light chain variable domain comprising the vlCDR1, vlCDR2, and vlCDR3 from CPA.9.086.H4(S241P) (SEQ ID NO:365).

In some embodiments, the anti-TIGIT antibody comprises: i) a heavy chain variable domain comprising the vhCDR1, vhCDR2, and vhCDR3 from CHA.9.547.18 (SEQ ID NO:1177; Figure 34QQQQ) and ii) a light chain variable domain comprising the vlCDR1, vlCDR2, and vlCDR3 from CHA.9.547.18 (SEQ ID NO:1181; Figure 34QQQQ).

In some embodiments, the anti-TIGIT antibody is selected from the group consisting of EOS-448 (GlaxoSmithKline, iTeos Therapeutics), BMS-986207, domvanalimab (AB154, Arcus Biosciences, Inc.), AB308 (Arcus Bioscience), Ociperlimab (aBGB-A1217, BeiGene), Tiragolumab (MTIG7192A, RocheGenentech), BAT6021 (Bio-Thera Solutions),BAT6005 (Bio-Thera Solutions), IBI939 (Innovent Biologics, US2021/00040201), JS006 (Junshi Bioscience/COHERUS), ASP8374 (Astellas Pharma Inc), Vibostolimab (MK-7684, Merck Sharp & Dohme), M6332 (Merck KGAA), Etigiliimab (OMP-313M32, Mereo BioPharma), SEA-TGT (Seagen)y, HB0030 (Huabo Biopharma), AK127 (AKESO), IBI939 (Innovent Biologics), and anti-TIGIT antibodies include the Genentech antibody (MTIG7192A).

In some embodiments, the anti-PD-1 antibody is selected from the group consisting of nivolumab (Opdivo^{®}; BMS; CheckMate078), pembrolizumab (KEYTRUDA^{®}; Merck), TSR-042 (Tesaro), cemiplimab (REGN2810; Regeneron Pharmaceuticals, see US20170174779), BMS-936559, Spartalizumab (PDR001, Novartis), pidilizumab (CT-011; Pfizer Inc), Tislelizumab (BGB-A317, BeiGene), Camrelizumab (SHR-1210, Incyte and Jiangsu HengRui), SHR-1210 (CTR20170299 and CTR20170322), SHR-1210 (CTR20160175 and CTR20170090), Sintilimab(Tyvyt^{®}; Eli lily and Innovent Biologics), Toripalimab (JS001, Shanghai Junshi Bioscience), JS-001 (CTR20160274), IBI308 (CTR20160735), BGB-A317 (CTR20160872), Penpulimab (AK105, Akeso Biopharma), Zimberelimab (Arcus), BAT1306 (Bio-Thera Solutions Ltd), Sasanlimab (PF-06801591, pfizer), Dostarlimab-gxly (GlaxoSmithKline LLC), Prolgolimab (Biocad), Cadonilimab (Akeso Inc), Geptanolimab (Genor BioPharma Co Ltd), Serplulimab (Shanghai Henlius Biotech Inc), Balstilimab (Agenus Inc), Retifanlimab (Incyte Corp), Cetrelimab (Johnson & Johnson), CS-1003 (EQRx Inc), IBI-318 (Innovent Biologics Inc), Ivonescimab (Akeso Inc), Pucotenlimab (Lepu Biopharma Co Ltd), QL-1604 (Qilu Pharmaceutical Co Ltd), SCTI-10A (SinoCelltech Group Ltd), Tebotelimab (MacroGenics Inc), AZD-7789 (AstraZeneca Plc), Budigalimab (AbbVie Inc), EMB-02 (EpimAb Biotherapeutics Inc), Ezabenlimab (Boehringer Ingelheim International GmbH), F-520 (Shandong New Time Pharmaceutical Co Ltd), HX-009 (Waterstone Hanxbio Pty Ltd), Zeluvalimab (Amgen), Peresolimab (Eli Lilly and Co), Rosnilimab (AnaptysBio Inc), Vudalimab (Xencor), Izuralimab (Xencor), Lorigerlimab (MacroGenics Inc), YBL-006 (Y-Biologics Inc), and ONO-4685 (Ono Pharmaceutical Co Ltd), LY-3434172 (Eli Lilly and Co).

In some embodiments, the anti-PD-L1 antibody is selected from the group consisting of atezolizumab (TECENTRIQ^{®}; MPDL3280A; IMpower110; Roche/Genentech), avelumab (BAVENCIO^{®}; MSB001071 8C; EMD Serono & Pfizer), and Durvalumab (MEDI4736; IMFINZI^{®}; AstraZeneca). And other antibodies under development, for example, Lodapolimab (LY3300054, Eli Lily), Pimivalimab (Jounce Therapeutics Inc), SHR-1316 (Jiangsu Hengrui Medicine Co Ltd), Envafolimab (Jiangsu Simcere Pharmaceutical Co Ltd), sugemalimab (CStone Pharmaceuticals Co Ltd), cosibelimab (Checkpoint Therapeutics Inc), pacmilimab (CytomX Therapeutics Inc), IBI-318, IBI-322, IBI-323 (Innovent Biologics Inc), INBRX-105 (Inhibrx Inc), KN-046 (Alphamab Oncology), 6MW-3211 (Mabwell Shanghai Bioscience Co Ltd), BNT-311 (BioNTech SE), FS-118 (F-star Therapeutics Inc), GNC-038 (Systimmune Inc), GR-1405 (Genrix (Shanghai) Biopharmaceutical Co Ltd), HS-636 (Zhejiang Hisun Pharmaceutical Co Ltd), LP-002 (Lepu Biopharma Co Ltd), PM-1003 (Biotheus Inc), PM-8001 (Biotheus Inc), STIA-1015 (ImmuneOncia Therapeutics LLC), ATG-101 (Antengene Corp Ltd), BJ-005 (BJ Bioscience Inc), CDX-527 (Celldex Therapeutics Inc), GNC-035 (Systimmune Inc), GNC-039( Systimmune Inc), HLX-20 (Shanghai Henlius Biotech Inc), JS-003 (Shanghai Junshi Bioscience Co Ltd), LY-3434172 (Eli Lilly and Co), MCLA-145 (Merus NV), MSB-2311 (Transcenta Holding Ltd), PF-07257876 (Pfizer Inc), Q-1802 (QureBio Ltd), QL-301 (QLSF Biotherapeutics Inc), QLF-31907 (Qilu Pharmaceutical Co Ltd), RC-98 (RemeGen Co Ltd), TST-005 (Transcenta Holding Ltd), Atezolizumab (IMpower133), BMS-936559/MDX-1105, and/or RG-7446/MPDL3280A, and YW243.55.S70.

In some embodiments, the anti-IL18-BP antibody and the second antibody are administered sequentially or simultaneously, in any order, and in one or more formulations.

In some embodiments, the anti-IL18-BP antibody is for use in combination with an immunostimulatory antibody, a cytokine therapy, or an immunomodulatory drug, cytotoxic agents, chemotherapeutic agents, growth inhibitory agents, anti-hormonal agents, kinase inhibitors, anti-angiogenic agents, cardioprotectants, immunosuppressive agents, agents that promote proliferation of hematological cells, angiogenesis inhibitors, protein tyrosine kinase (PTK) inhibitors, or other therapeutic agents.

In some embodiments, the method further comprises administering one or more inflammasome activators.

In some embodiments, the inflammasome activator is an chemotherapy agent.

In some embodiments, the chemotherapy agent is selected from the group consisting of Platinum, Paclitaxel (taxol), Sorafenib, Doxorubicin, Sorafenib, 5-FU, Gemcitabine, and Irinotecan (CPT-11).

In some embodiments, the Platinum chemotherapy agent is Oxaliplatin or Cisplatin.

In some embodiments, the inflammasome activator is a CD39 inhibitor.

In some embodiments, the CD39 inhibitor is an anti-CD39 antibody.

In some embodiments, the cancer is selected from the group consisting of vascularized tumors, melanoma, non-melanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, primary peritoneal ovarian carcinoma, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma, post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), melanoma, malignant melanoma, metastatic melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, pleural mesothelioma, chordoma, endometrial sarcoma, chondrosarcoma, uterine sarcoma, uveal melanoma, amyloidosis, AL-amyloidosis, astrocytoma, and/or Myelodysplastic syndromes (MDS).

In some embodiments, the cancer is selected from the group consisting of renal clear cell carcinoma (RCC), lung cancer, NSCLC, lung adenocarcinoma, lung squamous cell carcinoma, gastric adenocarcinoma, ovarian cancer, endometrial cancer, breast cancer, triple negative breast cancer (TNBC), head and neck tumor, colorectal adenocarcinoma, melanoma, and metastatic melanoma.

The present invention also provides for the anti-IL18BP antibody of the invention for use in the treatment of cancer by activating T cells, NK cells, NKT cells, Dendritic cells, MAIT T cells, γδ T cells, and/or innate lymphoid cells (ILCs), and/or modulating Myeloid cells in a patient. The present invention also provides for the anti-IL18BP antibody of the invention for use in increasing IL-18 mediated immuno-stimulating activity in the tumor microenvironment (TME), and/or lymph nodes.

The present invention also provides for the anti-IL18BP antibody of the invention for use in treating cancer in a recipient patient.

The present invention also provides for an anti-IL18BP antibody of the invention for use in combination with a second antibody. In some embodiments, the second antibody is selected from the group consisting of an anti-PVRIG antibody, an anti-PD-1 antibody, an anti-PD-L1 antibody, and an anti-TIGIT antibody.

The present invention also provides for an anti-IL18BP antibody of the invention wherein the anti-IL18-BP antibody exhibits a binding affinity or KD of less than 0.005 pM, 0.01 pM, 0.02 pM, 0.03 pM, 0.04 pM, 0.05 pM, 0.06 pM, 0.07 pM, 0.08 pM, 0.09 pM, 0.10 pM, 0.15 pM, 0.20 pM, 0.25 pM, 0.30 pM, 0.35 pM, 0.40 pM, 0.45 pM, 0.50 pM, 0.55 pM, 0.60 pM, 0.65 pM, 0.70 pM, 0.75 pM, 0.80 pM, 0.85 pM, 0.90 pM, 0.95 pM, or 1 pM.

Any references to methods of treatment of the human (or animal) body in the subsequent paragraphs of this description are to be interpreted, when read in the context of the claimed invention, as references to the claimed antibody and/or the claimed composition for use in the relevant methods of treatment of the human (or animal) body.

### BRIEF DESCRIPTION OF THE DRAWINGS

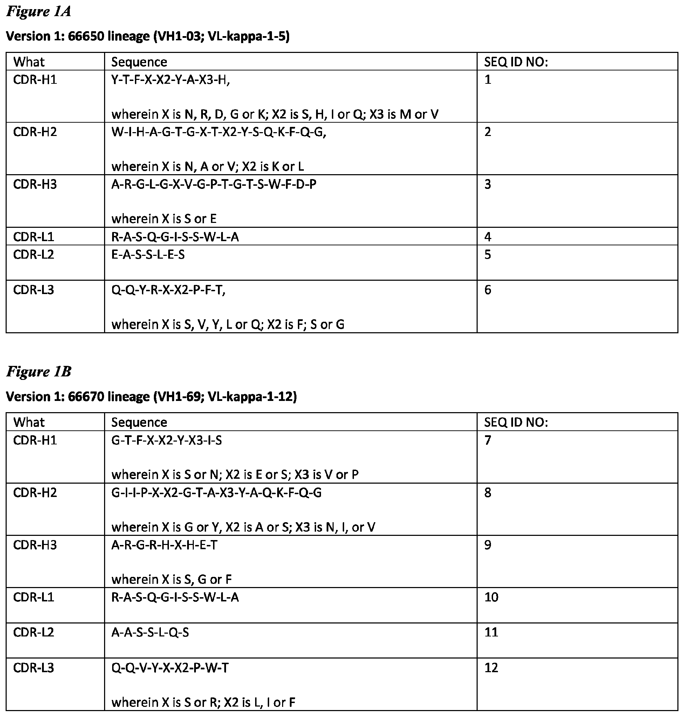

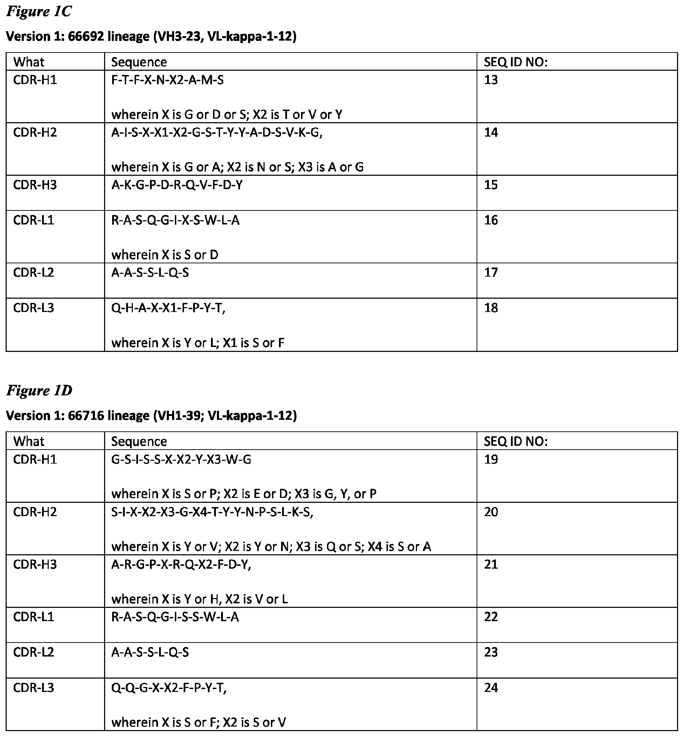

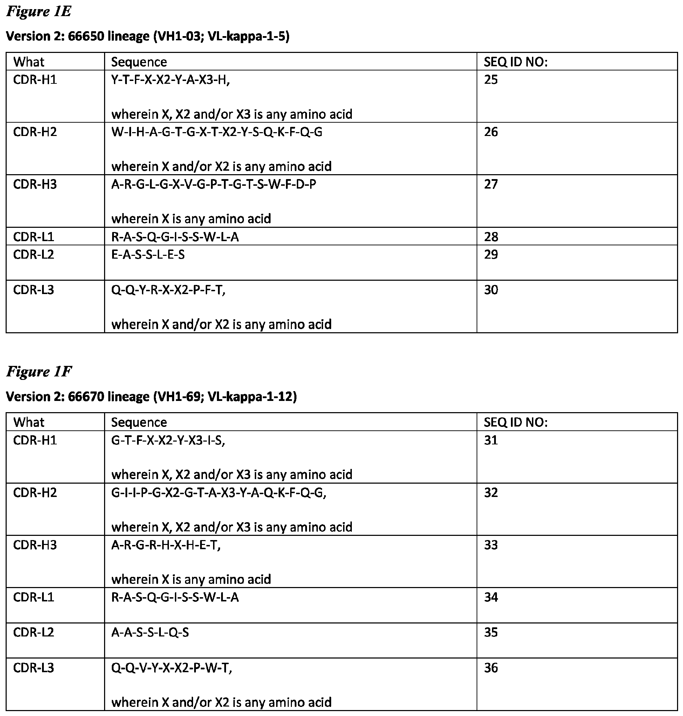

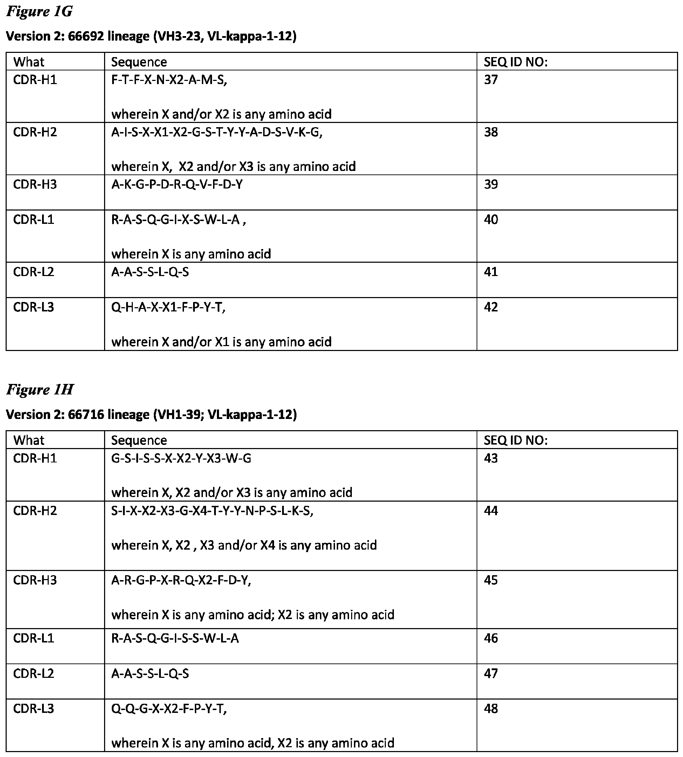

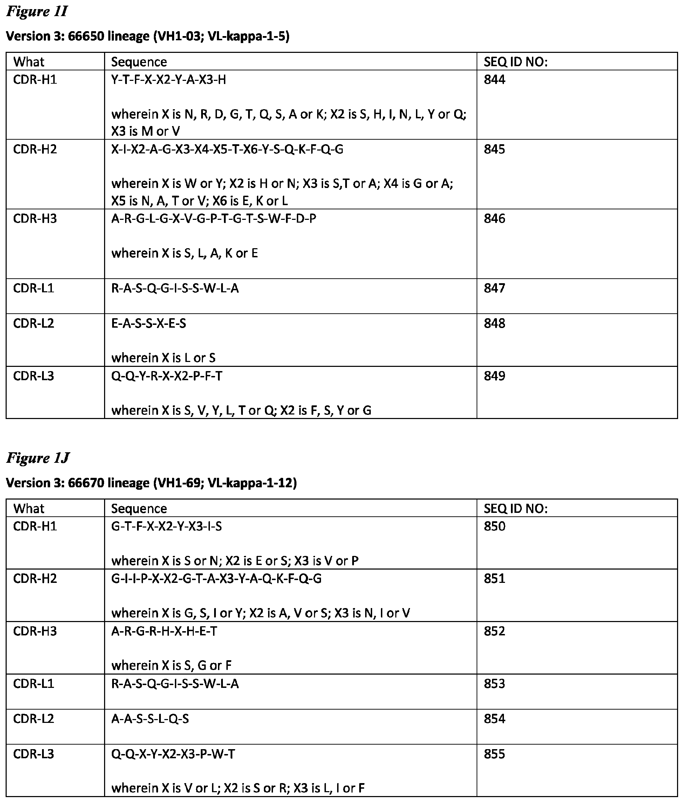

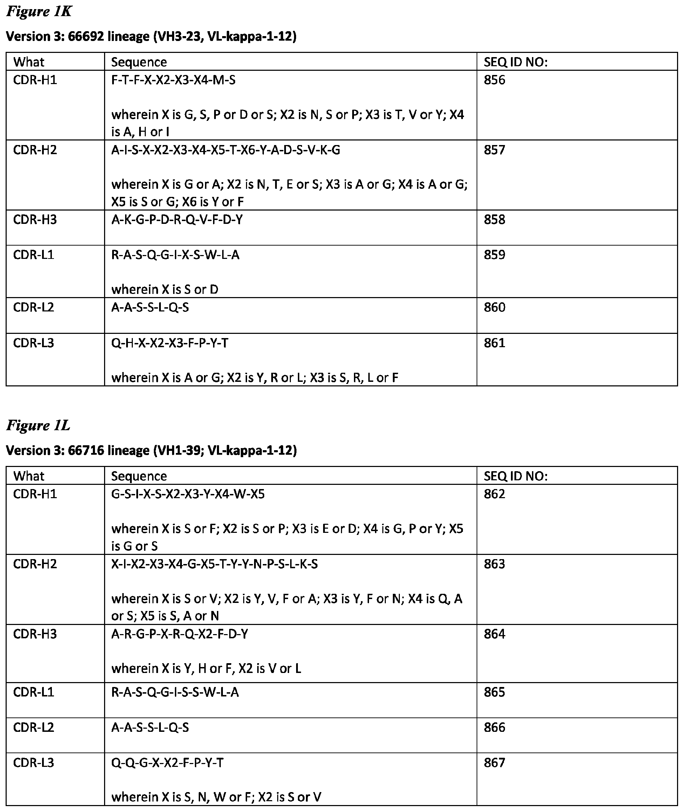

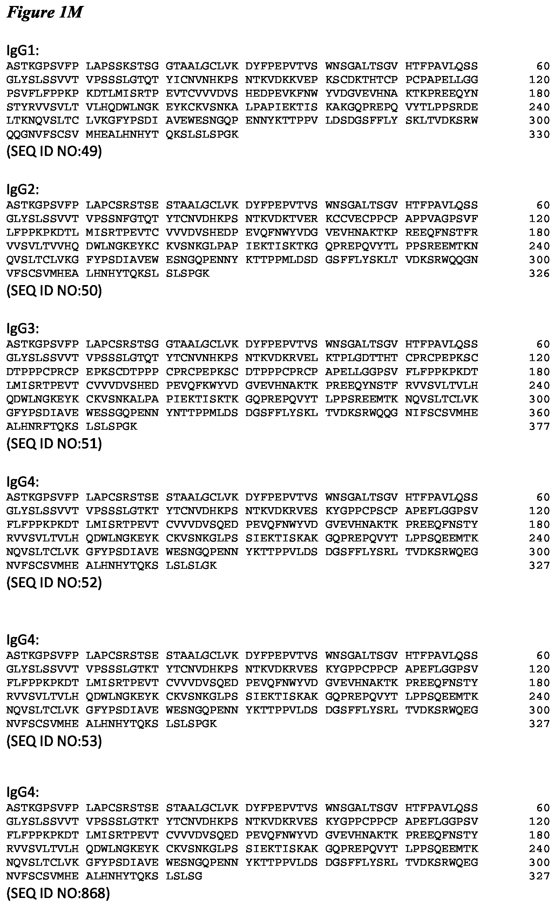

Figure 1A-1L depict the vhCDR1, vhCDR2, vhCDR3, vlCDR1, vlCDR2, vlCDR3 sequence of antibody 66650 (Figure 1A and 1E and 1I), 66670 (Figure 1B and 1F and 1J), 66692 (Figure 1C and 1G and 1K), 66716 (Figure 1D and 1H and 1L). Figure 1M provides IgG sequences, including IgG1, IgG2, IgG3 and IgG4.

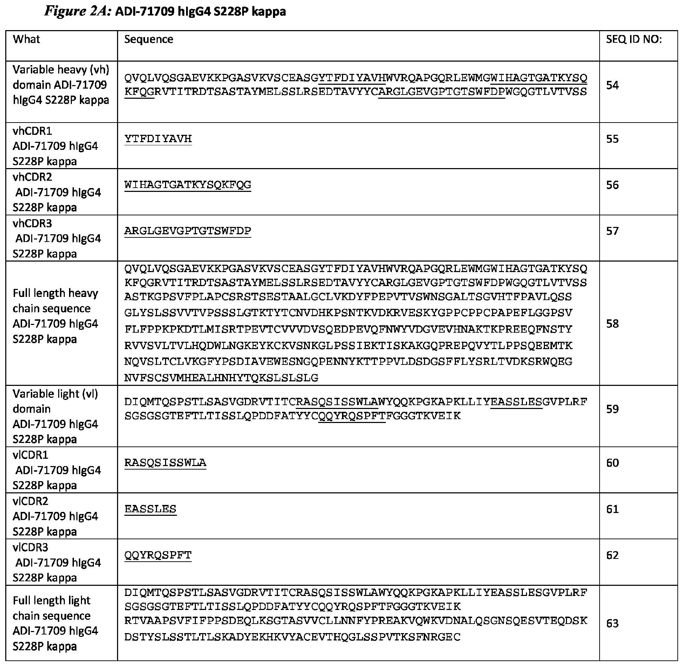

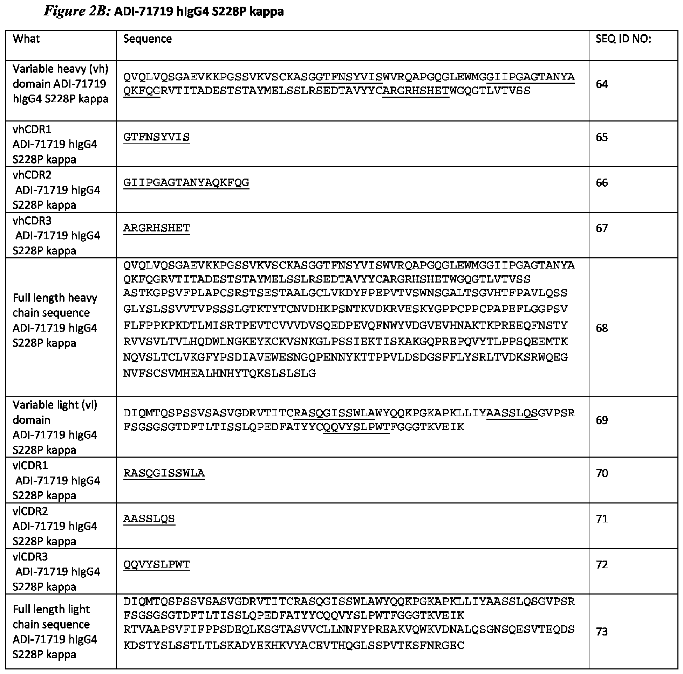

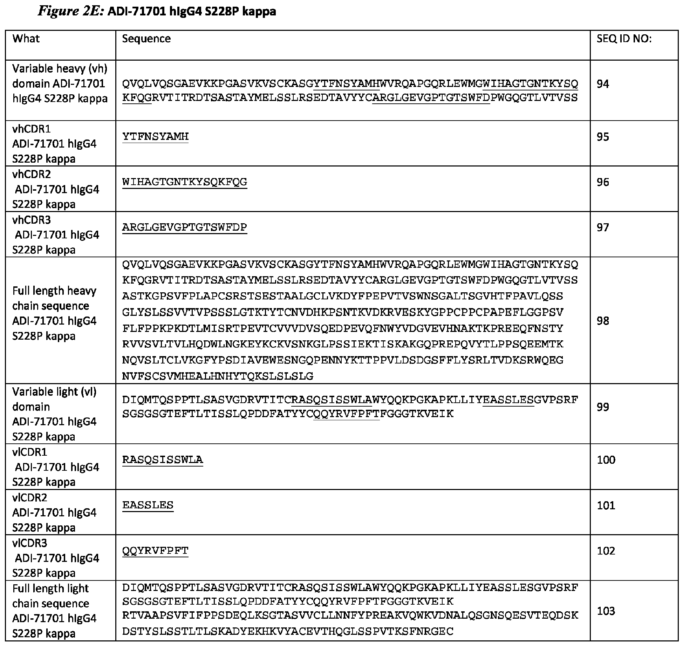

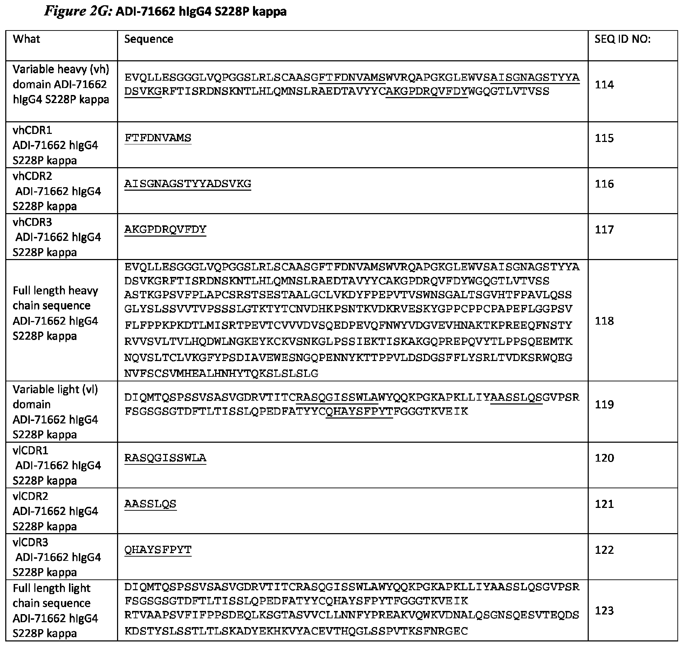

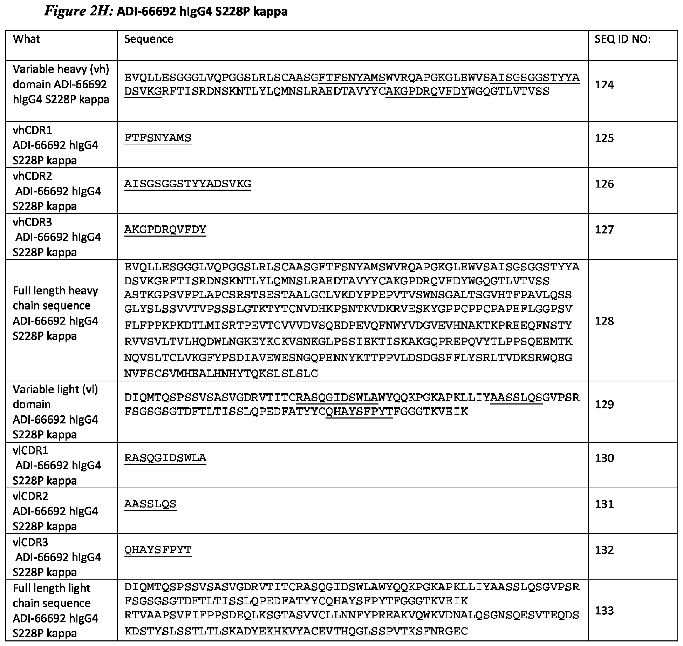

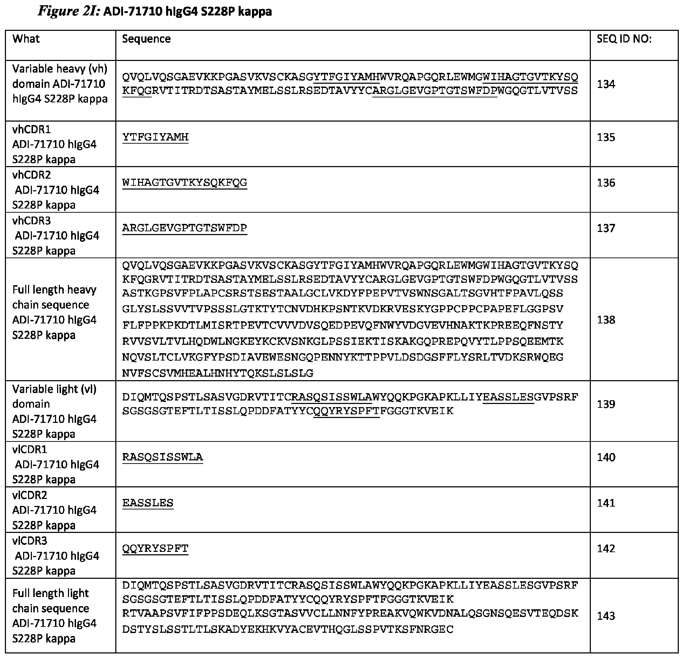

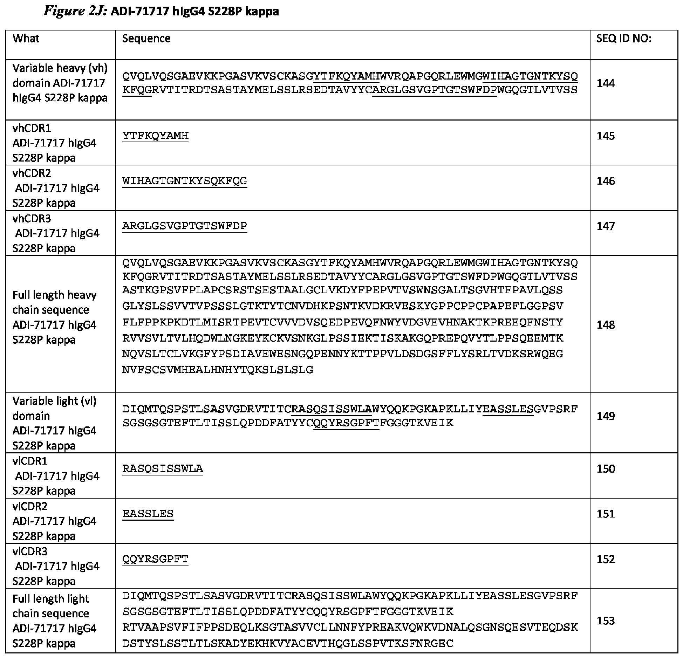

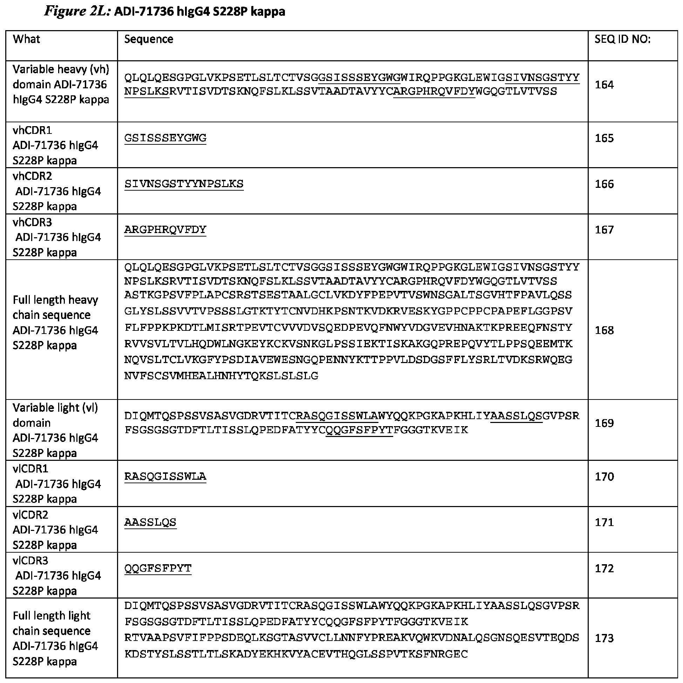

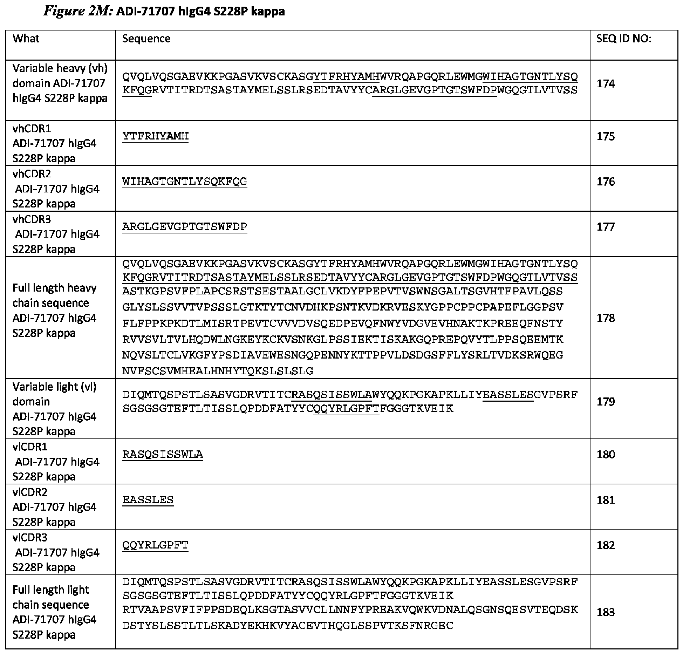

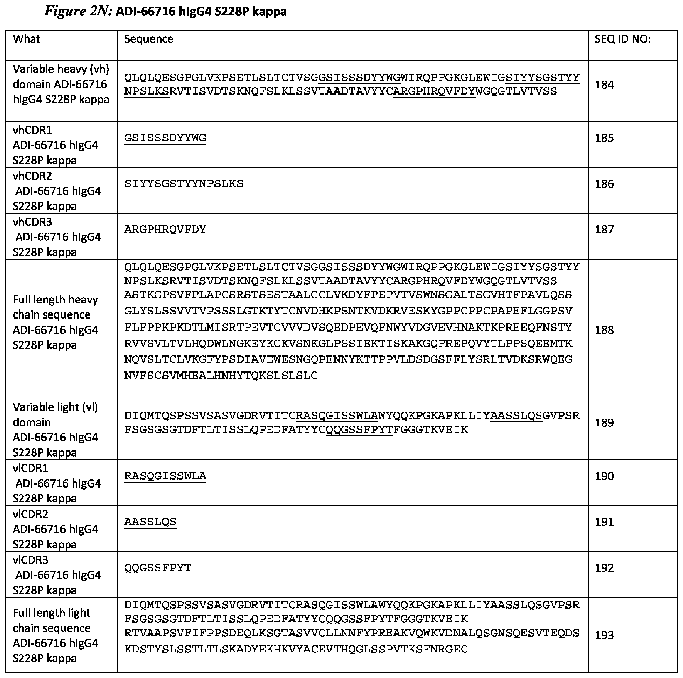

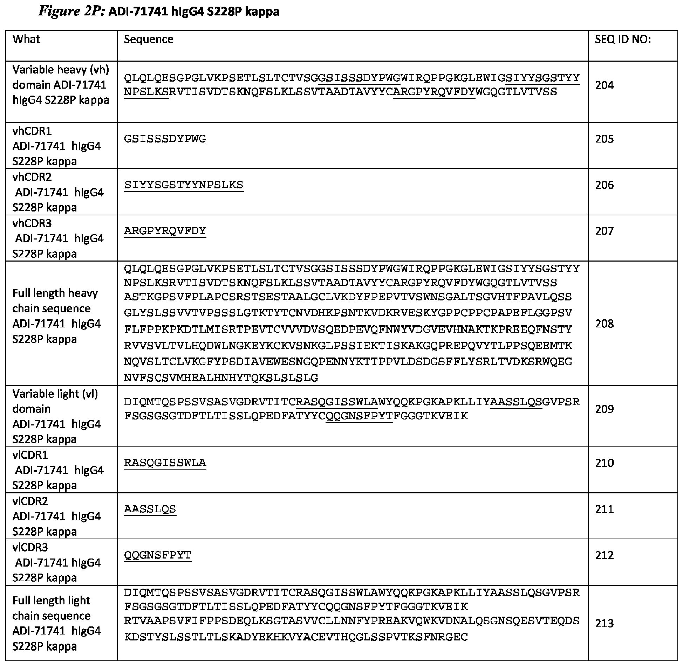

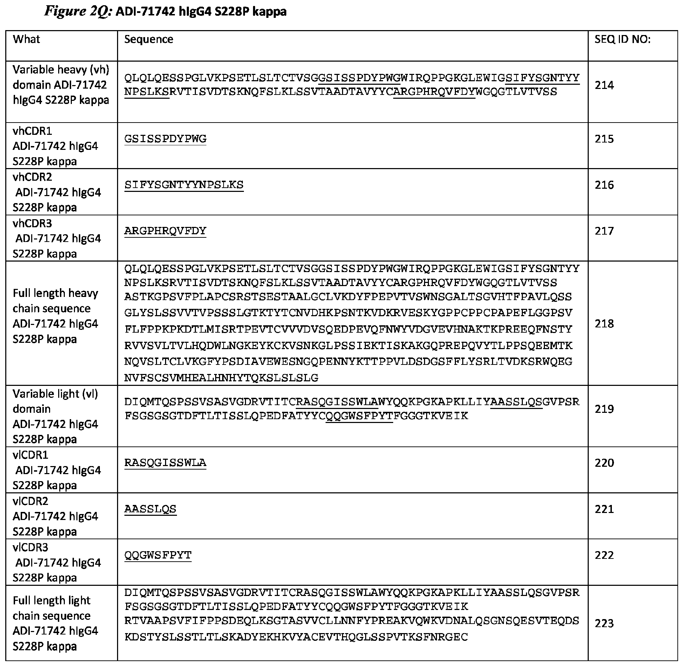

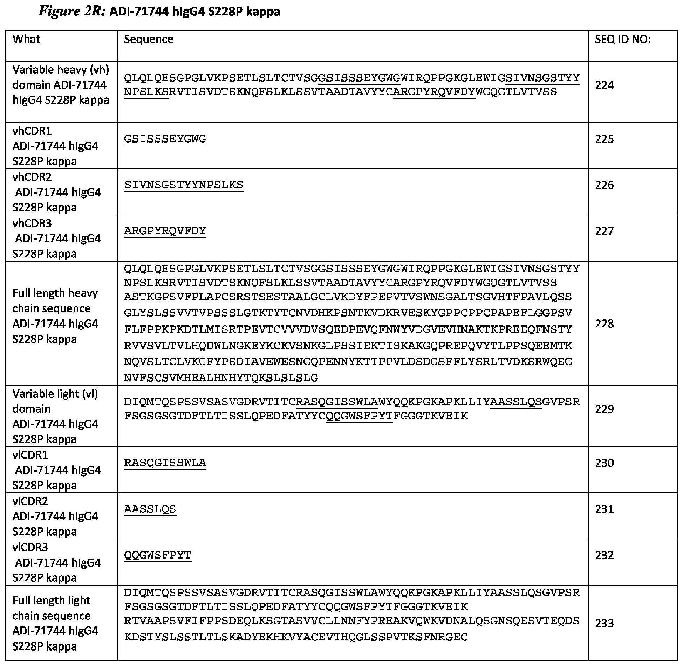

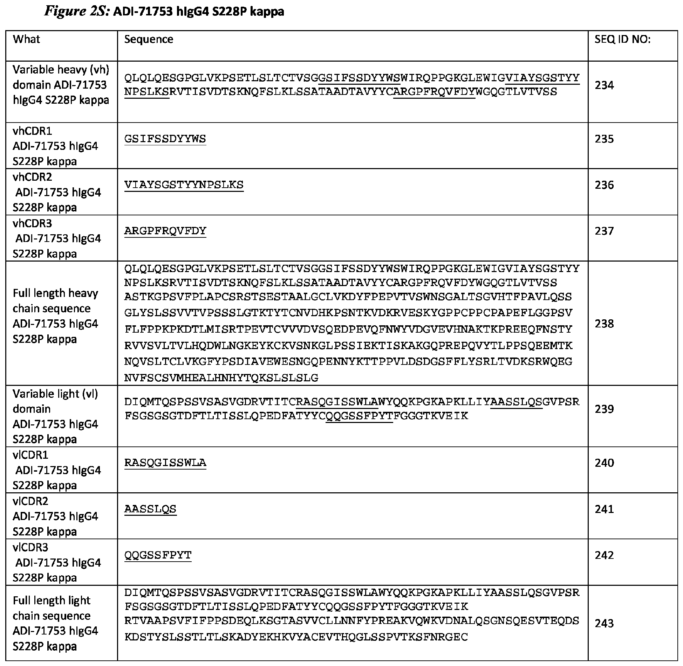

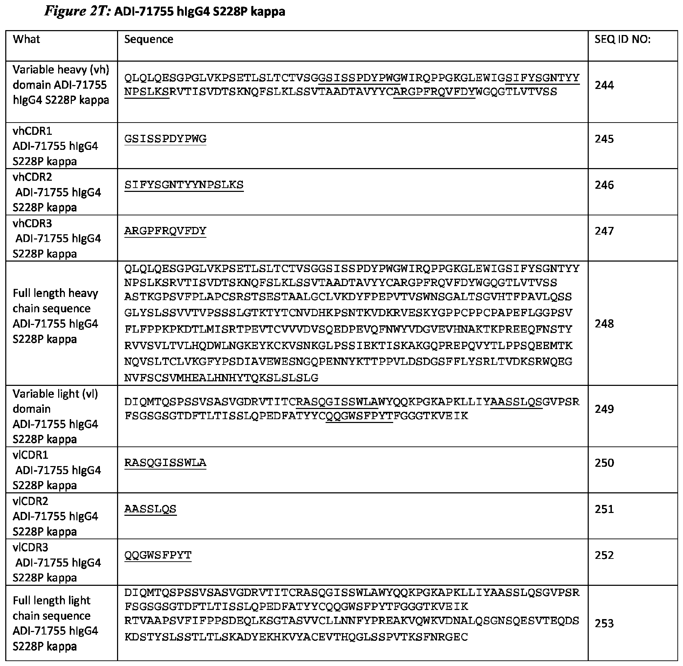

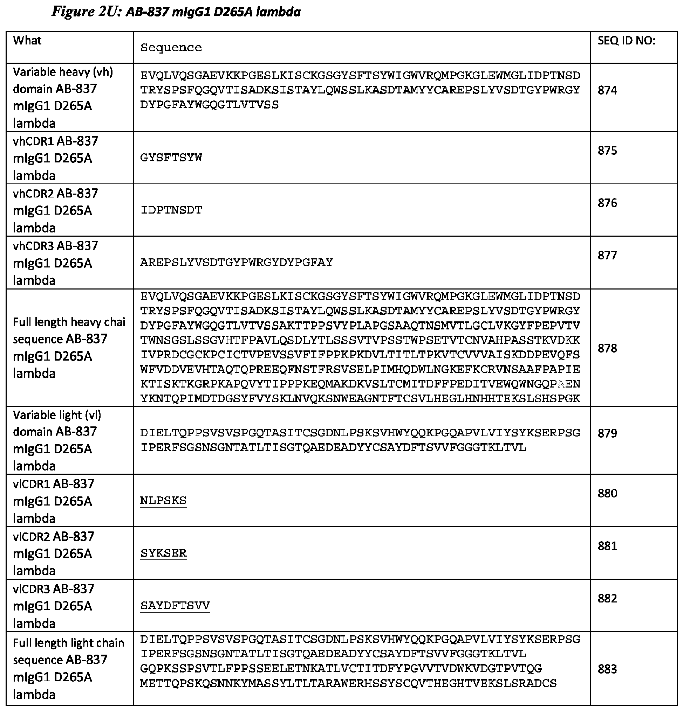

Figure 2A-2U depict the variable heavy and light chains, the vhCDR1, vhCDR2, vhCDR3, vlCDR1, vlCDR2, vlCDR3 sequences as well as the full length of the antibodies ADI-71709 (Figure 2A), ADI-71719 (Figure 2B), ADI-71720 (Figure 2C), ADI-71722 (Figure 2D), ADI-71701 (Figure 2E), ADI-71663 (Figure 2F), ADI-71662 (Figure 2G), ADI-66692 (Figure 2H), ADI-71710 (Figure 2I), ADI-71717 (Figure 2J), ADI-71739 (Figure 2K), ADI-71736 (Figure 2L), ADI-71707 (Figure 2M), ADI-66716 (Figure 2N), ADI-71728 (Figure 2O), ADI-71741 (Figure 2P), ADI-71742 (Figure 2Q), ADI-71744 (Figure 2R), ADI-71753 (Figure 2S), ADI-71755 (Figure 2T), and AB-837 (referred also as "AbD35328", "837", or "Ab837") (Figure 2U).

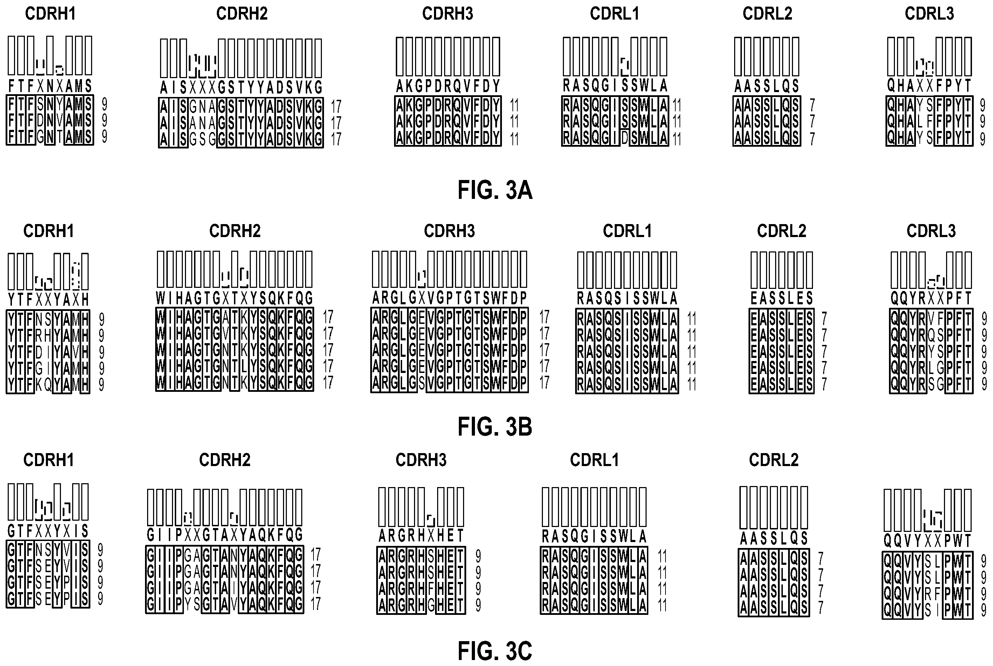

Figure 3A-3E: A) depicts the alignment of CDRH and CDRL sequence between VH3-23 and VL-kappa-1-12 germline sequences- 71663 and 71662 and 66692. B) depicts the alignment of CDRH and CDRL sequence between VH1-03 and VL-kappa-1-5 germline sequences 71701, 71707, 71709, 71710, and 71717. C) depicts the alignment of CDRH and CDRL sequence between VH1-69 and VL-kappa-1-2 germline sequences 71719, 71720, 71722 and 71728. D) depicts the alignment of CDRH and CDRL sequence between VH4-39 and VL-kappa-1-12 germline sequences- 71736, 71739 and 66716. E) depicts the alignment of CDRH and CDRL sequence between VH4-39 and VL-kappa-1-12 germline sequences-71736, 71739, 66716, 71742, 71744, 71741, 71753 and 71755.

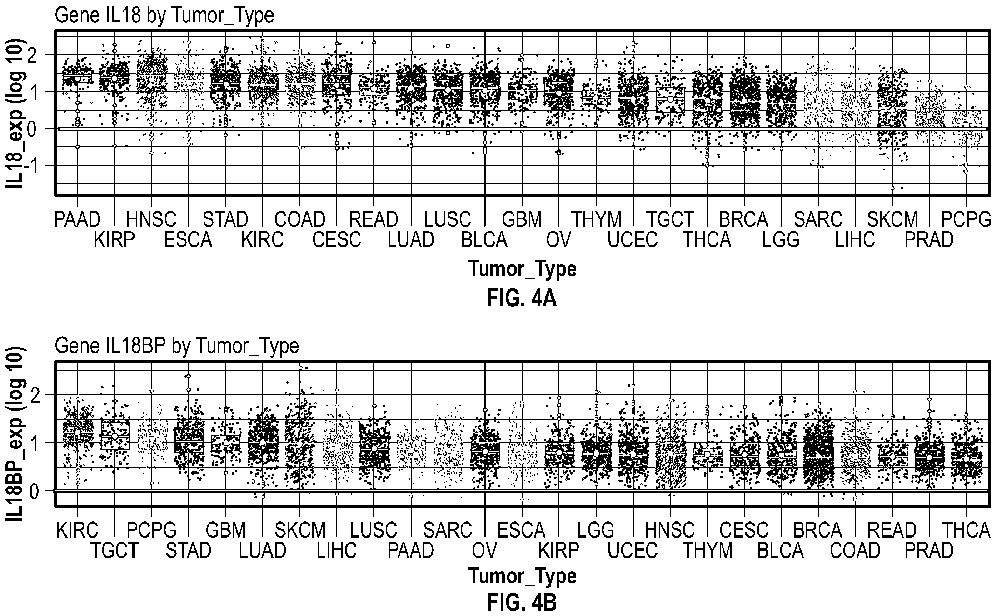

Figure 4A-4B: A) depicts the expression of IL18 across all TCGA tumors. and B) depicts the expression of IL18-BP across all TCGA tumors. Box plot of log10 RPKM for each TCGA tumors, reference line at 1 RPKM.

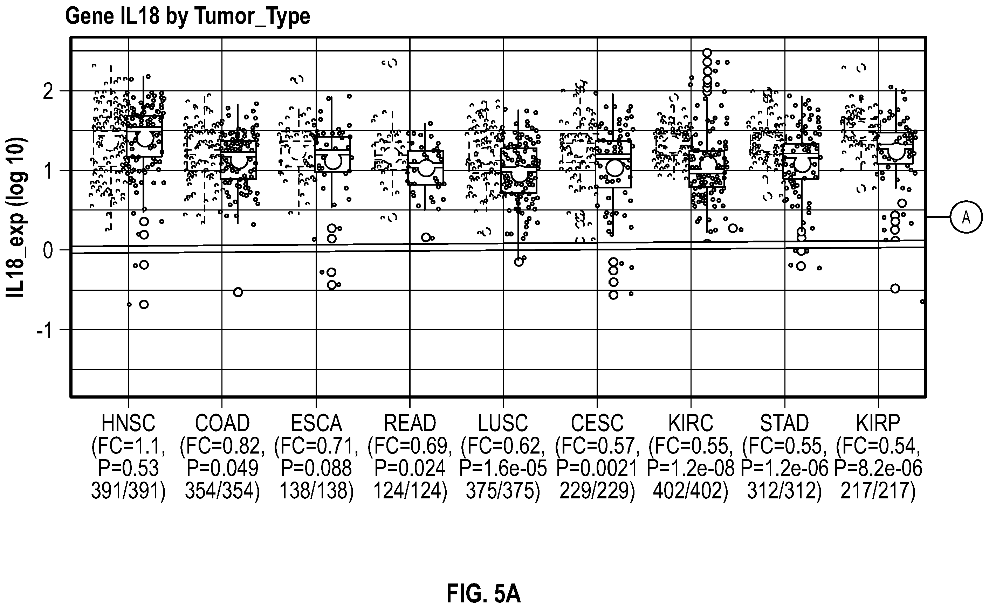

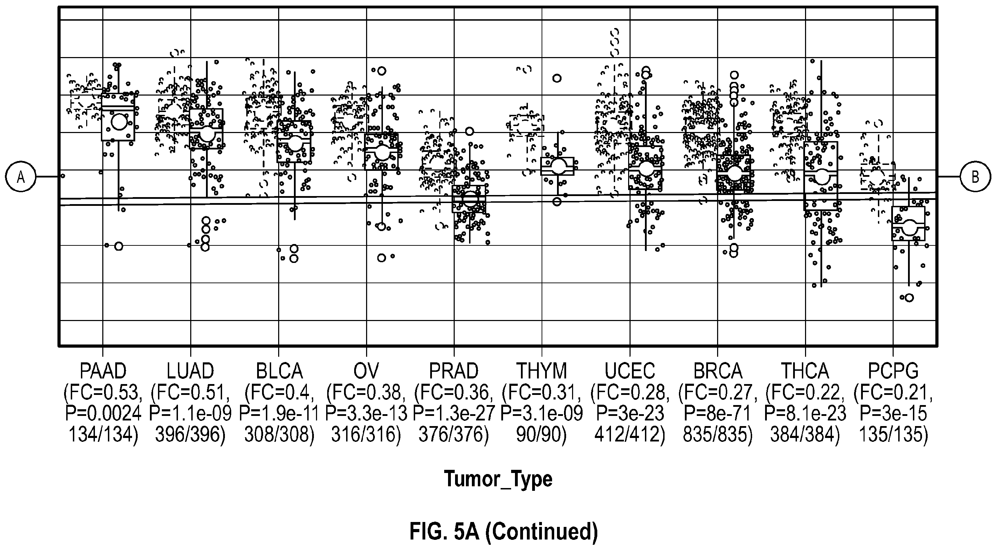

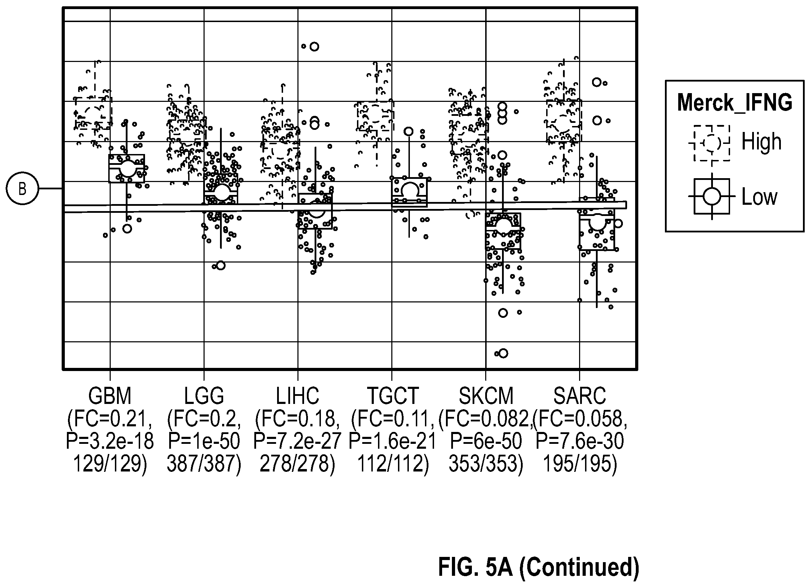

Figure 5A-5B: A) depicts IL18 stratified by IFNγ expression per tumor type in TCGA. B) depicts IL18-BP stratified by IFNγ expression per tumor type in TCGA. Box plot of log10 RPKM for each TCGA tumors, reference line at 1 RPKM. For tumor abbreviations see Table 1. IFNγ high represent the top quartile and IFNγ low represents the bottom quartile. FC - fold change, P - p-value of student's T-test between IFNγ high to IFNγ low. Fraction represents the number of samples in IFNγ high / IFNγ low.

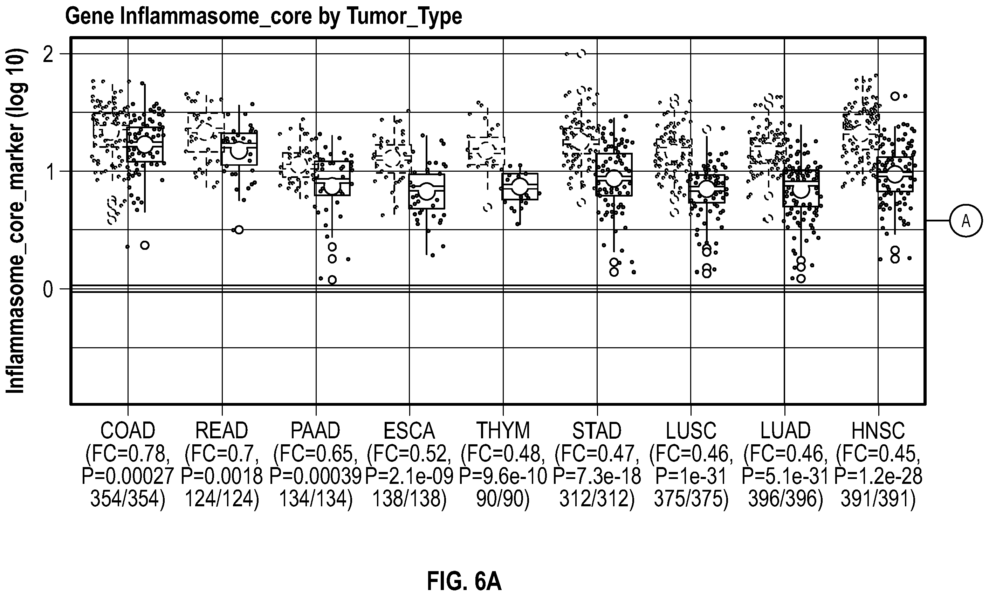

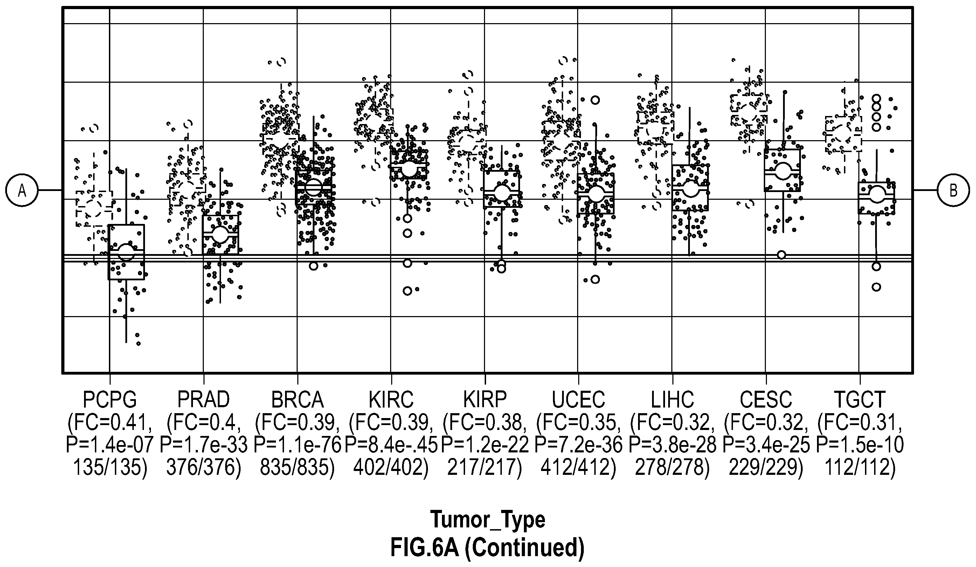

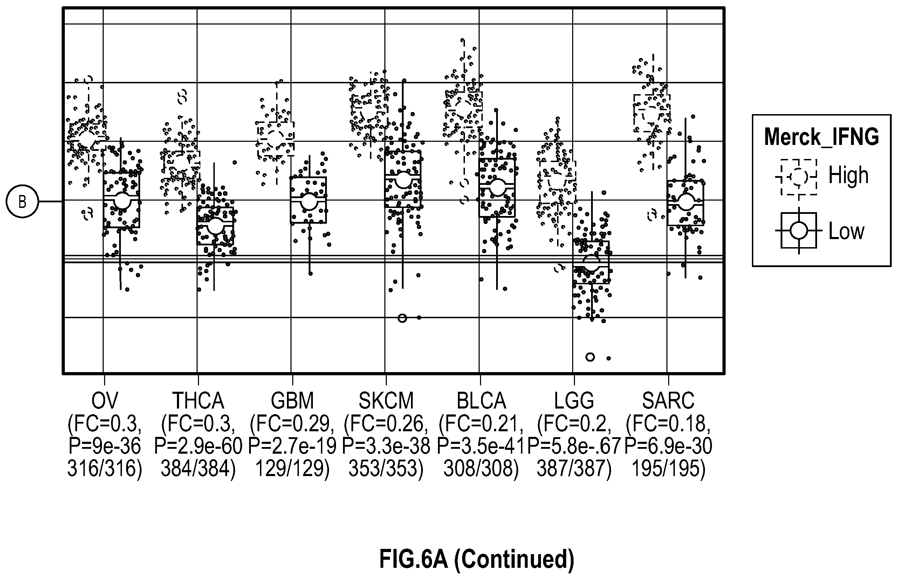

Figure 6A-6B: A) depicts core inflammasome signature, stratified by IFNγ expression per tumor type in TCGA. Box plot of log10 RPKM for each TCGA tumors, reference line at 1 RPKM. For tumor abbreviations see Table 1. IFNγ high represent the top quartile and IFNγ low represents the bottom quartile. FC - fold change, P - p-value of student's T-test between IFNγ high to IFNγ low. Fraction represents the number of samples in IFNγ high / IFNγ low. B) depicts cosine similarity heatmap and dandogram, between core inflammasome genes, IL18, IL18-BP, IL18R's, and additional upstream inflammasome genes.

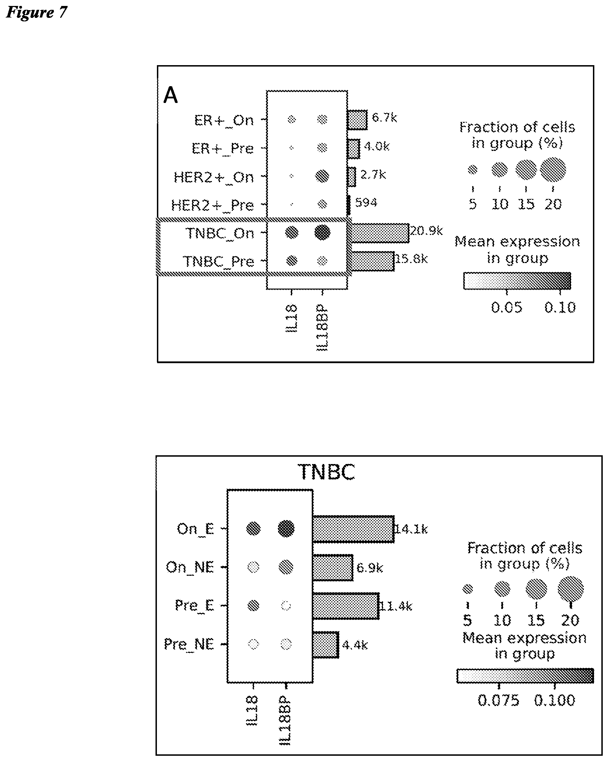

Figure 7A-7B: A) depicts DotPlot of IL18 and IL18-BP, in subtype of breast cancer, pre and on treatment, expression of the two genes pre and on treatment in TNBC. B) depicts DotPlot of IL18 and IL18-BP, expression of the two genes pre and on treatment in TNBC, divided also by expanding TCR clones (_E) and non-expanding TCR clones (_NE).

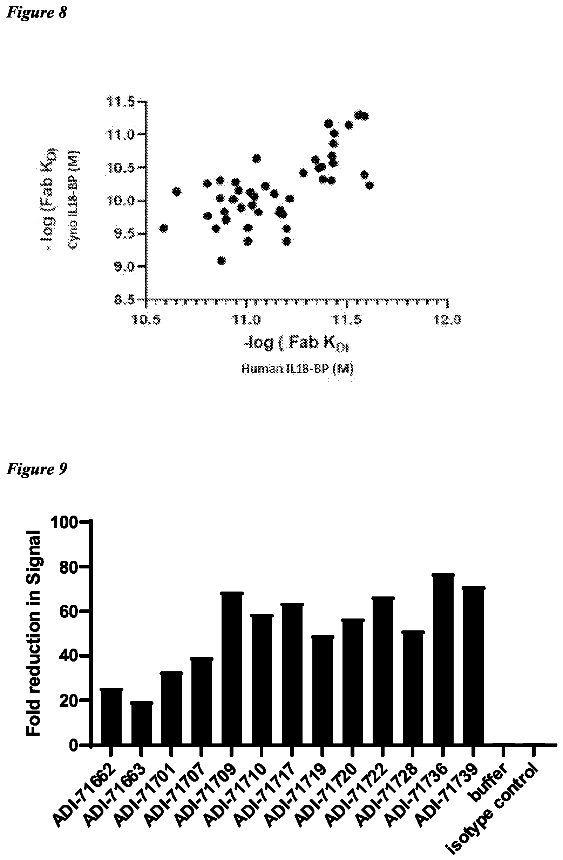

Figure 8: Affinity matrix for mAbs against human IL18-BP to human and cynomolgus monkey ("cyno") IL18-BP by Biacore
Figure 9: Competition with human IL18 for the binding of IL18-BP-Fc performed in AlfaLISA assay with 15nM of purified Ab with hIgG1 backbone.

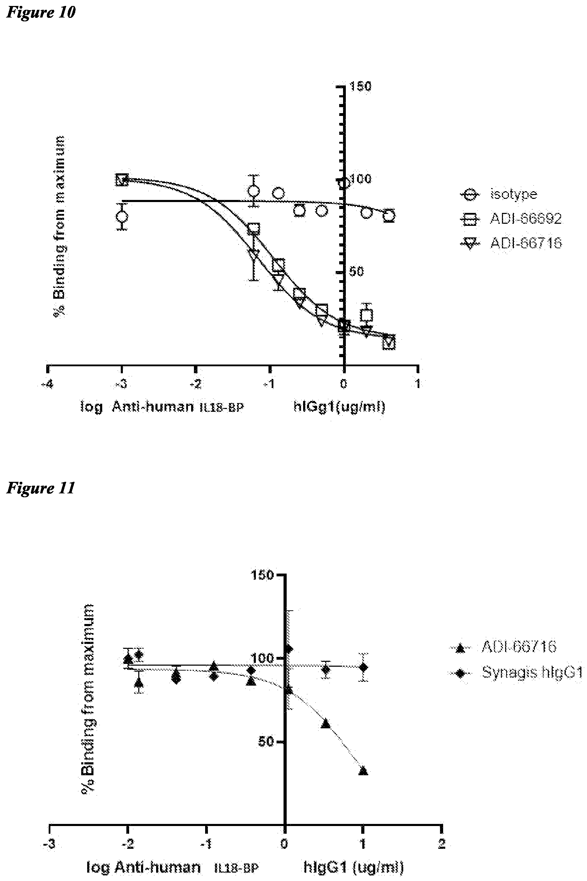

Figure 10: The blocking activity of the parental mAbs against human IL18-BP analyzed by ELISA
Figure 11: The blocking activity of the parental mAbs against cynomolgus monkey IL18-BP analyzed by ELISA

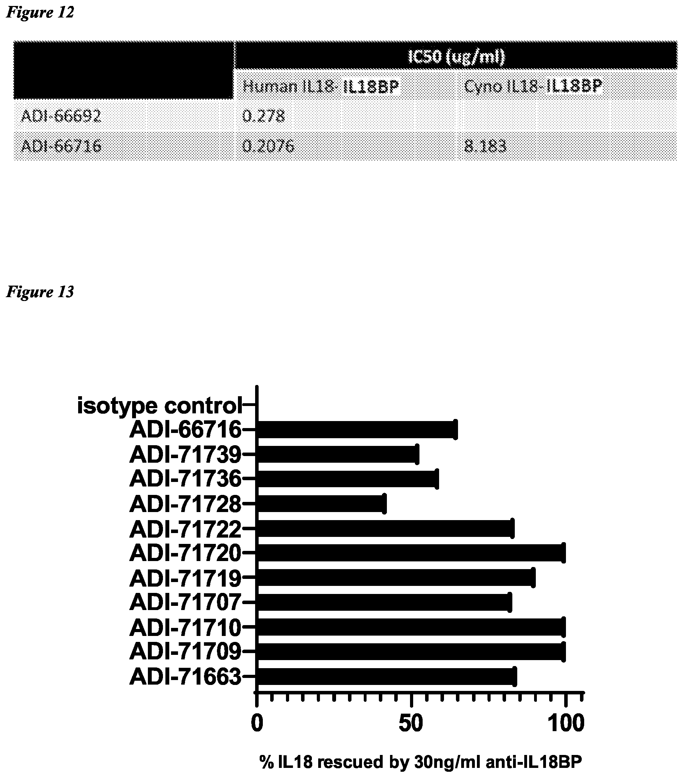

Figure 12: IC50 values for the anti-human IL18-BP Abs measured by ELISA
Figure 13: The ability of the mAbs against human IL18-BP to rescue human IL18 bound by IL18-BP-Fc protein demonstrated using IL18 HEK293 reporter cells.

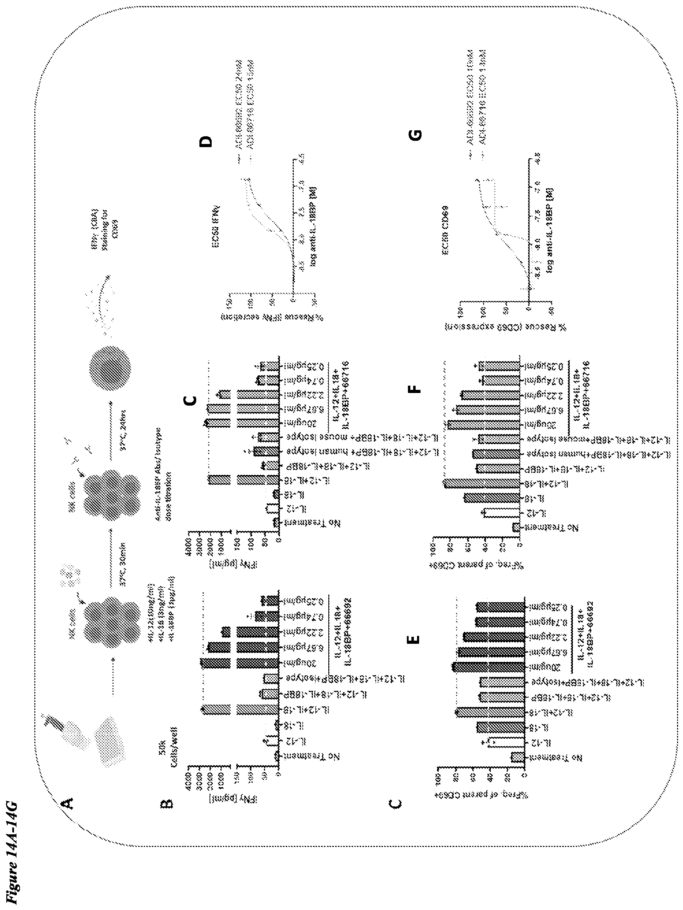

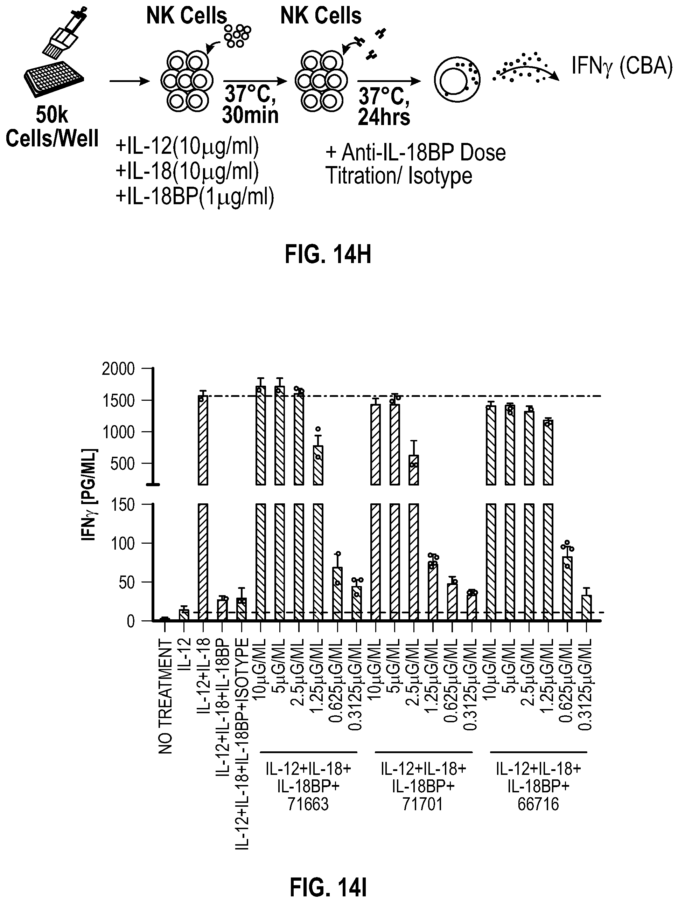

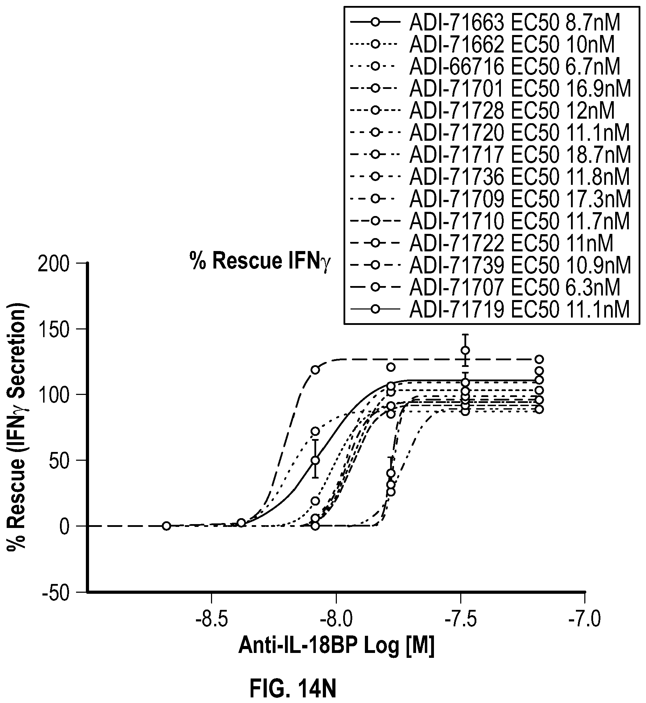

Figure 14A-14H: Anti-IL-18BP antibodies fully restored IL-18 activity on NK cells. Figure 14A and 14H show schematic representations of assay setup; thawed NK cells from four donors were cultuNed for 30 minutes with rhIL-18 (3 or 10 ng/ml) and rhIL-18BP (1µg/ml), in the presence of rhIL-12 (10ng/ml) to allow the formation of IL-18-IL-18BP complex. 30 minutes post incubation, the cells were treated with a dose titration of anti-IL-18BP antibodies (20µg/ml to 0.25µg/ml; dilution factor of 1:3 (Figures 14A-G); or 10µg/ml to 0.325µg/ml; dilution factor of 1:2 (Figures 14H-N)) or isotype control (20µg/ml (Figures 14A-G) or 10µg/ml (Figures 14A-G)). Figure 14I-N shows Anti-IL-18BP antibodies were able to fully restore IFNγ secretion (Figure 14B-D, 14I-N) and CD69 expression (Figure 14E-G) in a dose-dependent manner. Isotype controls were not able to restore IL-18 activity. Figure 14N shows the dose response curve of % rescue by Anti-IL-18BP antibodies and calculated EC50s. Representative data is from one donor. Rescue by anti-IL-18BP Ab is calculated as: [(IL-12+ IL-18+IL-18BP+ anti-IL-18BP Ab)- (IL-12+ IL-18+ IL-18BP+ Isotype)]/ [(IL-12+ IL-18)- (IL-12+ IL-18+ IL-18BP+ Isotype)].

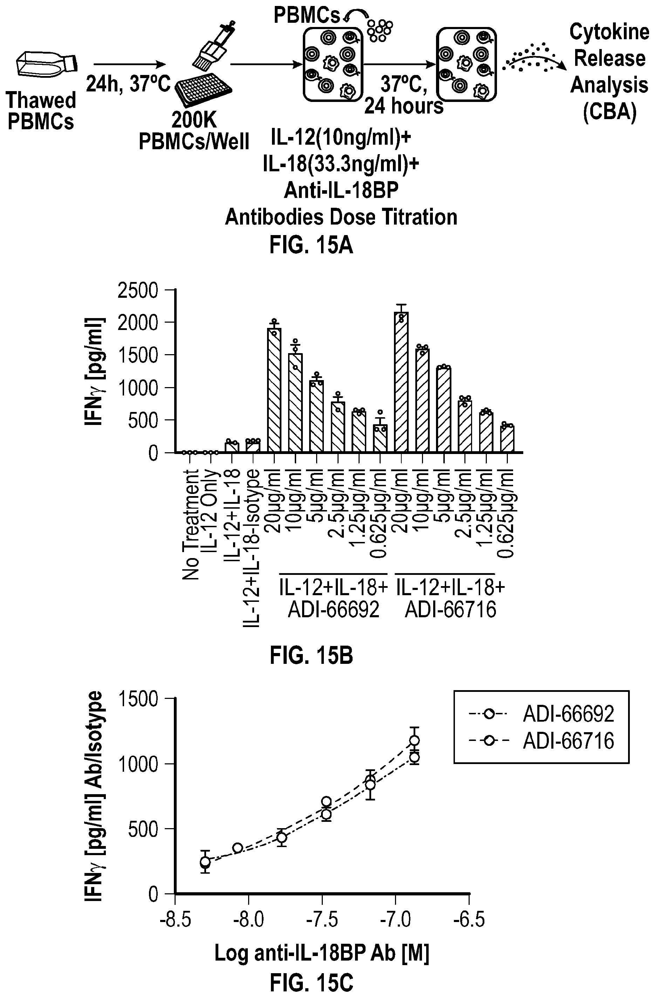

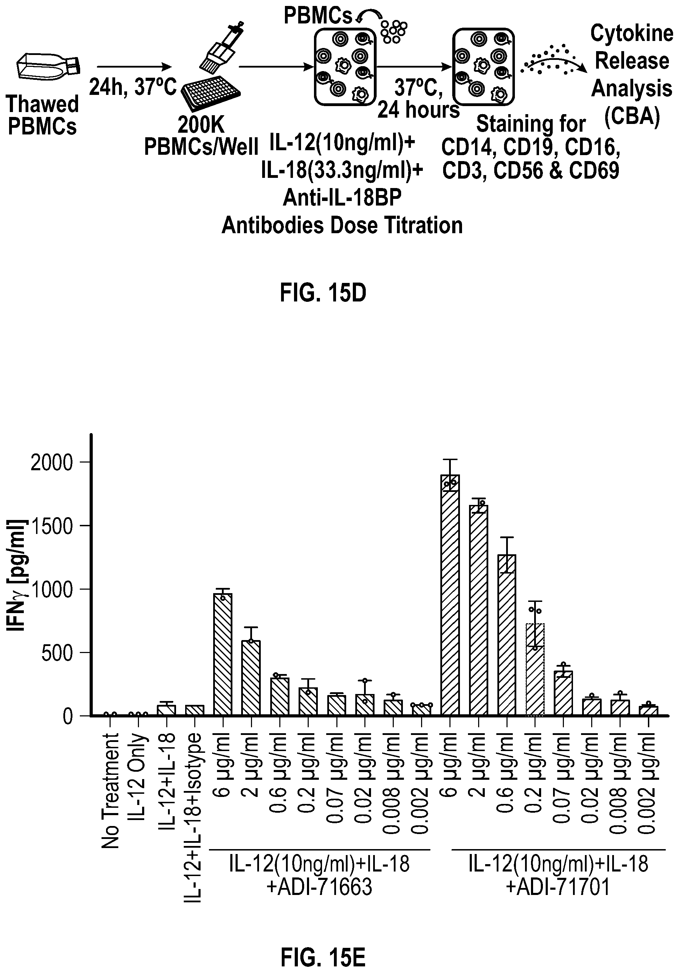

Figure 15A-15J: Anti-IL-18BP antibodies blocked IL-18BP secreted from PBMCs. Figures 15A, 15D show Schematic representation of assay setup; thawed PBMCs from two donors were cultured for 24 hours with rhIL-12 (10 ng/ml), rhIL-18 (33.3ng/ml) and a dose titration of anti-IL-18BP antibodies (Figure 15B: 20µg/ml to 0.625µg/ml; dilution factor of 1:2. Figure 15E-J: 6ug/ml to 0.002ug/ml; dilution factor of 1:3) or isotype control (20µg/ml). Figures 15B-C and 15E-J show Anti-IL-18BP antibodies were able to induce dose-dependent IFNγ secretion above the IL-12+IL-18 control levels, suggesting that the antibodies can block endogenous IL-18BP activity. Representative data is from one donor.

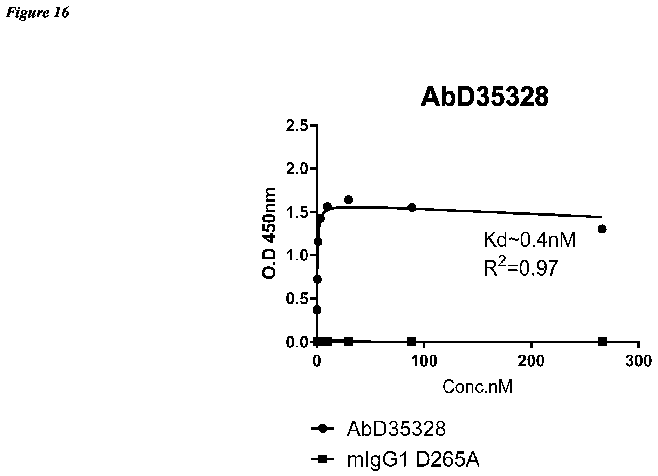

Figure 16 depicts affinity measurement of anti-mouse mIL18BP Ab to mouse IL18-BP protein by ELISA.

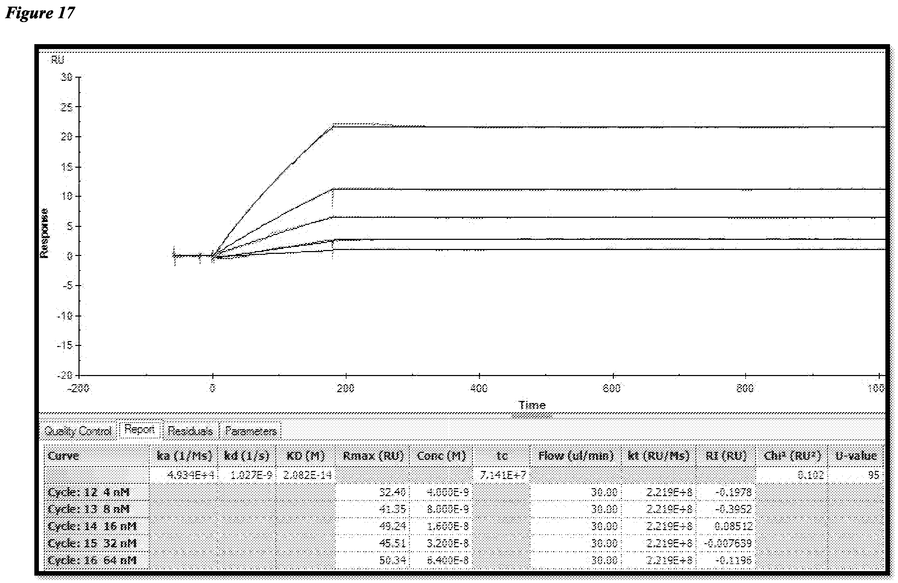

Figure 17 depicts SPR kinetic measurement of anti-mouse IL18-BP (AbD35328 (referred also as "837", "Ab837" or "AB-837")).

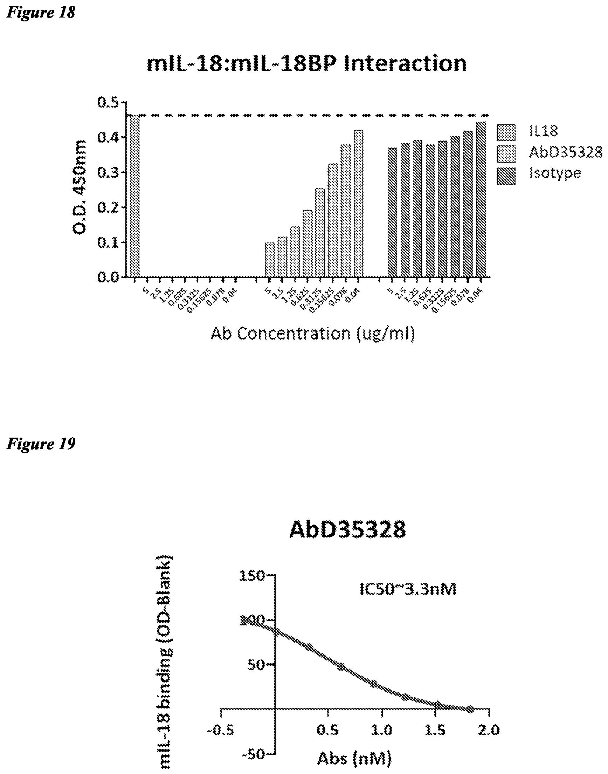

Figure 18 depicts analysis of mAbs performance in functional blocking of mIL18-BP- mIL-18 interaction by ELISA.
Figure 19 depicts IC50 analysis for anti-mouse IL18-BP (AbD35328).

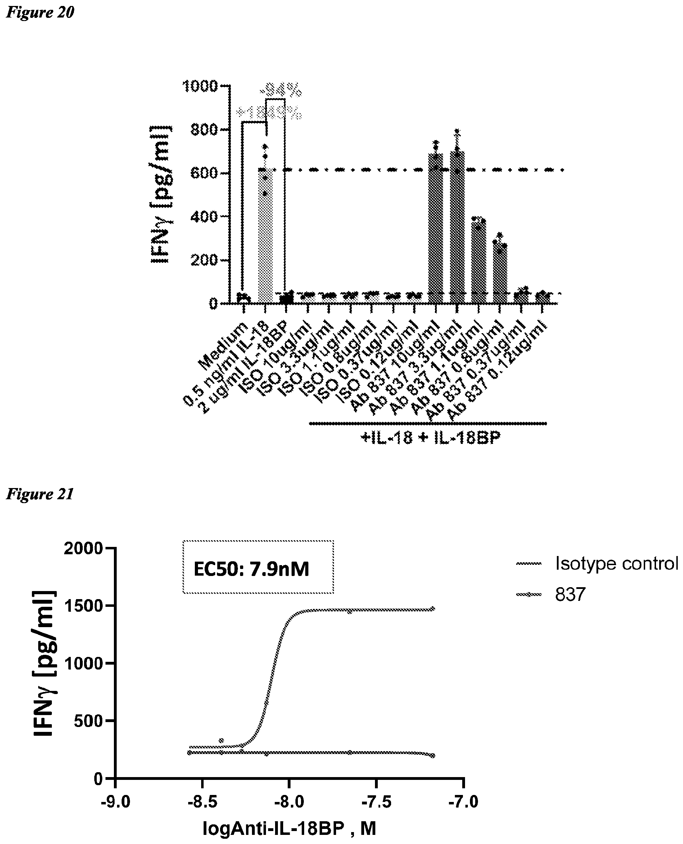

Figure 20 depicts the functional blocking activity of purified mAbs against mouse IL18-BP by IFNγ secretion.
Figure 21 depicts the EC50 analysis for anti-mouse IL18-BP.

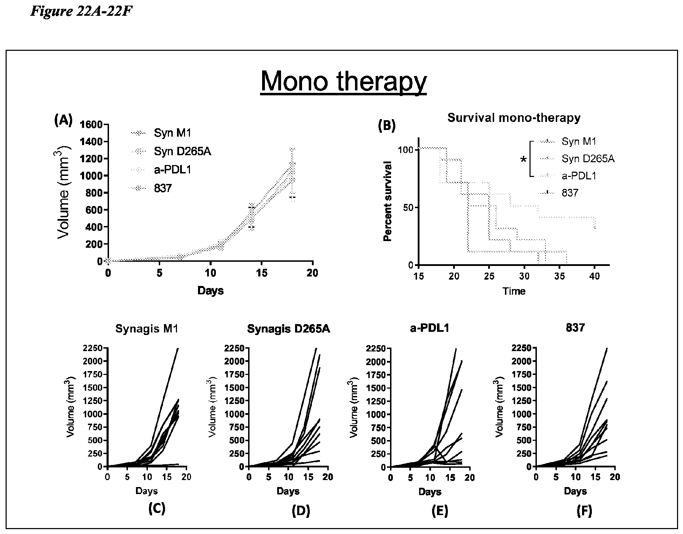

Figure 22A-22L depict assessment of anti-II,18-BP monotherapy or combo therapy with anti-PD-L1 Ab in mouse syngeneic CT26 tumor model. (A): tumor growth measurement of each group in monotherapy, (B): survival percentage analysis of each group in monotherapy, (C)-(F): overview of tumor growth measurement of individual mice in each group of monotherapy (G): tumor growth measurement of each group in combo therapy, (H): survival percentage analysis of combo therapy, (I)-(K): overview of tumor growth measurement of individual mice in each group of combo therapy, and (L):statistical analysis of the effects of combo therapy.

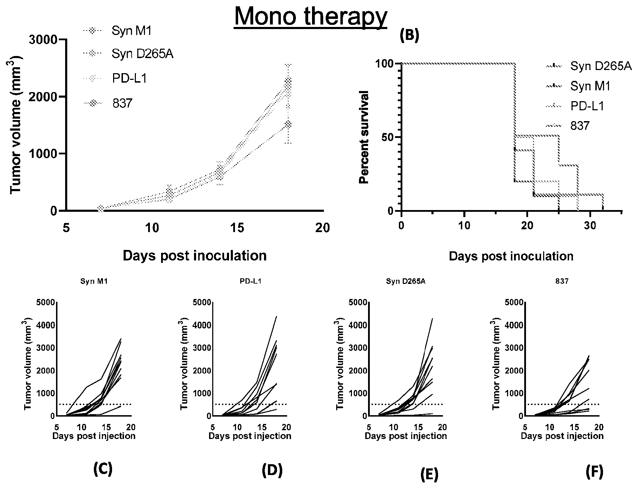

Figure 23A-23L depict assessment of anti-II,18-BP monotherapy or combo therapy with anti-PD-L1 Ab in mouse syngeneic B16/Db-hmgp100 mouse tumor model. (A): tumor growth measurement of each group in monotherapy, (B):survival percentage analysis of each group in monotherapy, (C)-(F):overview of tumor growth measurement of individual mice in each group of mono therapy, (G):tumor growth measurement of each group in combo therapy, (H): survival percentage analysis of each group in combo therapy (I)-(K): overview of tumor growth measurement of individual mice in each group of combo therapy, and (L):statistical analysis of the effects of combo therapy.

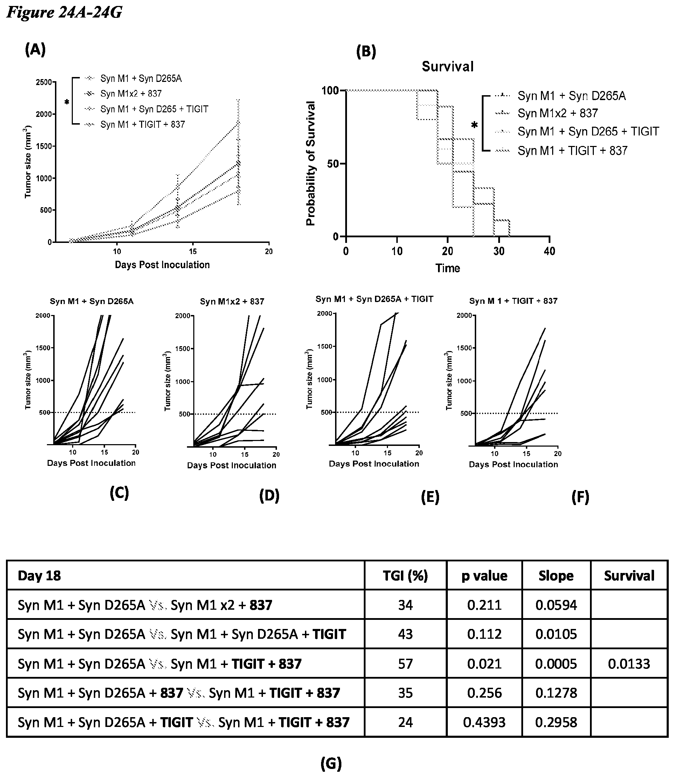

Figure 24A-24G depict activity of Anti-IL18-BP and anti-TIGIT Combination in B16/Db-hmgp100 Syngeneic Mouse Tumor Model. (A): tumor growth measurement of each group in combo therapy, (B): survival percentage analysis of each group combo therapy, (C)-(F): overview of tumor growth measurement of individual mice in each group of combo therapy, and (G): statistical analysis of the effects of combo therapy.

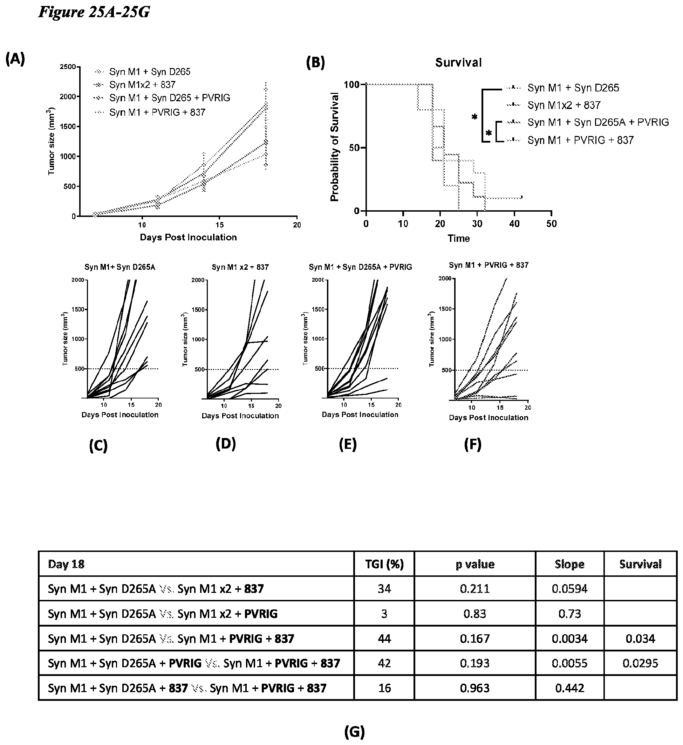

Figure 25A-25G depict activity of Anti-IL18-BP and anti-PVRIG Combination in B16/Db-hmgp100 Syngeneic Mouse Tumor Model. (A): tumor growth measurement of each group in combo therapy, (B): survival percentage analysis of each group in combo therapy (C)-(F): overview of tumor growth measurement of individual mice in each group of combo therapy, and (G): statistical analysis of the effects of combo therapy.

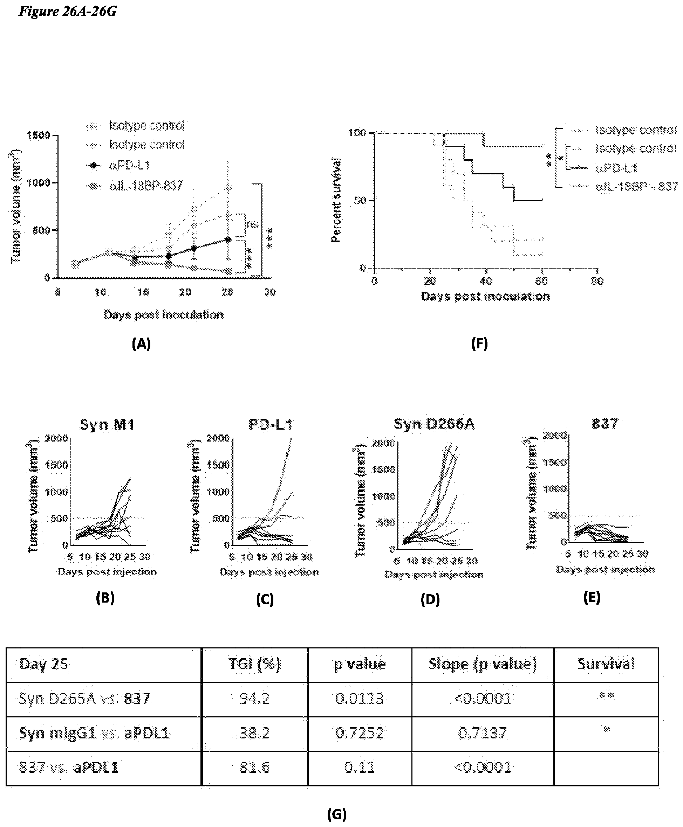

Figure 26A-26G depict monotherapy activity of anti-IL18-BP and anti-mPD-L1 in syngeneic E0771 orthotopic mouse tumor model. (A): tumor growth measurement of each group in monotherapy, (B)- (E): overview of tumor growth measurement of individual mice in each group of monotherapy, (F): survival percentage analysis of each group in monotherapy, and (G): statistical analysis of the effects of monotherapy.

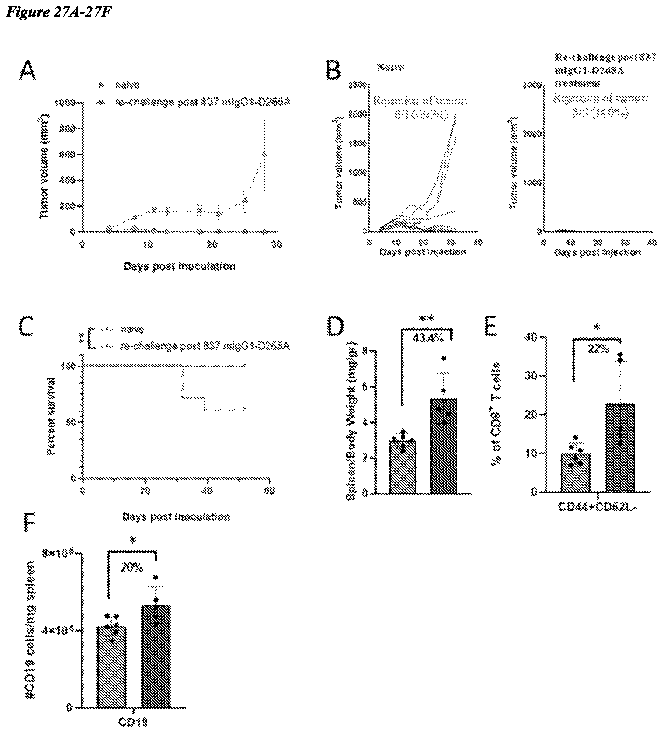

Figure 27A-27F depict tumor rechallenge experiment of E0771 TNBC model. Groups of 5-10 C57BL/6 tumor- naive age-matched mice were orthotopically inoculated with E0771 (0.5x10⁶ cells). When tumor reached the volume of 250mm³, mice were treated with designated mAb: AB-837 mIgG1-D265A or isotype control followed by 5 additional doses. After two months, tumor-free and naïve aged-matched mice were orthotopically re-inoculated with E0771. (A): Tumor volumes are represented as the mean volume ± SEM. (B) Individual tumors measurements for each mouse are depicted, CR-complete responders, PR- partially responders (TV<=500mm³). (C) Kaplan-Meier survival curves for each group are shown. (D) Spleen weight/body weight ratio. (E) percent of CD44⁺CD62L⁻CD8⁺ effector T cells. (F) number of CD19+ cells per mg spleen.

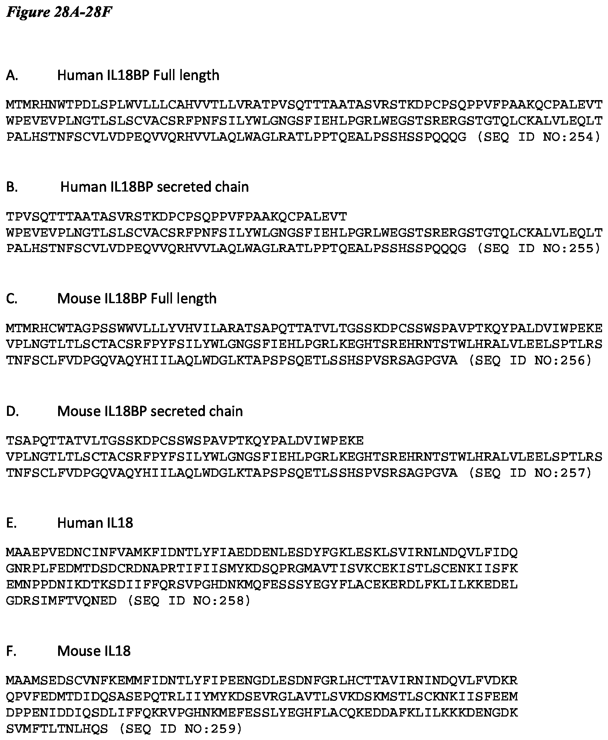

Figure 28A-28F depict the amino acid sequence of the human (A) and mouse (C) IL18-BP proteins. Signal Peptide sequence is highlighted. The secreted human and mouse IL18-BP protein chains are depicted in (B) and (D), respectively. Figure 28 E and F depict the amino acid sequence of human and mouse IL18 proteins, respectively.

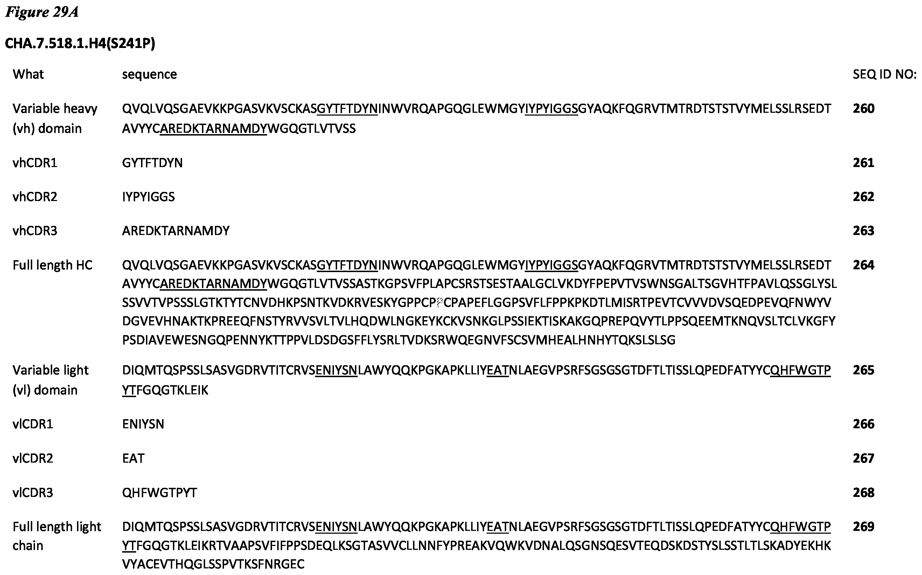

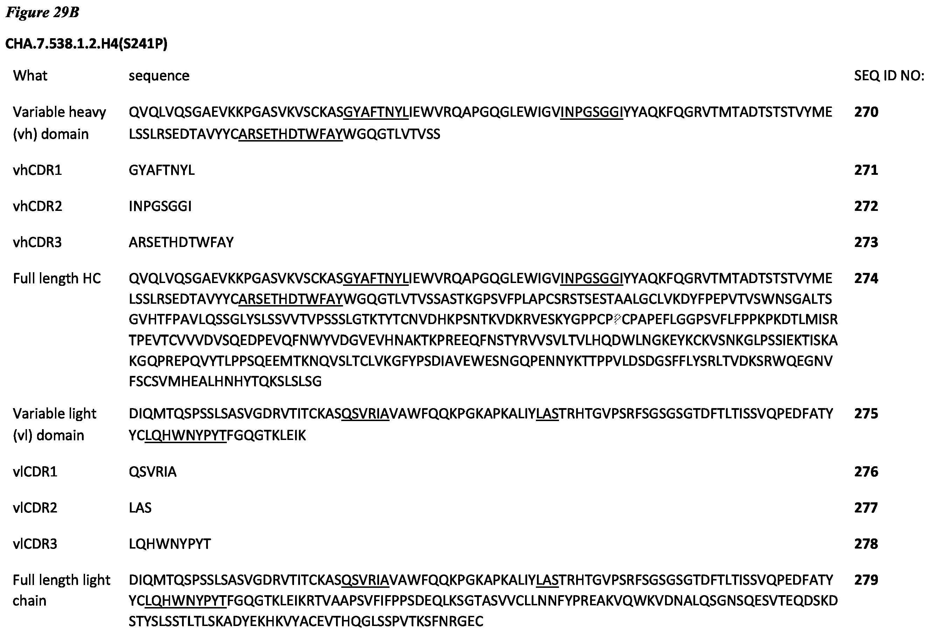

Figure 29A-29B depict the variable heavy and light chains as well as the vhCDR1, vhCDR2, vhCDR3, vlCDR1, vlCDR2 and vlCDR3 sequences of CHA.7.518.1.H4(S241P) and CHA.7.538.1.2.H4(S241P).

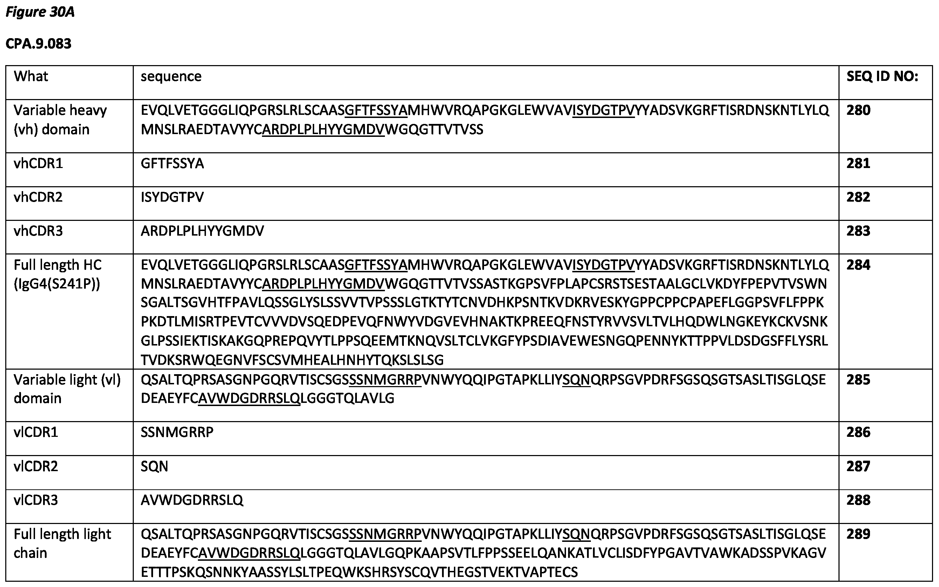

Figure 30A-30B depicts the variable heavy and light chains as well as the vhCDR1, vhCDR2, vhCDR3, vlCDR1, vlCDR2 and vlCDR3 sequences of CPA.9.083.H4(S241P) and CPA.9.086.H4(S241P).

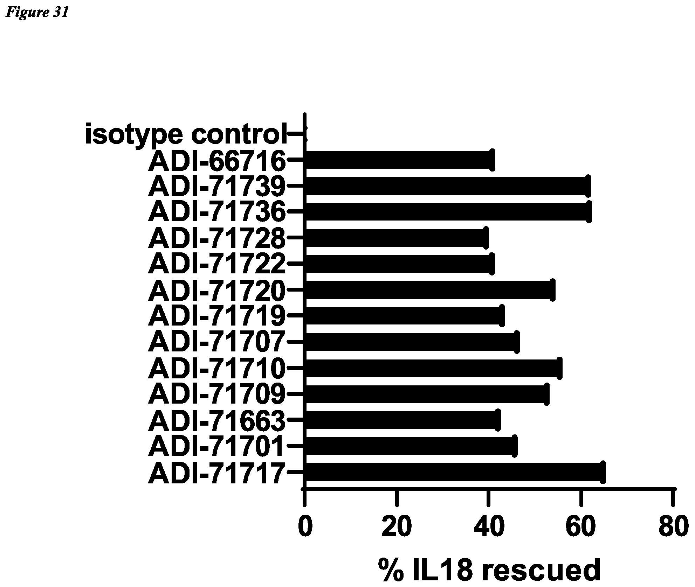

Figure 31 shows the ability of the mAbs against human IL18-BP to rescue human IL18 bound by IL18-BP in human serum demonstrated by ELISA

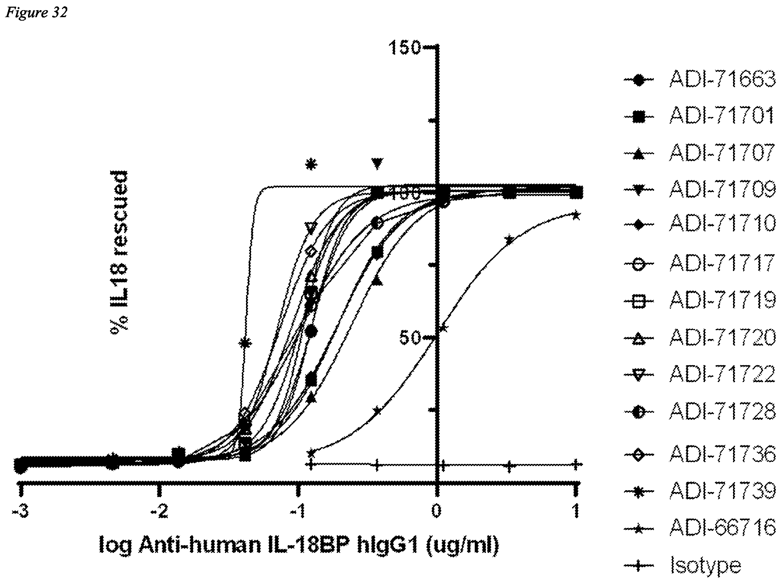

Figure 32 shows the ability of the mAbs against human IL18-BP to rescue cyno IL18 bound by cyno IL18-BP demonstrated using ELISA.

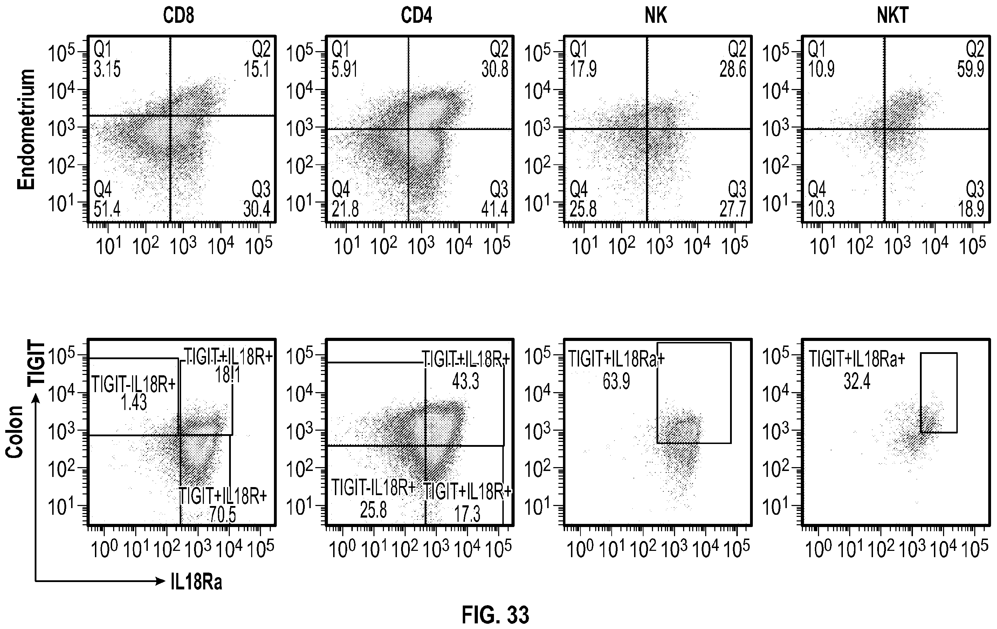

Figure 33 shows TIGIT and IL18Ra are co-expression within the TME.

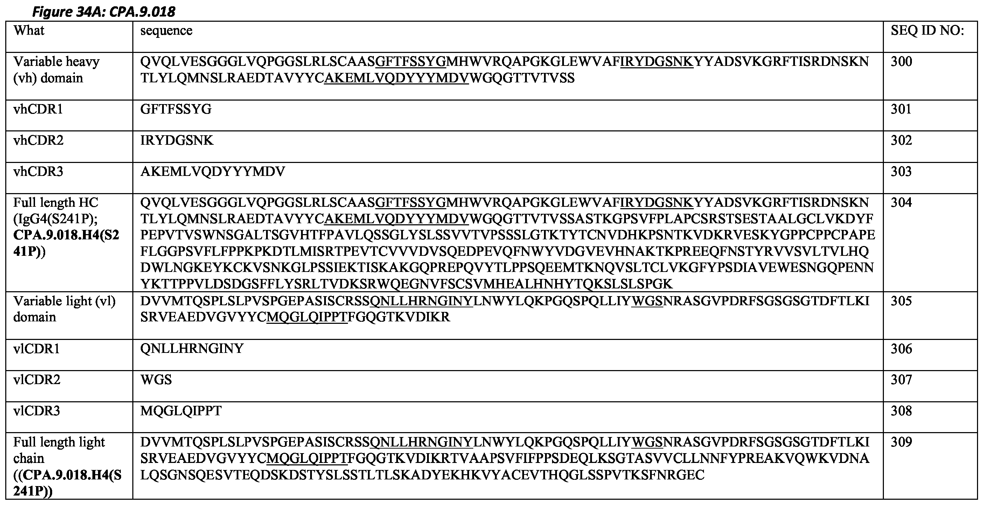

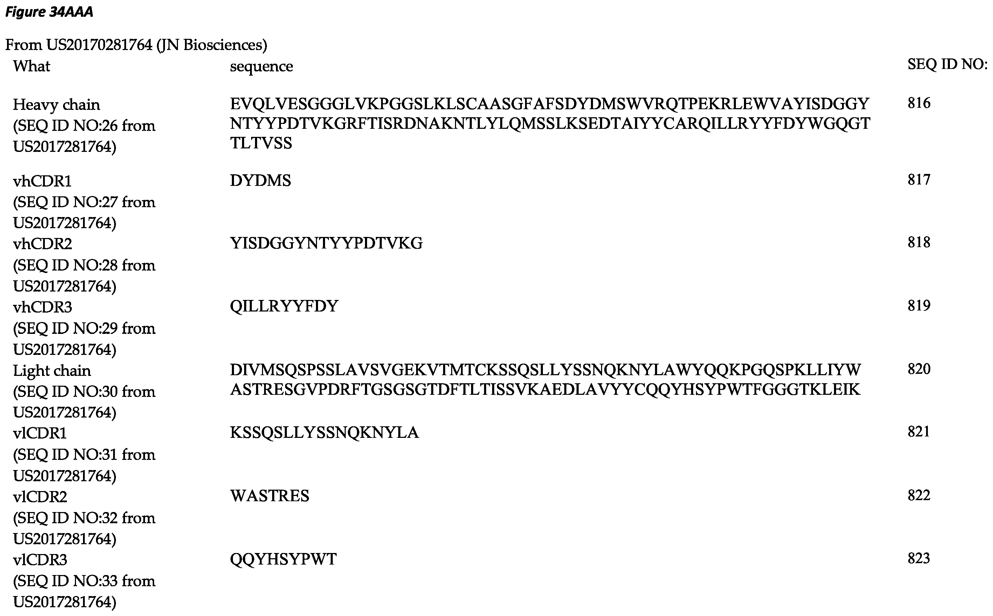

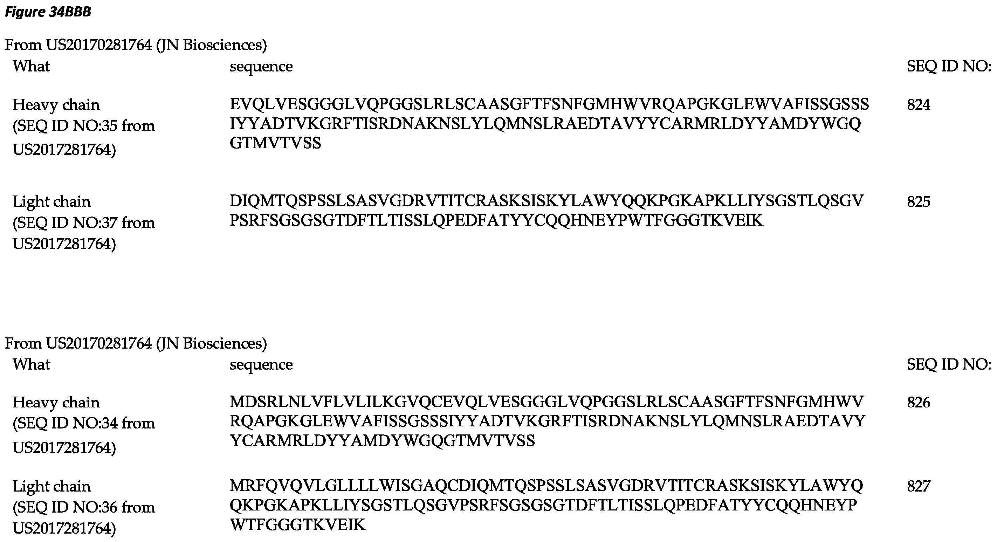

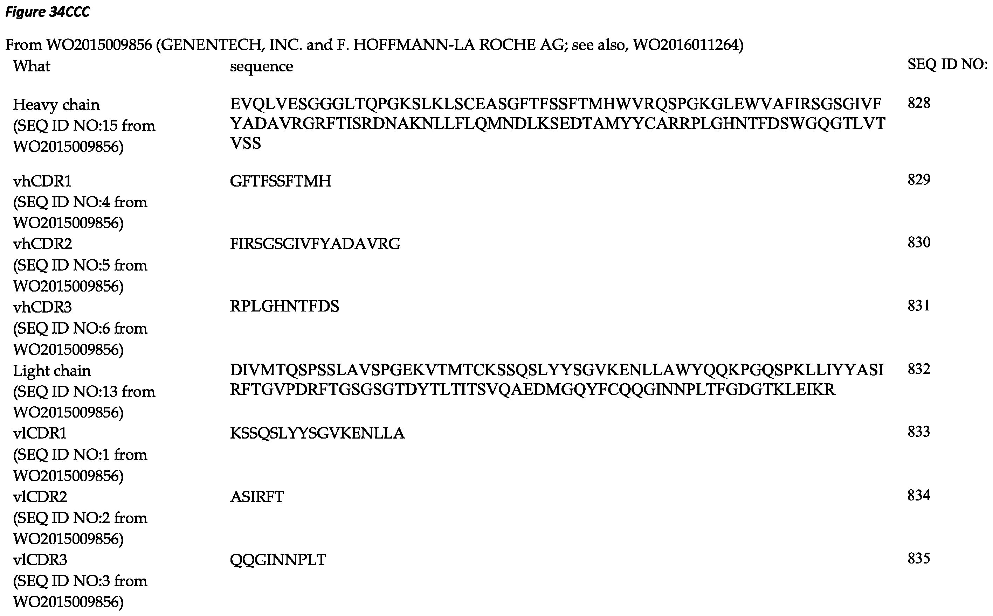

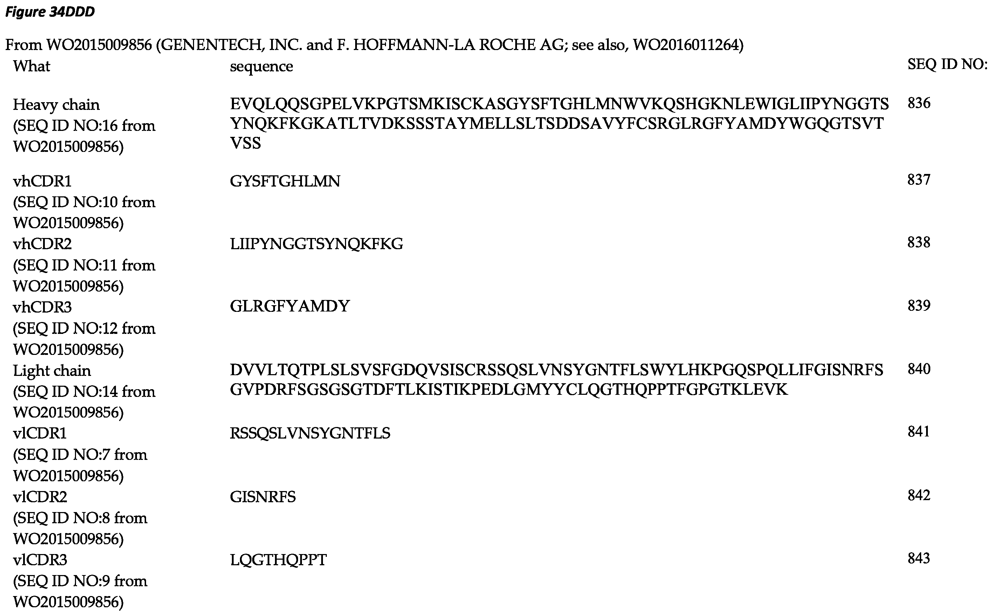

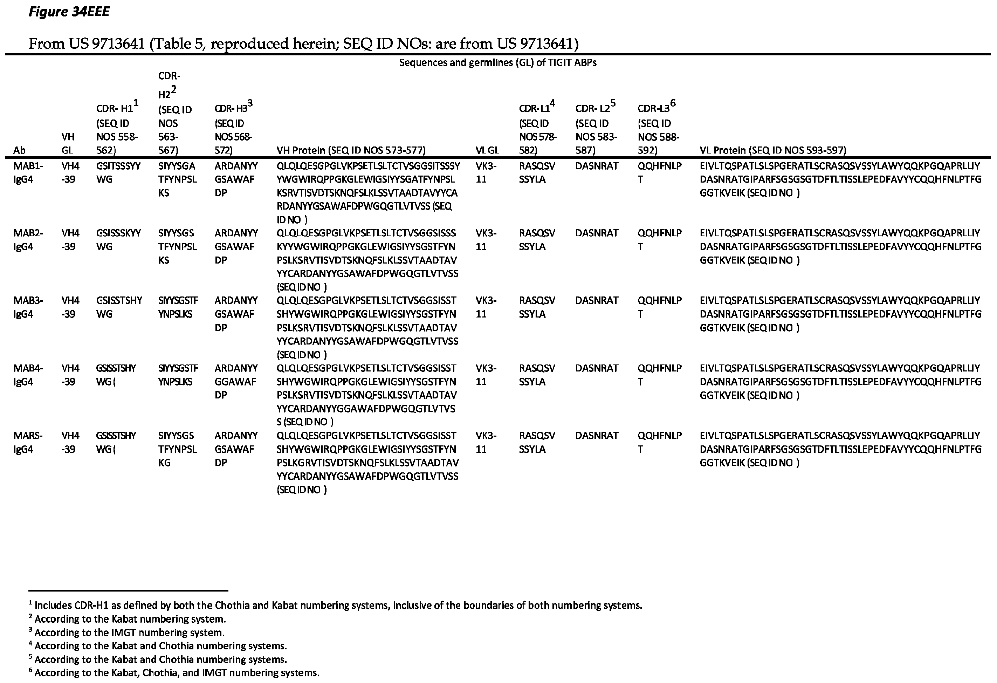

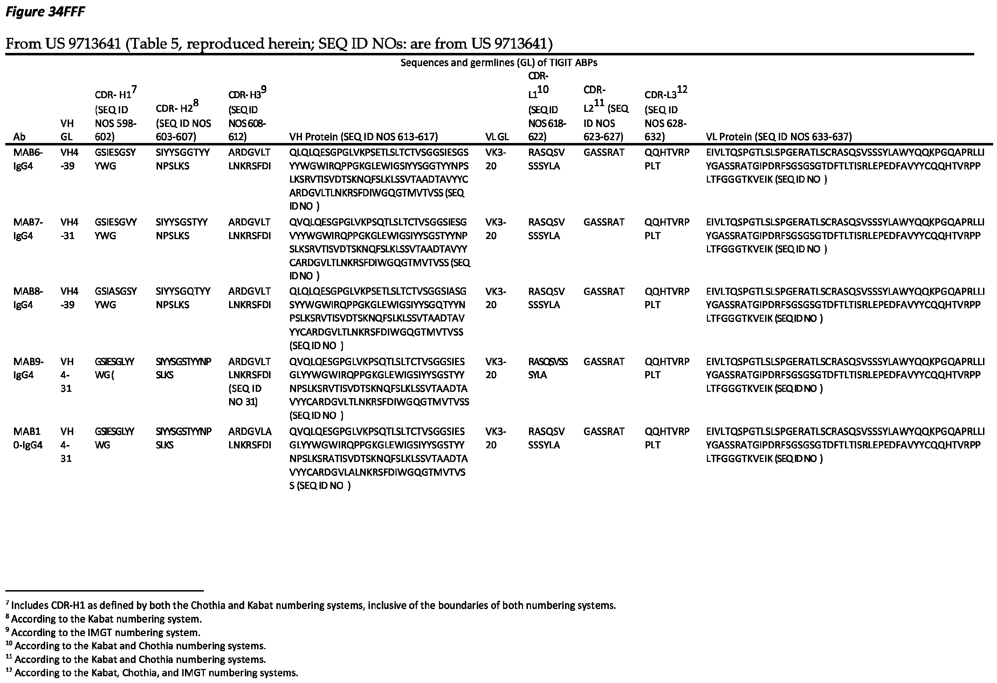

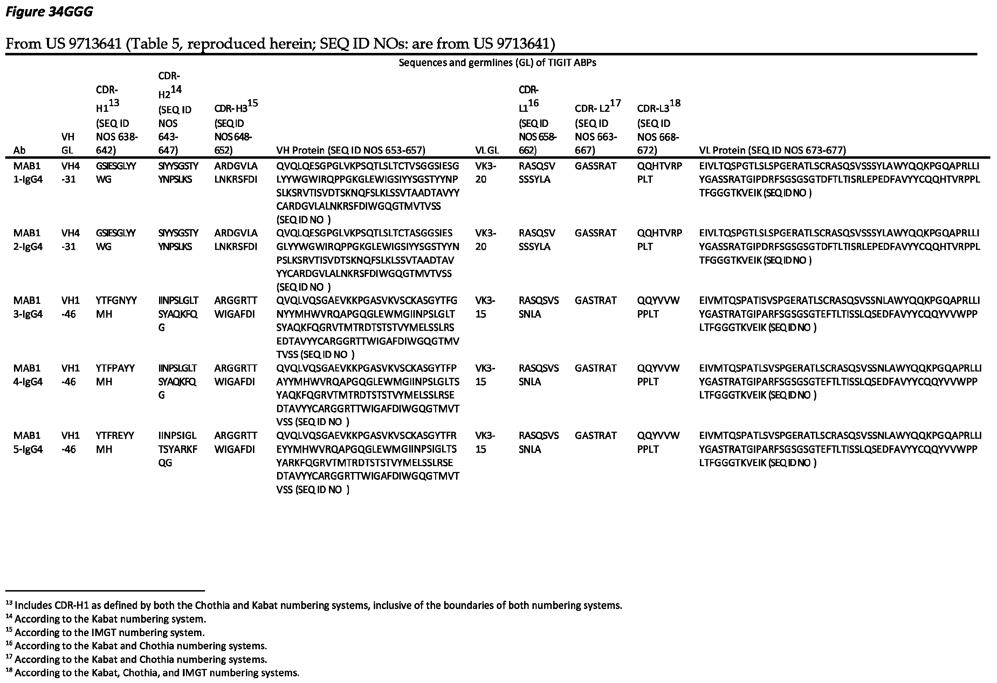

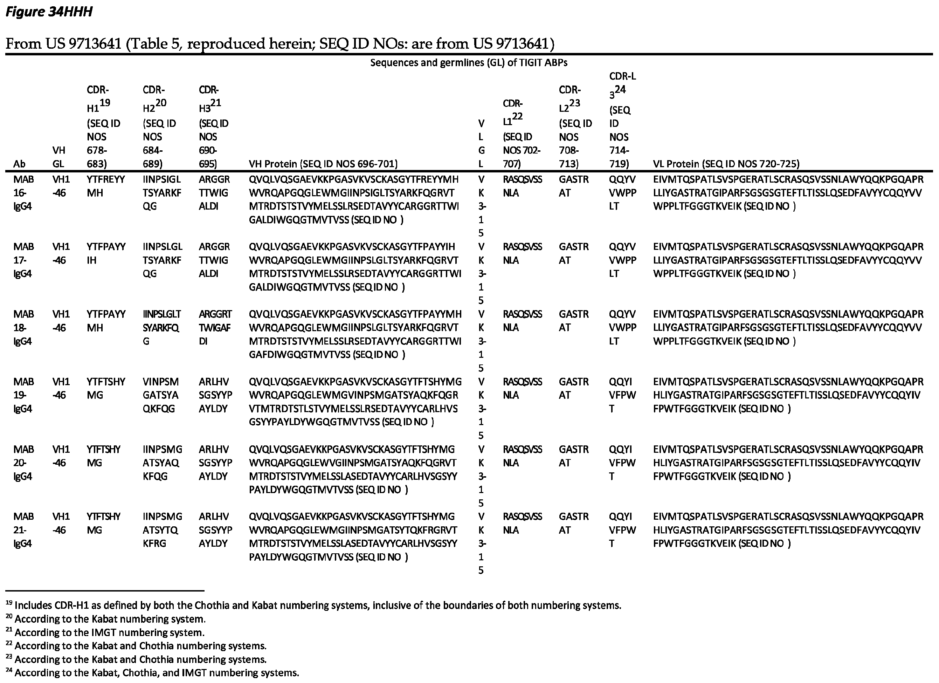

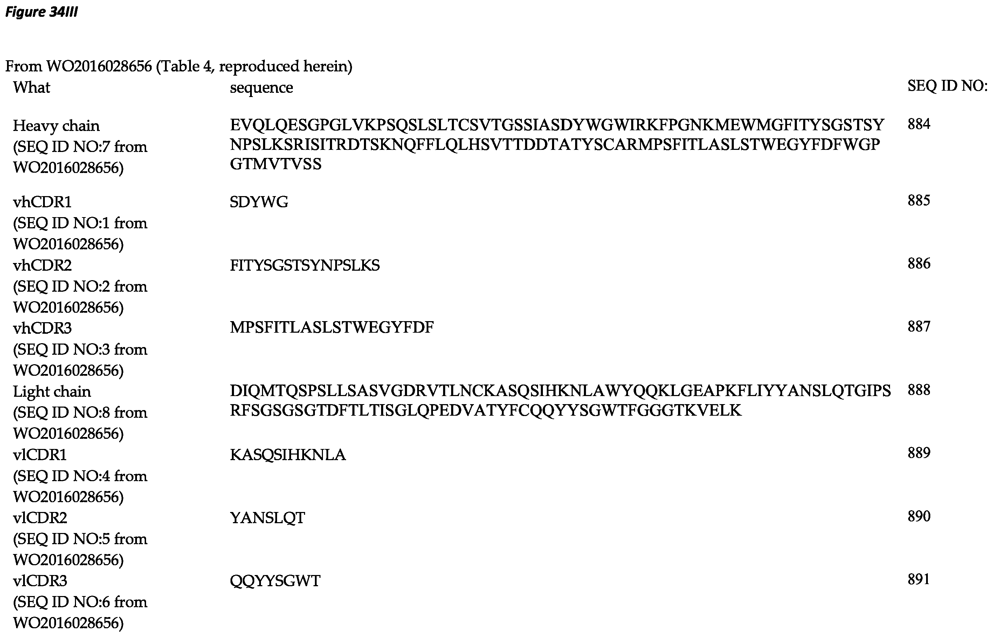

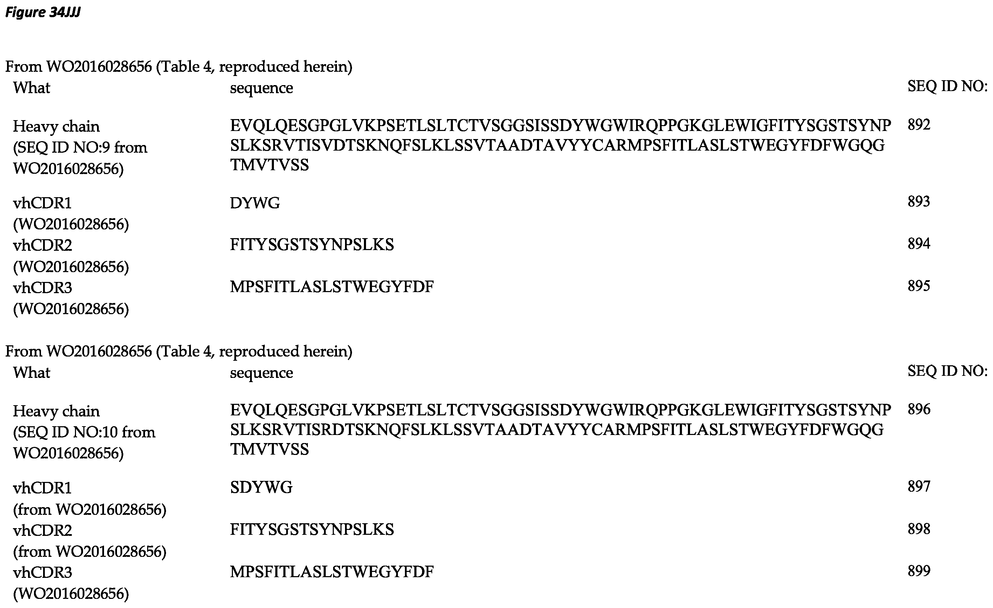

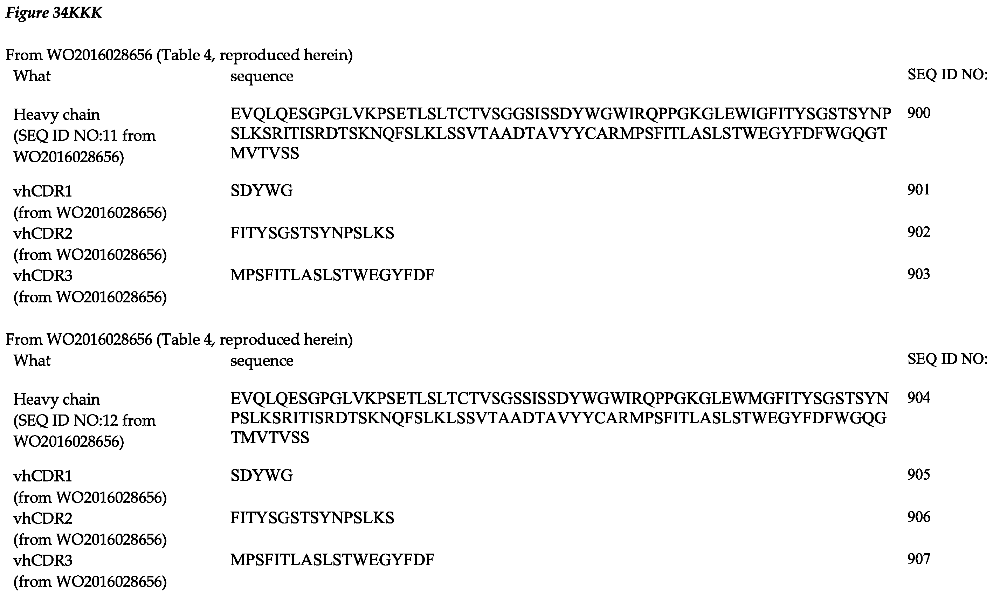

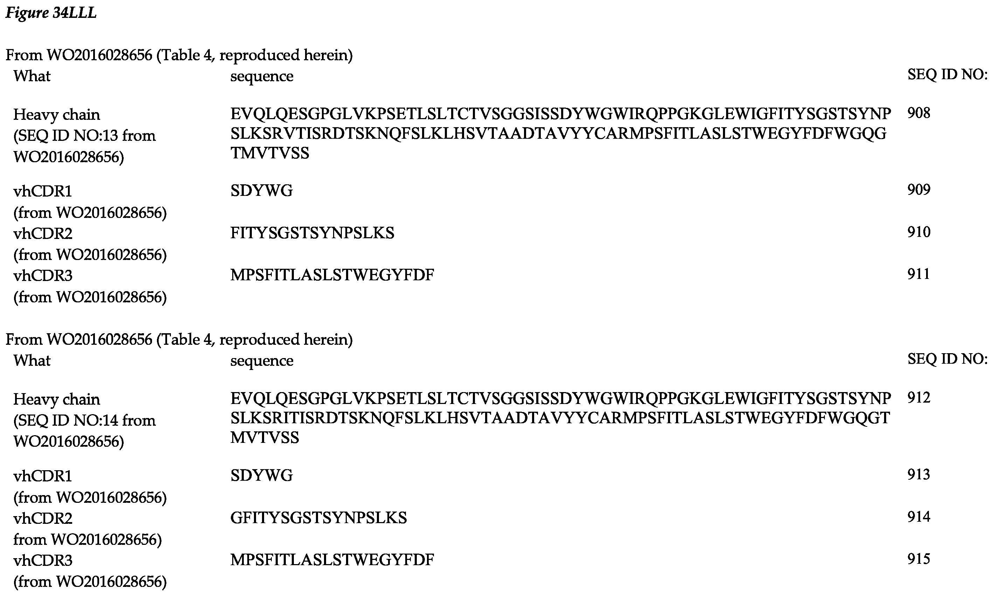

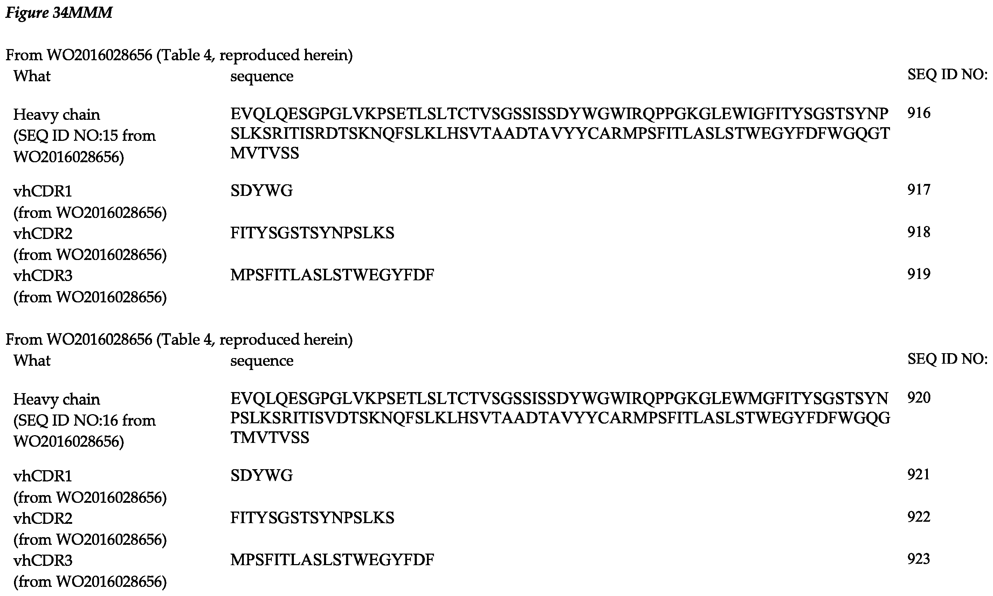

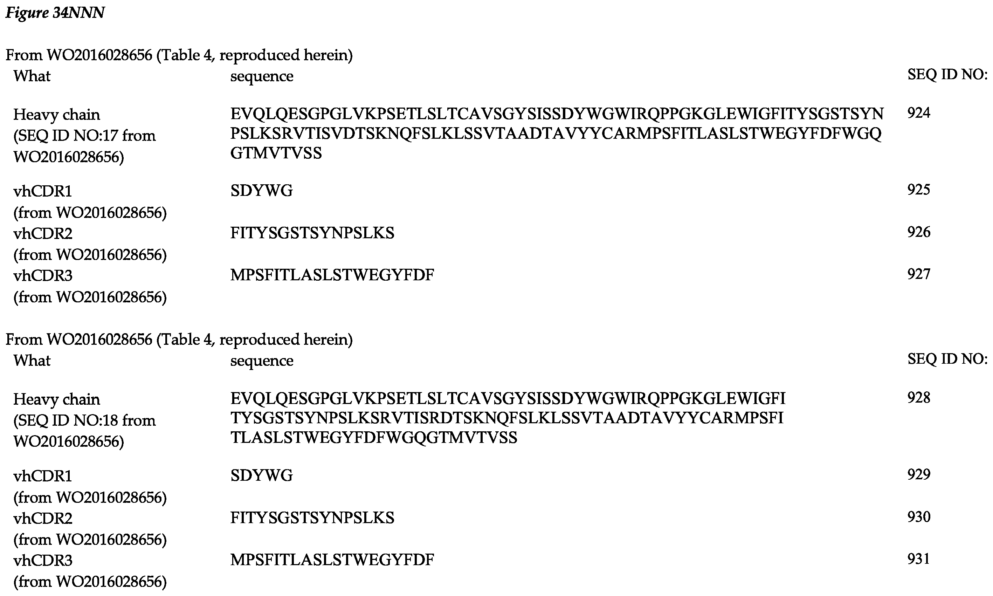

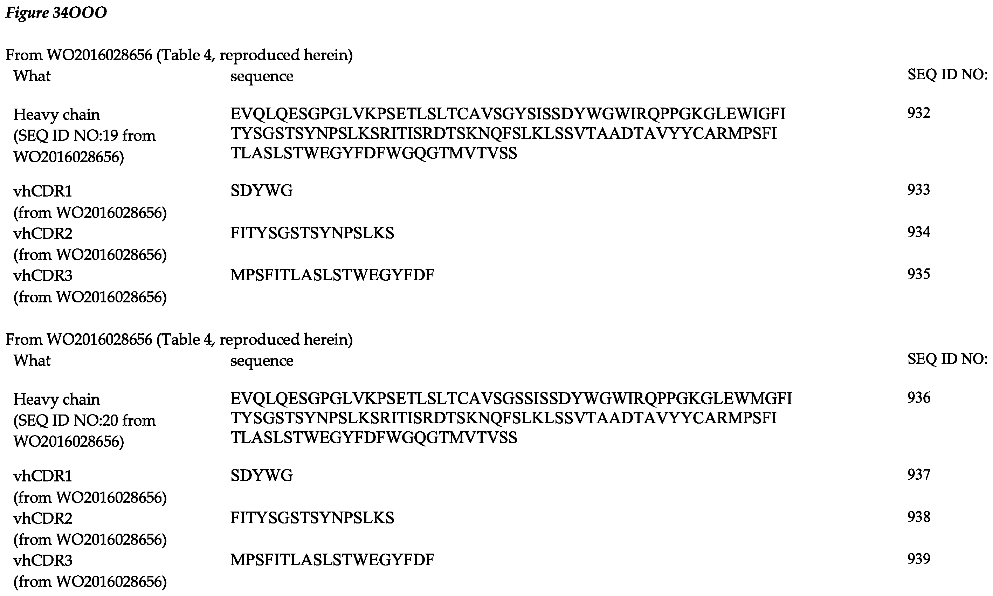

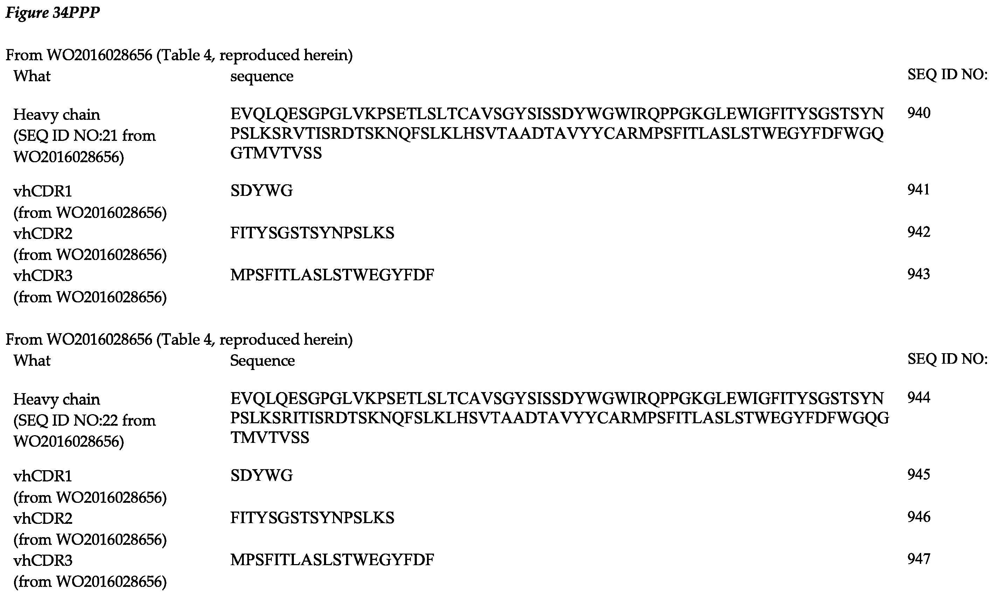

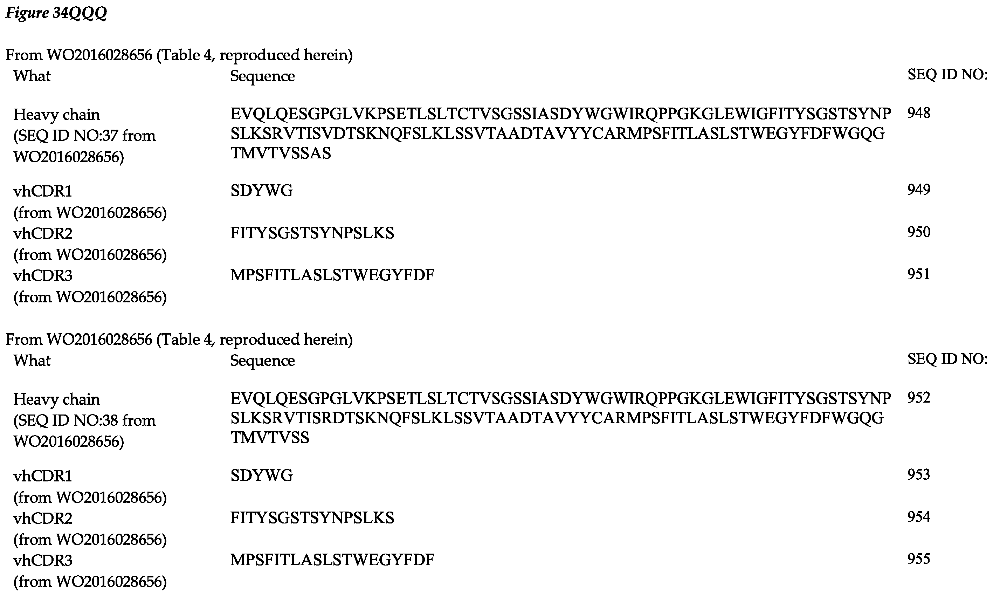

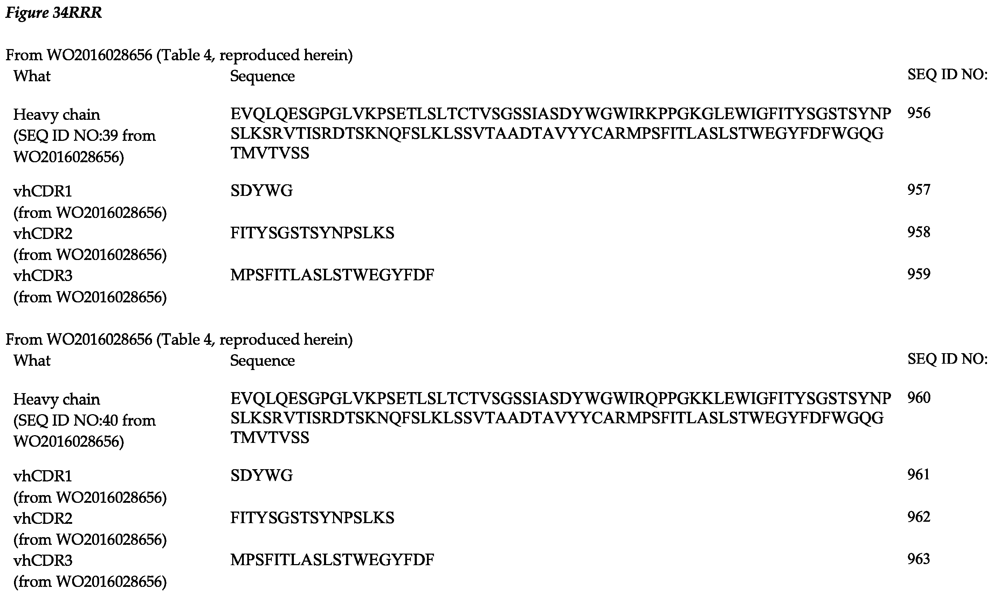

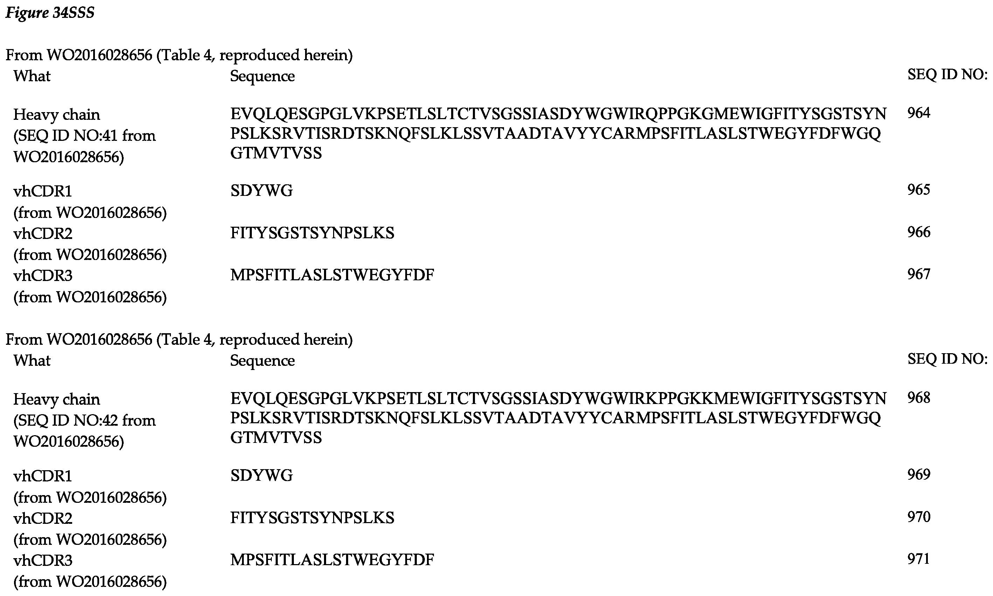

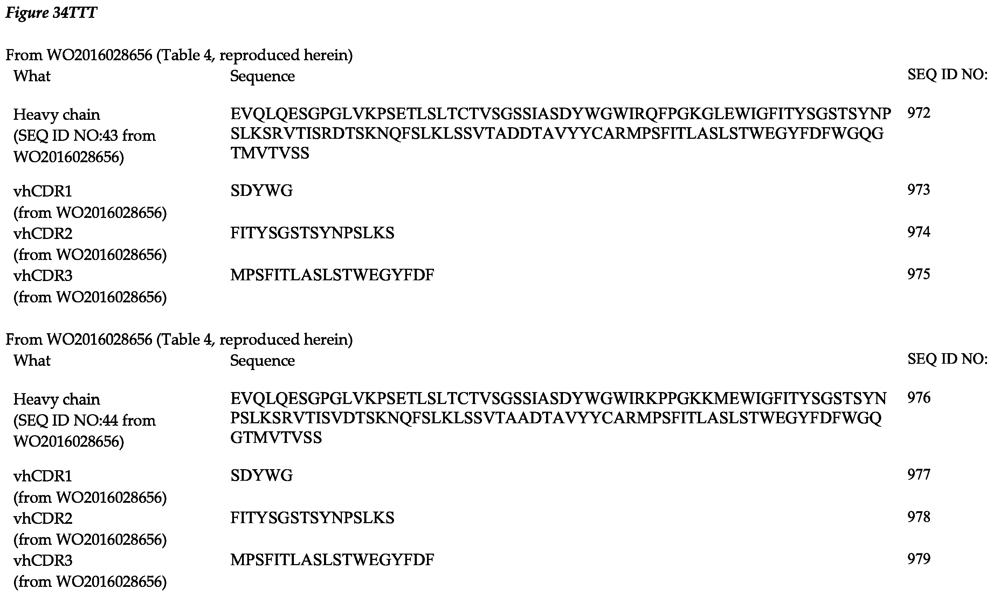

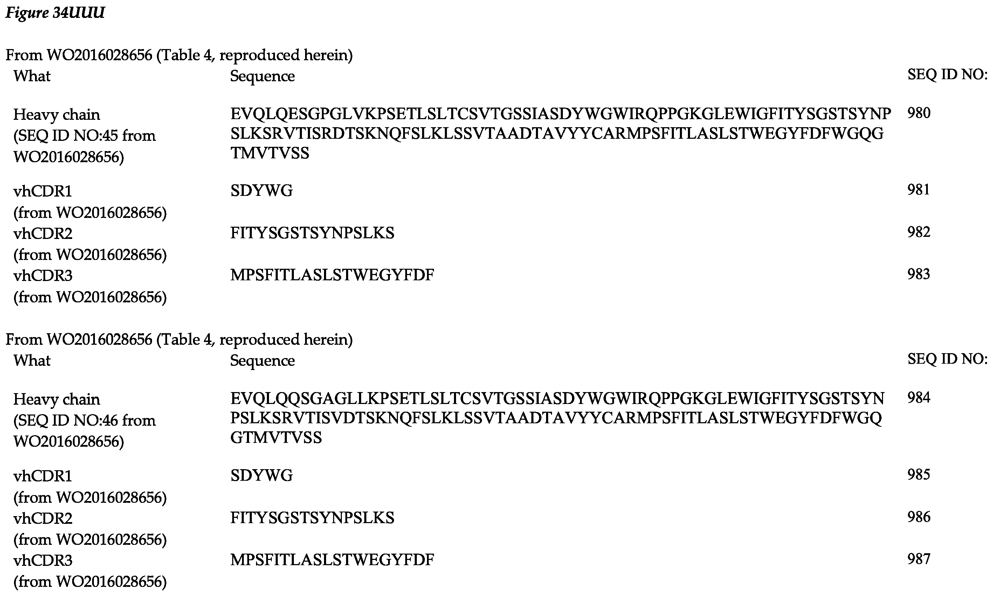

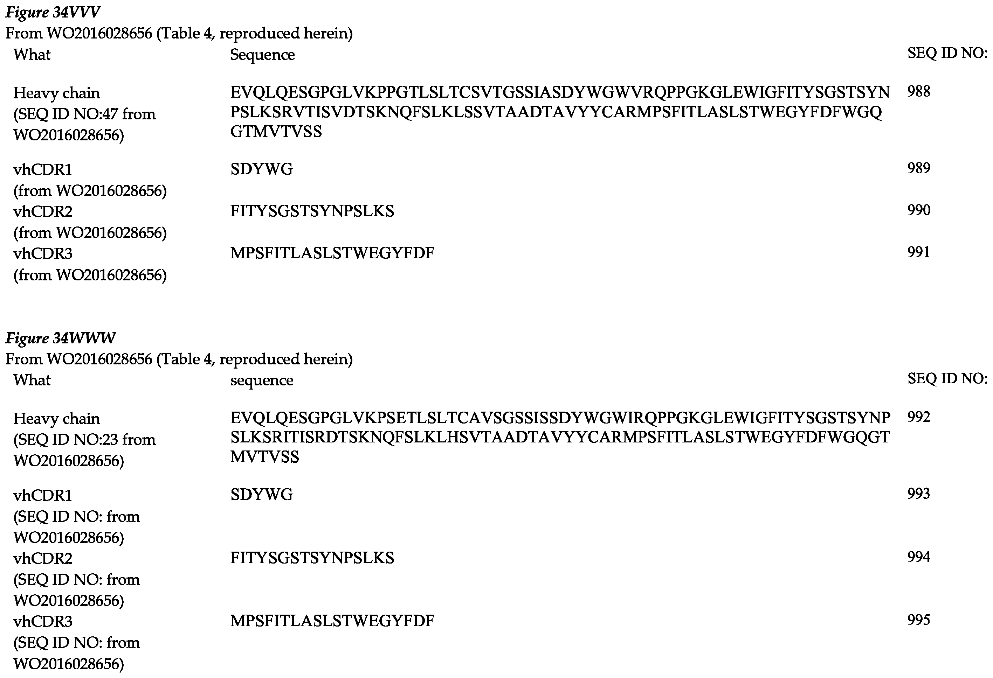

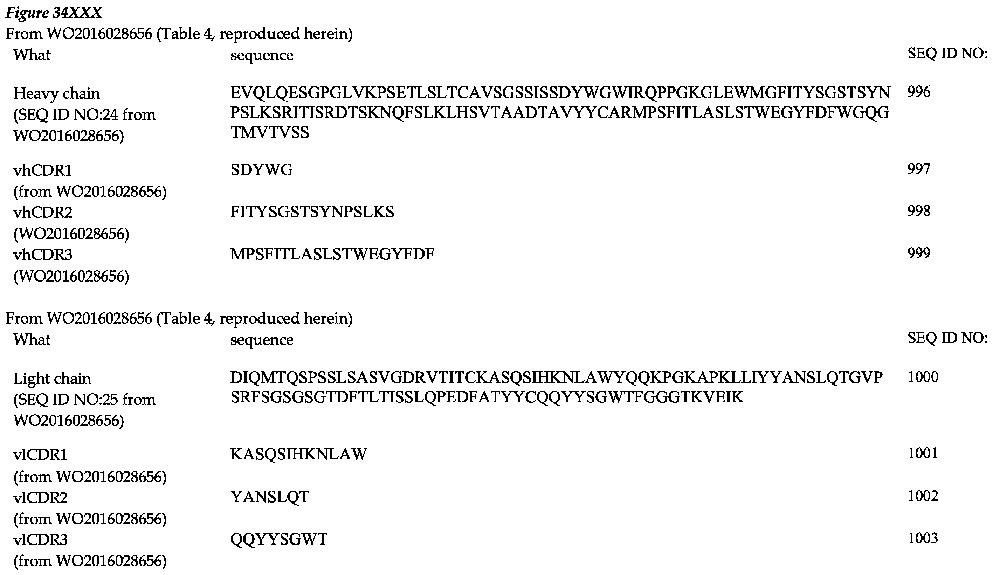

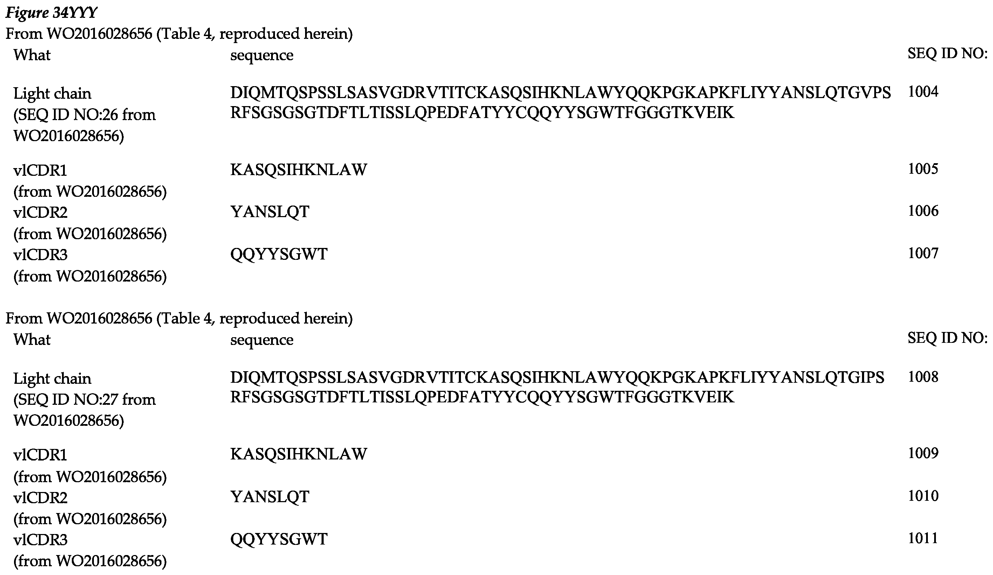

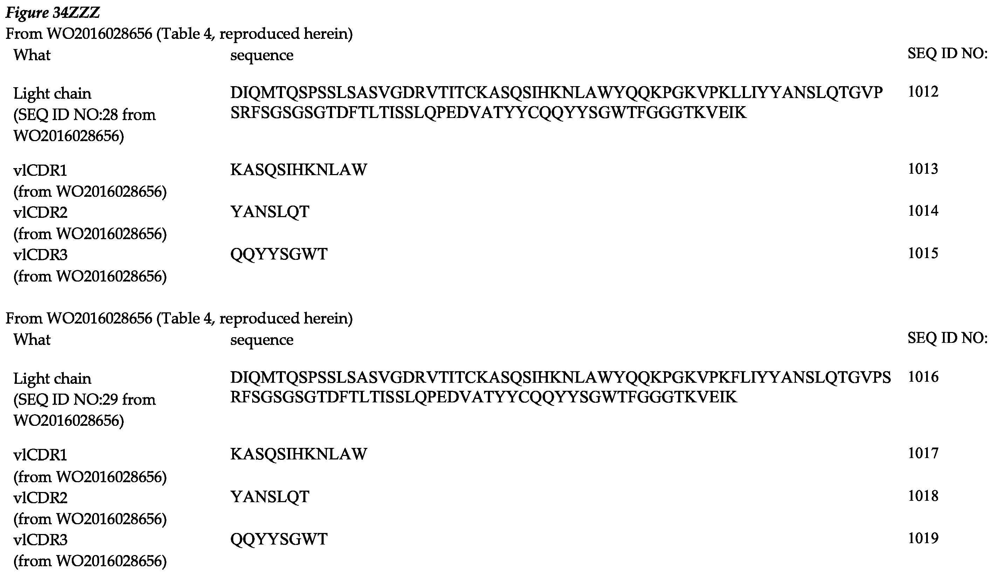

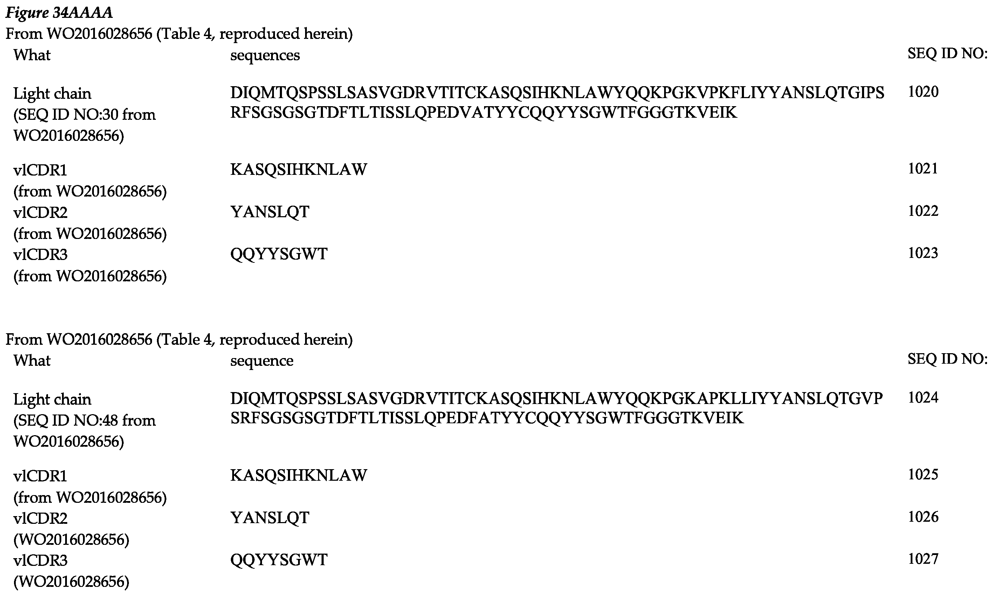

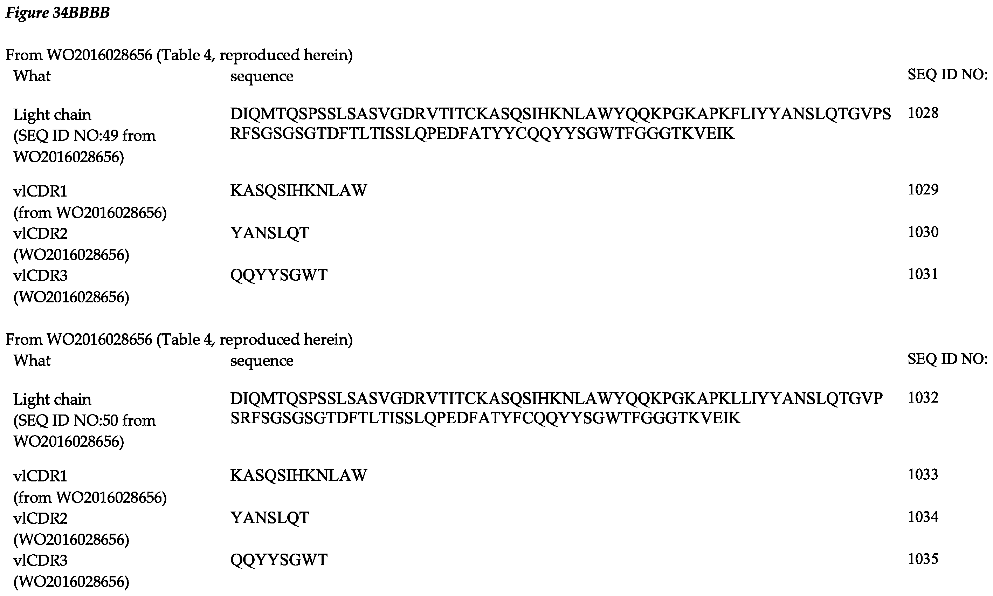

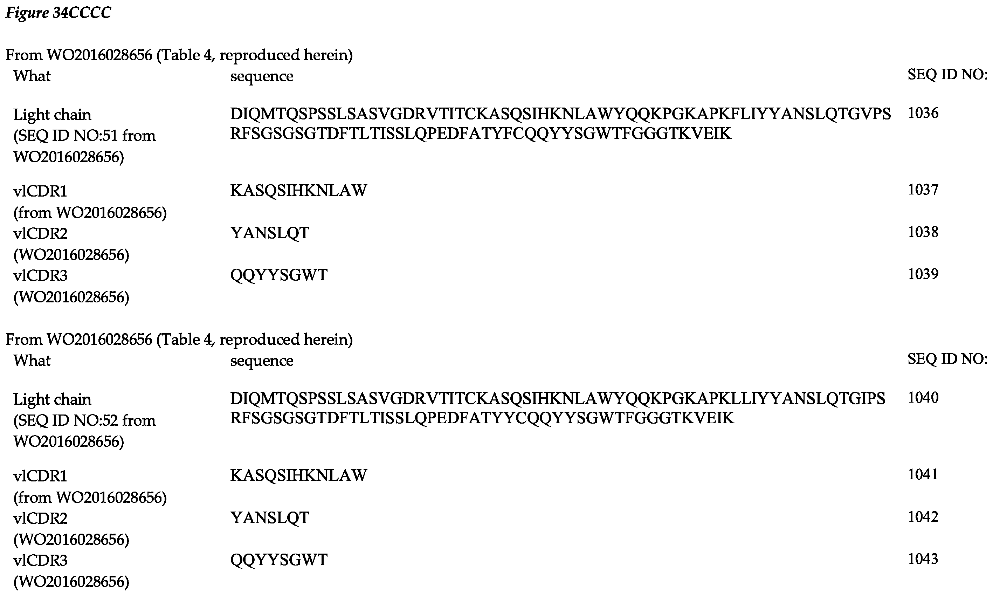

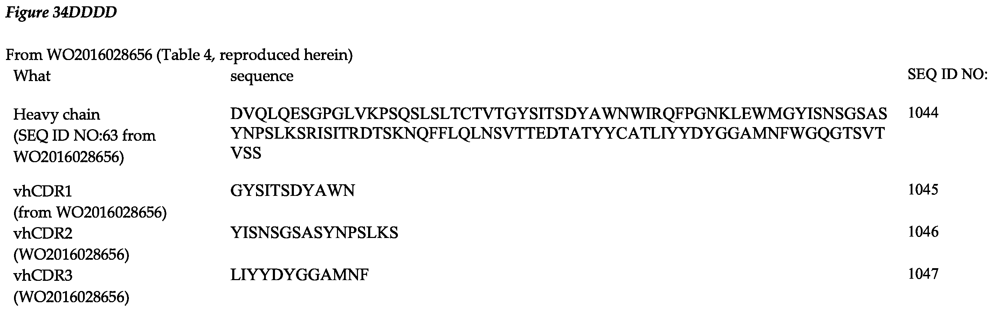

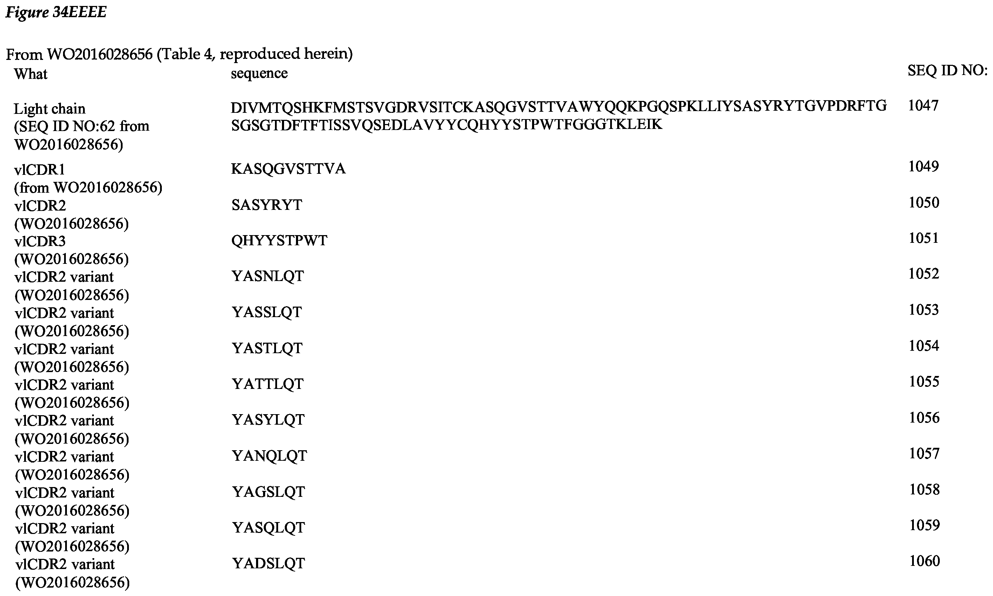

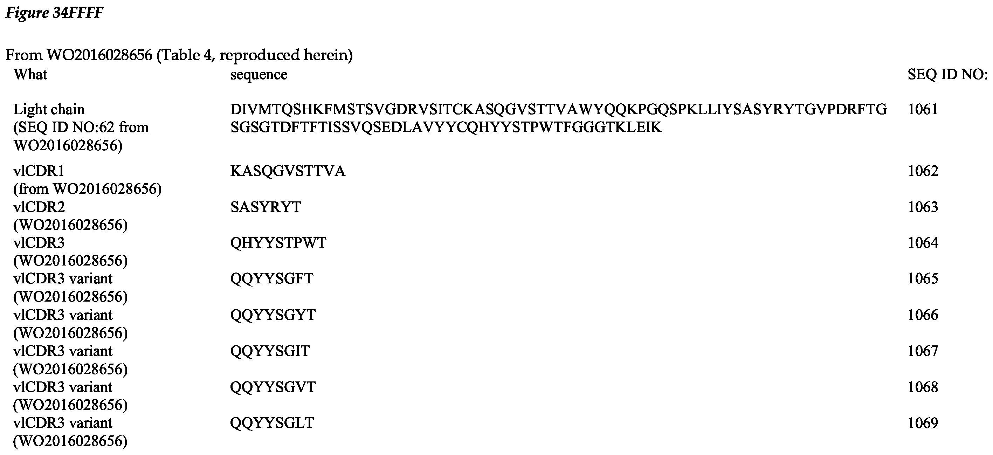

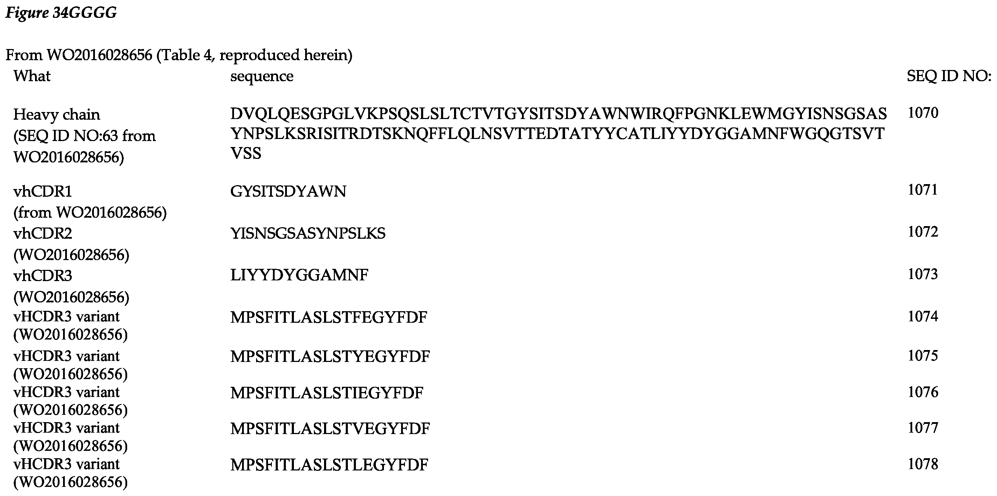

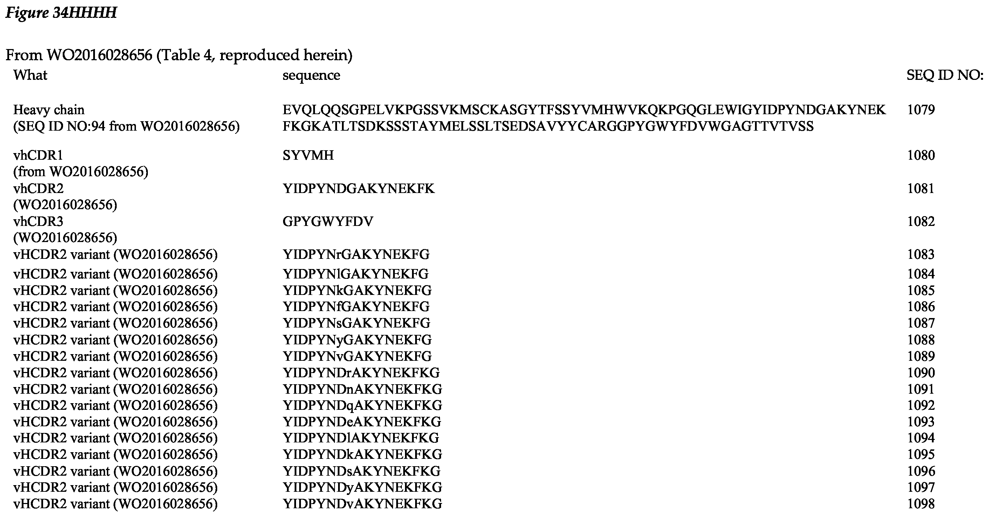

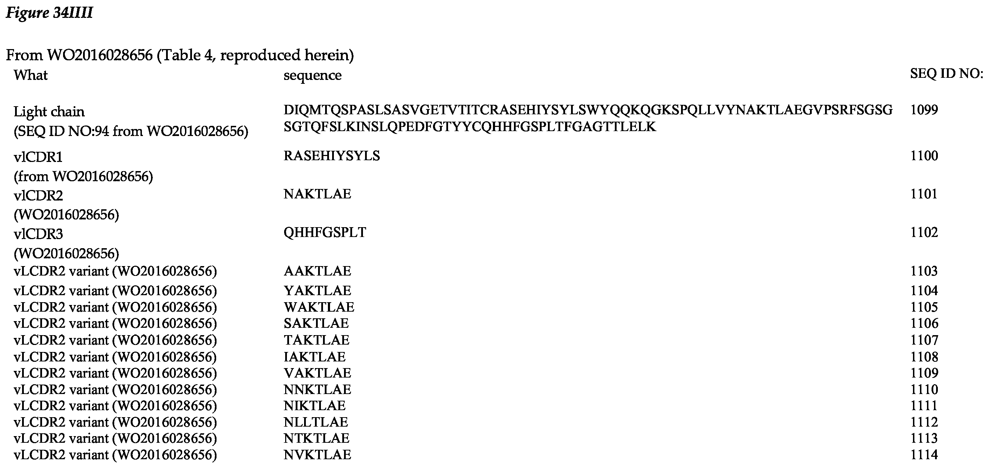

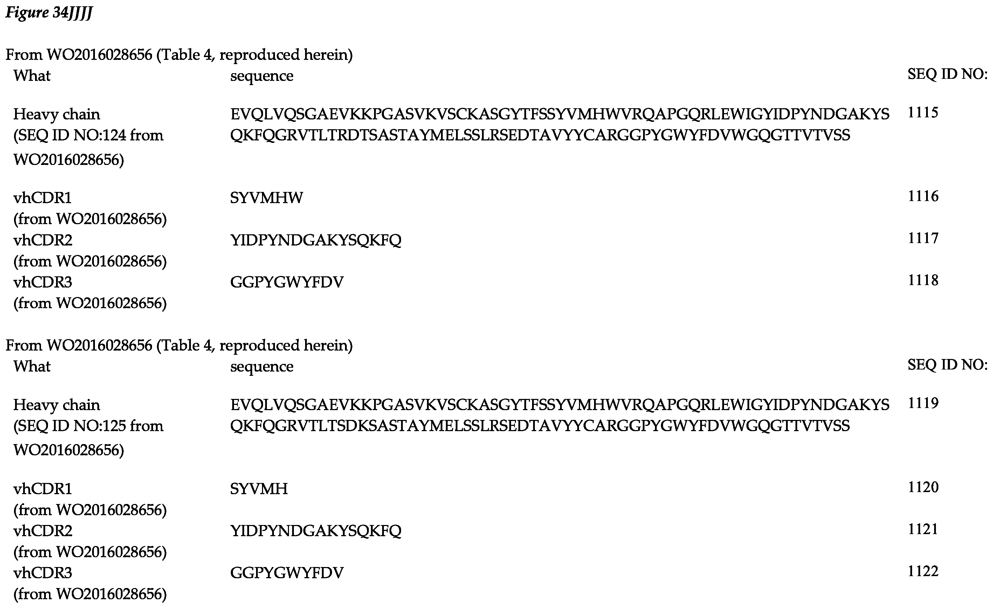

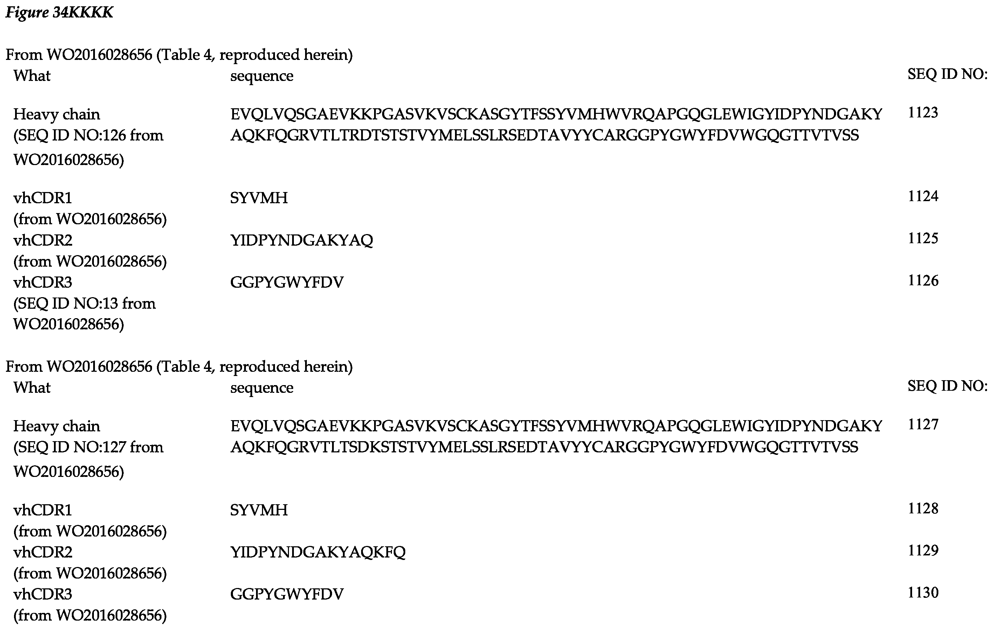

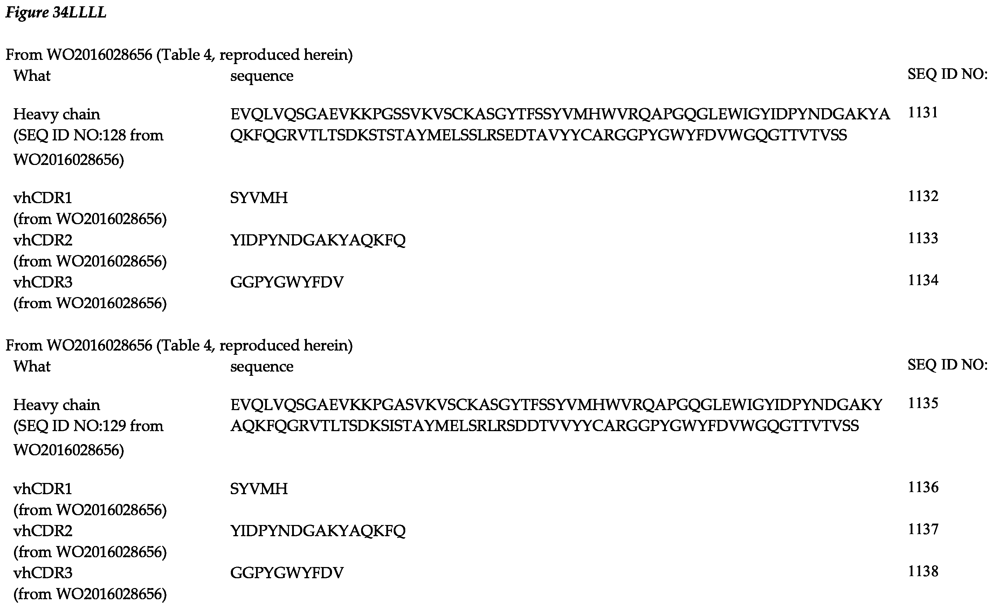

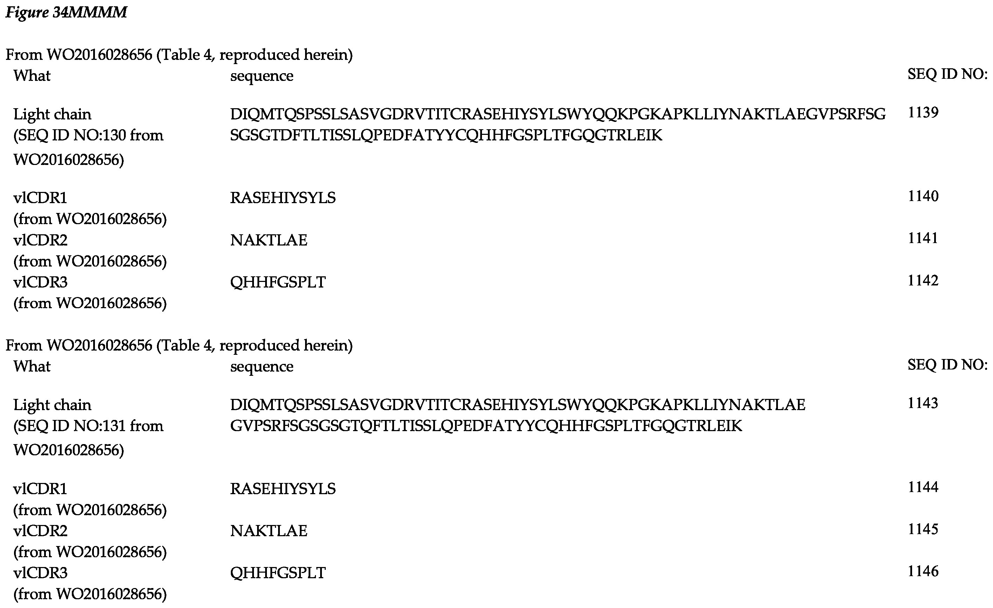

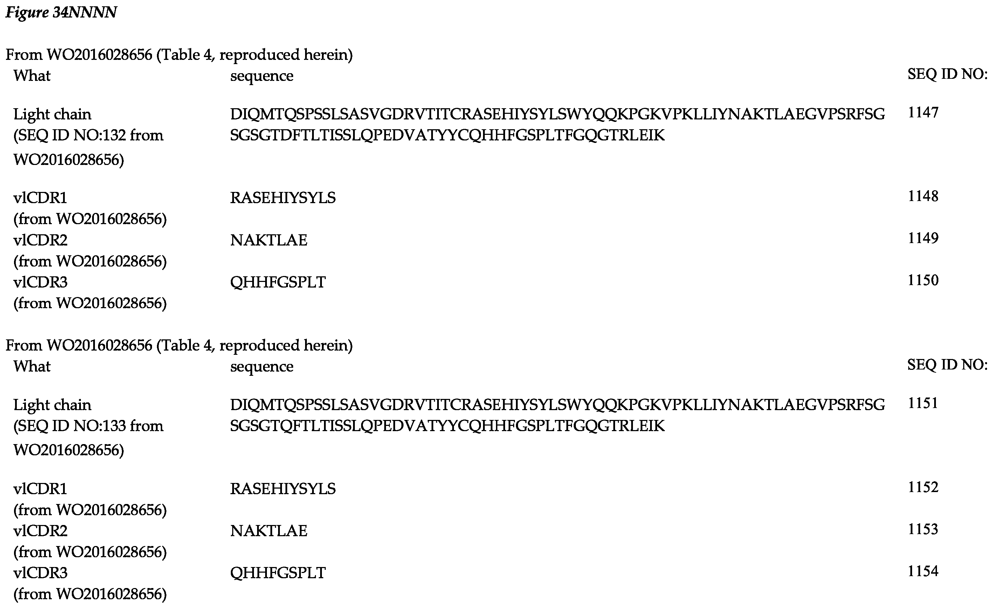

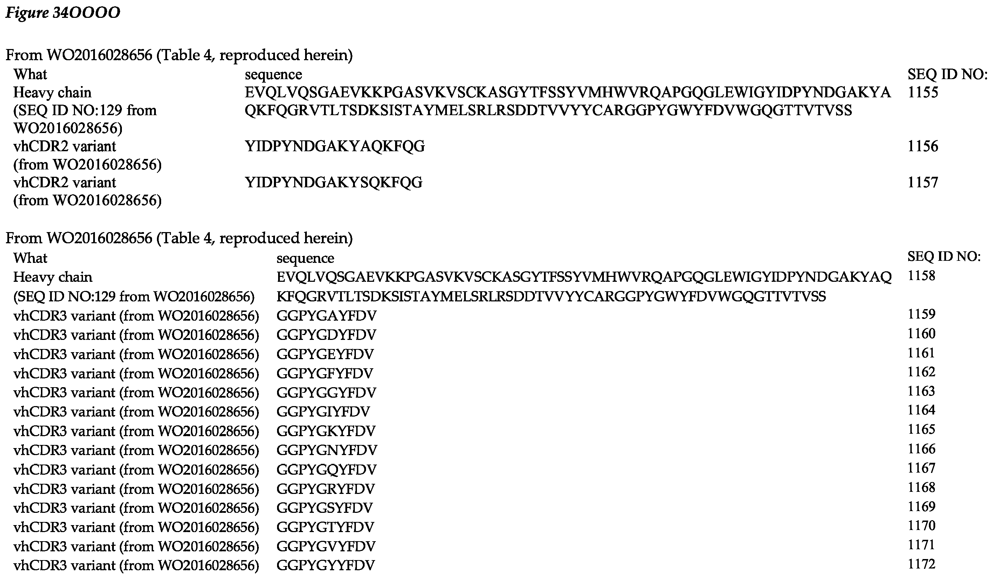

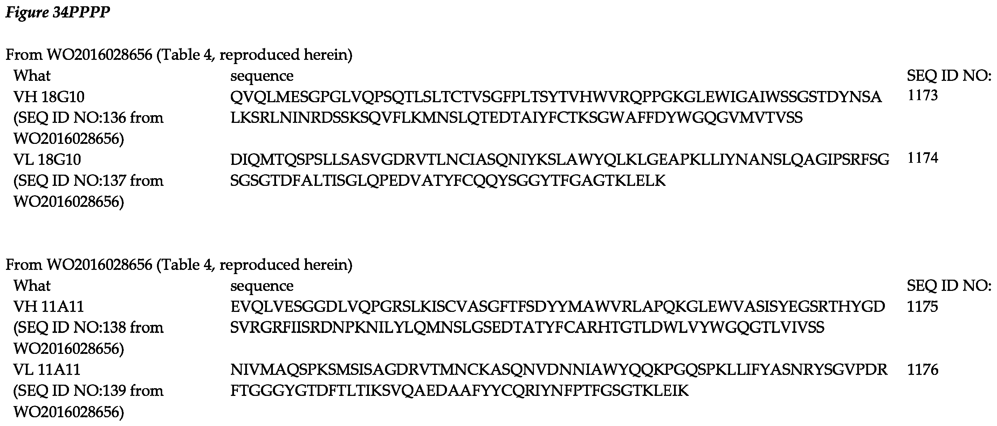

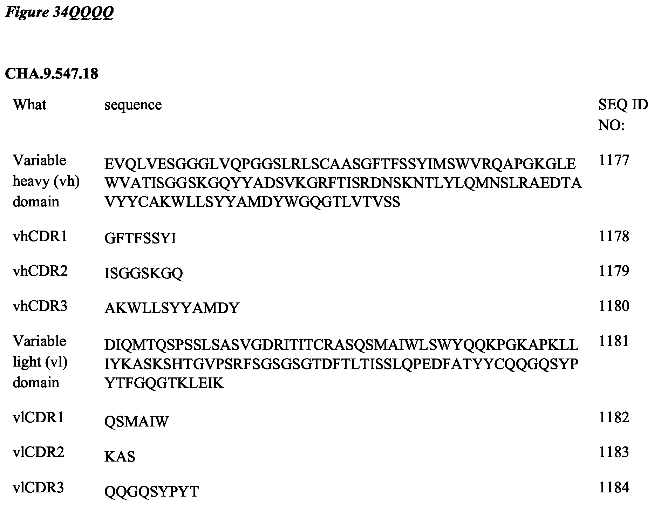

Figure 34A-34QQQQ depicts the sequences of four anti-TIGIT antibodies that block the interaction of TIGIT and PVR, CPA.9.083.H4(S241P), CPA.9.086.H4(S241P), CHA.9.547.7.H4(S241P) and CHA.9.547.13.H4(S241P), as well as benchmark antibodies, BM26 and BM29, and numerous other anti-TIGIT antibodies.

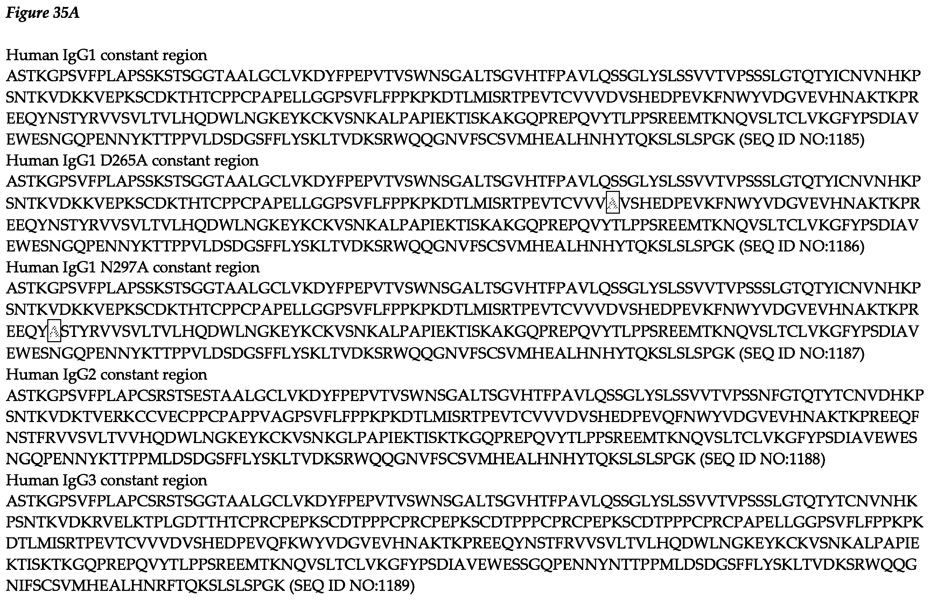

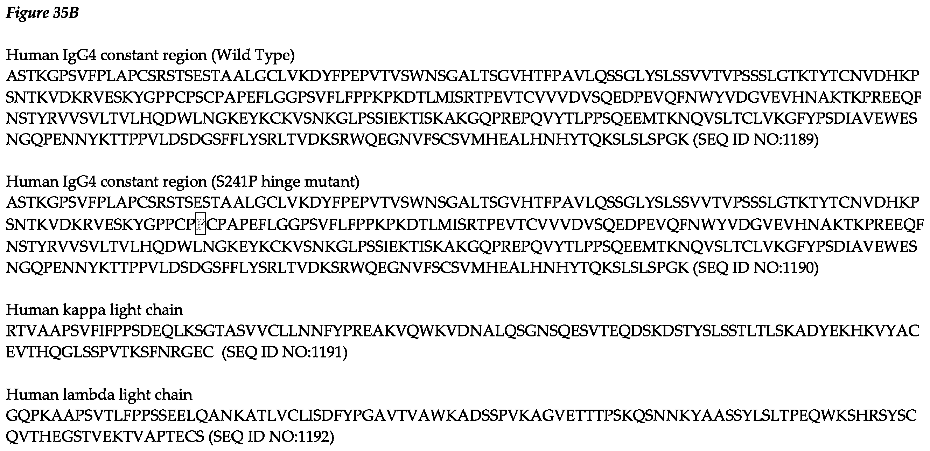

Figure 35A-35B depicts the amino acid sequences of the constant domains of human IgG1 (with some useful amino acid substitutions), IgG2, IgG3, IgG4, IgG4 with a hinge variant that finds particular use in the present invention, and the constant domains of the kappa and lambda light chains.

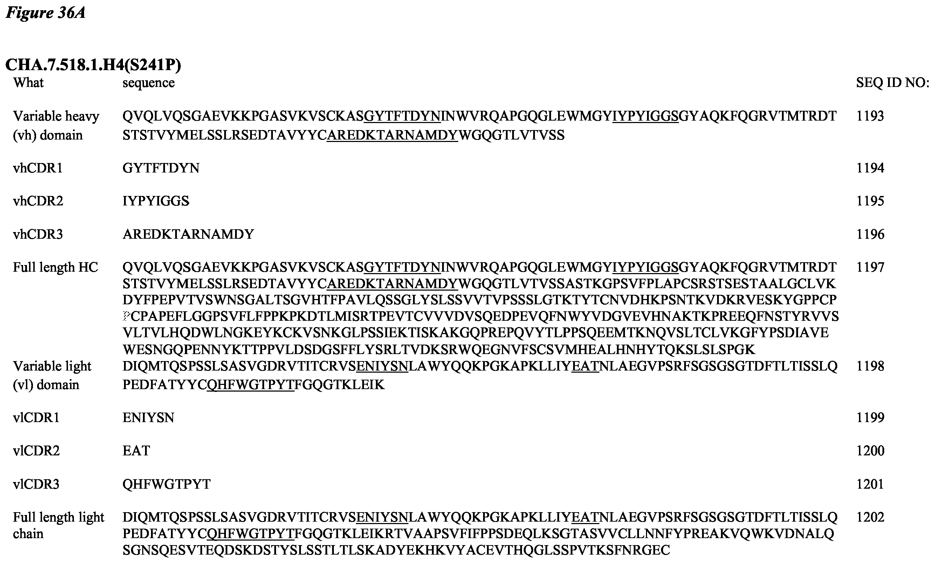

Figure 36A-36AG depicts the variable heavy and light chains as well as the vhCDR1, vhCDR2, vhCDR3, vlCDR1, vlCDR2 and vlCDR3 sequences of the anti-PVRIG antibodies described herein.

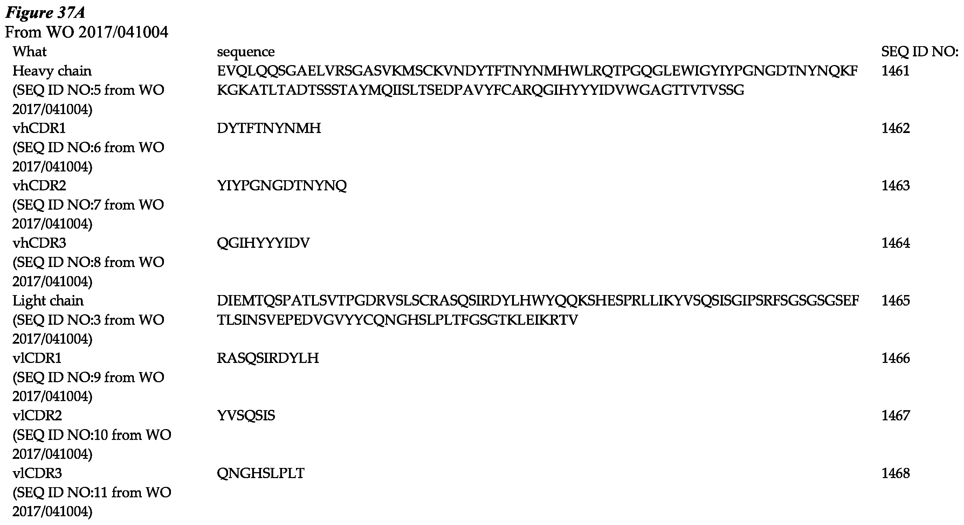

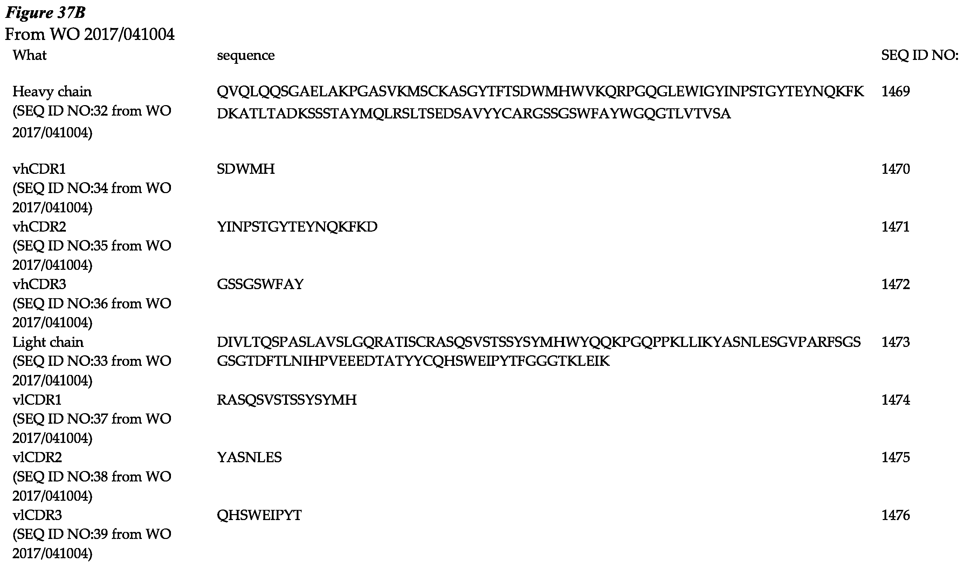

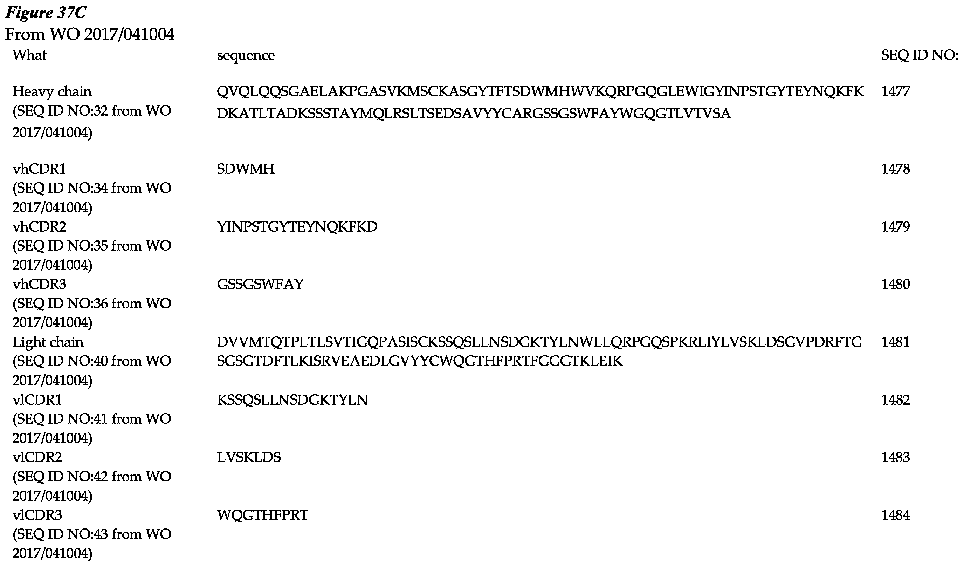

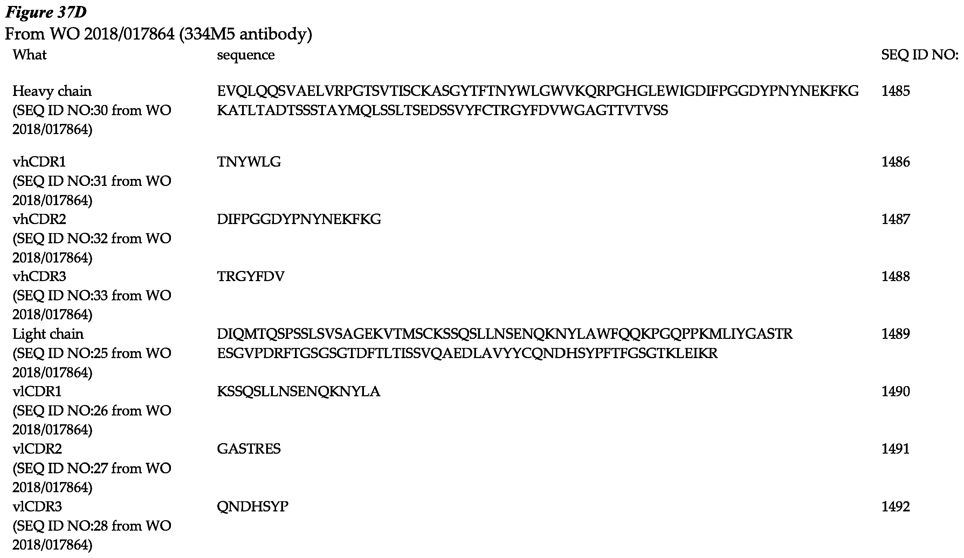

Figure 37A-37D depicts the sequences of other PVRIG antibodies described herein.

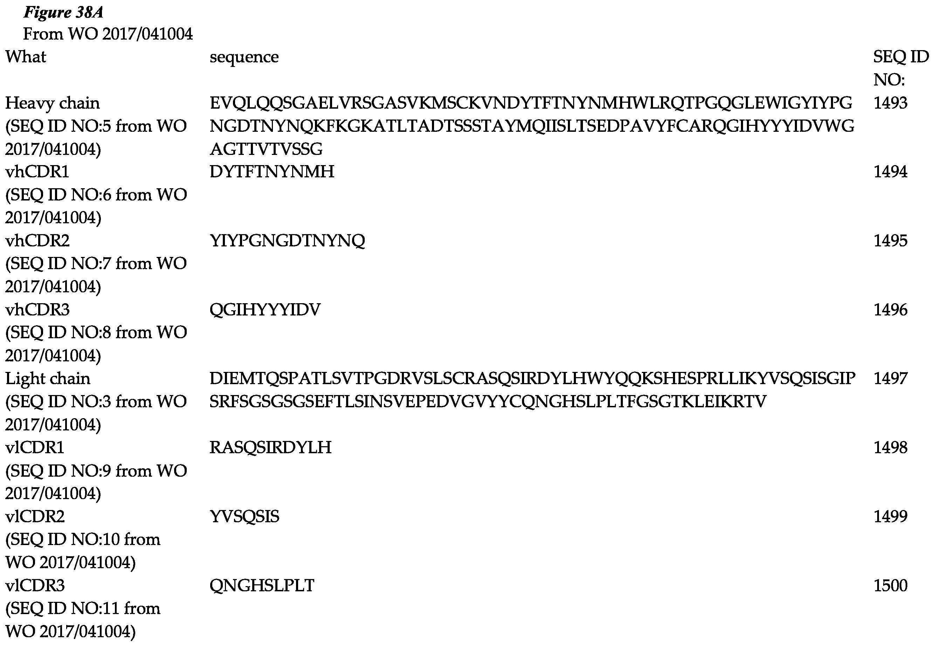

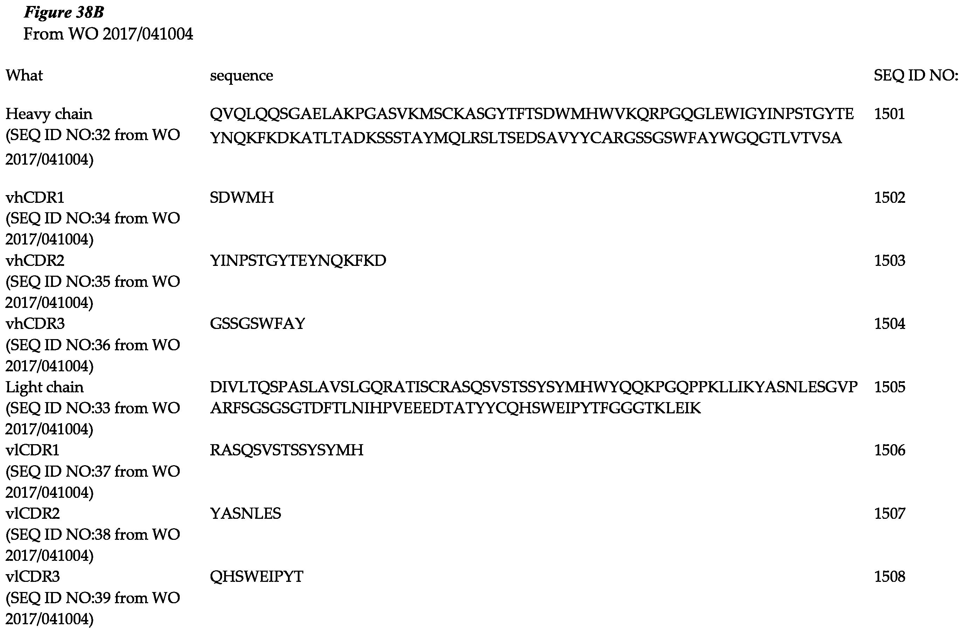

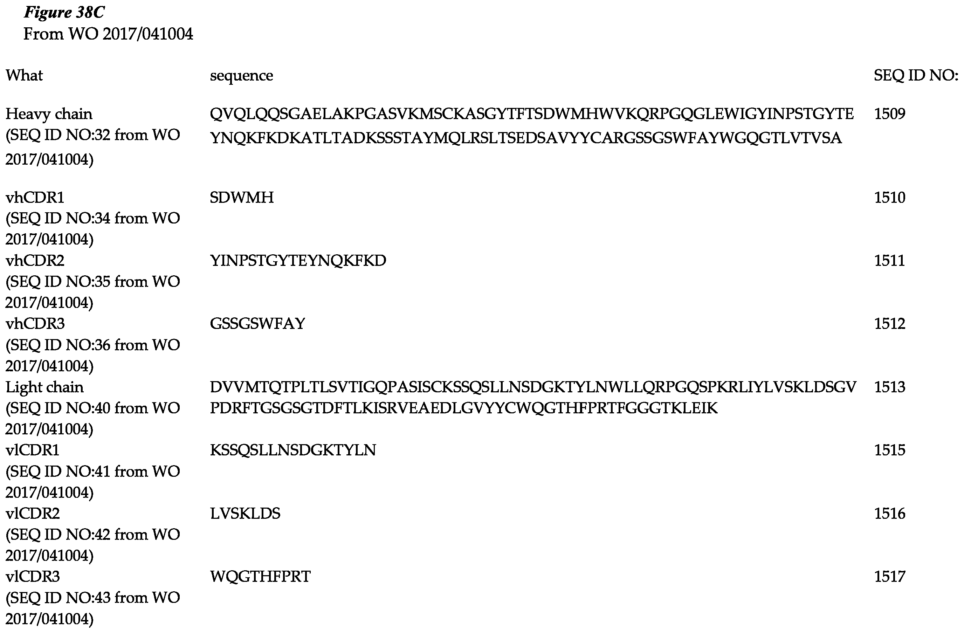

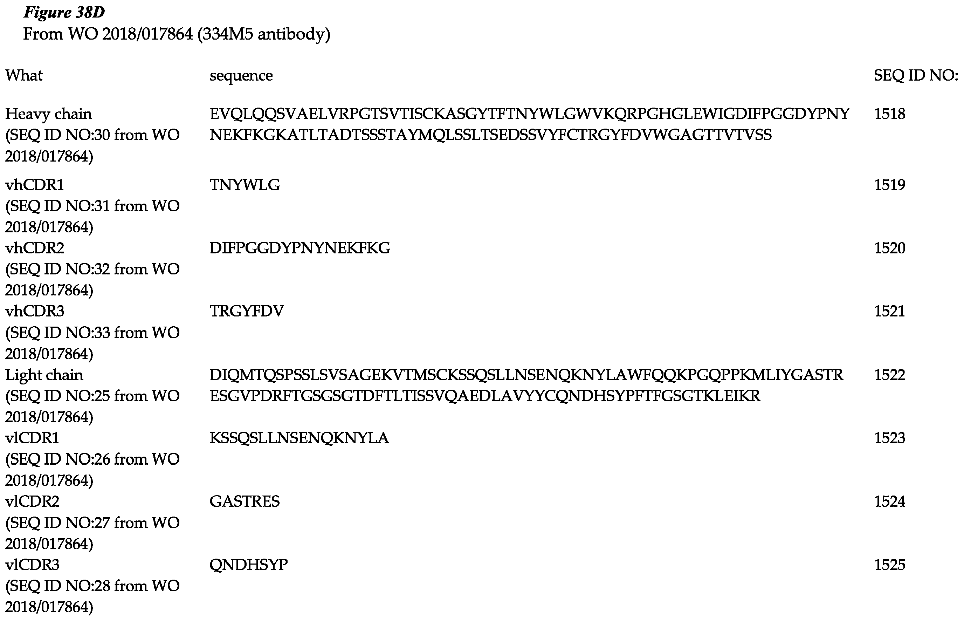

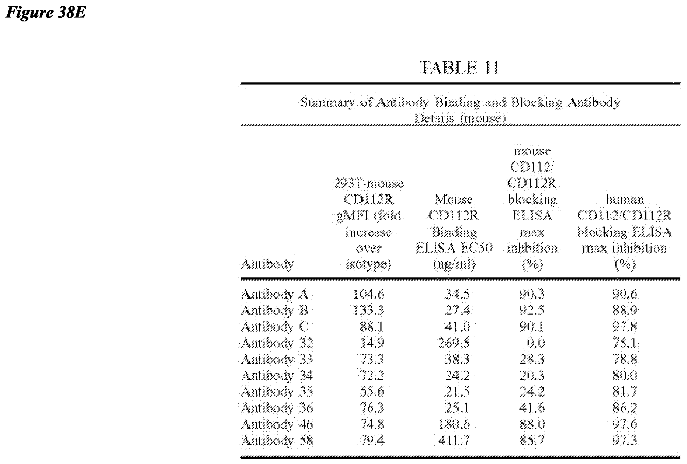

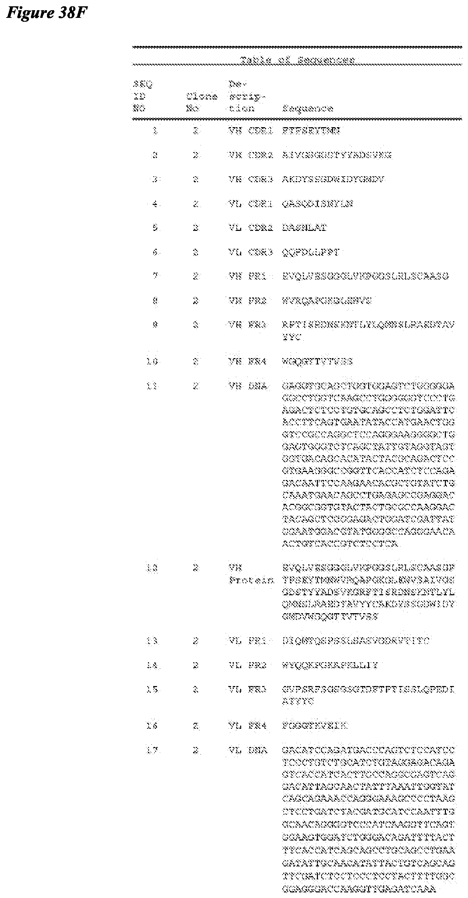

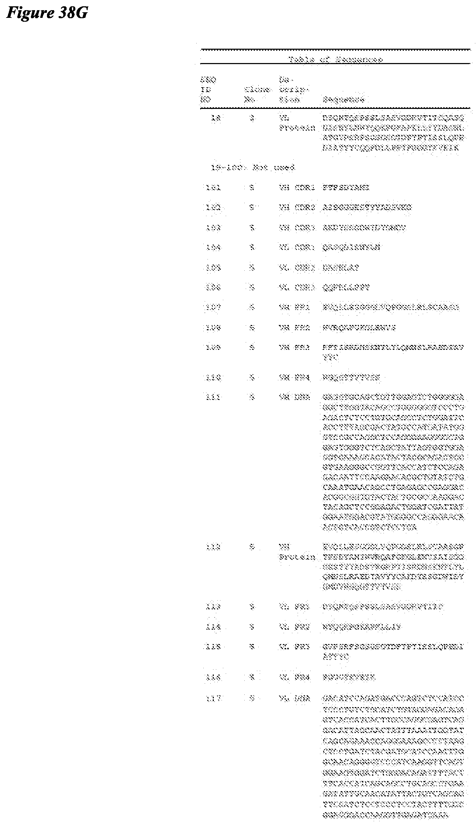

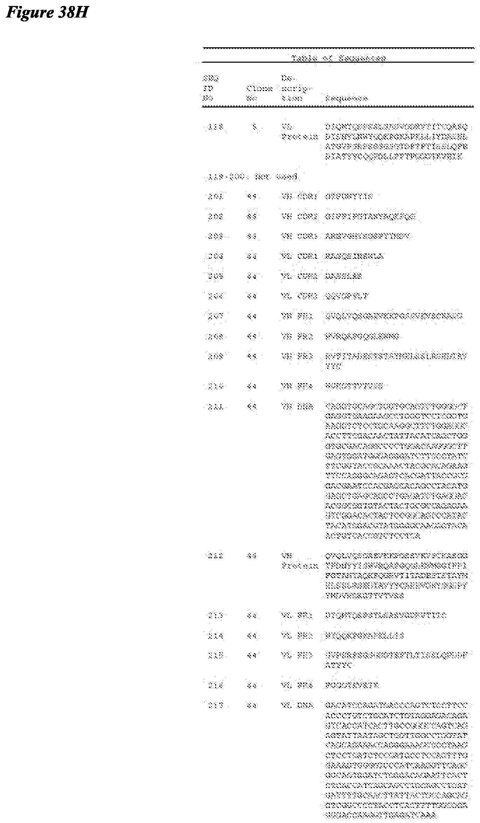

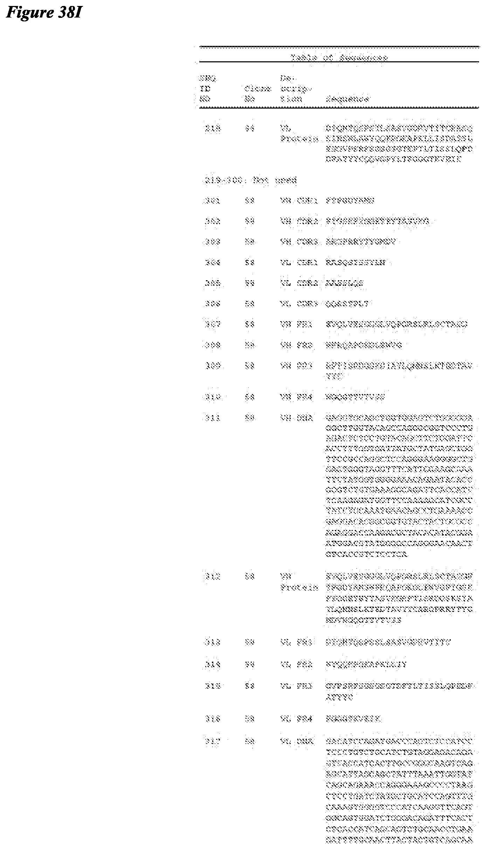

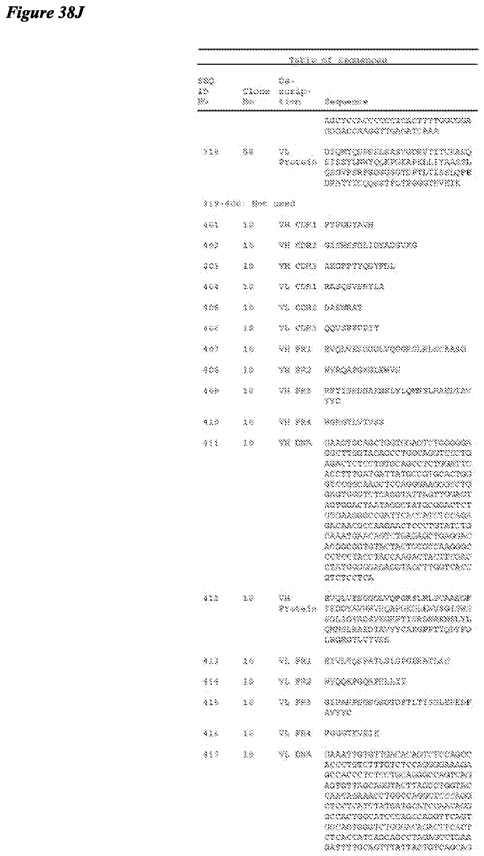

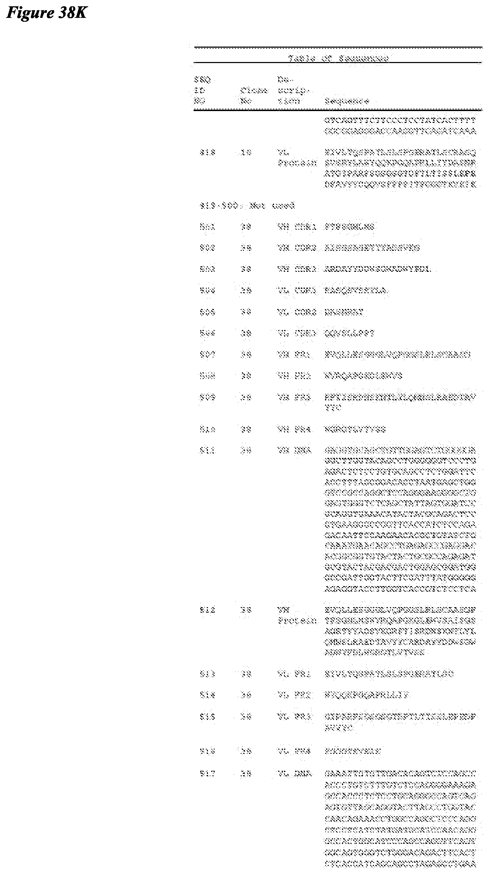

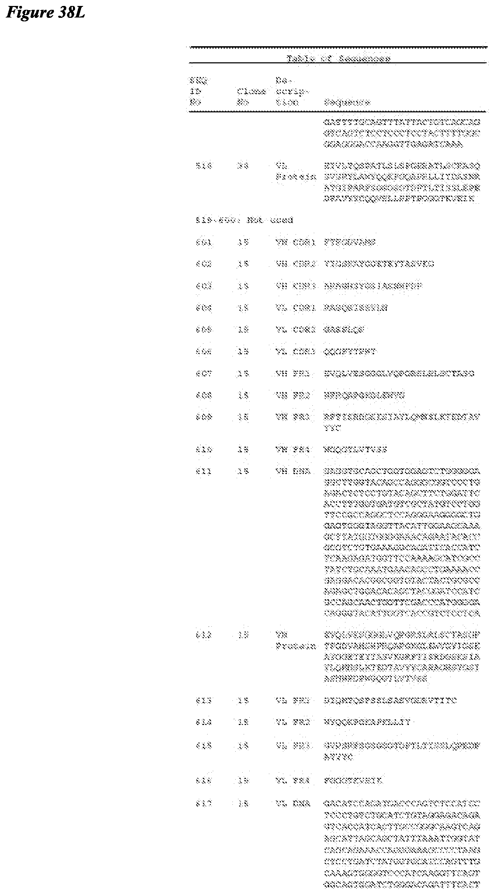

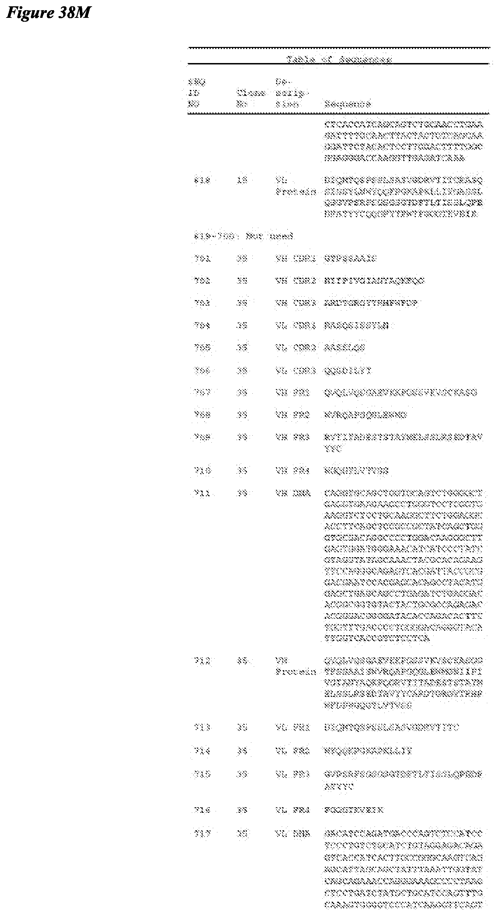

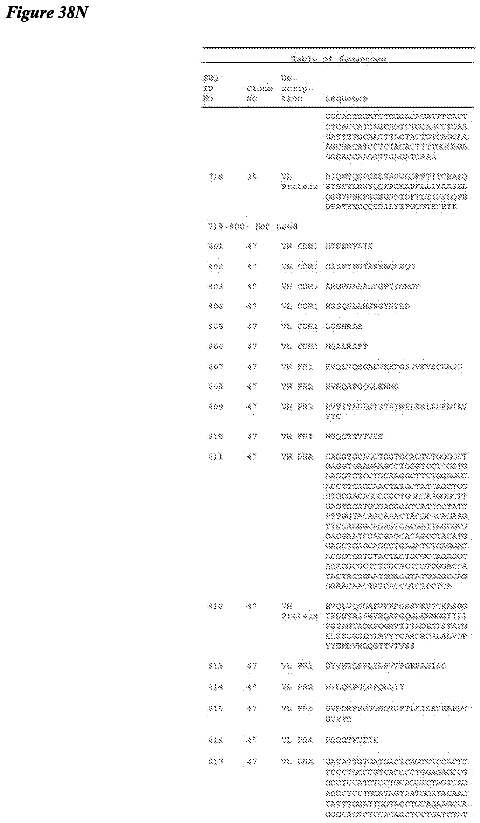

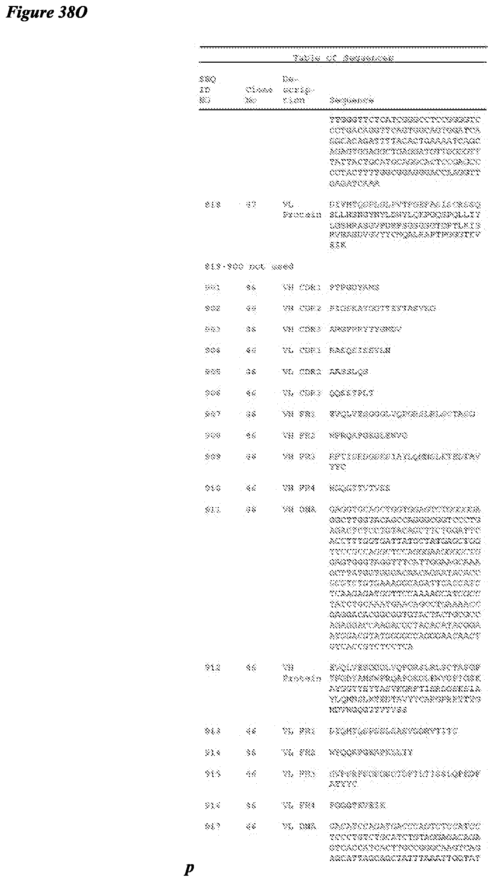

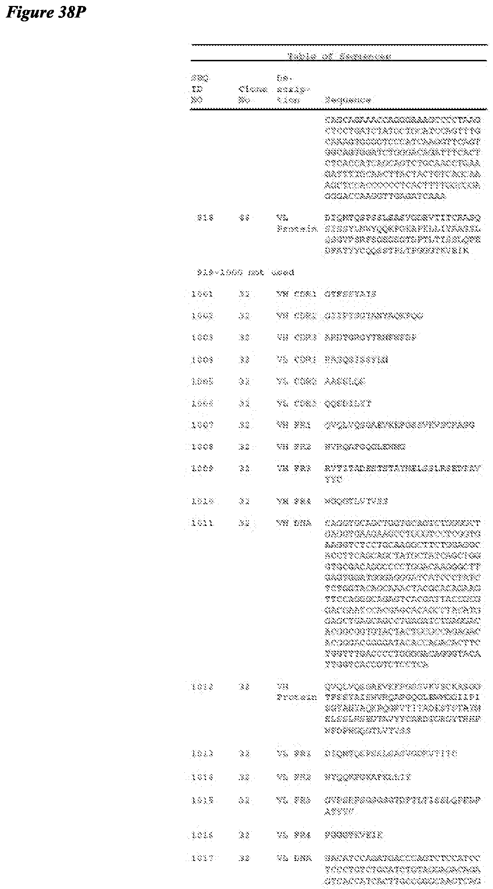

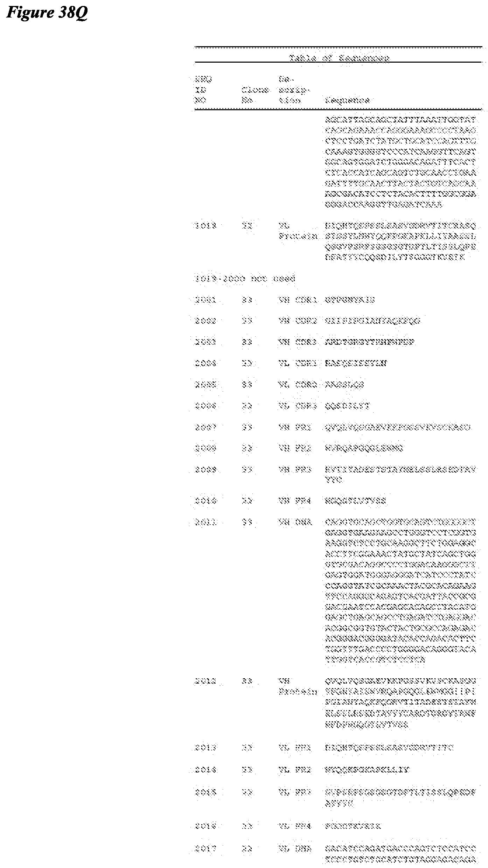

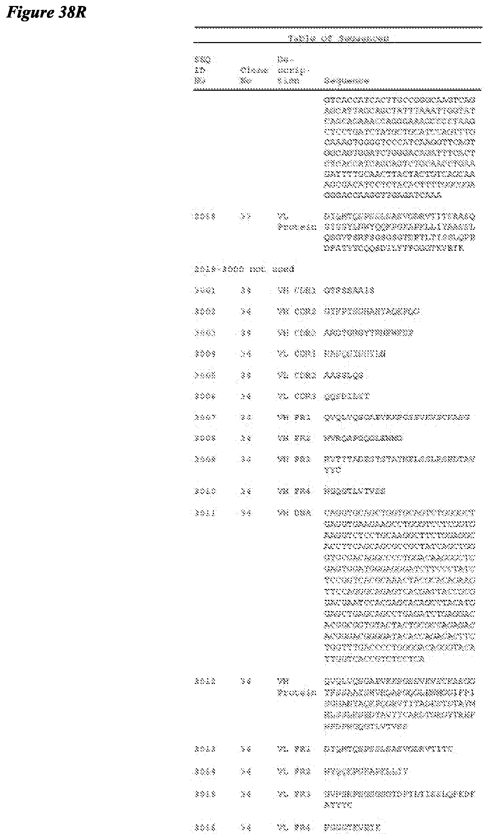

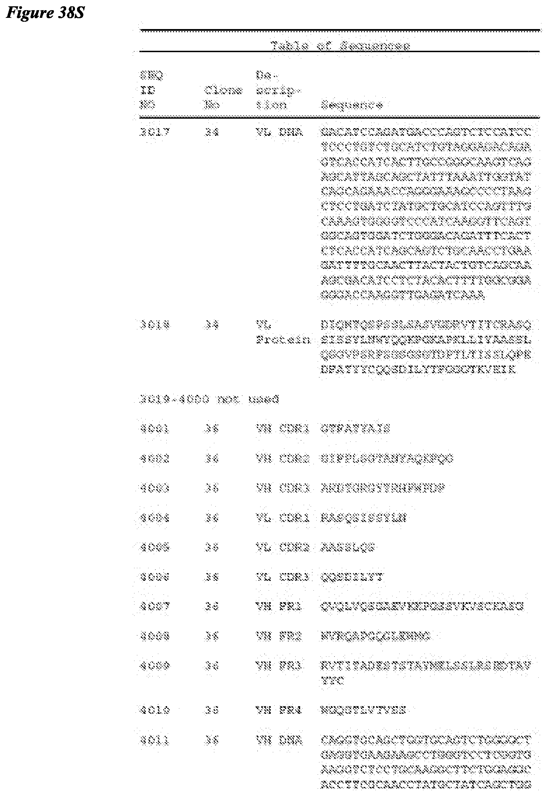

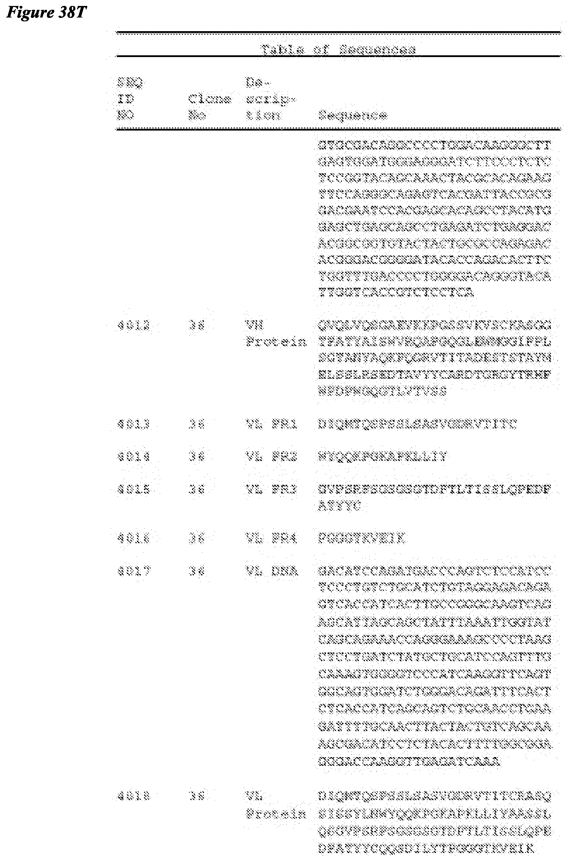

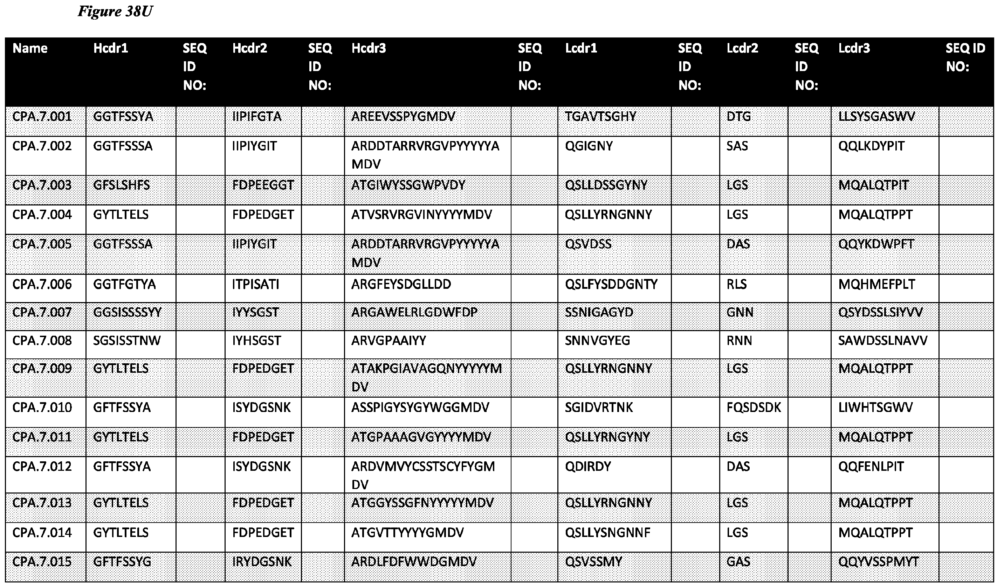

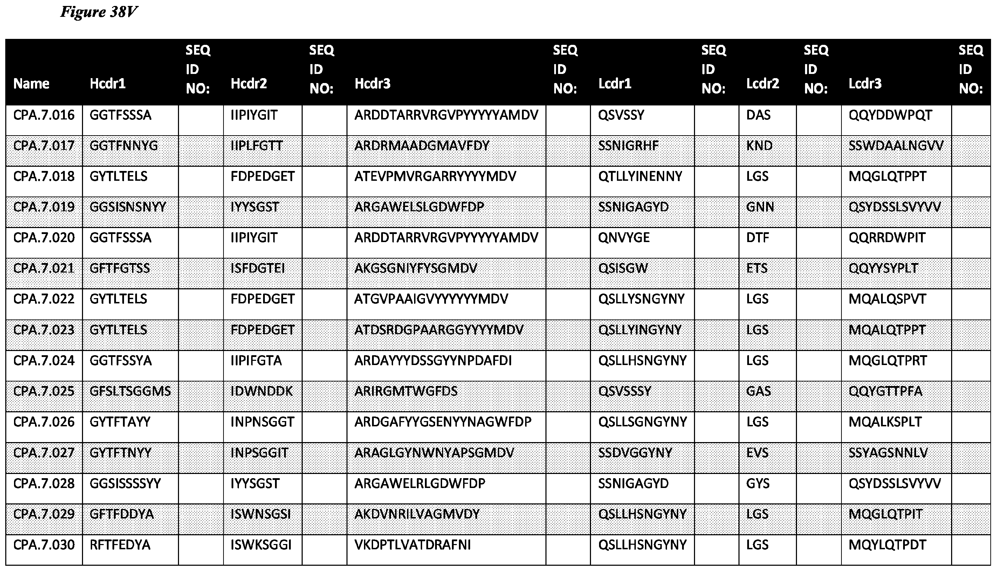

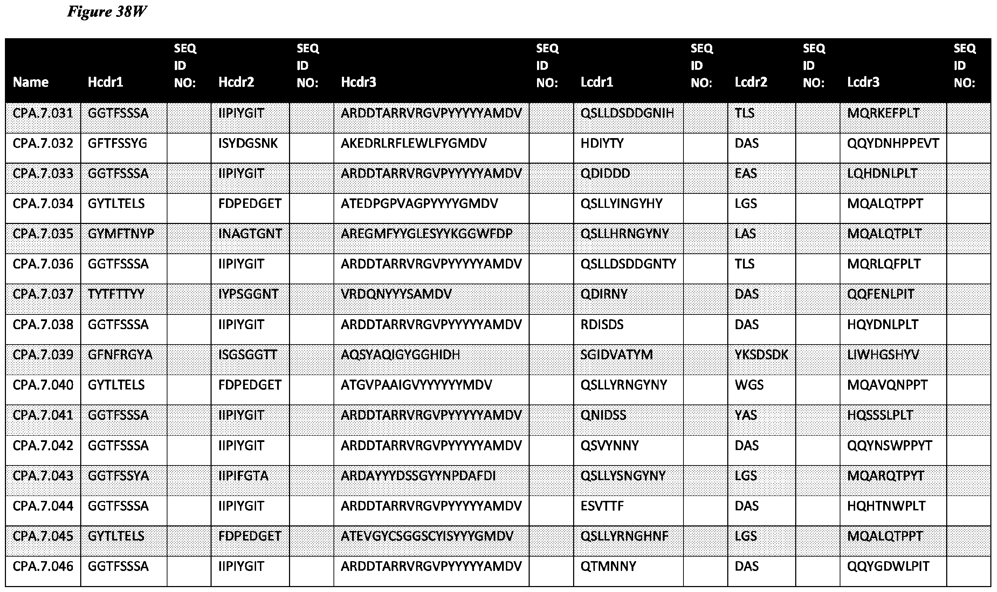

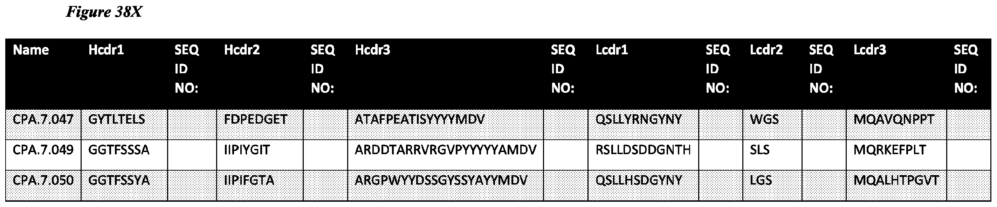

Figure 38A-38X provides additional anti-PVRIG antibodies for use described herein.

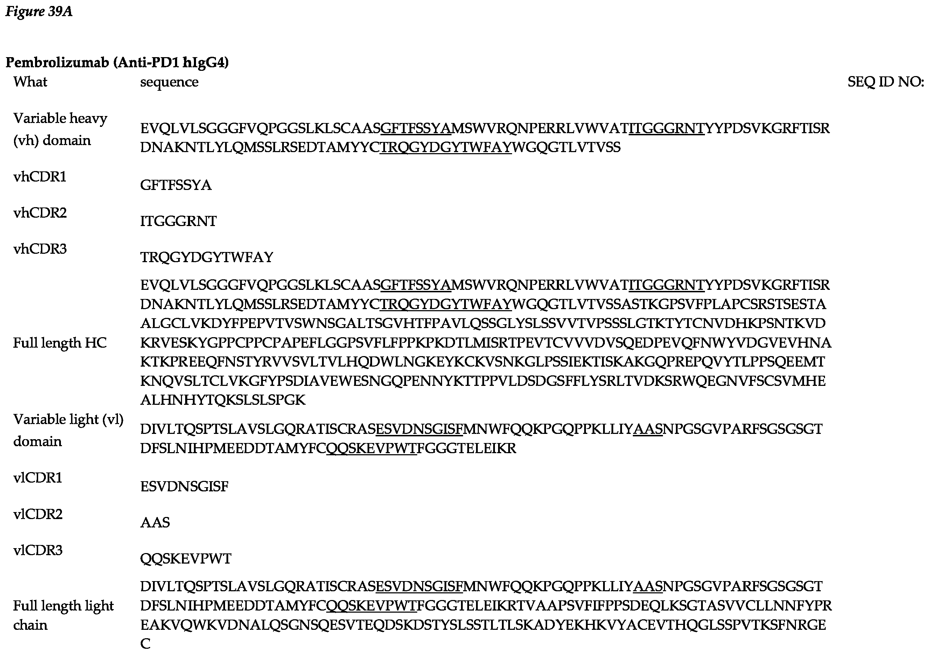

Figure 39A-39B depicts the sequences of exemplary anti-PD-1 antibodies.

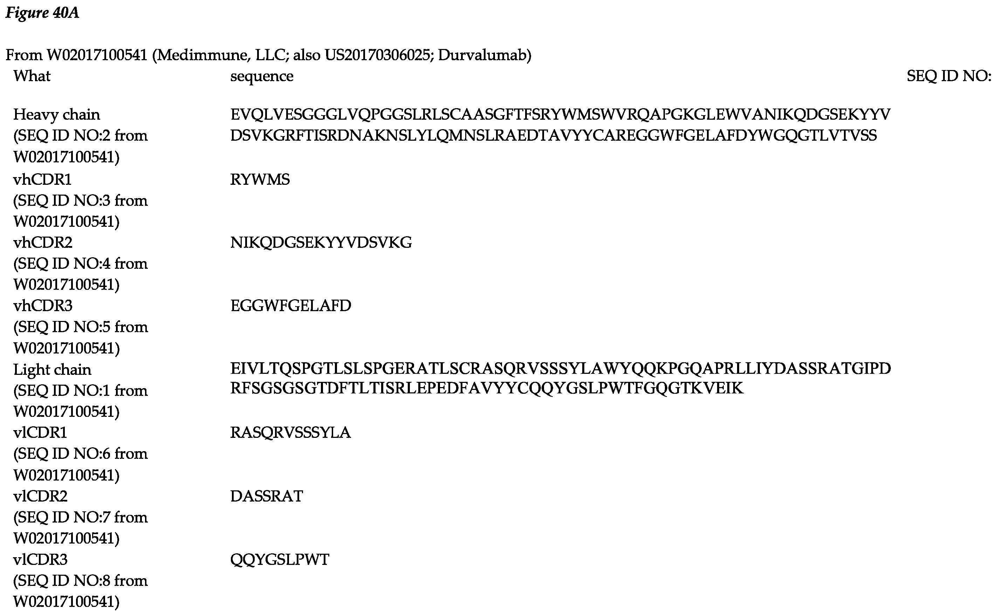

Figure 40A-40I depicts the sequences of exemplary anti-PD-L1 antibodies.

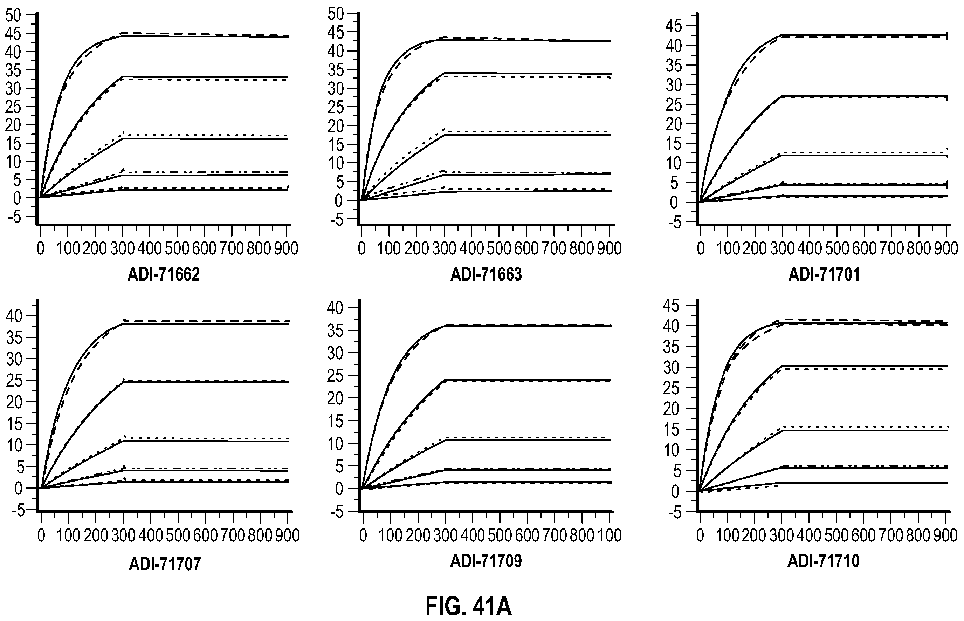

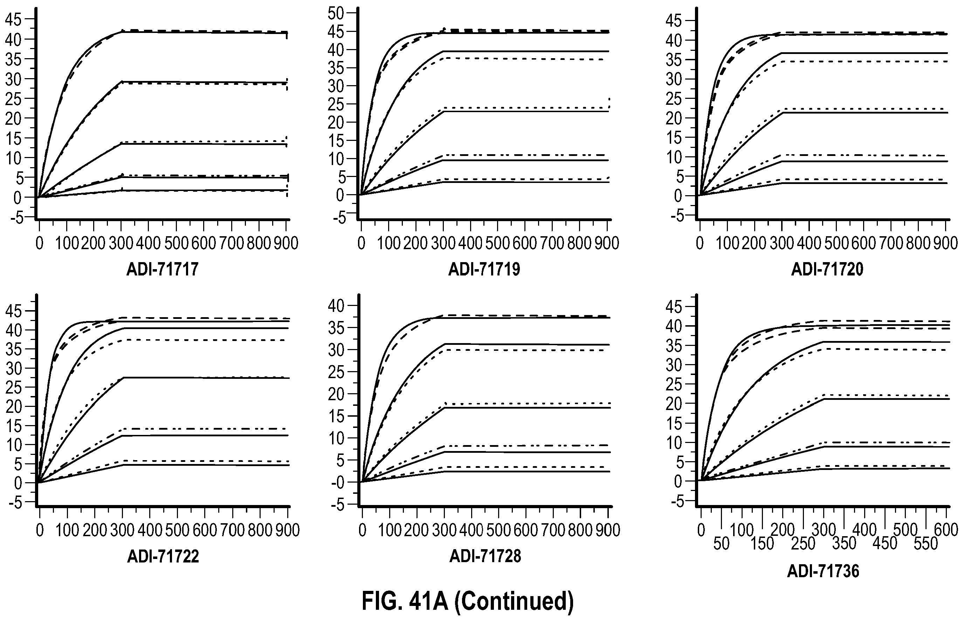

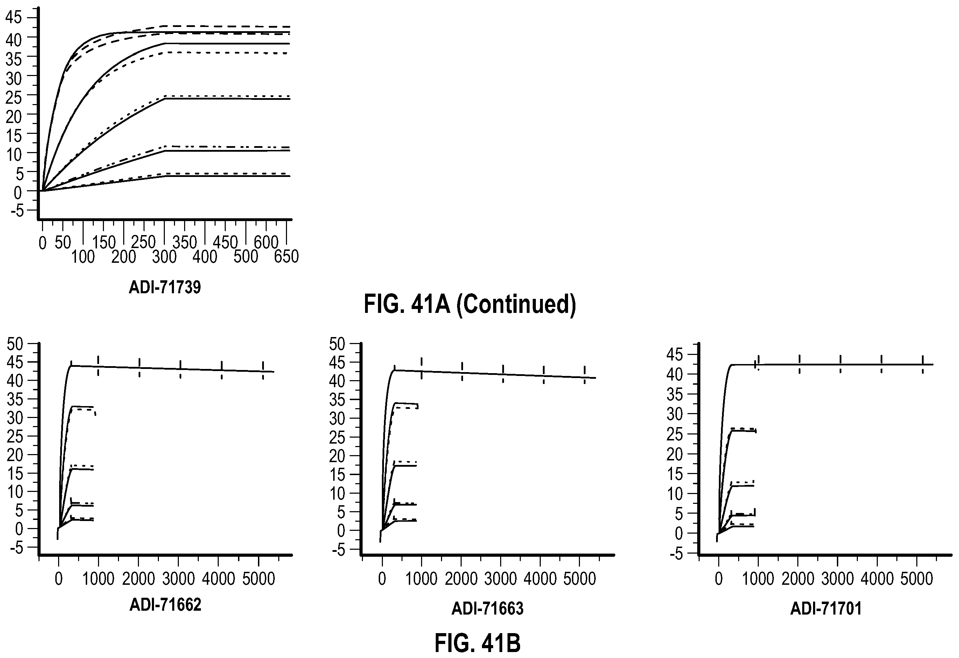

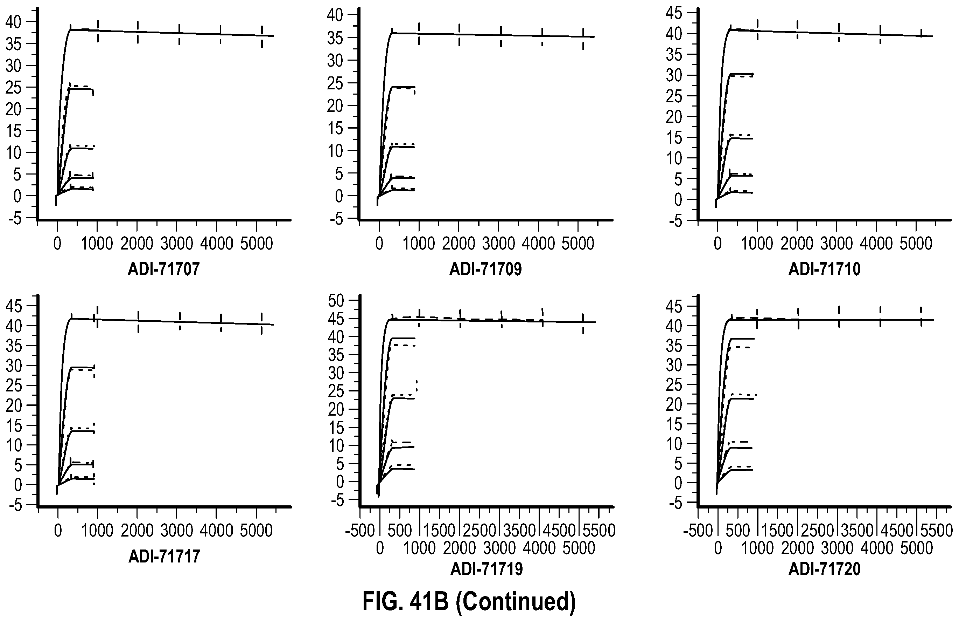

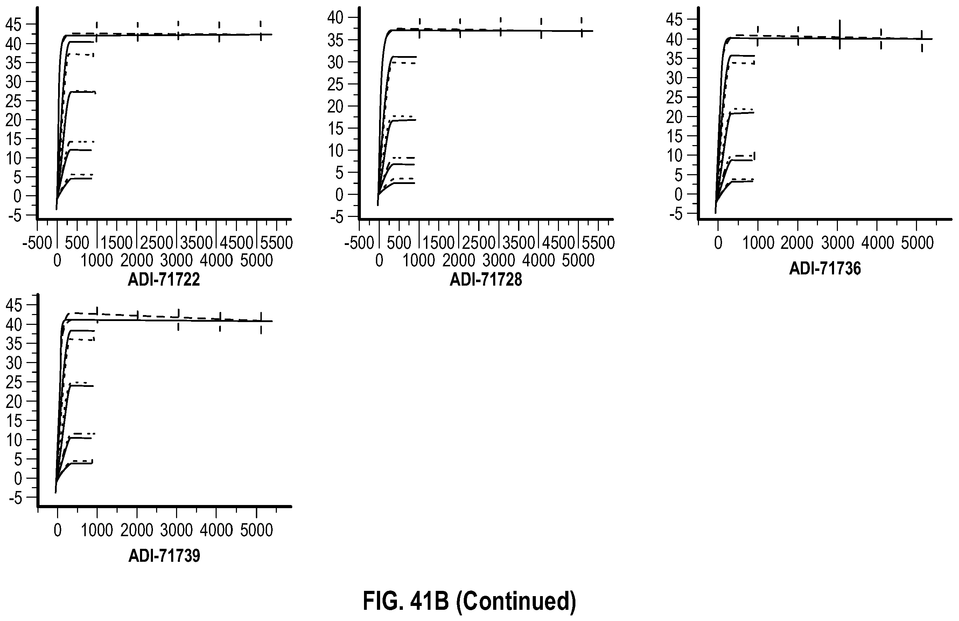

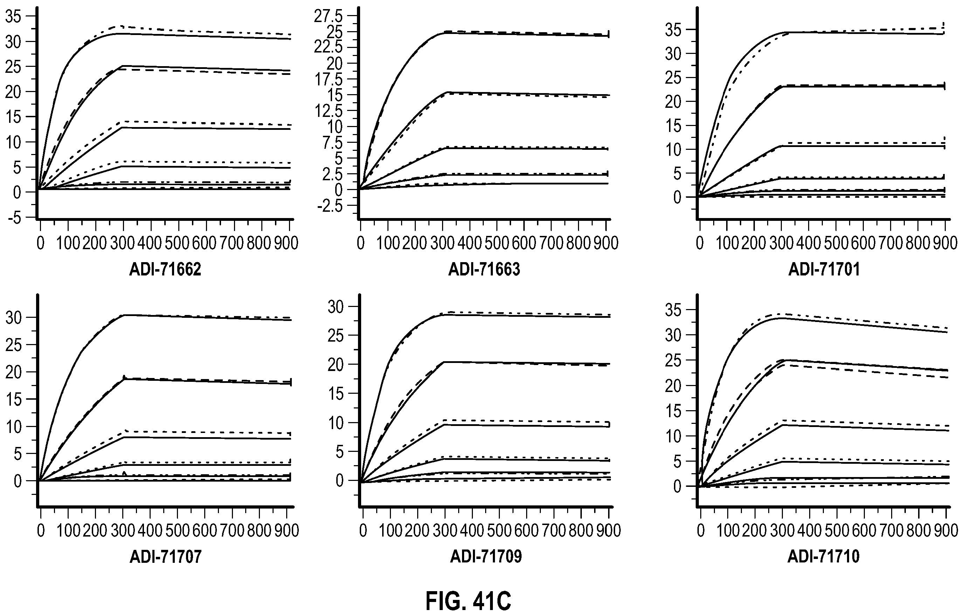

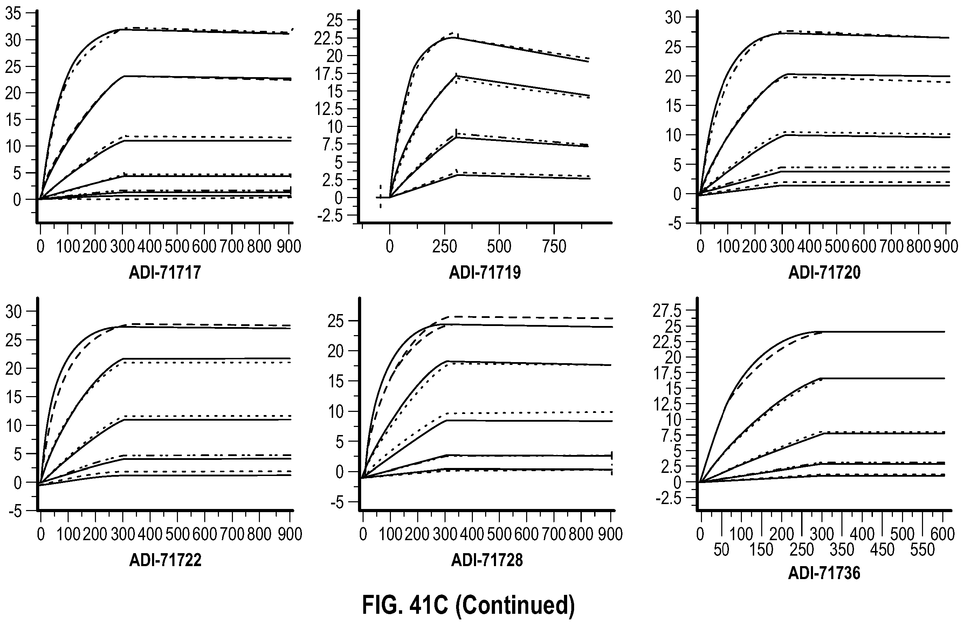

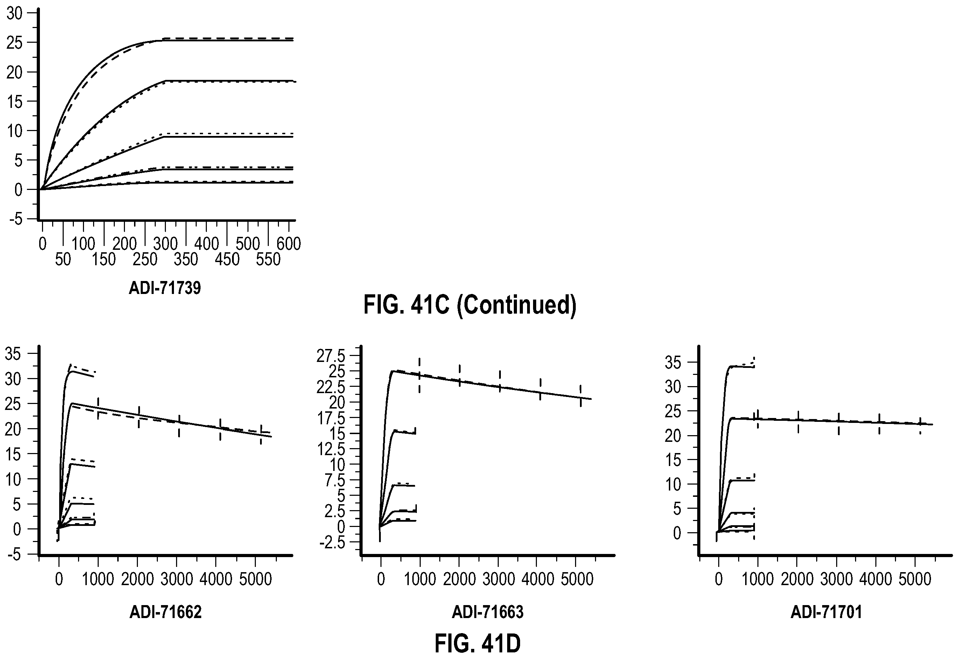

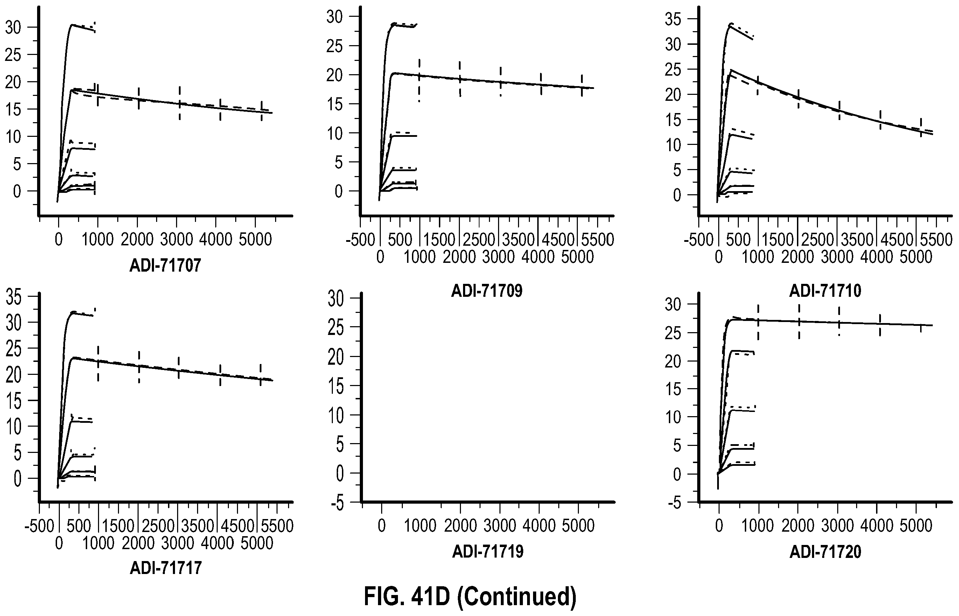

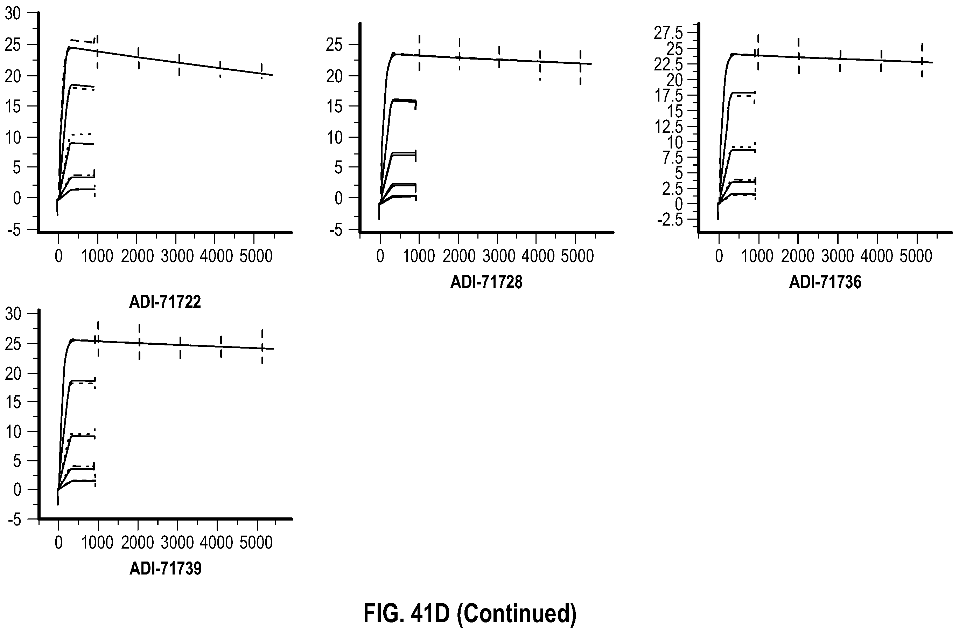

Figure 41A-41D depicts Biacore KD measurements, performed with biotinylated human/cyno IL18BP-Fc protein coated on the CM5 chip. Figure 41(A), (B): Biacore image of the anti-IL18BP Fab -human IL18BP interactions; 10min dissociation (41(A)), 85 min dissociation (41(B)). Figure 41(C), (D): Biacore image of the anti-IL18BP Fab -cyno IL18BP interactions, 10 min dissociation (41(C)), 85 min dissociation (41(D)).

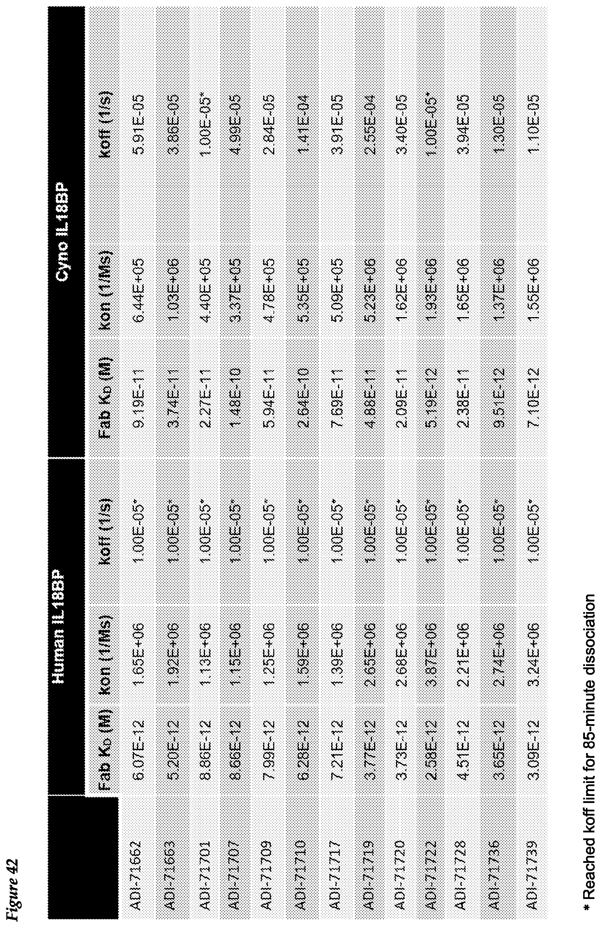

Figure 42 depicts a Table, showing KD values for human/cyno anti-IL18BP Fab -IL18BP interactions measured by Biacore.

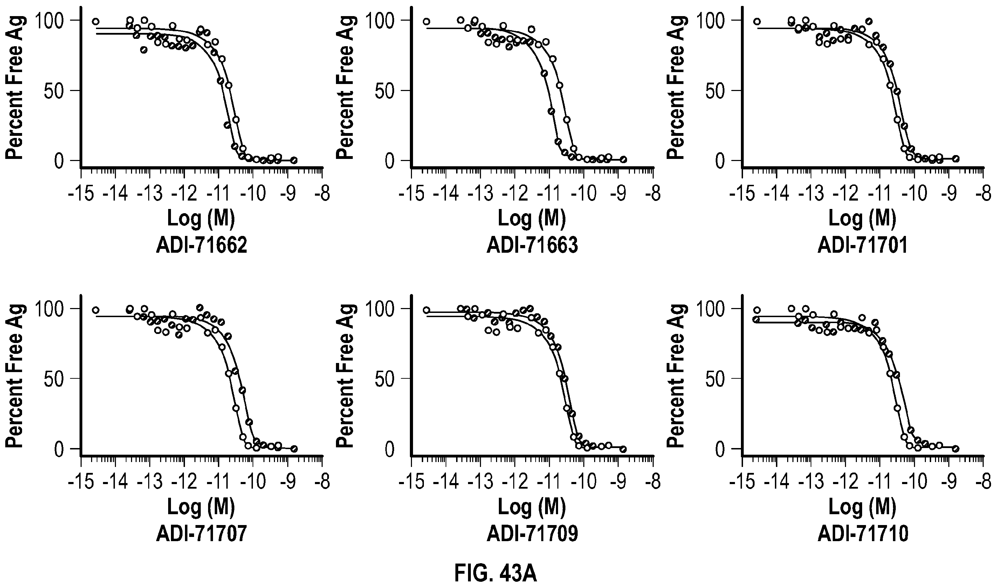

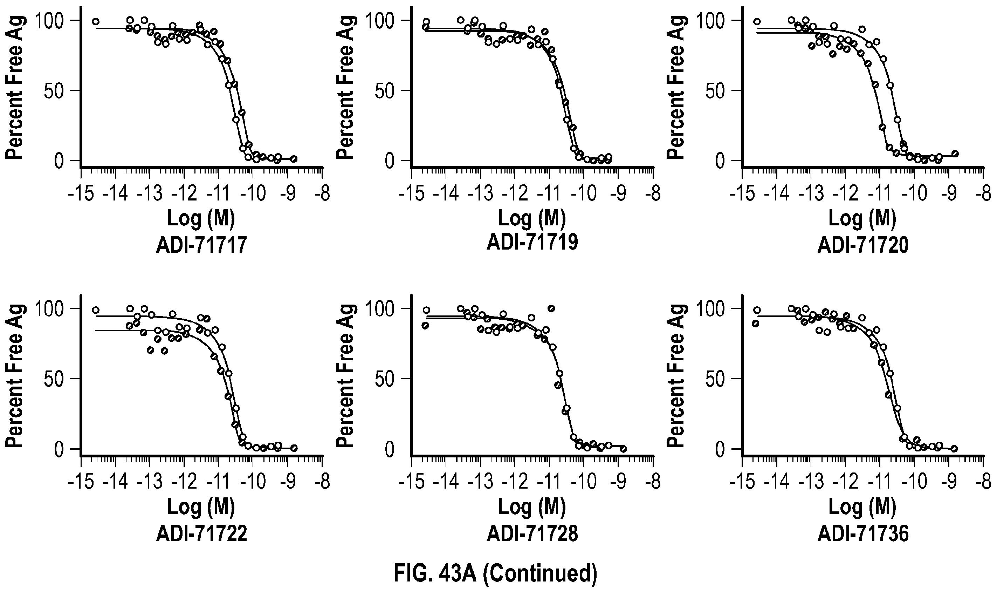

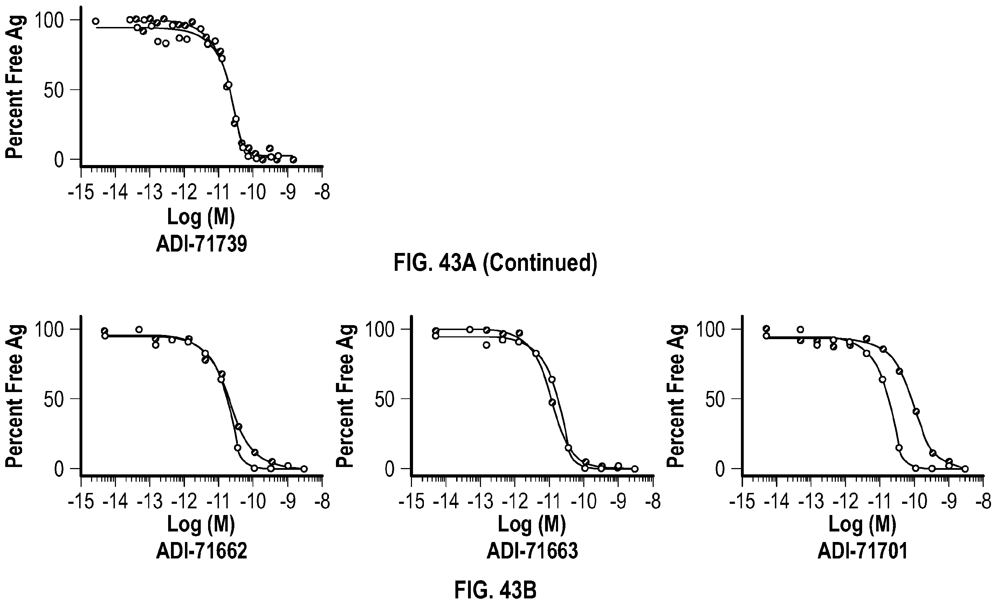

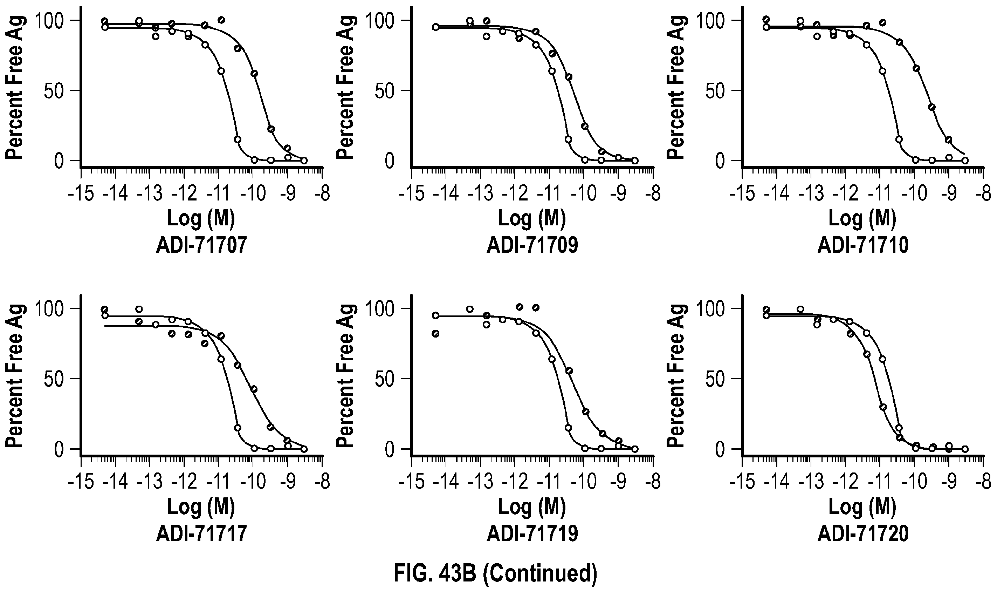

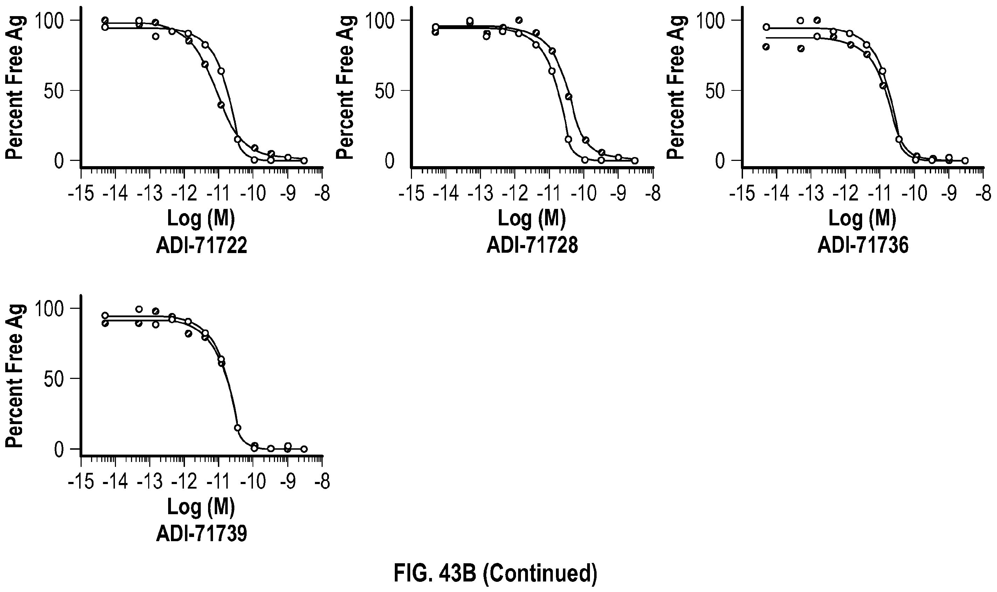

Figure 43A-43B presents the affinity of optimized IL18BP antibodies, accessed using MSD. Figure 43A shows an overlay of the Fab-IL18BP MSD Image (in Black) with the Human IL-18 - IL18BP MSD Image (in Green). Figure 43B shows an overlay of the Fab-IL18BP MSD Image (in Black) with the Cyno IL-18 - IL18BP MSD Image (in Green).

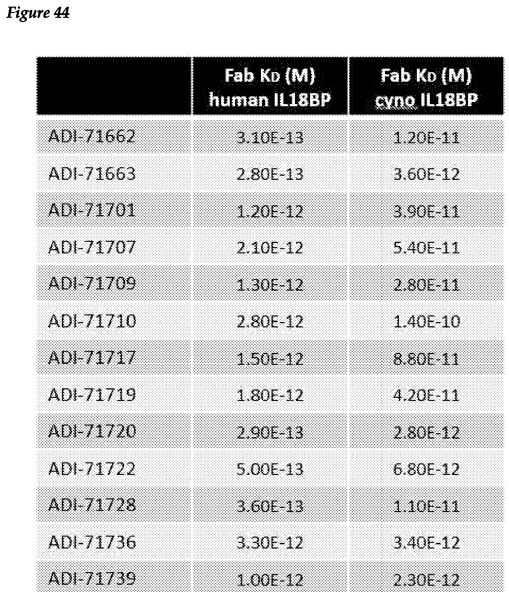

Figure 44 presents a Table, showing K_{D} values for human/cyno anti-IL18BP Fab -IL18BP interactions measured by MSD.

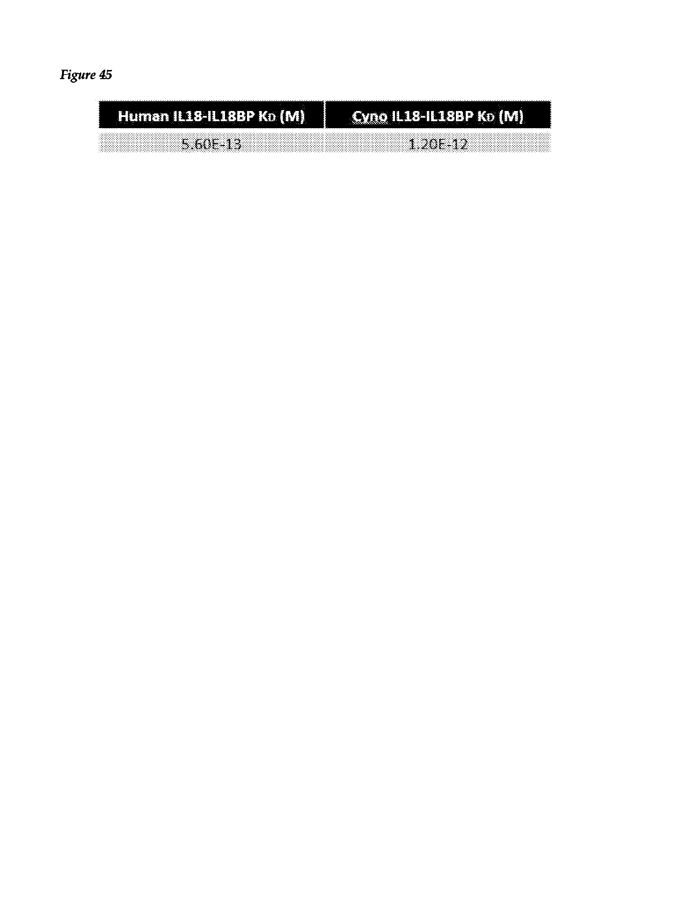

Figure 45 presents a Table, showing K_{D} values for human/cyno IL18-IL18BP interactions measured by MSD.

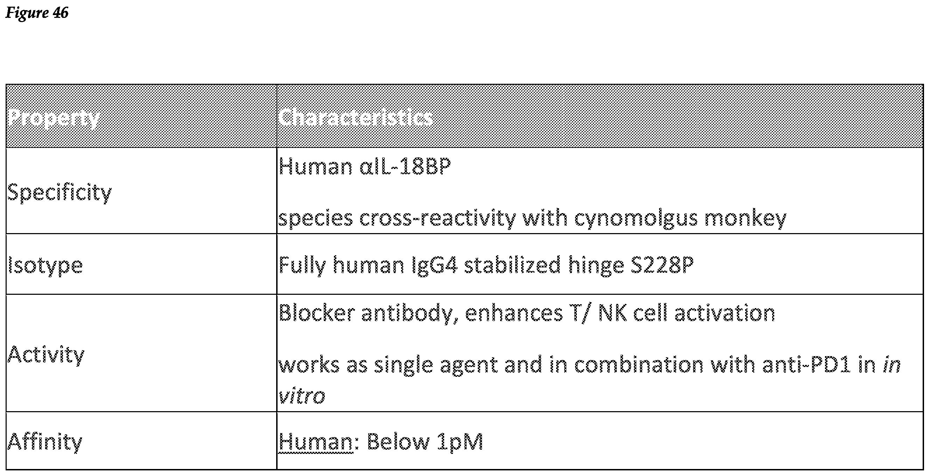

Figure 46 provide exemplary antibody characteristics for an αIL-18BP antibody (αIL-18BP Ab) of interest.

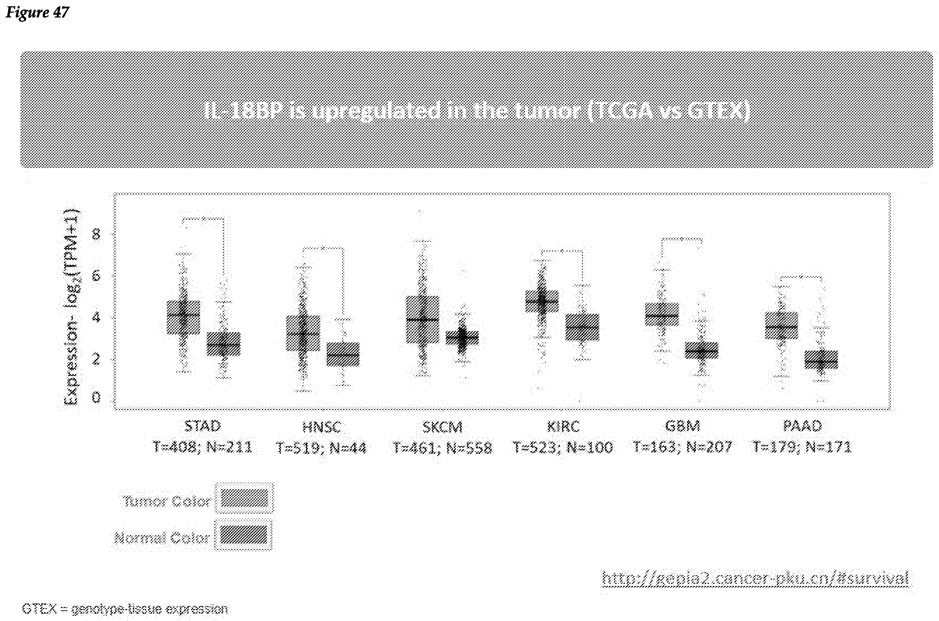

Figure 47 shows IL-18BP levels are elevated in human cancers. Expression of *IL18BP* transcripts in normal (green) or cancer (red) tissues from the TCGA database. GBM, glioblastoma multiforme; HSNC, head and neck squamous carcinoma; KIRC, kidney renal clear cell carcinoma; PAAD, pancreatic adenocarcinoma; SKCM, skin cutaneous melanoma; STAD, stomach adenocarcinoma (*P < 0.01).

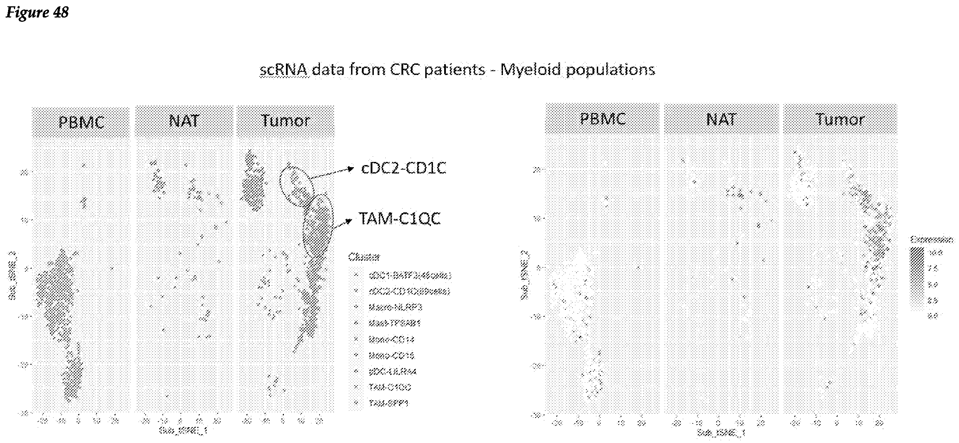

Figure 48 shows IL-18BP is expressed in suppressive myeloid populations in the TME suggesting resistance mechanism. Single-cell RNA analyses of tumor-infiltrating myeloid cells, including tumor associated macrophages (TAMs) and dendritic cells (DCs) in human Colorectal cancer (CRC) showing that IL-18BP is expressed in suppressive myeloid population in the tumor. This suggests a resistance mechanism to immune activation in the tumor microenvironment (TME). Left Panel: Myeloid cell population in the peripheral (PBMC), normal tumor (NAT) and in the tumor. Right Panel: IL18BP is mainly expressed in cDC2-CD1C and TAM-C1QC, suppressive myeloid populations suggestive that IL18BP could be a resistance mechanism to immune cell activation in the tumor.

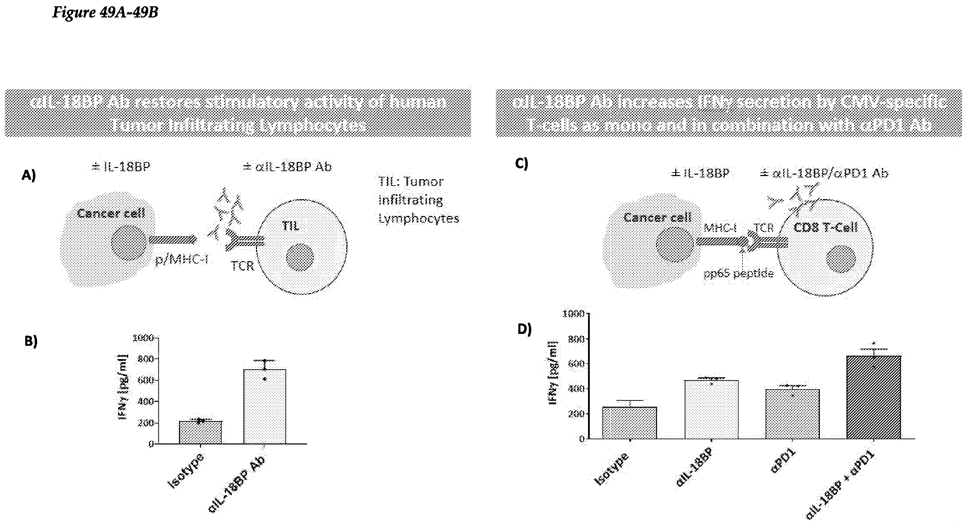

Figure 49A-49B provides that αIL-18BP Ab (ADI-71739) enhances stimulatory activity of human T cells. A) Schematic representation of assay setup; thawed tumor infiltrating lymphocytes (TILs), co-cultured with MEL624 cells in a 1:1 ratio, were treated for 30 minutes with rhIL-18 (R&D systems, 30 ng/ml) and rhIL-18BP (R&D systems, 1µg/ml), to allow the formation of IL-18-IL-18BP complex. 30 minutes post incubation, the cells were treated with ADI-71739 or isotype control (10ug/ml). B) shows that the anti-IL-18BP antibody was able to increase IFNγ secretion from TILs compared to isotype control. C) Schematic representation of assay setup; MEL-624 cells that overexpress PD-L1 were loaded with CMV pp65 peptide and seeded. The cells were cultured for 30 minutes with rhIL-18 (30 ng/ml) and rhIL-18BP (2µg/ml), to allow the formation of IL-18-IL-18BP complex. Then the cells were treated with ADI-71739, Pembrolizumab or isotype control (all antibodies were administered at same final concentration of 10µg/ml). 30 minutes post incubation with antibodies, CMV-reactive T-cells were added to the culture. D) ADI-71739 as mono was able to increase IFNγ secretion from CMV-reactive T-cells, and in a more potent manner when combined with Pembrolizumab.

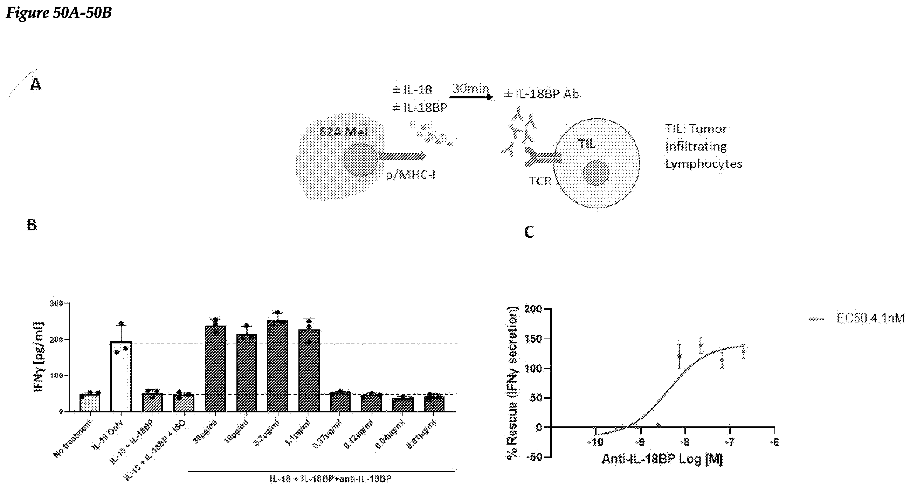

Figure 50A-50B shows anti-IL-18BP antibody fully restored IL-18 activity in MEL624:TIL assay. Figure 50A: Schematic representation of assay setup; thawed tumor infiltrating lymphocytes (TILs), co-cultured with MEL624 cells in a 1:1 ratio, were treated for 30 minutes with rhIL-18 (30 ng/ml) and rhIL-18BP (1 µg/ml), to allow the formation of IL-18-IL-18BP complex. 30 minutes post incubation, the cells were treated with a dose titration of anti-IL-18BP antibody (ADI-71722), (30 µg/ml to 0.01 µg/ml; dilution factor of 1:3) or isotype control (30 µg/ml). Figure 50B: shows that the anti-IL-18BP antibody was able to fully restore IFNγ secretion in a dose-dependent manner. The isotype control was not able to restore IL-18 activity. Figure 50C: shows the dose response curve of % rescue by anti-IL-18BP antibody and calculated EC50. Representative data is from one donor. Rescue by anti-IL-18BP Ab is calculated as: [(IL-18+IL-18BP+ anti-IL-18BP Ab)- (IL-18+ IL-18BP+ Isotype)]/ [(IL-18)- (IL-18+ IL-18BP+ Isotype)].

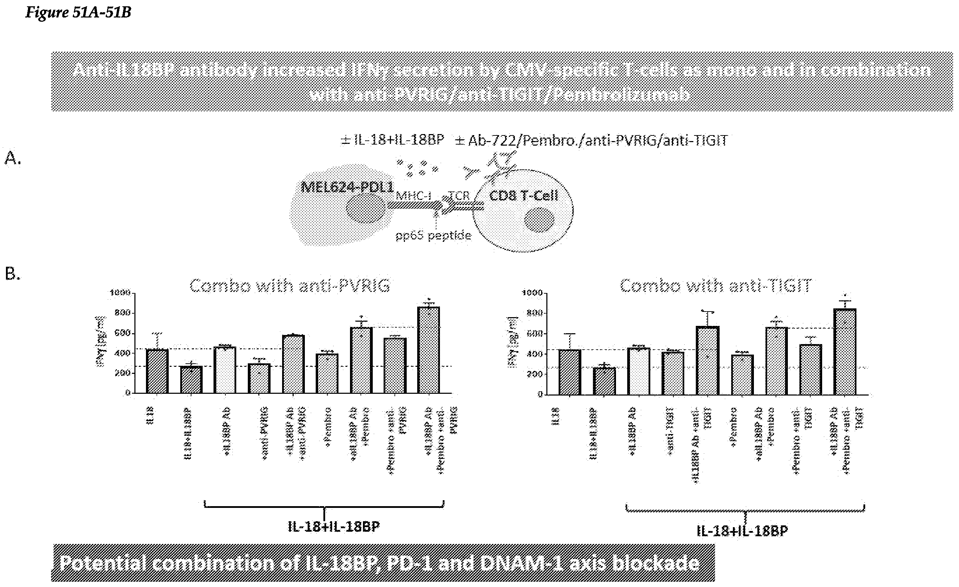

Figure 51A-51B shows anti-hIL-18BP antibody enhances activity of PD-1 and DNAM-1 axis blockade in an in-vitro CMV recall assay. Anti-IL-18BP antibody increased IFNg secretion by CMV-reactive T-cells as mono and in combination with aPVRIG/aTIGIT/Pembrolizumab. A) Schematic representation of assay setup; MEL-624 cells that overexpress PD-L1 were loaded with CMV pp65 peptide and seeded. The cells were cultured for 30 minutes with rhIL-18 (30 ng/ml) and rhIL-18BP (2µg/ml), to allow the formation of IL-18-IL-18BP complex. Then the cells were treated with anti-IL-18BP antibody (ADI-71722), anti-PVRIG, anti-TIGIT, Pembrolizumab or isotype control (all antibodies were administered at same final concentration of 10µg/ml). 30 minutes post incubation with antibodies, CMV-reactive T-cells were added to the culture. B) ADI-71722 increased IFNγ secretion by CMV-reactive T-cells as mono and in combination with anti-PVRIG/anti-TIGIT/Pembrolizumab. Left figure: Anti-IL-18BP antibody as mono was able to fully restore IFNγ secretion, and in a more potent manner when combined with Pembrolizumab/anti-PVRIG. Right figure: ADI-71722 as mono was able to fully restore IFNγ secretion, and in a more potent manner when combined with Pembrolizumab/anti-TIGIT.

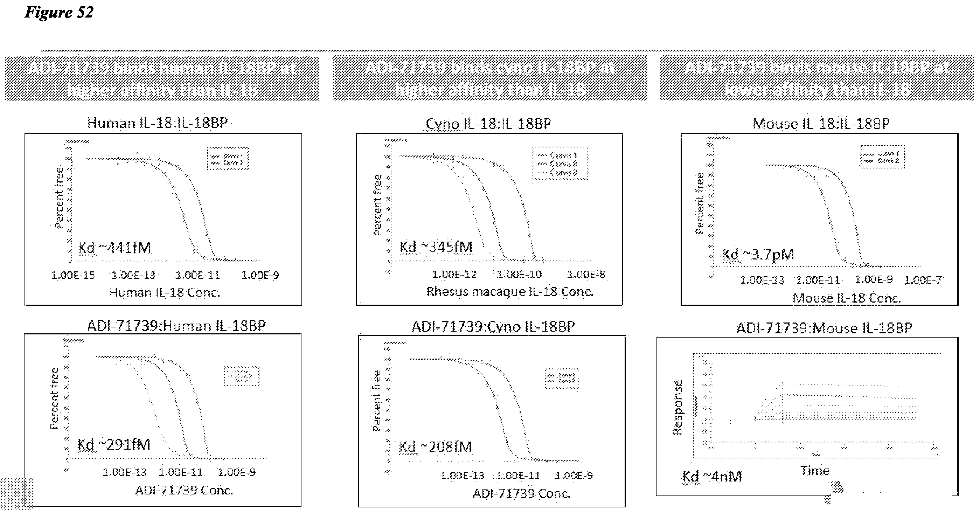

Figure 52 provides data showing ADI-71739 binds human and cyno IL-18BP at high affinity and mouse Il-18bp at low affinity. ADI-71739 binds human and cyno IL-18BP at high affinity and mouse Il-18bp at low affinity: Upper panel (from left to right): human IL18-IL18BP interaction, cyno IL18-IL18BP interaction and mouse IL18-IL18BP interaction measurements in KinExA, final KD are 441 fM, 345 fM and 3.7pM respectively. Lower panel (from left to right): ADI-71739- human IL18BP interaction, ADI-71739 - cyno IL18BP interaction measurement in KinExA and ADI-71739 - mouse IL18BP interaction measurement by Biacore. Final KD are 291fM, 209 fM and 4nM respectively. For each run in KinExA, two or three curves with different column binding protein (CBP) concentrations were run and analyzed using n-curve analysis to determine the Kd.

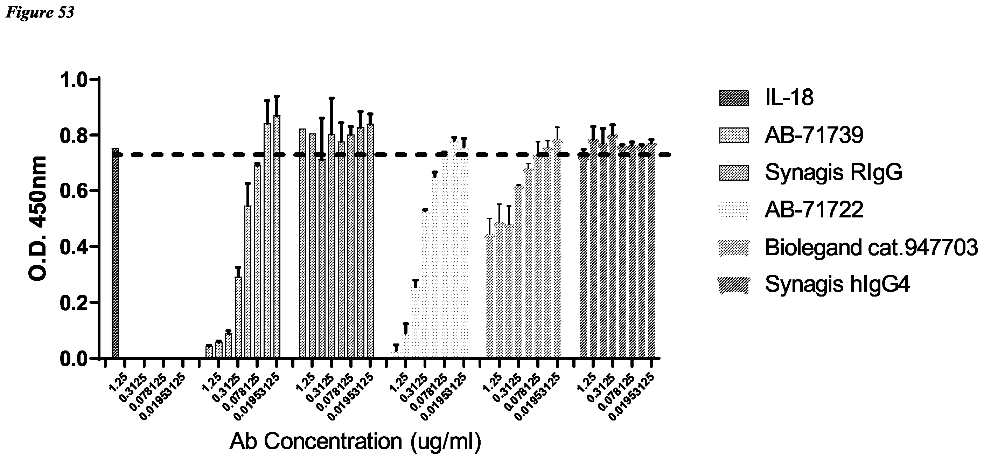

Figure 53 shows blocking effect of anti IL18BP Abs on the binding of human IL18BP to human IL-18. Blocking effect of anti-IL18BP Abs was tested by ELISA, using 1ng/ml human IL-18 protein.

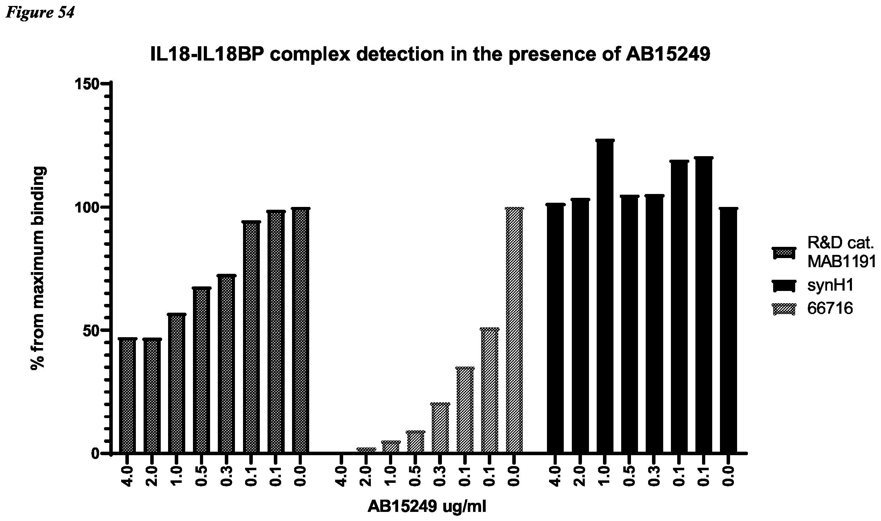

Figure 54 shows competition ELISA using complex of soluble IL18-IL18BP and anti IL18BP Abs. Blocking of IL18-IL18BP complex formation was tested by ELISA, MAB1191 shows reduced blocking activity compared to 66716 Ab.

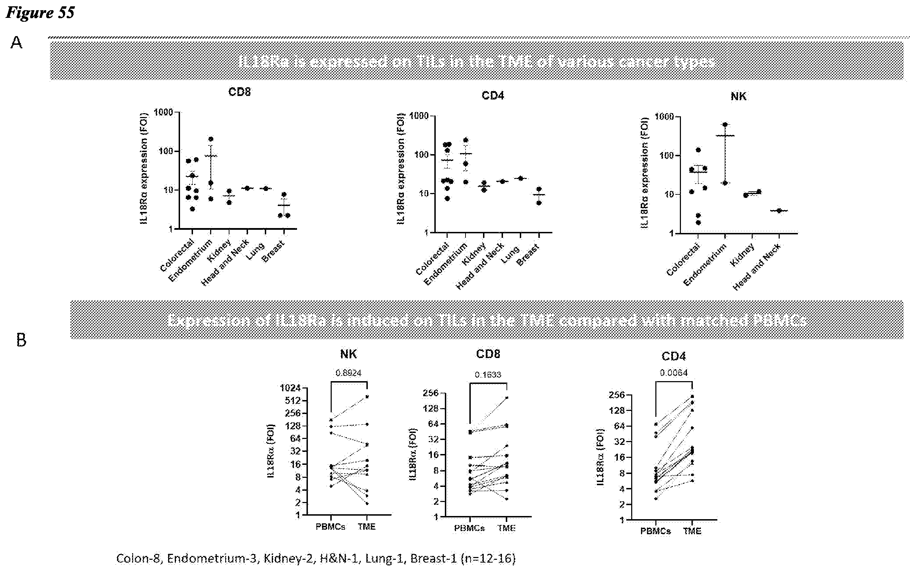

Figure 55 shows IL18Ra is expressed on TILs subsets in the TME and its expression is induced on TILs compared to periphery. IL18Ra is expressed on TILs in the TME and its expression is induced on CD4 TILs compared with periphery. A) Expression of IL18Ra on CD8+ and CD4+ and NK TILs from dissociated human tumors of various cancer types is shown. Each dot represents a distinct tumor from an individual patient. Fold expression value was calculated by dividing the MFI of a target by the MFI of the relevant isotype control. (FOI). Average and SEM is shown by the ticks. B) Expression of IL18Ra on CD4+ and CD8+ T and NK cells from donor-matched PBMCs and TME. Statistical analysis was preformed using paired t test (two tailed), P < 0.05; **p=0.0064

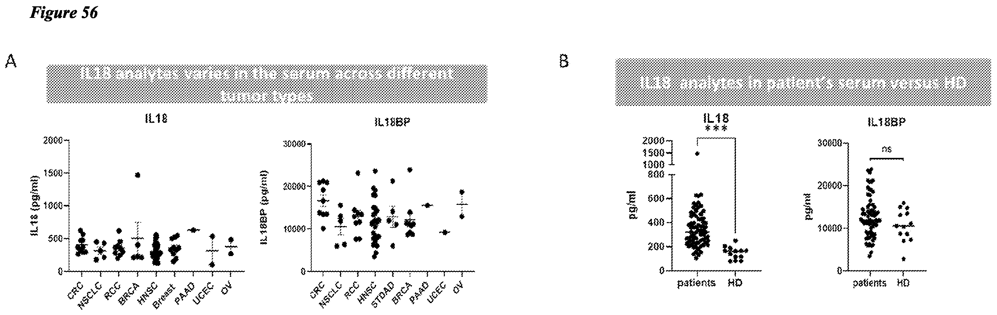

Figure 56 shows IL18 levels in serum of cancer patients is increased compared with levels in HD serum. A) Levels of IL18 analytes (IL18 and IL18BP) in patient's serum across indications. B) Dot plot representing IL18 analytes in serum samples from an individual patient or HD. Statistical analysis was preformed using t test (two tailed), *P* ≤ 0.0005***

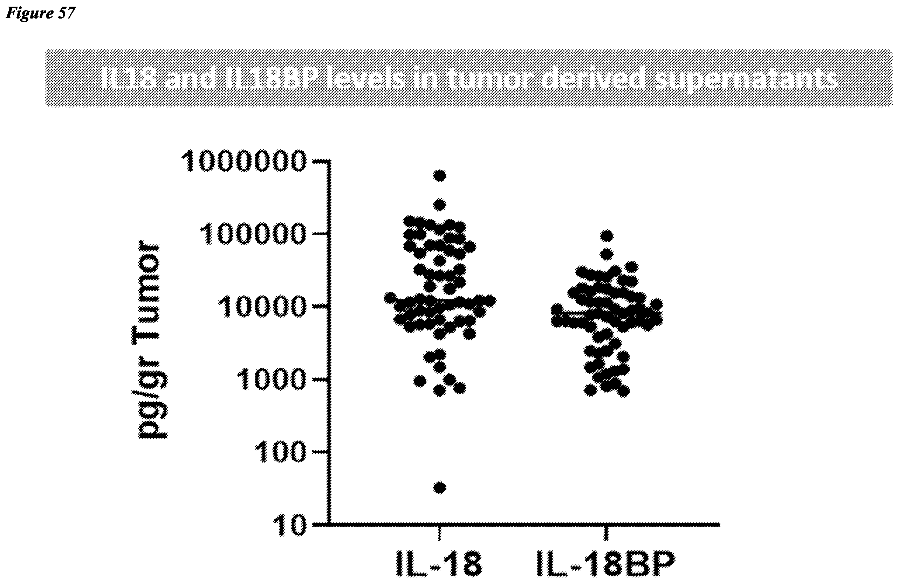

Figure 57 shows IL18 analytes (IL18 and IL18BP) levels in tumor derived supernatants (TDS) across indications. Dot plot representing IL 18 analytes in TDS samples. Each dot represents an individual patient's sample.

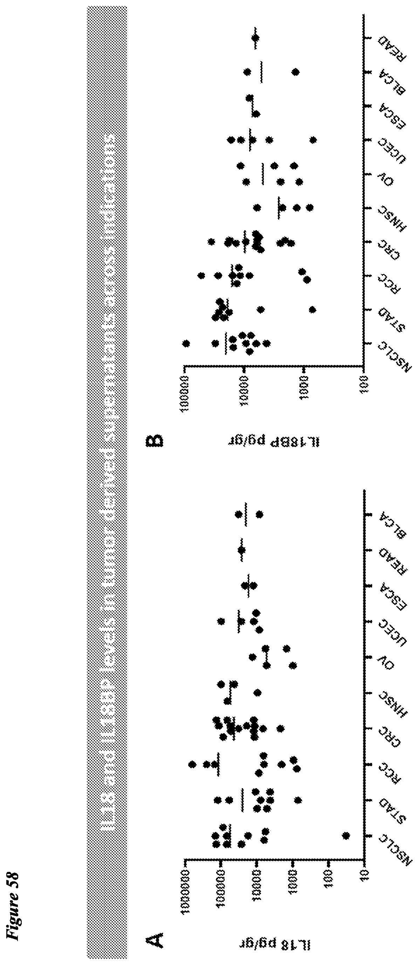

Figure 58A-58B shows levels of IL18 (A) and IL18BP (B) in patient's tumor derived supernatant (TDS) across indications. Mean levels are represented by black lines.

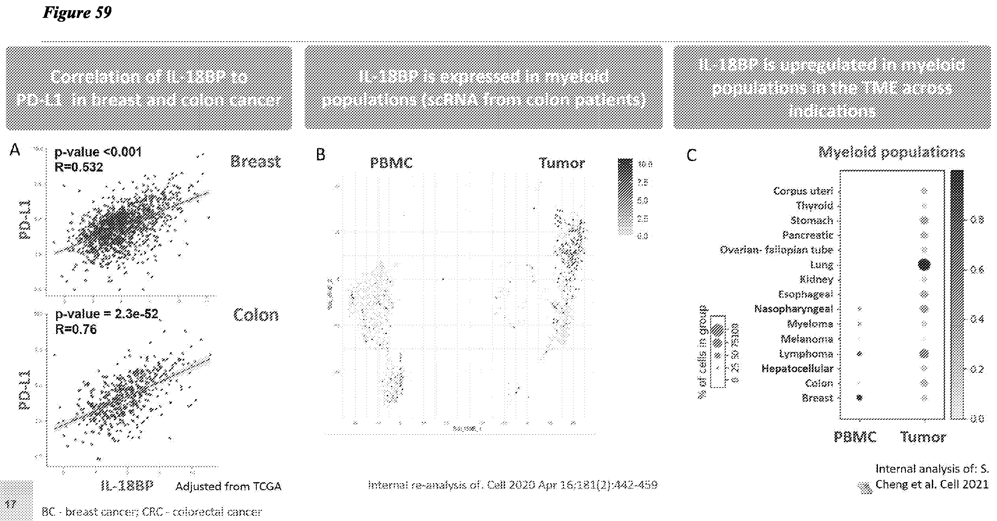

Figure 59A-59C shows IL-18BP is expressed in suppressive myeloid populations in the TME suggesting resistance mechanism. IL-18BP is Expressed in Suppressive Myeloid Populations and correlate to PD-L1 in the TME Suggesting Resistance Mechanism. A) IL-18BP correlates with PD-L1 at RNA level (TCGA) in colon and breast cancers suggesting a resistance mechanism to immune activation in the tumor microenvironment (TME). B) Single-cell RNA analyses of tumor-infiltrating myeloid cells, including tumor associated macrophages (TAMs) and dendritic cells (DCs) in colon cancer patients showing that IL-18BP is up-regulated in myeloid population in the TME compared to the periphery (PBMCs), suggesting a resistance mechanism to immune activation in the TME. C) Single-cell RNA analyses of tumor-infiltrating myeloid cells, including tumor associated macrophages (TAMs) and dendritic cells (DCs) across indications showing that IL-18BP is up-regulated in myeloid population in the TME compared to the periphery (PBMCs), suggesting a resistance mechanism to immune activation in the TME

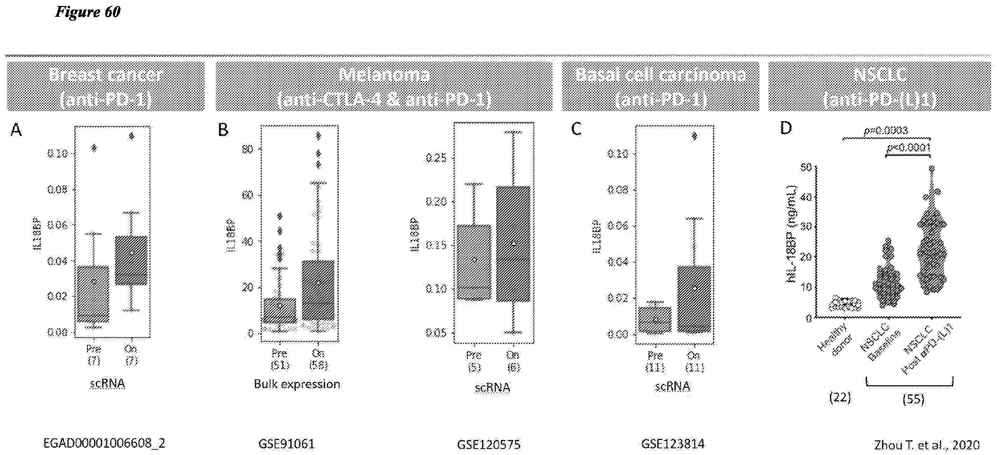

Figure 60A-60D shows IL-18BP is upregulated following immune checkpoint blockade (ICB) treatment. A-C) IL-18BP is upregulated (RNA level) following ICB treatment IL-18BP levels are upregulated in the tumor microenvironment (RNA) following treatment with anti-PD-1 (breast and basal cell carcinoma) or anti-PD-1 plus anti CTLA-4 (melanoma) suggesting a potential resistance mechanism. D) IL-18BP is elevated in NSCLC patient serum post aPD-(L)1 treatment Quantification of plasma IL-18BP protein level by ELISA for healthy donors (n = 22) and patients with NSCLC (n = 52) at baseline before treatment and after receiving treatment with anti-PD-(L)1 (n = 52).

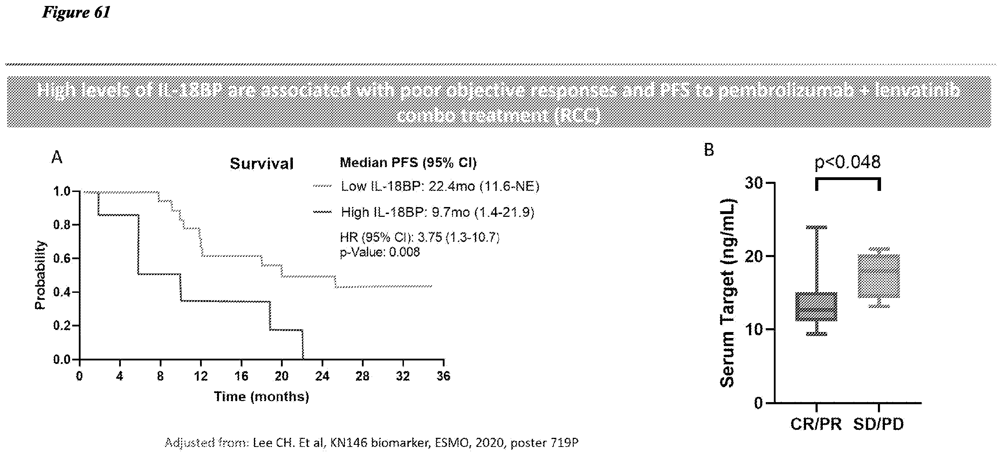

Figure 61A-61B shows IL-18BP baseline serum levels may be associated with poor response to anti-PD-1. A supportive data for the role of IL-18BP as a soluble ICP and a potential resistance mechanism to PD1 blockage in Renal Cell Carcinoma patients receiving Pembrolizumab plus Lenvatinib. A) High IL-18BP in patient serum pre-treated with Pembrolizumab plus Lenvatinib is associated with shorter progression free survival (PFS). B) High IL-18BP in patient serum is pre-treated with Pembrolizumab plus Lenvatinib associated with stable or progressive disease (SD/PD).

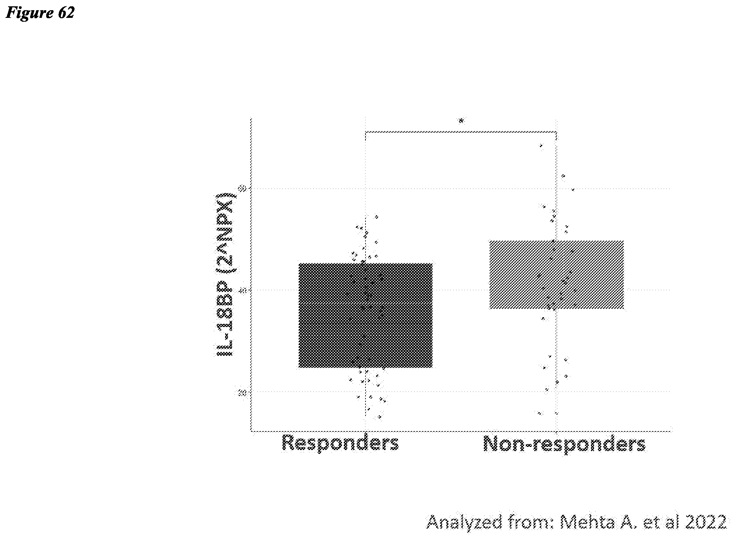

Figure 62 shows IL-18BP baseline serum levels may be associated with poor response to anti-PD-1. A supportive data for the role of IL-18BP as a soluble ICP and a potential resistance mechanism to PD1 blockage in melanoma cancer patients receiving anti PD-1 treatment. High IL-18BP in serum of melanoma cancer patients pre-treated with anti PD-1 is associated with poor response. Raw Olink data (NPX format) Student's T-test was performed for IL 18BP protein after intensity normalization for Target products.

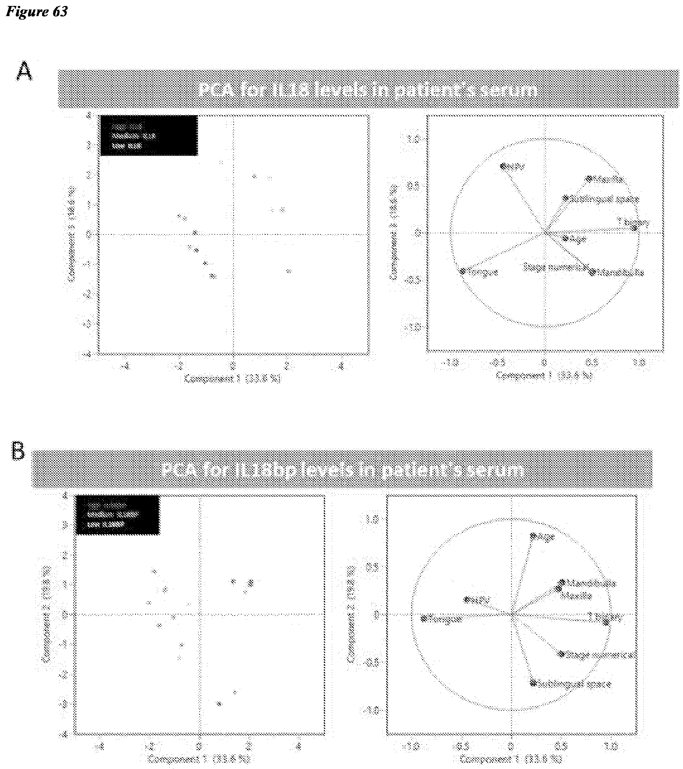

Figure 63A-63B shows Principal Component Analysis (PCA) of IL-18 and IL-18BP levels in serum of Head & Neck cancer. PCA shows that mainly tumor's sites separate between samples with high levels of IL-18 Vs. low levels. A-B. Location of tumor in tongue correlates with high levels of IL-18 and lower levels of IL18BP compared with other sites.

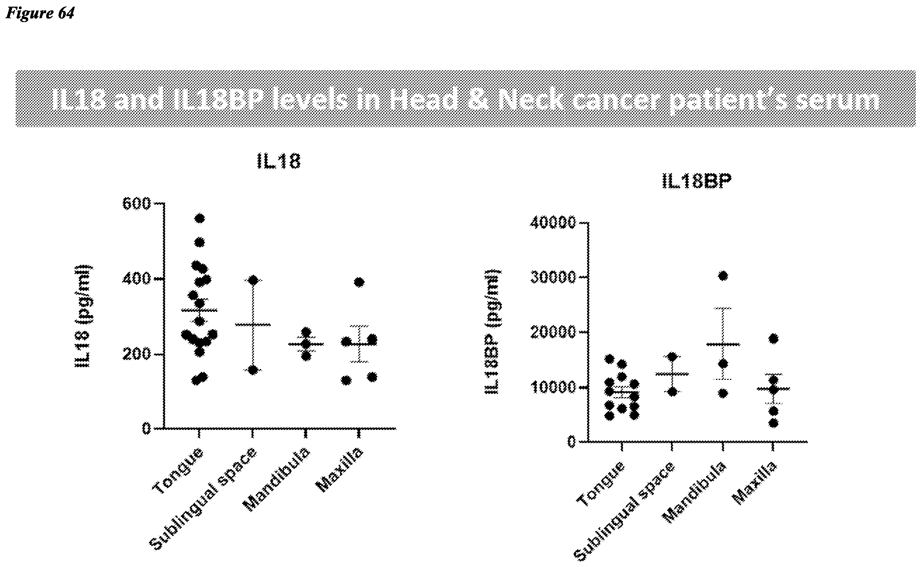

Figure 64 shows IL-18 and IL-18BP levels (dotplots) in Head & Neck patient's serum in different tumor's sites. Higher levels of IL-18 in Head & Neck patient's serum are shown in tongue.

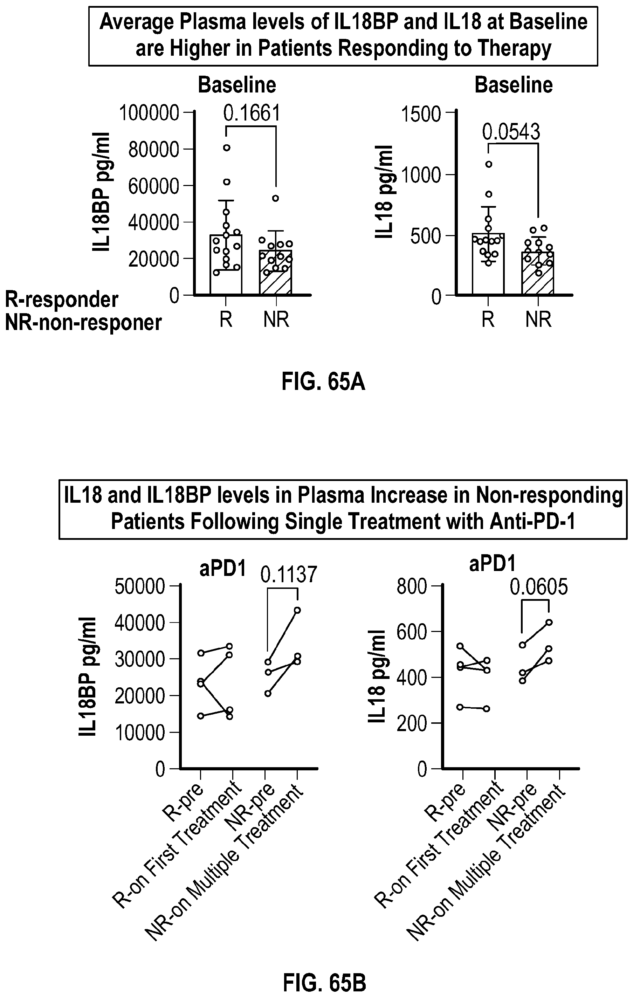

Figure 65A-65C shows IL18 and IL18BP plasma levels in NSCLC patients are increased following anti-PD-1 monotherapy or anti-PD-1+chemotherapy combination. Average plasma levels of IL18 and IL18BP are higher in responder patients at baseline and increase in NR patients treated with anti-PD1. A) IL18 and IL18BP levels in plasma of R/NR NSCLC patients at baseline. B) IL18 and IL18BP levels in plasma of individual NSCLC patients (R/NR) at baseline and following single anti-PD-1 treatment. C) IL18 and IL18BP levels in plasma of individual NSCLC patients (R/NR) at baseline and following single anti PD1 treatment or following combined treatment of chemotherapy+anti-PD-1. D) Percentage of change from baseline of IL18 and IL18BP in R/NR NSCLC patients following single anti-PD-1 treatment or chemotherapy combined with anti-PD-1. P values in A-C graphs were obtained following paired T test.

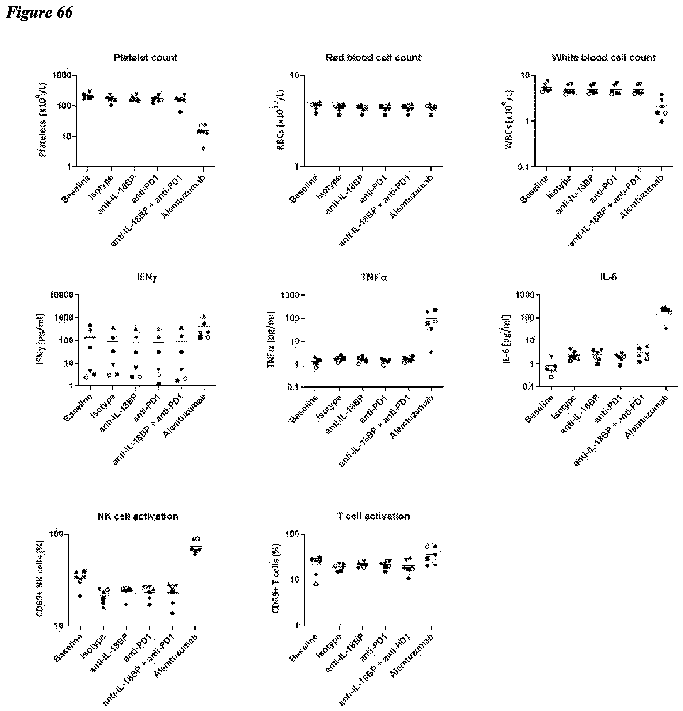

Figure 66 shows the whole blood assay data. Anti-IL-18BP antibody Ab-71709, as mono or in combination with Nivolumab, did not show signs of systemic immune activation in ID.Flow, an ex vivo system that mimics the human blood circulation. Fresh whole blood was taken from six healthy volunteers and immediately transferred to a whole blood loop system. The test items were administered, and the blood was set to circulate at 37°C to prevent clotting. Blood samples collected at the 24hr time point were analyzed for hematology and flow cytometry parameters and then processed to plasma for cytokine analysis. The anti-CD52 antibody Alemtuzumab was included as a reference antibody with manageable cytokine release in the clinic. As opposed to Alemtuzumab, according to the various readouts employed, the anti-IL-18BP antibody did not induce any signs of systemic immune activation, as mono or in combination with the anti-PD1 antibody Nivolumab.

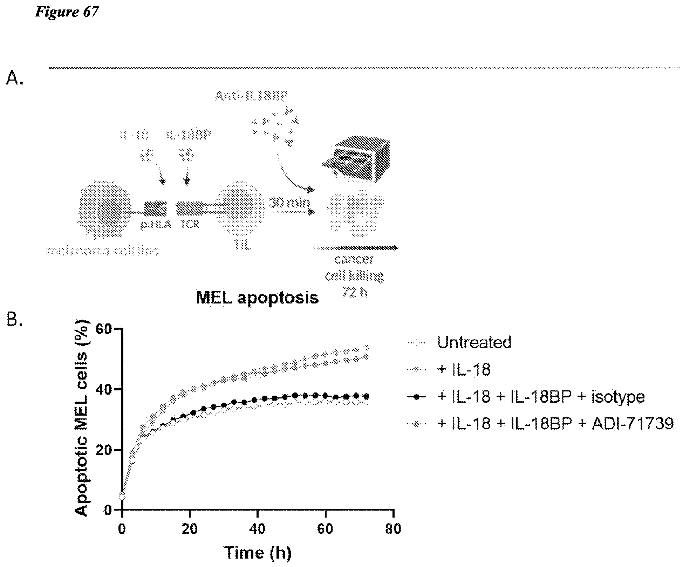

Figure 67A-67B shows in vitro studies testing the effects of ADI-71739 on killing of melanoma cells by human TILs. Anti-IL18-BP antibody ADI-71739 increased killing of melanoma cells by tumor infiltrating lymphocytes. A) Schematic representation of assay setup. MEL624 cells were co-cultured with human TILs that were previously enriched for MART1 or gp100 peptide-specific clones. rhIL-18 (R&D systems, 50 ng/ml) and rhIL-18BP (R&D systems, 1µg/ml) were added to the co-culture for 30 minutes to allow the formation of IL-18:IL-18BP complex prior to treatment with 10µg/ml ADI-71739 or isotype control. The co-culture was monitored for 72 hours using an IncuCyte live cell imaging instrument. B) Addition of IL-18 (grey) enhanced tumor cell killing as indicated by lower confluence (left) and increased apoptosis (right) over time of the MEL624 cells. In the presence of the isotype control antibody (black), IL-18BP abrogated the effects of IL-18, while the anti-IL-18BP antibody (turquoise) was able to completely restore these effects.

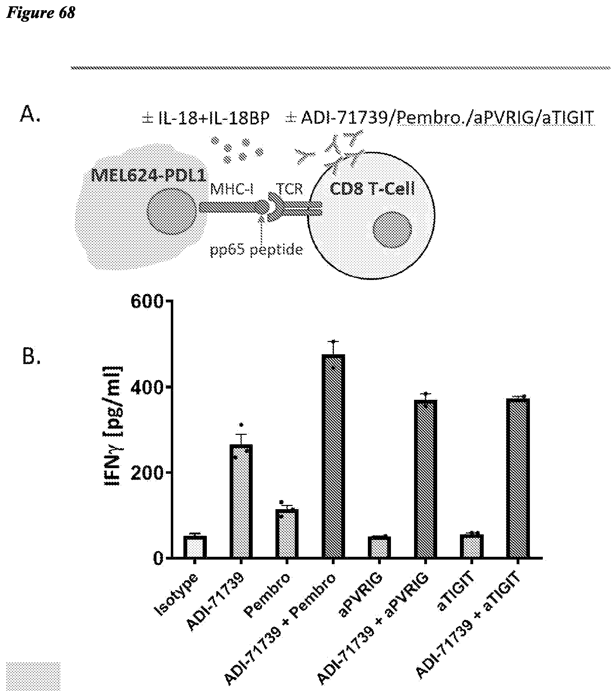

Figure 68A-68B shows in vitro studies testing the effects of combination of ADI-71739 with other checkpoint blocking antibodies. Anti-IL18-BP antibody ADI-71739 increased IFNg secretion by CMV-specific T cells as mono and in combination with aPVRIG/aTIGIT/Pembrolizumab. A) Schematic representation of assay setup. MEL624 cells that overexpress PD-L1 were loaded with CMV peptide pp65. The cells were cultured for 30 minutes with rhIL-18 (R&D systems, 30 ng/ml) and rhIL-18BP (R&D systems, 2µg/ml) to allow the formation of IL-18:IL-18BP complex, and the cells were then treated with 10µg/ml ADI-71739 or aPVRIG (anti-PVRIG) or aTIGIT (anti-TIGIT) or Pembrolizumab (anti-PD-L1) or isotype control, as mono or in various combinations. CMV-specific T-cells were then added to the culture and IFNg secretion was measured after an overnight incubation. B) The anti-IL-18BP antibody alone was able to increase IFNγ secretion by the T cells, and this effect was augmented upon combination with Pembrolizumab/aPVRIG/aTIGIT.

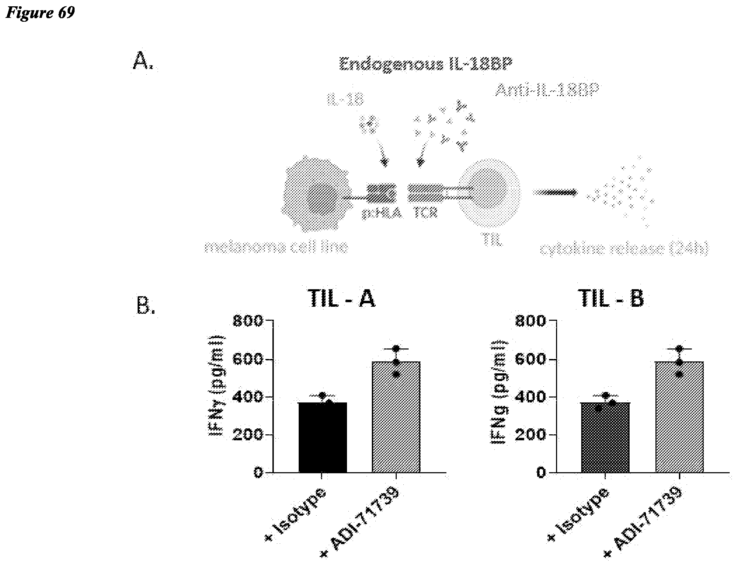

Figure 69A-69B shows in vitro studies testing the effects of ADI-71739 on human TIL function in the presence of endogenous IL-18BP levels. Anti-IL18BP antibody ADI-71739 increased IFNg release by tumor infiltrating lymphocytes. A) Schematic representation of assay setup. MEL624 cells were co-cultured with human TILs that were previously enriched for MART1 or gp100 peptide-specific clones. IL-18 (3.7 ng/ml) was added to the co-culture along with 5µg/ml ADI-71739 or isotype control. The co-culture was set for 18 hours following which IFNg levels were measured in supernatants. B) IFNγ levels were increased in co-cultures treated with ADI-71739 (turquoise) as compared with isotype-treated samples (black). Representative examples from two TIL donors are shown.

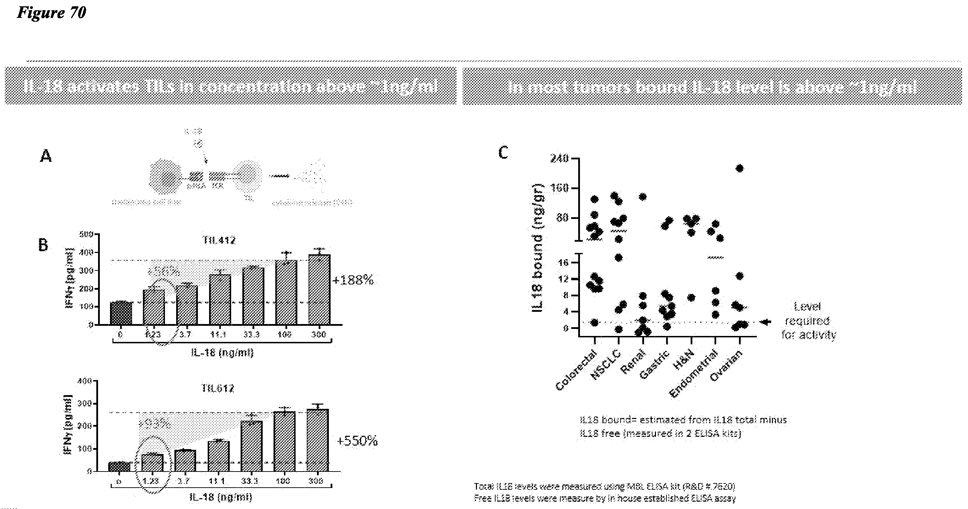

Figure 70A-70C shows that Bound IL-18 levels in the TME are above required amount for T cell activation in vitro. A) Schematic representation of assay setup; thawed tumor infiltrating lymphocytes (TILs), co-cultured with MEL624 cells in a 1:1 ratio, were treated with rhIL-18 (R&D systems, 1.23-300 ng/ml) for 24hr. B) rhIL-18 increased IFNγ secretion in a dose-dependent manner. rhIL-18 activates TILs in concentration above ~1ng/ml and reached saturation at ~100ng/ml. C) Levels of bound IL-18 in TDS across indications are mostly above the level required for *in vitro* T cell activation. Bound IL18 levels were calculated by deducting IL18 free from total IL-18 measured for each sample by two separate ELISA kits. Dashed red line represent the level required for functional activity (1.5ng/gr). Black lines represent the median level bound IL-18 for each tumor type.

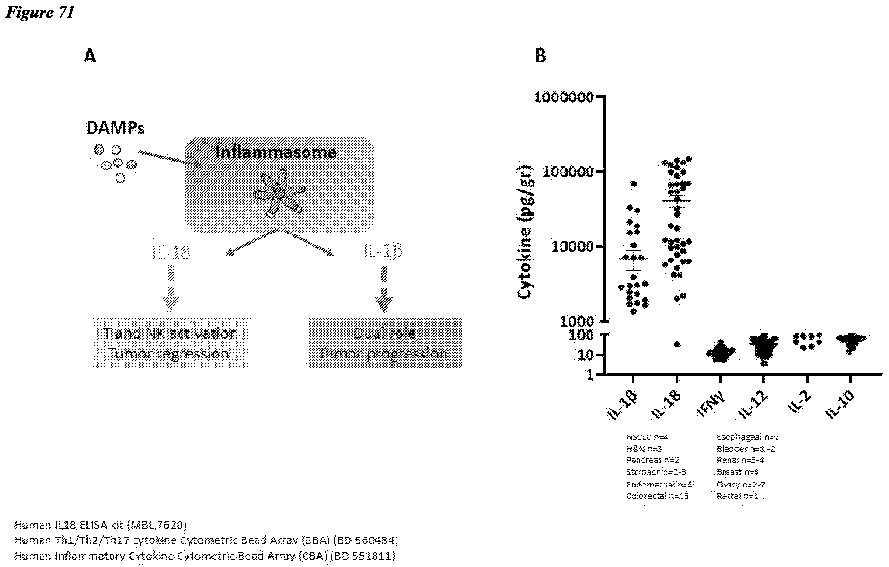

Figure 71A-71B shows that unlike other cytokines, inflammasome induced cytokines such as IL-18 and IL-1b are abundant in the TME. A) IL-18 and IL-1b are inflammasome derived cytokines with opposite effects in the TME. While IL-18 promotes T and NK cell activation and lead to anti tumorigenic activity, IL1b has a dual role and in sum of effects lead to pro-tumorigenic activity. B) Dotplot shows levels of cytokines in tumor derived supernatants measured across various indications. Each dot represents one sample. The mean is depicted by the short black lines. Dashed red lines represent the limit of detection for each cytokine.

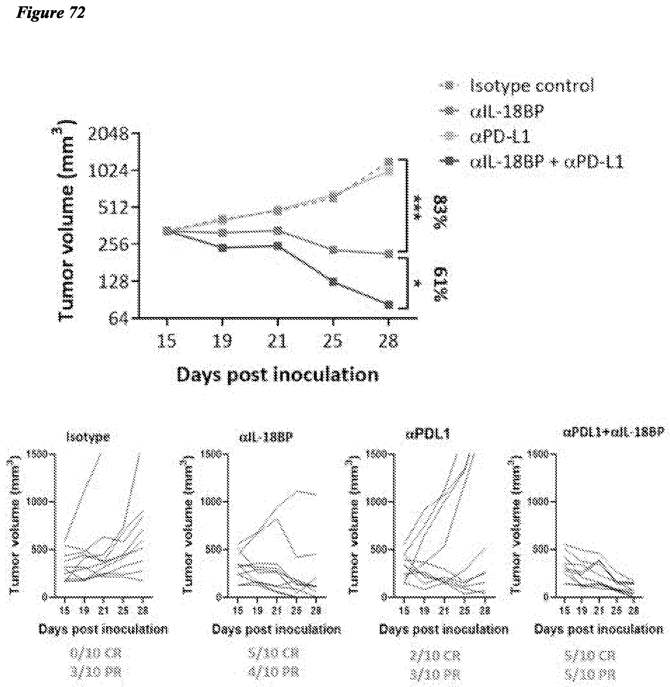

Figure 72 shows anti-IL-18BP antibody and anti-PD-L1 antibody combination studies in mouse tumor models. Anti-IL-18BP Ab in combination with anti-PD-L1 Ab increase tumor growth inhibition and survival in mouse tumor model. Groups of ten 6 weeks old female C57BL/6 mice were subcutaneously injected with E0771 and were administered with mIgG1 Synagis isotype control, anti-mouse IL-18BP Ab, anti PDL1 ab or combination of anti-mouse IL-18BP Ab with anti PD-L1 ab (IP) followed by 6 additional doses. Tumor volumes are represented as the Mean volume + SEM. Tumor volumes were measured twice weekly.

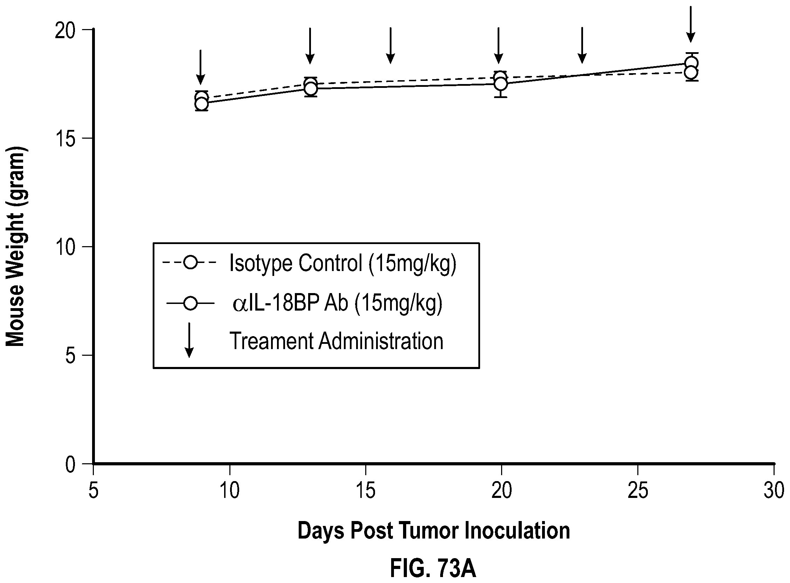

Figure 73A-73C shows administration of anti-IL18BP is expected to have a better therapeutic window than engineered IL-18. C57BL/6 mice were subcutaneously injected with MC38ova cells and treated with designated mAb Synagis mIgG1 (IP), anti-IL18bp mIgG1 (IP), PBS (SC), or Engineered IL-18 (SC) twice weekly. A) Mice were weighed once weekly. B) Mice were bled before the 4th treatment, 4 hours after the 4th treatment, and 24 hours after the 4th treatment. Serum was analyzed for presence of indicated molecules - IFNg, TNF, MCP1, IL6. C) Serum was analyzed for levels of IL-18. D. Spleens were harvested from mice 24 hours after the 4th treatment and weighed. E. Spleens harvested from mice treated with IL15 or ILI5+ILRa were weighed.

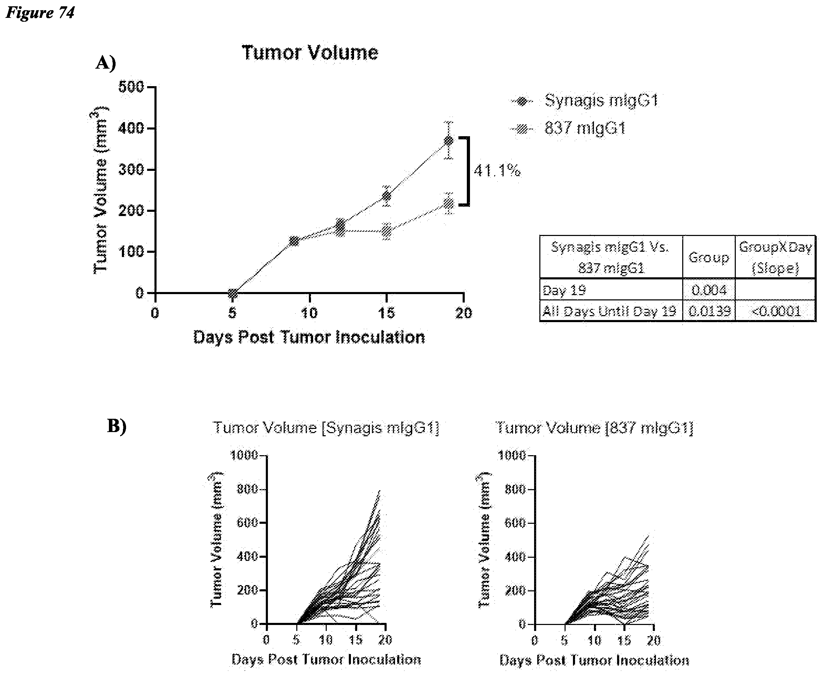

Figure 74A-74B depict assessment of anti-IL18-BP monotherapy in mouse syngeneic MC38ova tumor model. C57BL/6 mice were subcutaneously injected with 1.2M MC38ova cells and treated with designated mAb Synagis mIgG1 (IP), anti-IL18bp mIgG1 (IP) twice weekly. A) tumor growth measurement of each group, B) overview of tumor growth measurement of individual mice in each group.

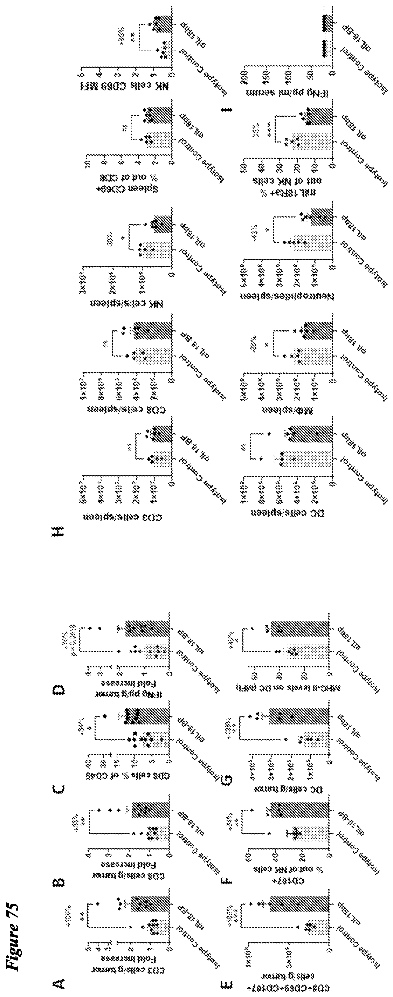

Figure 75 shows that Anti-IL18bp antibody modulates tumor microenvironment without effecting periphery in MC38ova tumor model. C57BL/6 mice were subcutaneously injected with MC38ova^{dim} and were treated with anti-mouse IL-18BP Ab (IP). Tumors, spleens and serum were harvested, and immune composition and cytokine concentrations were determined. A-G) representing tumor microenvironment, H) represents spleen, I) represents serum.

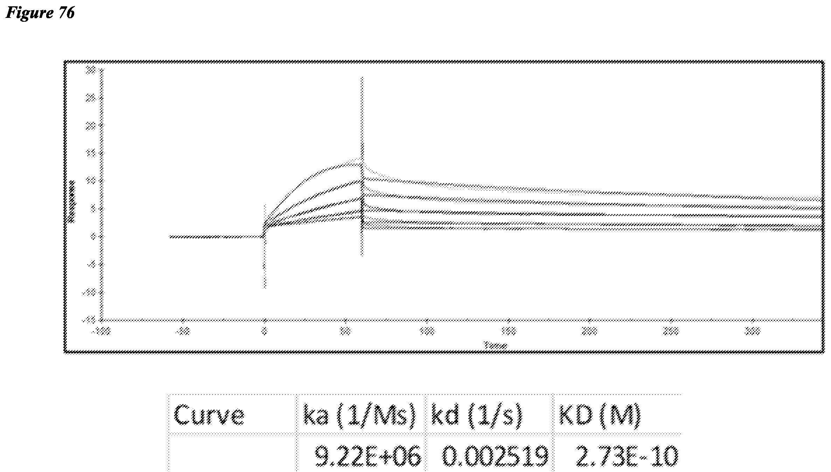

Figure 76. Binding of MAB1191 Ab to human IL18BP, affinity measurement using Biacore.

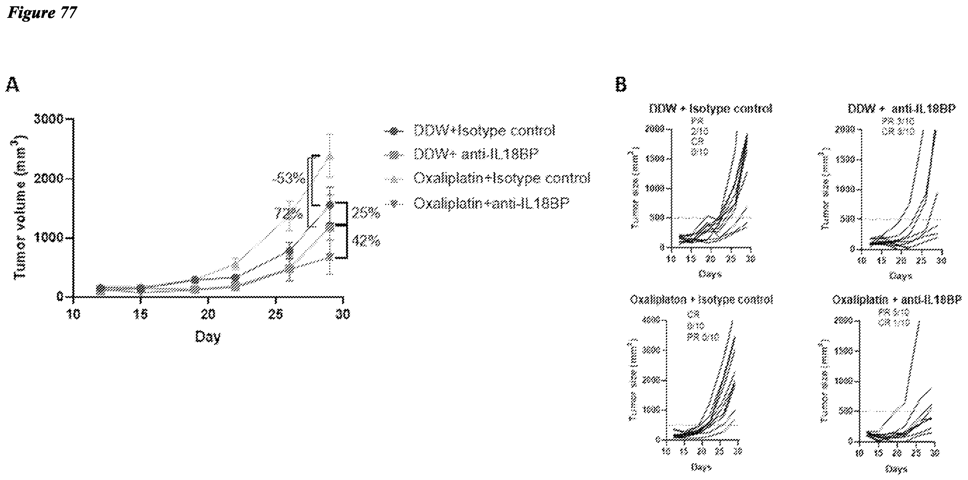

Figure 77: Effect of combination of anti-IL18BP antibody with oxaliplatin in MC38ovadim tumor model. Groups of 10 C57BL/6 mice were inoculated with MC38OVAdim. At tumor volume (TV) of 110mm3, mice were treated with 5mg/kg of oxaliplatin or control DDW. At TV 140mm3 mice were treated with 15mg/kg of anti-IL18BP mIgG1 Ab or isotype control, followed by 5 additional doses. (A) TVs are represented as the mean volume ± SEM. (B) Individual tumors measurements for each mouse are depicted (n=10 per group). CR-complete responders, PR- partially responders (TV<=500mm3) CR-complete responders, PR- partially responders.

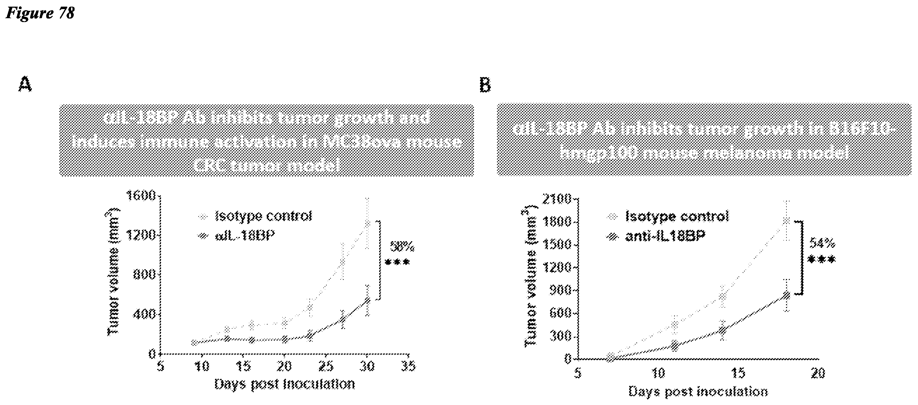

Figure 78. The anti-tumor activity of anti-mouse IL18BP as a single agent in MC380VAdim and B16F10-hmgp100 mouse tumor models. Groups of 10 C57BL/6 mice were inoculated with MC38ovadim or B16F10-hmgp100 cells. Mice were treated with designated mAb: anti IL-18BP Ab or isotype control. (A-B) anti-IL-18BP Ab inhibits tumor growth in MC38ova (A) or B16F10-hmgp100 (B) moues tumor models. Tumor volumes are represented as the mean volume ± SEM.

### DETAILED DESCRIPTION OF THE INVENTION

### I. INTRODUCTION

### A. Interleukin 18 binding protein

The present invention provides antibodies that specifically bind to interleukin 18 binding protein (IL18-BP). "Protein" in this context is used interchangeably with "polypeptide" and includes peptides as well. The present invention provides antibodies that specifically bind to IL18-BP.

The IL18-BP gene is localized to the human chromosome 11, and no exon coding for a transmembrane domain could be found in the 8.3 kb genomic sequence comprising the IL18-BP gene. Four isoforms of IL18-BP generated by alternative mRNA splicing have been identified in humans so far. They were designated IL18-BP a, b, c, and d, all sharing the same N-terminus and differing in the C-terminus (Novick, D. et al., Immunity, 10:127-136, (1999)). These isoforms vary in their ability to bind IL18 (Kim, S.-H. et al., PNAS, 97(3): 1190-1195 (2000)). Of the four human IL18-BP (hIL18-BP) isoforms, isoforms a and c are known to have a neutralizing capacity for IL18. The most abundant IL18-BP isoform, isoform a, exhibits a high affinity for IL 18 with a rapid on-rate and a slow off-rate, and a dissociation constant (K_{d}) of approximately 0.4 nM (Kim, S.-H. et al., PNAS, 97(3): 1190-1195 (2000)). Others have reported that the affinity of IL18-BP to IL-18 is approximately ~1pM or ~25pM (Zhou T. et al., Nature, 583(7817): 609-614, (2020), Girard C. et al., Rheumatology 2016;55:2237-2247 (2016)) IL18-BPb and IL18-BPd isoforms lack a complete Ig domain and lack the ability to bind or neutralize IL18. Two mouse isoforms of IL18-BP, resulting from mRNA splicing and found in various cDNA libraries and have been expressed, purified, and assessed for binding and neutralization of IL 18 biological activities (Kim, S.-H. et al., PNAS, 97(3): 1190-1195 (2000)). Human and mouse IL18-BP share 60.8% amino acid similarity. Murine IL18-BPc and IL18-BPd isoforms, possessing the identical Ig domain, also neutralize >95% murine IL18 at a molar excess of two. However, murine IL18-BPd, which shares a common C-terminal motif with human IL18-BPa, also neutralizes human IL18. Molecular modeling identified a large mixed electrostatic and hydrophobic binding site in the Ig domain of IL18-BP, which could account for its high affinity binding to the ligand (Kim, S.-H. et al., PNAS, 97(3): 1190-1195 (2000)).

IL18-BP is a secreted protein of 194 amino acids in length, with a signal peptide (spanning from amino acid 1 to 30), and a secreted chain (spanning from amino acid 41 to 171) and 4 potential N-glycosylation sites but no transmembrane domains. The full length human IL18-BP isoform a protein is shown in Figure 28 (SEQ ID NO:254). The present invention provides formulations comprising antibodies that specifically bind to IL 18-BP proteins. "Protein" in this context is used interchangeably with "polypeptide", and includes peptides as well. The present invention provides antibodies that specifically bind to IL18-BP proteins. IL18-BP is a secreted protein of 194 amino acids in length, with a signal peptide (spanning from amino acid 1 to 30), and a secreted chain (spanning from amino acid 41 to 171).

Accordingly, as used herein, the term "IL18 BP", "IL-18BP", "IL18BP", "IL18-BP", "IL18 binding protein", or "Interleukin 18 binding protein" may optionally include any such protein, or variants, conjugates, or fragments thereof, including but not limited to known or wild type IL18-BP, as described herein, as well as any naturally occurring splice variants, amino acid variants or isoforms. The term of IL18-BP is used interchangeably with "IL18 binding protein", "Interleukin 18 binding protein", "IL18 BPa", "interleukin-18-binding protein isoform a", "interleukin-18 binding protein isoform a precursor", The term "soluble" form of IL18-BP is also used interchangeably with the terms "IL18 BP soluble" or "fragments of IL18-BP polypeptides", which may refer broadly to one or more of the following optional polypeptides. The term "soluble" with regard to the form of IL18-BP is also used interchangeably with the terms "secreted" as well as "fragments of IL18-BP polypeptides", which may refer broadly to one or more of the IL18-BP polypeptides disclosed herein.

IL18-BP is constitutively expressed in the spleen and belongs to the immunoglobulin superfamily. The residues involved in the interaction of IL18 with IL18-BP have been described through the use of computer modelling (Kim, S.-H. et al., PNAS, 97(3): 1190-1195 (2000)) and based on the interaction between the similar protein IL-1β with the IL-1R type I (Vigers, G. P. A. et al., Nature, 386:190-194 (1997)). IL18-BP functions as an inhibitor of the proinflammatory cytokine, IL18. IL-18 modulates immune system functions including induction of IFNγ production, Th1 differentiation, NK cell activation, and cytotoxic T lymphocytes (CTL) responses (Tominaga, K., et al., International Immunology, 12(2): 151-160 (2000) and Senju, H., et al., Int J Biol Sci., 14(3):331-340 (2018)). IL18-BP binds IL18, prevents the binding of IL18 to its receptor, and thus inhibits IL18 induced T and NK cell activation and proliferation, and pro-inflammatory cytokine production, resulting in reduced T and NK cell activity and T-helper type 1 immune responses. IL18-BP abolishes IL18 induction of IFN-γ and IL18 activation of NF-κB *in vitro.* In addition, IL18-BP inhibits induction of IFN-γ in mice injected with LPS terminus (Novick, D. et al., Immunity, 10:127-136, (1999)).

IL18 is constitutively present in many cells (Puren et al., PNAS, 96:2256-2261 (1999)) and circulates in healthy humans (Urushihara et al. 2000), representing a unique phenomenon in cytokine biology. Due to the high affinity of IL18 to IL18-BP (Kd~1pM) as well as the high concentration of IL18-BP found in the circulation (20-fold molar excess over IL18), it has been hypothesized that most, if not all of the IL18 molecules in the circulation are bound to IL18-BP. Thus, the circulating IL18-BP that competes with cell surface receptors for IL18 may act as a natural anti-inflammatory and an immunosuppressive molecule.

Anti-IL18-BP antibodies (including antigen-binding fragments) that bind to IL18-BP and block the interaction of IL18 and IL18-BP, thereby releasing increased levels of free IL18 can be used to enhance T cells, NK cells, NKT cells, Myeloid cells, Dendritic cells, MAIT T cells, γδ T cells, and/or innate lymphoid cells (ILCs) activation, proliferation, cytokines and/or chemokines secretion, and can be used in treating diseases such as cancer and pathogen infection. These anti-IL18-BP antibodies find use in treating diseases such as cancer.

Accordingly, described herein are anti-IL18-BP antibodies as provided in Figures 1, 2, and/or 3 (*e.g.,* including anti-IL18-BP antibodies including those with CDRs identical to those shown in Figures 1, 2, and/or 3). IL18-BP, also called Interleukin-18 binding protein, UniProtKB/Swiss-Prot (O95998) or HGNC (5987) NCBI Entrez Gene (10068), relates to amino acid and nucleic acid sequences shown in RefSeq accession identifier: NG_029021.1, NM_001039659.1, NP_001034748.1, NC_000011.10 Chromosome 11 Reference GRCh38.p13 Primary Assembly accession identifier: NM_001039660.2 and NP_001034749.1 and NC_000011.9 Chromosome 11 Reference GRCh38.p13 Primary Assembly accession identifier: NP_001034748.1, NM_001039659.2, NP_005690.2NM_005699.3 NP_001034748.1NM_001039659.2, NP_005690.2, NM_005699.3, NP_001138529.1 NM_001145057.1, NP_001138527.1, NM_001145055.1. The antibodies of the invention are specific for the IL18-BP.

### II. ANTI-IL18-BP ANTIBODIES

Described herein are anti-IL18-BP antibodies as provided in Figures 1, 2 and 3 (e.g., including anti-IL18-BP antibodies including those with CDRs identical to those shown in Figures 1, 2, and/or 3), as well as antibodies that compete for binding with the antibodies enumerated in Figures 1, 2, and/or 3.

As is discussed below, the term "antibody" is used generally. Antibodies that find use in the present invention can take on a number of formats as described herein, including traditional antibodies as well as antibody derivatives, fragments and mimetics, described below. In general, the term "antibody" includes any polypeptide that includes at least one antigen binding domain, as more fully described below. Antibodies may be polyclonal, monoclonal, xenogeneic, allogeneic, syngeneic, or modified forms thereof, as described herein, with monoclonal antibodies finding particular use in many embodiments. The antibodies of the claimed invention bind specifically to IL18-BP molecules. The terms "monoclonal antibodies" and "monoclonal antibody composition", as used herein, refer to a population of antibody molecules that contain only one species of an antigen-binding site capable of immunoreacting with a particular epitope of an antigen, whereas the term "polyclonal antibodies" and "polyclonal antibody composition" refer to a population of antibody molecules that contain multiple species of antigen-binding sites capable of interacting with a particular antigen. A monoclonal antibody composition typically displays a single binding affinity for a particular antigen with which it immunoreacts.

Traditional full length antibody structural units typically comprise a tetramer. Each tetramer is typically composed of two identical pairs of polypeptide chains, each pair having one "light" (typically having a molecular weight of about 25 kDa) and one "heavy" chain (typically having a molecular weight of about 50-70 kDa). Human light chains are classified as kappa and lambda light chains. The present invention is directed to the IgG class, which has several subclasses, including, but not limited to IgG1, IgG2, IgG3, and IgG4. Thus, "isotype" as used herein is meant any of the subclasses of immunoglobulins defined by the chemical and antigenic characteristics of their constant regions. While the exemplary antibodies herein designated "CPA" are based on IgG1 heavy constant regions, as shown in Figure 4, the anti-IL18-BP antibodies of the invention include those using IgG2, IgG3 and IgG4 sequences, or combinations thereof. For example, as is known in the art, different IgG isotypes have different effector functions which may or may not be desirable. Accordingly, the CPA antibodies of the invention can also swap out the IgG1 constant domains for IgG2, IgG3 or IgG4 constant domains (depicted in Figure 1E), with IgG2 and IgG4 finding particular use in a number of situations, for example for ease of manufacture or when reduced effector function is desired, the latter being desired in some situations.

The amino-terminal portion of each chain includes a variable region of about 100 to 110 or more amino acids primarily responsible for antigen recognition, generally referred to in the art and herein as the "Fv domain" or "Fv region". In the variable region, three loops are gathered for each of the V domains of the heavy chain and light chain to form an antigen-binding site. Each of the loops is referred to as a complementarity-determining region (hereinafter referred to as a "CDR"), in which the variation in the amino acid sequence is most significant. "Variable" refers to the fact that certain segments of the variable region differ extensively in sequence among antibodies. Variability within the variable region is not evenly distributed. Instead, the V regions consist of relatively invariant stretches called framework regions (FRs) of 15-30 amino acids separated by shorter regions of extreme variability called "hypervariable regions".

Each VH and VL is composed of three hypervariable regions ("complementary determining regions," "CDRs") and four FRs, arranged from amino-terminus to carboxy-terminus in the following order: FR1-CDR1-FR2-CDR2-FR3-CDR3-FR4.

The hypervariable region generally encompasses amino acid residues from about amino acid residues 24-34 (LCDR1; "L" denotes light chain), 50-56 (LCDR2) and 89-97 (LCDR3) in the light chain variable region and around about 31-35B (HCDR1; "H" denotes heavy chain), 50-65 (HCDR2), and 95-102 (HCDR3) in the heavy chain variable region, although sometimes the numbering is shifted slightly as will be appreciated by those in the art; Kabat et al., SEQUENCES OF PROTEINS OF IMMUNOLOGICAL INTEREST, 5 th Ed. Public Health Service, National Institutes of Health, Bethesda, Md. (1991) and/or those residues forming a hypervariable loop (*e.g*. residues 26-32 (LCDR1), 50-52 (LCDR2) and 91-96 (LCDR3) in the light chain variable region and 26-32 (HCDR1), 53-55 (HCDR2) and 96-101 (HCDR3) in the heavy chain variable region; Chothia and Lesk (1987) J. Mol. Biol. 196:901-917. Specific CDRs are described below and shown in Figure 6A-6D.

The carboxy-terminal portion of each chain defines a constant region primarily responsible for effector function. Kabat et al. collected numerous primary sequences of the variable regions of heavy chains and light chains. Based on the degree of conservation of the sequences, they classified individual primary sequences into the CDR and the framework and made a list thereof (see SEQUENCES OF IMMUNOLOGICAL INTEREST, 5 th edition, NIH publication, No. 91-3242, E. A. Kabat et al.).

In the IgG subclass of immunoglobulins, there are several immunoglobulin domains in the heavy chain. By "immunoglobulin (Ig) domain" herein is meant a region of an immunoglobulin having a distinct tertiary structure. Of interest in the present invention are the heavy chain domains, including, the constant heavy (CH) domains and the hinge domains. In the context of IgG antibodies, the IgG isotypes each have three CH regions. Accordingly, "CH" domains in the context of IgG are as follows: "CH1" refers to positions 118-220 according to the EU index as in Kabat. "CH2" refers to positions 237-340 according to the EU index as in Kabat, and "CH3" refers to positions 341-447 according to the EU index as in Kabat.

Accordingly, described herein are variable heavy domains, variable light domains, heavy constant domains, light constant domains and Fc domains to be used as outlined herein. By "variable region" as used herein is meant the region of an immunoglobulin that comprises one or more Ig domains substantially encoded by any of the Vκ or Vλ, and/or VH genes that make up the kappa, lambda, and heavy chain immunoglobulin genetic loci respectively. Accordingly, the variable heavy domain comprises vhFR1-vhCDR1-vhFR2-vhCDR2-vhFR3-vhCDR3-vhFR4, and the variable light domain comprises vlFR1-vlCDR1-vlFR2-vlCDR2-vlFR3-vlCDR3-vlFR4. By "heavy constant region" herein is meant the CH1-hinge-CH2-CH3 portion of an antibody. By "Fc" or "Fc region" or "Fc domain" as used herein is meant the polypeptide comprising the constant region of an antibody excluding the first constant region immunoglobulin domain and in some cases, part of the hinge. Thus Fc refers to the last two constant region immunoglobulin domains of IgA, IgD, and IgG, the last three constant region immunoglobulin domains of IgE and IgM, and the flexible hinge N-terminal to these domains. For IgA and IgM, Fc may include the J chain. For IgG, the Fc domain comprises immunoglobulin domains Cγ2 and Cγ3 (Cγ2 and Cγ3) and the lower hinge region between Cγ1 (Cγ1) and Cγ2 (Cγ2). Although the boundaries of the Fc region may vary, the human IgG heavy chain Fc region is usually defined to include residues C226 or P230 to its carboxyl-terminus, wherein the numbering is according to the EU index as in Kabat. In some embodiments, as is more fully described below, amino acid modifications are made to the Fc region, for example to alter binding to one or more FcγR receptors or to the FcRn receptor.

Thus, "Fc variant" or "variant Fc" as used herein is meant a protein comprising an amino acid modification in an Fc domain. The Fc variants of the present invention are defined according to the amino acid modifications that compose them. Thus, for example, N434S or 434S is an Fc variant with the substitution serine at position 434 relative to the parent Fc polypeptide, wherein the numbering is according to the EU index. Likewise, M428L/N434S defines an Fc variant with the substitutions M428L and N434S relative to the parent Fc polypeptide. The identity of the WT amino acid may be unspecified, in which case the aforementioned variant is referred to as 428L/434S. It is noted that the order in which substitutions are provided is arbitrary, that is to say that, for example, 428L/434S is the same Fc variant as M428L/N434S, and so on. For all positions discussed in the present invention that relate to antibodies, unless otherwise noted, amino acid position numbering is according to the EU index.

By "Fab" or "Fab region" as used herein is meant the polypeptide that comprises the VH, CH1, VL, and CL immunoglobulin domains. Fab may refer to this region in isolation, or this region in the context of a full length antibody, antibody fragment or Fab fusion protein. By "Fv" or "Fv fragment" or "Fv region" as used herein is meant a polypeptide that comprises the VL and VH domains of a single antibody. As will be appreciated by those in the art, these generally are made up of two chains.

Throughout the present specification, either the IMTG numbering system or the Kabat numbering system is generally used when referring to a residue in the variable domain (approximately, residues 1-107 of the light chain variable region and residues 1-113 of the heavy chain variable region) (e.g, Kabat et al., supra (1991)). EU numbering as in Kabat is generally used for constant domains and/or the Fc domains.

The CDRs contribute to the formation of the antigen-binding, or more specifically, epitope binding site of antibodies. "Epitope" refers to a determinant that interacts with a specific antigen binding site in the variable region of an antibody molecule known as a paratope. Epitopes are groupings of molecules such as amino acids or sugar side chains and usually have specific structural characteristics, as well as specific charge characteristics. A single antigen may have more than one epitope.

The epitope may comprise amino acid residues directly involved in the binding (also called immunodominant component of the epitope) and other amino acid residues, which are not directly involved in the binding, such as amino acid residues which are effectively blocked by the specifically antigen binding peptide; in other words, the amino acid residue is within the footprint of the specifically antigen binding peptide.

Epitopes may be either conformational or linear. A conformational epitope is produced by spatially juxtaposed amino acids from different segments of the linear polypeptide chain. A linear epitope is one produced by adjacent amino acid residues in a polypeptide chain. Conformational and nonconformational epitopes may be distinguished in that the binding to the former but not the latter is lost in the presence of denaturing solvents.

An epitope typically includes at least 3, and more usually, at least 5 or 8-10 amino acids in a unique spatial conformation. Antibodies that recognize the same epitope can be verified in a simple immunoassay showing the ability of one antibody to block the binding of another antibody to a target antigen, for example "binning". Specific bins are described below.

Included within the definition of "antibody" is an "antigen-binding portion" of an antibody (also used interchangeably with "antigen-binding fragment", "antibody fragment" and "antibody derivative"). That is, for the purposes of the invention, an antibody of the invention has a minimum functional requirement that it bind to a IL18-BP antigen. As will be appreciated by those in the art, there are a large number of antigen fragments and derivatives that retain the ability to bind an antigen and yet have alternative structures, including, but not limited to, (i) the Fab fragment consisting of VL, VH, CL and CH1 domains, (ii) the Fd fragment consisting of the VH and CH1 domains, (iii) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., 1988, Science 242:423-426, Huston et al., 1988, Proc. Natl. Acad. Sci. U.S.A. 85:5879-5883), (iv) "diabodies" or "triabodies", multivalent or multispecific fragments constructed by gene fusion (Tomlinson et. al., 2000, Methods Enzymol. 326:461-479; WO94/13804; Holliger et al., 1993, Proc. Natl. Acad. Sci. U.S.A. 90:6444-6448), (v) "domain antibodies" or "dAb" (sometimes referred to as an "immunoglobulin single variable domain", including single antibody variable domains from other species such as rodent (for example, as disclosed in WO 00/29004), nurse shark and Camelid V-HH dAbs, (vi) SMIPs (small molecule immunopharmaceuticals), camelbodies, nanobodies and IgNAR.

Still further, an antibody or antigen-binding portion thereof (antigen-binding fragment, antibody fragment, antibody portion) may be part of a larger immunoadhesion molecules (sometimes also referred to as "fusion proteins"), formed by covalent or noncovalent association of the antibody or antibody portion with one or more other proteins or peptides. Examples of immunoadhesion molecules include use of the streptavidin core region to make a tetrameric scFv molecule and use of a cysteine residue, a marker peptide and a C-terminal polyhistidine tag to make bivalent and biotinylated scFv molecules. Antibody portions, such as Fab and F(ab')₂ fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques, as described herein.

In general, the anti-IL18-BP antibodies of the invention are recombinant. "Recombinant" as used herein, refers broadly with reference to a product, *e.g.,* to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (non-recombinant) form of the cell or express native genes that are otherwise abnormally expressed, under expressed or not expressed at all.

The term "recombinant antibody", as used herein, includes all antibodies that are prepared, expressed, created or isolated by recombinant means, such as (a) antibodies isolated from an animal (*e.g.,* a mouse) that is transgenic or transchromosomal for human immunoglobulin genes or a hybridoma prepared therefrom (described further below), (b) antibodies isolated from a host cell transformed to express the human antibody, e.g., from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable regions in which the framework and CDR regions are derived from human germline immunoglobulin sequences. In certain embodiments, however, such recombinant human antibodies can be subjected to *in vitro* mutagenesis (or, when an animal transgenic for human Ig sequences is used, in vivo somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

### A. ANTI-IL18-BP BINDING ANTIBODY

The present invention provides anti-IL18-BP antibodies, as defined in the claims. (For convenience, "anti-IL18-BP antibodies" and "IL18-BP antibodies" are used interchangeably). The anti-IL18-BP antibodies of the invention specifically bind to human IL18-BP, and preferably the secreted chain of human IL18-BP, as depicted in Figure 28. Described herein are anti-IL18-BP antibodies that specifically bind to human IL18-BP, and preferably the secreted chain of human IL18-BP, as depicted in Figure 28, including, e.g., anti-IL18-BP antibodies including those with CDRs identical to those shown in Figures 1, 2 and 3.

As noted herein and more fully described below, the anti-IL18-BP antibodies (including antigen-binding fragments) that both bind to IL 18-BP and block the interaction of IL18-BP and IL18, thereby releasing increased levels of free IL 18, are used to enhance T cells, NK cells, NKT cells, Myeloid cells, dendritic cells, MAIT T cells, γδ T cells, and/or innate lymphoid cells (ILCs) activation, proliferation, cytokines and/or chemokines secretion, and can be used in treating diseases such as cancer and pathogen infection.

Specific binding for IL18-BP or a IL18-BP epitope can be exhibited, for example, by an antibody having a KD of at least about 10⁻⁵ M, at least about 10⁻⁶ M, at least about 10⁻⁷ M, at least about 10⁻⁸ M, at least about 10⁻⁹ M, alternatively at least about 10⁻¹⁰ M, at least about 10⁻¹¹ M, at least about 10⁻¹² M, at least about 10⁻¹³ M, at least about 10⁻¹⁴ M, or greater, where KD refers to a dissociation rate of a particular antibody-antigen interaction. Typically, an antibody that specifically binds an antigen will have a KD that is 20-, 50-, 100-, 500-, 1000-, 5,000-, 10,000-, 100,000- or more times greater for a control molecule relative to the IL18-BP antigen or epitope.

However, as supported by the Examples, for optimal binding to IL18-BP, the antibodies described herein have a KD (also referred to as the binding affinity) less than 0.01 nM, less 10 nM and most preferably less than 0.1 pM, with less than 1 pM, less than 0.1 pM, and less than 0.01 pM, finding use in the methods of the invention. In some instances, the anti-IL-18BP antibodies described herein exhibit a KD less than 900 pM, less than 850 pM, less than 800 pM, less than 750 pM, less than 700 pM, less than 650 pM, less than 600 pM, less than 550 pM, less than 500 pM, less than 450 pM, less than 400 pM, less than 350 pM, less than 300 pM, less than 250 pM, less than 200 pM, less than 150 pM, less than 100 pM, less than 50 pM, or less than 10 pM. In some embodiments, the anti-IL-18BP antibodies exhibit a KD less than 750 pM. In some instances, the anti-IL18-BP antibodies described herein can bind to human IL18-BP with a K_{D} of 50 nM or less, 10 nM or less, or 1 nM or less (that is, higher binding affinity), 100pM or less, 10pM or less, 1pM or less, 0.1pM or less, or 0.01 pM or less, wherein K_{D} is determined by known methods, e.g. surface plasmon resonance (SPR, e.g. Biacore instrument), ELISA, KinExA, and most typically SPR at 25° or 37° C. In some instances, the anti-IL18-BP antibodies described herein bind to human IL18-BP with a a KD less than 900 pM, less than 850 pM, less than 800 pM, less than 750 pM, less than 700 pM, less than 650 pM, less than 600 pM, less than 550 pM, less than 500 pM, less than 450 pM, less than 400 pM, less than 350 pM, less than 300 pM, less than 250 pM, less than 200 pM, less than 150 pM, less than 100 pM, less than 50 pM, or less than 10 pM, wherein K_{D} is determined by known methods, *e.g.* surface plasmon resonance (SPR, *e.g.* Biacore instrument), ELISA, KinExA, and most typically SPR at 25° or 37° C. The anti-IL18-BP antibodies of the claimed invention exhibit a binding affinity or KD equal to or lower than 1pM, as measured by solution equilibrium titration. In some embodiments, the antibodies preferably have a KD or binding affinity less than 0.005 pM, 0.01 pM, 0.02 pM, 0.03 pM, 0.04 pM, 0.05 pM, 0.06 pM, 0.07 pM, 0.08 pM, 0.09 pM, 0.10 pM, 0.15 pM, 0.20 pM, 0.25 pM, 0.30 pM, 0.35 pM, 0.40 pM, 0.45 pM, 0.50 pM, 0.55 pM, 0.60 pM, 0.65 pM, 0.70 pM, 0.75 pM, 0.80 pM, 0.85 pM, 0.90 pM, 0.95 pM, or 1 pM.

Also, specific binding for a particular antigen or an epitope can be exhibited, for example, by an antibody having a KA or Ka for an IL18-BP antigen or epitope of at least 20-, 50-, 100-, 500-, 1000-, 5,000-, 10,000-, 100,000- or more times greater for the epitope relative to a control, where KA or Ka refers to an association rate of a particular antibody-antigen interaction.

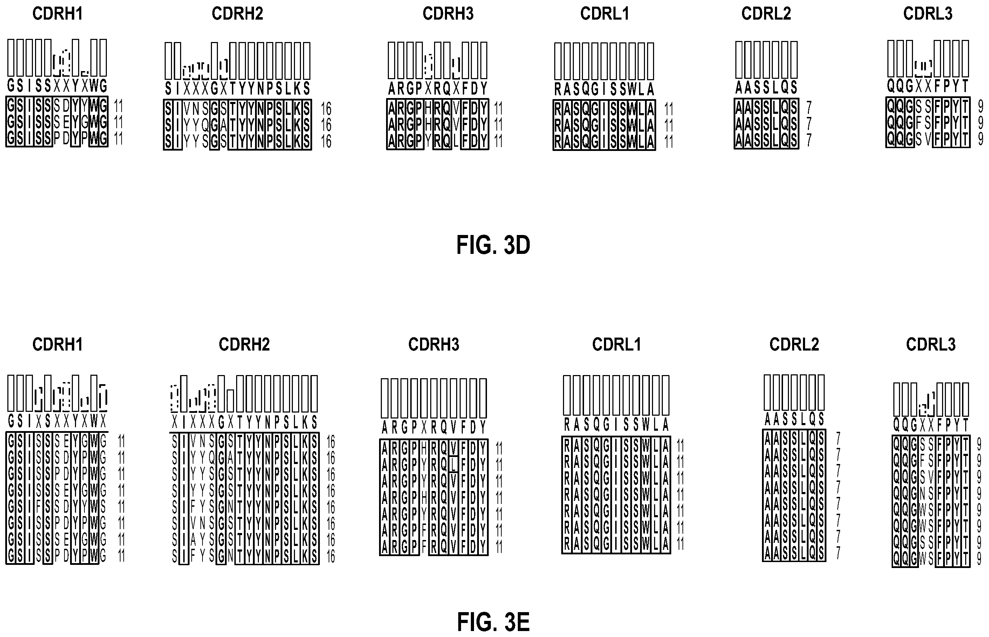

The invention relates to antigen binding domains, including full length antibodies, which contain a number of specific, enumerated sets of 6 CDRs, as defined in the claims. Described herein are antigen binding domains, including full length antibodies, which contain a number of specific, enumerated sets of 6 CDRs, as provided in Figures 1, 2, and/or 3. Also described herein are antigen binding domains, including full length antibodies, which contain a number of specific, enumerated sets of 6 CDRs, as provided in Figure 3.

The invention further provides variable heavy and light domains for the anti-IL18-BP antibodies of the invention, as defined in the claims, as well as full length heavy and light chains for the anti-IL18-BP antibodies of the invention, as defined in the claims.

The disclosure further includes variants of the above components, including variants in the CDRs. Variable heavy chains can be at least 80%, at least 90%, at least 95%, at least 98% or at least 99% identical to the "VH" sequences herein, and/or contain from 1, 2, 3, 4, 5, 6, 7, 8, 9, 10 amino acid changes, or more, when Fc variants are used. Variable light chains can be at least 80%, at least 90%, at least 95%, at least 98% or at least 99% identical to the "VL" sequences herein, and/or contain from 1, 2, 3, 4, 5, 6, 7, 8, 9, 10 amino acid changes, or more, when Fc variants are used. Similarly, heavy and light chains can be at least 80%, at least 90%, at least 95%, at least 98% or at least 99% identical to the "HC" and "LC" sequences herein, and/or contain from 1, 2, 3, 4, 5, 6, 7, 8, 9, 10 amino acid changes, or more, when Fc variants are used.

Accordingly, described herein are antibodies, usually full length or scFv domains, that comprise the following CHA sets of CDRs, the sequences of which are shown in Figure 1 through 3.

The 66650 lineage (VH1-03; VL-kappa-1-5) consensus sequence of CDRs (Figure 1A) was generated using ADI-71701, ADI-71709, ADI-71710, ADI-71707 and ADI-71717 antibodies. The respective sequence alignment is shown in Figure 3B.

The 66650 lineage (VH1-03; VL-kappa-1-5) consensus sequence comprises:
- CDR-H1 having the sequence Y-T-F-X-X2-Y-A-X3-H, wherein X is N, R, D, G or K; X2 is S, H, I or Q; X3 is M or V;
- CDR-H2 having the sequence W-I-H-A-G-T-G-X-T-X2-Y-S-Q-K-F-Q-G, wherein X is N, A or V; X2 is K or L;
- CDR-H3 having the sequence A-R-G-L-G-X-V-G-P-T-G-T-S-W-F-D-P, wherein X is S or E;
- CDR-L1 having the sequence R-A-S-Q-G-I-S-S-W-L-A;
- CDR-L2 having the sequence E-A-S-S-L-E-S; and
- CDR-L3 having the sequence Q-Q-Y-R-X-X2-P-F-T, wherein X is S, V, Y, L or Q; X2 is F, S, or G.

Described herein is an anti-IL18-BP antibody comprising the CDRs:
- CDR-H1 having the sequence Y-T-F-X-X2-Y-A-X3-H, wherein X is N, R, D, G or K; X2 is S,H,I or Q; X3 is M or V;
- CDR-H2 having the sequence W-I-H-A-G-T-G-X-T-X2-Y-S-Q-K-F-Q-G, wherein X is N, A or V; X2 is K or L;
- CDR-H3 having the sequence A-R-G-L-G-X-V-G-P-T-G-T-S-W-F-D-P, wherein X is S or E;
- CDR-L1 having the sequence R-A-S-Q-G-I-S-S-W-L-A;
- CDR-L2 having the sequence E-A-S-S-L-E-S; and
- CDR-L3 having the sequence Q-Q-Y-R-X-X2-P-F-T, wherein X is S, V, Y, L or Q; X2 is F, S, or G.

The 66670 lineage (VH1-69; VL-kappa-1-12) consensus sequence of CDRs (Figure 1B) was generated using ADI-71719, ADI-71720, ADI-71722, and ADI-71728 antibodies. The respective sequence alignment is shown in Figure 3C.

The 66670 lineage (VH1-69; VL-kappa-1-12) consensus sequence comprises:
- CDR-H1 having the sequence G-T-F-X-X2-Y-X3-I-S, wherein X is S or N; X2 is E or S; X3 is V or P;
- CDR-H2 having the sequence G-I-I-P-G-X2-G-T-A-X3-Y-A-Q-K-F-Q-G, wherein X is G or Y, X2 is A or S; X3 is N, I or V;
- CDR-H3 having the sequence A-R-G-R-H-X-H-E-T, wherein X is S, G or F;
- CDR-L1 having the sequence R-A-S-Q-G-I-S-S-W-L-A;
- CDR-L2 having the sequence A-A-S-S-L-Q-S;
- CDR-L3 having the sequence Q-Q-V-Y-X-X2-P-W-T, wherein X is S or R; X2 is L, I or F.

Described herein is an anti-IL18-BP antibody comprising the CDRs:
- CDR-H1 having the sequence G-T-F-X-X2-Y-X3-I-S, wherein X is S or N; X2 is E or S; X3 is V or P;
- CDR-H2 having the sequence G-I-I-P-G-X2-G-T-A-X3-Y-A-Q-K-F-Q-G, wherein X is G or Y, X2 is A or S; X3 is N, I, or V;
- CDR-H3 having the sequence A-R-G-R-H-X-H-E-T, wherein X is S, G or F;
- CDR-L1 having the sequence R-A-S-Q-G-I-S-S-W-L-A;
- CDR-L2 having the sequence A-A-S-S-L-Q-S; and
- CDR-L3 having the sequence Q-Q-V-Y-X-X2-P-W-T, wherein X is S or R; X2 is **L, I,** or F.

The 66692 lineage (VH3-23, VL-kappa-1-12) consensus sequence of CDRs (Figure 1C) was generated using ADI-71662, ADI-71663 and ADI-66692 antibodies. The respective sequence alignment is shown in Figure 3A.

The 66692 lineage (VH3-23, VL-kappa-1-12) consensus sequence comprises:
- CDR-H1 having the sequence F-T-F-X-N-X2-A-M-S, wherein X is G or D or S; X2 is T or V or Y;
- CDR-H2 having the sequence A-I-S-X-X1-X2-G-S-T-Y-Y-A-D-S-V-K-G, wherein X is G or A; X2 is N or S; X3 is A or G;
- CDR-H3 having the sequence A-K-G-P-D-R-Q-V-F-D-Y;
- CDR-L1 having the sequence R-A-S-Q-G-I-X-S-W-L-A, wherein X is S or D;
- CDR-L2 having the sequence A-A-S-S-L-Q-S; and
- CDR-L3 having the sequence Q-H-A-X-XI-F-P-Y-T, wherein X is Y or L; X2 is S or F.

Described herein is an anti-IL18-BP antibody comprising the CDRs:
- CDR-H1 having the sequence F-T-F-X-N-X2-A-M-S, wherein X is G or D or S; X2 is T or V or Y;
- CDR-H2 having the sequence A-I-S-X-X1-X2-G-S-T-Y-Y-A-D-S-V-K-G, wherein X is G or A; X2 is N or S; X3 is A or G;
- CDR-H3 having the sequence A-K-G-P-D-R-Q-V-F-D-Y;
- CDR-L1 having the sequence R-A-S-Q-G-I-X-S-W-L-A, wherein X is S or D;
- CDR-L2 having the sequence A-A-S-S-L-Q-S; and
- CDR-L3 having the sequence Q-H-A-X-XI-F-P-Y-T, wherein X is Y or L; X2 is S or F.

The 66716 lineage (VH1-39; VL-kappa-1-12) consensus sequence of CDRs (Figure 1D) was generated using ADI-71736, ADI-71739 and ADI-66716 antibodies. The respective sequence alignment is shown in Figure 3D.

The 66716 lineage (VH1-39; VL-kappa-1-12) consensus sequence comprises:
- CDR-H1 having the sequence G-S-I-S-S-X-X2-Y-X3-W-G, wherein X is S or P; X2 is E or D; X3 is G, P or Y;
- CDR-H2 having the sequence S-I-X-X2-X3-G-X4-T-Y-Y-N-P-S-L-K-S, wherein X is Y or V; X2 is Y or N; X3 is Q or S; X4 is S or A;
- CDR-H3 having the sequence A-R-G-P-X-R-Q-X2-F-D-Y, wherein X is Y or H, X2 is V or L;
- CDR-L1 having the sequence R-A-S-Q-G-I-S-S-W-L-A;
- CDR-L2 having the sequence A-A-S-S-L-Q-S; and
- CDR-L3 having the sequence Q-Q-G-X-X2-F-P-Y-T, wherein X is S or F; X2 is S or V.

Described herein is an anti-IL18-BP antibody comprising the CDRs:
- CDR-H1 having the sequence G-S-I-S-S-X-X2-Y-X3-W-G, wherein X is S or P; X2 is E or D; X3 is G, P or Y;
- CDR-H2 having the sequence S-I-X-X2-X3-G-X4-T-Y-Y-N-P-S-L-K-S, wherein X is Y or V; X2 is Y or N; X3 is Q or S; X4 is S or A;
- CDR-H3 having the sequence A-R-G-P-X-R-Q-X2-F-D-Y, wherein X is Y or H, X2 is V or L;
- CDR-L1 having the sequence R-A-S-Q-G-I-S-S-W-L-A;
- CDR-L2 having the sequence A-A-S-S-L-Q-S; and
- CDR-L3 having the sequence Q-Q-G-X-X2-F-P-Y-T, wherein X is S or F; X2 is S or V.

Described herein is an anti-IL18-BP antibody comprising the CDRs:
- CDR-H1 having the sequence Y-T-F-X-X2-Y-A-X3-H, wherein X is any amino acid; X2 is any amino acid; X3 is any amino acid;
- CDR-H2 having the sequence W-I-H-A-G-T-G-X-T-X2-Y-S-Q-K-F-Q-G, wherein X is any amino acid; X2 is any amino acid;
- CDR-H3 having the sequence A-R-G-L-G-X-V-G-P-T-G-T-S-W-F-D-P, wherein X is any amino acid;
- CDR-L1 having the sequence R-A-S-Q-G-I-S-S-W-L-A;
- CDR-L2 having the sequence E-A-S-S-L-E-S; and
- CDR-L3 having the sequence Q-Q-Y-R-X-X2-P-F-T, wherein X is any amino acid; X2 is any amino acid.

Described herein is an anti-IL18-BP antibody comprising the CDRs:
- CDR-H1 having the sequence G-T-F-X-X2-Y-X3-I-S, wherein X is any amino acid; X2 is any amino acid; X3 is any amino acid;
- CDR-H2 having the sequence G-I-I-P-G-X2-G-T-A-X3-Y-A-Q-K-F-Q-G, wherein X is any amino acid, X2 is any amino acid;
- CDR-H3 having the sequence A-R-G-R-H-X-H-E-T, wherein X is any amino acid;
- CDR-L1 having the sequence R-A-S-Q-G-I-S-S-W-L-A;
- CDR-L2 having the sequence A-A-S-S-L-Q-S;
- CDR-L3 having the sequence Q-Q-V-Y-X-X2-P-W-T, wherein X is any amino acid; X2 is any amino acid.

Described herein is an anti-IL18-BP antibody comprising the CDRs:
- CDR-H1 having the sequence G-T-F-X-X2-Y-X3-I-S, wherein X is any amino acid; X2 is any amino acid; X3 is any amino acid;
- CDR-H2 having the sequence G-I-I-P-G-X2-G-T-A-X3-Y-A-Q-K-F-Q-G, wherein X is any amino acid; X2 is any amino acid; X3 is any amino acid;
- CDR-H3 having the sequence A-R-G-R-H-X-H-E-T, wherein X is any amino acid;
- CDR-L1 having the sequence R-A-S-Q-G-I-S-S-W-L-A;
- CDR-L2 having the sequence A-A-S-S-L-Q-S;
- CDR-L3 having the sequence Q-Q-V-Y-X-X2-P-W-T, wherein X is any amino acid; X2 is any amino acid.

Described herein is an anti-IL18-BP antibody comprising the CDRs:
- CDR-H1 having the sequence F-T-F-X-N-X2-A-M-S, wherein X is any amino acid; X2 is any amino acid;
- CDR-H2 having the sequence A-I-S-X-X1-X2-G-S-T-Y-Y-A-D-S-V-K-G, wherein X is any amino acid; X2 is any amino acid; X3 is any amino acid;
- CDR-H3 having the sequence A-K-G-P-D-R-Q-V-F-D-Y;
- CDR-L1 having the sequence R-A-S-Q-G-I-X-S-W-L-A, wherein X is any amino acid;
- CDR-L2 having the sequence A-A-S-S-L-Q-S; and
- CDR-L3 having the sequence Q-H-A-X-XI-F-P-Y-T, wherein X is any amino acid; X2 is any amino acid.

Described herein is an anti-IL18-BP antibody comprising the CDRs:
- CDR-H1 having the sequence G-S-I-S-S-X-X2-Y-X3-W-G, wherein X is any amino acid; X2 is any amino acid; X3 is any amino acid;
- CDR-H2 having the sequence S-I-X-X2-X3-G-X4-T-Y-Y-N-P-S-L-K-S, wherein X is any amino acid; X2 is any amino acid; X3 is any amino acid; X4 is any amino acid;
- CDR-H3 having the sequence A-R-G-P-X-R-Q-X2-F-D-Y, wherein X is any amino acid; X2 is any amino acid;
- CDR-L1 having the sequence R-A-S-Q-G-I-S-S-W-L-A;
- CDR-L2 having the sequence A-A-S-S-L-Q-S; and
- CDR-L3 having the sequence Q-Q-G-X-X2-F-P-Y-T, wherein X is any amino acid; X2 is any amino acid.

Described herein is an antibody comprising:
i. a heavy chain variable domain, comprising:
   a) CDR-H1 having the sequence Y-T-F-X-X2-Y-A-X3-H, wherein X is N, R, D, G, T, Q, S, A or K; X2 is S, H, I, N, L, Y or Q; X3 is M or V ;
   b) CDR-H2 having the sequence X-I-X2-A-G-X3-X4-X5-T-X6-Y-S-Q-K-F-Q-G, wherein X is W or Y; X2 is H or N; X3 is S,T or A; X4 is G or A; X5 is N, A, T or V; X6 is E, K or L ; and
   c) CDR-H3 having the sequence A-R-G-L-G-X-V-G-P-T-G-T-S-W-F-D-P, wherein X is S, L, A, K or E; and
ii. a light chain variable domain, comprising:
   a) CDR-L1 having the sequence R-A-S-Q-G-I-S-S-W-L-A;
   b) CDR-L2 having the sequence E-A-S-S- -E-S, wherein X is L or S; and
   c) CDR-L3 having the sequence Q-Q-Y-R-X-X2-P-F-T, wherein X is S, V, Y, L, T or Q; X2 is F, S, Y or G.

Described herein is an antibody comprising:
i. a heavy chain variable domain, comprising:
   a) CDR-H1 having the sequence G-T-F-X-X2-Y-X3-I-S, wherein X is S or N; X2 is E or S; X3 is V or P
   b) CDR-H2 having the sequence G-I-I-P-X-X2-G-T-A-X3-Y-A-Q-K-F-Q-G, wherein X is G, S, I or Y; X2 is A, V or S; X3 is N, I or V ; and
   c) CDR-H3 having the sequence A-R-G-R-H-X-H-E-T, wherein X is S, G, or F; and
ii. a light chain variable domain, comprising:
   a) CDR-L1 having the sequence R-A-S-Q-G-I-S-S-W-L-A;
   b) CDR-L2 having the sequence A-A-S-S-L-Q-S; and
   c) CDR-L3 having the sequence Q-Q-X-Y-X2-X3-P-W-T, wherein X is V or L; X2 is S or R; X3 is L, I or F.

Described herein is an antibody comprising:
i. a heavy chain variable domain, comprising:
   a) CDR-H1 having the sequence F-T-F-X-X2-X3-X4-M-S, wherein X is G, S, P or D or S; X2 is N, S or P; X3 is T, V or Y; X4 is A, H or I;
   b) a CDR-H2 having the sequence A-I-S-X-X2-X3-X4-X5-T-X6-Y-A-D-S-V-K-G, wherein X is G or A; X2 is N, T, E or S; X3 is A or G; X4 is A or G; X5 is S or G; X6 is Y or F; and
   c) a CDR-H3 having the sequence A-K-G-P-D-R-Q-V-F-D-Y; and
ii. a light chain variable domain, comprising:
   a) a CDR-L1 having the sequence R-A-S-Q-G-I-X-S-W-L-A, wherein X is S or D;
   b) a CDR-L2 having the sequence A-A-S-S-L-Q-S; and
   c) a CDR-L3 having the sequence Q-H-X-X2-X3-F-P-Y-T, wherein X is A or G; X2 is Y, R or L; X3 is S, R, L or F.

Described herein is an antibody comprising:
i. a heavy chain variable domain, comprising:
   a) CDR-H1 having the sequence G-S-I-X-S-X2-X3-Y-X4-W-X5, wherein X is S or F; X2 is S or P; X3 is E or D; X4 is G,P or Y; X5 is G or S;
   b) CDR-H2 having the sequence X-I-X2-X3-X4-G-X5-T-Y-Y-N-P-S-L-K-S, wherein X is S or V; X2 is Y, V, F or A; X3 is Y,F or N; X4 is Q, A or S; X5 is S, A or N; and
   c) CDR-H3 having the sequence A-R-G-P-X-R-Q-X2-F-D-Y, wherein X is Y, H or F; X2 is V or L; and
ii. a light chain variable domain, comprising:
   a) CDR-L1 having the sequence R-A-S-Q-G-I-S-S-W-L-A;
      a) CDR-L2 having the sequence A-A-S-S-L-Q-S; and
   b) CDR-L3 having the sequence Q-Q-G-X-X2-F-P-Y-T, wherein X is S N, W or F; X2 is S or V.

The anti-IL18-BP antibodies also comprise framework regions. The framework regions of the variable heavy and variable light chains can be humanized as is known in the art (with occasional variants generated in the CDRs as needed), and thus humanized variants of the VH and VL chains of Figures 1, 2, and/or 3 can be generated. Furthermore, the humanized variable heavy and light domains can then be fused with human constant regions, such as the constant regions from IgG1, IgG2, IgG3 and IgG4.

In addition, also included are sequences that may have the identical CDRs but changes in the variable domain (or entire heavy or light chain). For example, IL18-BP antibodies include those with CDRs identical to those shown in Figure 1-3 but whose identity along the variable region can be lower, for example 85%, 88%, 90%, 92%, 95 or 98% percent identical. For example, IL18-BP antibodies include those with CDRs identical to those shown in Figure 3 but whose identity along the variable region can be lower, for example 95 or 98% percent identical, and in some embodiments at least 95% or at least 98%.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol. Biol. 48:444-453 (1970)) algorithm which has been incorporated into the GAP program in the GCG software package (available commercially), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J. Mol. Biol. 215:403-10. BLAST protein searches can be performed with the XBLAST program, score=50, wordlength=3 to obtain amino acid sequences homologous to the antibody molecules according to at least some embodiments of the invention. To obtain gapped alignments for comparison purposes, Gapped BLAST can be utilized as described in Altschul et al., (1997) Nucleic Acids Res. 25(17):3389-3402. When utilizing BLAST and Gapped BLAST programs, the default parameters of the respective programs (e.g., XBLAST and NBLAST) can be used.

In general, the percentage identity for comparison between IL18-BP antibodies is at least 75%, at least 80%, at least 90%, with at least about 95, 96, 97, 98 or 99% percent identity being preferred. The percentage identity may be along the whole amino acid sequence, for example the entire heavy or light chain or along a portion of the chains. For example, included within the definition of the anti-IL18-BP antibodies of the invention are those that share identity along the entire variable region (for example, where the identity is 95 or 98% identical along the variable regions, and in some embodiments at least 95% or at least 98%), or along the entire constant region, or along just the Fc domain.

### B. Specific anti-IL18-BP antibodies

The invention relates to antigen binding domains, including full length antibodies, which contain a number of specific, enumerated sets of 6 CDRs, as recited in the claims. Described herein are antigen binding domains, including full length antibodies, which contain consensus CDRs (*see, e.g.,* those listed in Figure 1A-1D).

The antibodies described herein are labeled as follows. The antibodies have reference numbers, for example "66650 lineage (VH1-03; VL-kappa-1-5)" or "VH1-03" or "ADI-71663 hIgG4 S228P kappa". This represents the combination of the CDRs and/or the variable heavy and variable light chains, as depicted in Figures 1, 2, and/or 3. "ADI-71663.VH" refers to the variable heavy portion of ADI-71663 hIgG4 S228P kappa, while "ADI-71663.VL" is the variable light chain. "ADI-71663.vhCDR1", "ADI-71663.vhCDR2", "ADI-71663.vhCDR3", "ADI-71663.vlCDR1", "ADI-71663.vICDR2", and "ADI-71663.vICDR3", refers to the CDRs are indicated. "ADI-71663.HC" refers to the entire heavy chain *(e.g.,* variable and constant domain) of this molecule, and "ADI-71663.LC" refers to the entire light chain (*e.g*., variable and constant domain) of the same molecule.

The invention further provides variable heavy and light domains for the anti-IL18-BP antibodies of the invention, as defined in the claims, as well as full length heavy and light chains for the anti-IL18-BP antibodies of the invention, as defined in the claims.

In many embodiments, the antibodies of the invention are human (derived from phage) and block IL18-BP. Figures 1A-1D and 2A-2P show anti-IL18-BP antibodies, with their components outlined as well:
CDR-H1, CDR-H2, CDR-H3, CDR-L1, CDR-L2, and CDR-L3 from 66650 lineage (VH1-03; VL-kappa-1-5);
CDR-H1, CDR-H2, CDR-H3, CDR-L1, CDR-L2, and CDR-L3 from 66670 lineage (VH1-69; VL-kappa-1-12);
CDR-H1, CDR-H2, CDR-H3, CDR-L1, CDR-L2, and CDR-L3 from 66692 lineage (VH3-23, VL-kappa-1-12);
CDR-H1, CDR-H2, CDR-H3, CDR-L1, CDR-L2, and CDR-L3 from 66716 lineage (VH1-39; VL-kappa-1-12);
ADI-71663, ADI-71663.VH, ADI-71663.VL, ADI-71663.HC, ADI-71663.LC and ADI-71663.H1, ADI-71663.H2, ADI-71663.H3, ADI-71663.H4; ADI-71663.vhCDR1, ADI-71663.vhCDR2, ADI-71663.vhCDR3, ADI-71663.vlCDR1, ADI-71663.vlCDR2, and ADI-71663.vlCDR3;
ADI-71662, ADI-71662.VH, ADI-71662.VL, ADI-71662.HC, ADI-71662.LC and ADI-71662.H1, ADI-71662.H2, ADI-71662.H3, ADI-71662.H4; ADI-71662.vhCDR1, ADI-71664. 71662, ADI-71662.vhCDR3, ADI-71662.vlCDR1, ADI-71662.vlCDR2, and ADI-71662.vlCDR3;
ADI-71701, ADI-71701.VH, ADI- 71701.VL, ADI- 71701.HC, ADI- 71701.LC and ADI-71701.H1, ADI-71701.H2, ADI-71701.H3, ADI-71701.H4; ADI- 71701.vhCDR1, ADI-71701.vhCDR2, ADI- 71701.vhCDR3, ADI- 71701.vlCDR1, ADI- 71701.vlCDR2, and ADI- 71701.vlCDR3;
ADI-71709, ADI- 71709.VH, ADI- 71709.VL, ADI- 71709.HC, ADI- 71709.LC and ADI-71709.H1, ADI-71709.H2, ADI-71709.H3, ADI-71709.H4; ADI- 71709.vhCDR1, ADI-71709.vhCDR2, ADI-71709.vhCDR3, ADI-71709.vlCDR1, ADI- 71709.vlCDR2, and ADI-71709.vlCDR3;
ADI-71710, ADI-71710.VH, ADI-71710.VL, ADI-71710.HC, ADI- 71710.LC and ADI- 71710.H1, ADI-71710.H2, ADI- 71710.H3, ADI-71710.H4; ADI- 71710.vhCDR1, ADI-71710.vhCDR2, ADI-71710.vhCDR3, ADI-71710.vlCDR1, ADI- 71710.vlCDR2, and ADI- 71710.vlCDR3;
ADI-71719, ADI-71719.VH, ADI-71719.VL, ADI-71719.HC, ADI- 71719.LC and ADI-71719.H1, ADI-71719.H2, ADI-71719.H3, ADI-71719.H4; ADI- 71719.vhCDR1, ADI-71719.vhCDR2, ADI- 71719.vhCDR3, ADI-71719.vlCDR1, ADI- 71719.vlCDR2, and ADI-71719.vlCDR3;
ADI-71720, ADI-71720.VH, ADI-71720.VL, ADI- 71720.HC, ADI- 71720.LC and ADI-71720.H1, ADI-71720.H2, ADI-71720.H3, ADI- 71720.H4; ADI- 71720.vhCDR1, ADI-71720.vhCDR2, ADI-71720.vhCDR3, ADI-71720.vlCDR1, ADI- 71720.vlCDR2, and ADI-71720.vlCDR3;
ADI-71722, ADI-71722.VH, ADI-71722.VL, ADI-71722.HC, ADI-71722.LC and ADI-71722.H1, ADI71722.H2, ADI-71722.H3, ADI-71722.H4; ADI-71722.vhCDR1, ADI-71722.vhCDR2, ADI-71722.vhCDR3, ADI-71722.vlCDR1, ADI-71722.vlCDR2, and ADI-71722.vlCDR3;
ADI-71717, ADI-71717.VH, ADI-71717.VL, ADI-71717.HC, ADI-71717.LC and ADI-71717.H1, ADI71717.H2, ADI-71717.H3, ADI-71717.H4; ADI-71717.vhCDR1, ADI-71717.vhCDR2, ADI-71717.vhCDR3, ADI-71717.vlCDR1, ADI-71717.vlCDR2, and ADI-71717.vlCDR3;
ADI-71739, ADI-71739.VH, ADI-71739.VL, ADI-71739.HC, ADI-71739.LC and ADI-71739.H1, ADI71739.H2, ADI-71739.H3, ADI-71739.H4; ADI-71739.vhCDR1, ADI-71739.vhCDR2, ADI-71739.vhCDR3, ADI-71739.vlCDR1, ADI-71739.vlCDR2, and ADI-71739.vlCDR3;
ADI-71736, ADI-71736.VH, ADI-71736.VL, ADI-71736.HC, ADI-71736.LC and ADI-71736.H1, ADI71736.H2, ADI-71736.H3, ADI-71736.H4; ADI-71736.vhCDR1, ADI-71736.vhCDR2, ADI-71736.vhCDR3, ADI-71736.vlCDR1, ADI-71736.vlCDR2, and ADI-71736.vlCDR3;
ADI-71707, ADI-71707.VH, ADI-71707.VL, ADI-71707.HC, ADI-71707.LC and ADI-71707.H1, ADI-71707.H2, ADI-71707.H3, ADI-71707.H4; ADI-71707.vhCDR1, ADI-71707.vhCDR2, ADI-71707.vhCDR3, ADI-71707.vlCDR1, ADI-71707.vlCDR2, and ADI-71707.vlCDR3;
AB-837, AB-837.VH, AB-837.VL, AB-837.HC, AB-837.LC and AB-837..H1, AB-837.H2, AB-837.H3, AB-837.H4; AB-837.vhCDR1, AB-837.vhCDR2, AB-837.vhCDR3, AB-837.vlCDR1, AB-837.vlCDR2, and AB-837.vlCDR3;
ADI-66692, ADI-66692.VH, ADI-66692.VL, ADI-66692.HC, ADI-66692.LC and ADI-66692.H1, ADI-66692.H2, ADI-66692.H3, ADI-66692.H4; ADI-66692.vhCDR1, ADI-66692.vhCDR2, ADI-66692.vhCDR3, ADI-66692.vlCDR1, ADI-66692.vlCDR2, and ADI-66692.vlCDR3;
ADI-66716, ADI-66716.VH, ADI-66716.VL, ADI-66716.HC, ADI-66716.LC and ADI-66716.H1, ADI-66716.H2, ADI-66716.H3, ADI-66716.H4; ADI-66716.vhCDR1, ADI-66716.vhCDR2, ADI-66716.vhCDR3, ADI-66716.vlCDR1, ADI-66716.vlCDR2, and ADI-66716.vlCDR3;
ADI-71728, ADI-71728.VH, ADI-71728.VL, ADI-71728.HC, ADI-71728.LC and ADI-71728.H1, ADI-71728.H2, ADI-71728.H3, ADI-71728.H4; ADI-71728.vhCDR1, ADI-71728.vhCDR2, ADI-71728.vhCDR3, ADI-71728.vlCDR1, ADI-71728.vlCDR2, and ADI-71728.vlCDR3; or
ADI-71741, ADI-71741.VH, ADI-71741.VL, ADI-71741.HC, ADI-71741.LC and ADI- 71741.H1, ADI71741.H2, ADI-71741.H3, ADI-71741.H4; ADI-71741.vhCDR1, ADI-71741.vhCDR2, ADI-71741.vhCDR3, ADI-71741.vlCDR1, ADI-71741.vlCDR2, and ADI-71741.vlCDR3;
ADI-71742, ADI-71742.VH, ADI-71742.VL, ADI-71742.HC, ADI-71742.LC and ADI-71742.H1, ADI71742.H2, ADI-71742.H3, ADI-71742.H4; ADI-71742.vhCDR1, ADI-71742.vhCDR2, ADI-71742.vhCDR3, ADI-71742.vlCDR1, ADI-71742.vlCDR2, and ADI-71742.vlCDR3;
ADI-71744, ADI-71744.VH, ADI-71744.VL, ADI-71744.HC, ADI-71744.LC and ADI-71744.H1, ADI71744.H2, ADI-71744.H3, ADI-71744.H4; ADI-71744.vhCDR1, ADI-71744.vhCDR2, ADI-71744.vhCDR3, ADI-71744.vlCDR1, ADI-71744.vlCDR2, and ADI-71744.vlCDR3;
ADI-71753, ADI-71753.VH, ADI-71753.VL, ADI-71753.HC, ADI-71753.LC and ADI-71753.H1, ADI71753.H2, ADI-71753.H3, ADI-71753.H4; ADI-71753.vhCDR1, ADI-71753.vhCDR2, ADI-71753.vhCDR3, ADI-71753.vlCDR1, ADI-71753.vlCDR2, and ADI-71753.vlCDR3; or
ADI71755, ADI-71755.VH, ADI-71755.VL, ADI-71755.HC, ADI-71755.LC and ADI-71755.H1, ADI71755.H2, ADI-71755.H3, ADI-71755.H4; ADI-71755.vhCDR1, ADI-71755.vhCDR2, ADI-71755.vhCDR3, ADI-71755.vlCDR1, ADI-71755.vlCDR2, and ADI-71755.vlCDR3.

### A. IL18-BP Antibodies that Compete for binding with Enumerated Antibodies

Described herein are not only the enumerated antibodies but additional antibodies that compete with the enumerated antibodies (the VH and ADI numbers as enumerated herein that specifically bind to IL18-BP) to specifically bind to the IL18-BP molecule. The IL18-BP antibodies described herein include antibodies that compete for binding with one or more of the enumerated antibodies, including VH1-03.66650, VH1-69.66670, VH3-23.66692, VH1-39.66716, VL-kappa-1-5-66650, VL-kappa-1-12, 66670, VL-kappa-1-12, 66692, VL-kappa-1-12, ADI-71663, ADI-71662, ADI-66692, ADI-71701, ADI-71709, ADI-71710, ADI-71707, ADI-71717, ADI-71719, ADI-71220, ADI-71722, ADI-71736, ADI-71739, ADI-71728, ADI-66716, ADI-71741, ADI-71742, ADI-71744, ADI-71753, or ADI-71755.

### B. Generation of Additional Antibodies

Additional antibodies to human IL18-BP can be done as is well known in the art, using well known methods such as those outlined in the examples. Thus, additional anti-IL18-BP antibodies can be generated by traditional methods such as immunizing mice (sometimes using DNA immunization, for example, such as is used by Aldevron), followed by screening against IL18-BP (including human IL18-BP) protein and hybridoma generation, with antibody purification and recovery.

### C. Optional Antibody Engineering

The anti-IL18-BP antibodies herein (*e.g.,* anti-IL18-BP antibodies including those with CDRs identical to those shown in Figures 1, 2, and/or 3) can be modified, or engineered, to alter the amino acid sequences by amino acid substitutions.

By "amino acid substitution" or "substitution" herein is meant the replacement of an amino acid at a particular position in a parent polypeptide sequence with a different amino acid. In particular, in some embodiments, the substitution is to an amino acid that is not naturally occurring at the particular position, either not naturally occurring within the organism or in any organism. For example, the substitution E272Y refers to a variant polypeptide, in this case an Fc variant, in which the glutamic acid at position 272 is replaced with tyrosine. For clarity, a protein which has been engineered to change the nucleic acid coding sequence but not change the starting amino acid (for example exchanging CGG (encoding arginine) to CGA (still encoding arginine) to increase host organism expression levels) is not an "amino acid substitution"; that is, despite the creation of a new gene encoding the same protein, if the protein has the same amino acid at the particular position that it started with, it is not an amino acid substitution.

As discussed herein, amino acid substitutions can be made to alter the affinity of the CDRs for the IL18-BP protein (including both increasing and decreasing binding, as is more fully outlined below), as well as to alter additional functional properties of the antibodies. For example, the antibodies may be engineered to include modifications within the Fc region, typically to alter one or more functional properties of the antibody, such as serum half-life, complement fixation, Fc receptor binding, and/or antigen-dependent cellular cytotoxicity. Furthermore, an antibody according to at least some embodiments of the invention may be chemically modified (*e.g.,* one or more chemical moieties can be attached to the antibody) or be modified to alter its glycosylation, again to alter one or more functional properties of the antibody. Such embodiments are described further below. The numbering of residues in the Fc region is that of the EU index of Kabat.

In some embodiments, the hinge region of C_{H1} is modified such that the number of cysteine residues in the hinge region is altered, *e.g*., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired *Staphylococcyl* protein A (SpA) binding relative to native Fc-hinge domain SpA binding. This approach is described in further detail in U.S. Pat. No. 6,165,745 by Ward et al.

In some embodiments, amino acid substitutions can be made in the Fc region, in general for altering binding to FcγR receptors. By "Fc gamma receptor", "FcγR" or "FcgammaR" as used herein is meant any member of the family of proteins that bind the IgG antibody Fc region and is encoded by an FcγR gene. In humans this family includes but is not limited to FcγRI (CD64), including isoforms FcγRIa, FcγRIb, and FcγRIc; FcγRII (CD32), including isoforms FcγRIIa (including allotypes H131 and R131), FcγRIIb (including FcγRIIb-1 and FcγRIIb-2), and FcγRIIc; and FcγRIII (CD16), including isoforms FcγRIIIa (including allotypes V158 and F158) and FcγRIIIb (including allotypes FcγRIIIb-NA1 and FcγRIIIb-NA2) (Jefferis et al., 2002, Immunol Lett 82:57-65), as well as any undiscovered human FcγRs or FcγR isoforms or allotypes. An FcγR may be from any organism, including but not limited to humans, mice, rats, rabbits, and monkeys. Mouse FcγRs include but are not limited to FcγRI (CD64), FcγRII (CD32), FcγRIII-1 (CD16), and FcγRIII-2 (CD16-2), as well as any undiscovered mouse FcγRs or FcγR isoforms or allotypes.

There are a number of useful Fc substitutions that can be made to alter binding to one or more of the FcγR receptors. Substitutions that result in increased binding as well as decreased binding can be useful. For example, it is known that increased binding to FcγRIIIa generally results in increased ADCC (antibody dependent cell-mediated cytotoxicity; the cell-mediated reaction wherein nonspecific cytotoxic cells that express FcγRs recognize bound antibody on a target cell and subsequently cause lysis of the target cell. Similarly, decreased binding to FcγRIIb (an inhibitory receptor) can be beneficial as well in some circumstances. Amino acid substitutions that find use in the present invention include those listed in U.S. 2006/0024298 A1 (particularly FIG. 41) and U.S. Patent No. 6,737,056. Particular variants that find use include, but are not limited to, 236A, 239D, 239E, 332E, 332D, 239D/332E, 267D, 267E, 328F, 267E/328F, 236A/332E, 239D/332E/330Y, 239D, 332E/330L, 299T and 297N.

In addition, the antibodies of the invention are modified to increase its biological half-life. Various approaches are possible. For example, one or more of the following mutations can be introduced: T252L, T254S, T256F, as described in U.S. Pat. No. 6,277,375 to Ward. Alternatively, to increase the biological half-life, the antibody can be altered within the C_{H1} or C_{L} region to contain a salvage receptor binding epitope taken from two loops of a CH2 domain of an Fc region of an IgG, as described in U.S. Pat. Nos. 5,869,046 and 6,121,022 by Presta et al. Additional mutations to increase serum half-life are disclosed in U.S. Patent Nos. 8,883,973, 6,737,056 and 7,371,826, and include 428L, 434A, 434S, and 428L/434S.

In yet other embodiments, the Fc region is altered by replacing at least one amino acid residue with a different amino acid residue to alter the effector functions of the antibody. For example, one or more amino acids selected from amino acid residues 234, 235, 236, 237, 297, 318, 320 and 322 can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another example, one or more amino acids selected from amino acid residues 329, 331 and 322 can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another example, one or more amino acid residues within amino acid positions 231 and 239 are altered to thereby alter the ability of the antibody to fix complement. This approach is described further in PCT Publication WO 94/29351 by Bodmer et al.

In yet another example, the Fc region is modified to increase the ability of the antibody to mediate antibody dependent cellular cytotoxicity (ADCC) and/or to increase the affinity of the antibody for an Fcy receptor by modifying one or more amino acids at the following positions: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 329, 330, 331, 333, 334, 335, 337, 338, 340, 360, 373, 376, 378, 382, 388, 389, 398, 414, 416, 419, 430, 434, 435, 437, 438 or 439. This approach is described further in PCT Publication WO 00/42072 by Presta. Moreover, the binding sites on human IgG1 for FcyRI, FcyRII, FcyRIII and FcRn have been mapped and variants with improved binding have been described (see Shields, R. L. et al. (2001) J. Biol. Chem. 276:6591-6604). Specific mutations at positions 256, 290, 298, 333, 334 and 339 are shown to improve binding to FcyRIII. Additionally, the following combination mutants are shown to improve FcγRIII binding: T256A/S298A, S298A/E333A, S298A/K224A and S298A/E333A/K334A. Furthermore, mutations such as M252Y/S254T/T256E or M428L/N434S improve binding to FcRn and increase antibody circulation half-life (see Chan CA and Carter PJ (2010) Nature Rev Immunol 10:301-316).

In still another embodiment, the antibody can be modified to abrogate *in vivo* Fab arm exchange. Specifically, this process involves the exchange of IgG4 half-molecules (one heavy chain plus one light chain) between other IgG4 antibodies that effectively results in bispecific antibodies which are functionally monovalent. Mutations to the hinge region and constant domains of the heavy chain can abrogate this exchange (*see,* Aalberse, RC, Schuurman J., 2002, Immunology 105:9-19).

In still another embodiment, the glycosylation of an antibody is modified. For example, an aglycosylated antibody can be made (*i.e.,* the antibody lacks glycosylation). Glycosylation can be altered to, for example, increase the affinity of the antibody for antigen or reduce effector function such as ADCC. Such carbohydrate modifications can be accomplished by, for example, altering one or more sites of glycosylation within the antibody sequence, for example N297. For example, one or more amino acid substitutions can be made that result in elimination of one or more variable region framework glycosylation sites to thereby eliminate glycosylation at that site.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies according to at least some embodiments of the invention to thereby produce an antibody with altered glycosylation. For example, the cell lines Ms704, Ms705, and Ms709 lack the fucosyltransferase gene, FUT8 (α (1,6) fucosyltransferase), such that antibodies expressed in the Ms704, Ms705, and Ms709 cell lines lack fucose on their carbohydrates. The Ms704, Ms705, and Ms709 FUT8 cell lines are created by the targeted disruption of the FUT8 gene in CHO/DG44 cells using two replacement vectors (see U.S. Patent Publication No. 20040110704 by Yamane et al. and Yamane-Ohnuki et al. (2004) Biotechnol Bioeng 87:614-22). As another example, EP 1,176,195 by Hanai et al. describes a cell line with a functionally disrupted FUT8 gene, which encodes a fucosyl transferase, such that antibodies expressed in such a cell line exhibit hypofucosylation by reducing or eliminating the α 1,6 bond-related enzyme. Hanai et al. also describe cell lines which have a low enzyme activity for adding fucose to the N-acetylglucosamine that binds to the Fc region of the antibody or does not have the enzyme activity, for example the rat myeloma cell line YB2/0 (ATCC CRL 1662). PCT Publication WO 03/035835 by Presta describes a variant CHO cell line, Lec13 cells, with reduced ability to attach fucose to Asn(297)-linked carbohydrates, also resulting in hypofucosylation of antibodies expressed in that host cell (see also Shields, R. L. et al. (2002) J. Biol. Chem. 277:26733-26740). PCT Publication WO 99/54342 by Umana et al. describes cell lines engineered to express glycoprotein-modifying glycosyl transferases (*e.g*., β(1,4)-N-acetylglucosaminyltransferase III (GnTIII)) such that antibodies expressed in the engineered cell lines exhibit increased bisecting GlcNac structures which results in increased ADCC activity of the antibodies (see also Umana et al. (1999) Nat. Biotech. 17:176-180). Alternatively, the fucose residues of the antibody may be cleaved off using a fucosidase enzyme. For example, the fucosidase α-L-fucosidase removes fucosyl residues from antibodies (Tarentino, A. L. et al. (1975) Biochem. 14:5516-23).

Another modification of the antibodies herein that is contemplated by the invention is pegylation or the addition of other water-soluble moieties, typically polymers, *e.g*., in order to enhance half-life. An antibody can be pegylated to, for example, increase the biological (*e.g*., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG groups become attached to the antibody or antibody fragment. Preferably, the pegylation is carried out via an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C₁-C₁₀) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies according to at least some embodiments of the invention. See for example, EP 0 154 316 by Nishimura et al. and EP 0 401 384 by Ishikawa et al.

In addition to substitutions made to alter binding affinity to FcγRs and/or FcRn and/or increase in vivo serum half-life, additional antibody modifications can be made, as described in further detail below.

In some cases, affinity maturation is done. Amino acid modifications in the CDRs are sometimes referred to as "affinity maturation". An "affinity matured" antibody is one having one or more alteration(s) in one or more CDRs which results in an improvement in the affinity of the antibody for antigen, compared to a parent antibody which does not possess those alteration(s). In some cases, although rare, it may be desirable to decrease the affinity of an antibody to its antigen, but this is generally not preferred.

In some instances, one or more amino acid modifications can be made in one or more of the CDRs of the IL18-BP antibodies disclosed herein. In general, only 1 or 2 or 3-amino acids are substituted in any single CDR, and generally no more than from 1, 2, 3, 4, 5, 6, 7, 8 9 or 10 changes are made within a set of CDRs. However, it should be appreciated that any combination of no substitutions, 1, 2 or 3 substitutions in any CDR can be independently and optionally combined with any other substitution.

Affinity maturation can be done to increase the binding affinity of the antibody for the IL18-BP antigen by at least about 100% or more, or at least about 10⁴ or more, 10⁵ or more, 10⁶ or more, 10⁷ or more, as compared to the "parent" antibody. Preferred affinity matured antibodies will have nanomolar or even picomolar affinities for the IL18-BP antigen. Affinity matured antibodies are produced by known procedures. See, for example, Marks et al., 1992, Biotechnology 10:779-783 that describes affinity maturation by variable heavy chain (VH) and variable light chain (VL) domain shuffling. Random mutagenesis of CDR and/or framework residues is described in: Barbas, et al., PNAS, USA 91:3809-3813 (1994); Shier et al., Gene, 169:147-155 (1995); Yelton et al., J. Immunol., 155: 1994-2004 (1995); Jackson et al., J. Immunol. 154(7):3310-9 (1995); and Hawkins et al., J. Mol. Biol., 226:889-896 (1992), for example.

Alternatively, amino acid modifications can be made in one or more of the CDRs of the antibodies of the invention that are "silent", *e.g.* that do not significantly alter the affinity of the antibody for the antigen. These can be made for a number of reasons, including optimizing expression (as can be done for the nucleic acids encoding the antibodies of the invention).

Thus, included within the definition of the CDRs and antibodies disclosed herein are variant CDRs and antibodies; that is, the antibodies disclosed herein can include amino acid modifications in one or more of the CDRs of the enumerated antibodies disclosed herein. In addition, as outlined below, amino acid modifications can also independently and optionally be made in any region outside the CDRs, including framework and constant regions.

### III. NUCLEIC ACID COMPOSITIONS

Nucleic acid compositions encoding the anti-IL18-BP antibodies of the invention are also described, as well as expression vectors containing the nucleic acids and host cells transformed with the nucleic acid and/or expression vector compositions. As will be appreciated by those in the art, the protein sequences depicted herein can be encoded by any number of possible nucleic acid sequences, due to the degeneracy of the genetic code.

The nucleic acid compositions that encode the IL18-BP antibodies will depend on the format of the antibody. For traditional, tetrameric antibodies containing two heavy chains and two light chains are encoded by two different nucleic acids, one encoding the heavy chain and one encoding the light chain. These can be put into a single expression vector or two expression vectors, as is known in the art, transformed into host cells, where they are expressed to form the antibodies of the invention. In some embodiments, for example when scFv constructs are used, a single nucleic acid encoding the variable heavy chain-linker-variable light chain is generally used, which can be inserted into an expression vector for transformation into host cells. The nucleic acids can be put into expression vectors that contain the appropriate transcriptional and translational control sequences, including, but not limited to, signal and secretion sequences, regulatory sequences, promoters, origins of replication, selection genes, etc.

Preferred mammalian host cells for expressing the recombinant antibodies according to at least some embodiments of the invention include Chinese Hamster Ovary (CHO cells), PER.C6, HEK293 and others as is known in the art.

The nucleic acids may be present in whole cells, in a cell lysate, or in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art.

To create a scFv gene, the V_{H}- and V_{L}-encoding DNA fragments are operatively linked to another fragment encoding a flexible linker, *e.g*., encoding the amino acid sequence (Gly₄-Ser)₃ (SEQ ID NO: 150), such that the V_{H} and V_{L} sequences can be expressed as a contiguous single-chain protein, with the V_{L} and V_{H} regions joined by the flexible linker (*see, e.g.,* Bird et al., Science 242:423-426 (1988); Huston et al. PNAS, 85:5879-5883 (1988); McCafferty et al., Nature 348:552-554 (1990)).

### IV. ADMINISTRATION OF FORMULATIONS OF ANTI-IL18-BP ANTIBODIES

Administration of the pharmaceutical composition comprising the anti-IL18-BP antibodies herein (e.g., anti-IL18-BP antibodies including those with CDRs identical to those shown in Figures 1, 2, and/or 3), preferably in the form of a sterile aqueous solution, may be done in a variety of ways. As is known in the art, protein therapeutics are often delivered by IV infusion. The antibodies of the present invention may also be delivered using such methods. For example, administration may be veinous or by intravenous infusion with 0.9% sodium chloride as an infusion vehicle. Such techniques are disclosed in Remington's Pharmaceutical Sciences 16th edition, Osol, A. Ed., 1980.

The dosing amounts and frequencies of administration are, in some embodiments, selected to be therapeutically or prophylactically effective. As is known in the art, adjustments for protein degradation, systemic versus localized delivery, and rate of new protease synthesis, as well as the age, body weight, general health, sex, diet, time of administration, drug interaction and the severity of the condition may be necessary, and will be ascertainable with routine experimentation by those skilled in the art. In order to treat a patient, a therapeutically effective dose of the Fc variant of the present invention may be administered. By "therapeutically effective dose" herein is meant a dose that produces the effects for which it is administered.

### V. METHODS OF USING THE ANTI-IL18-BP ANTIBODY

### A. Therapeutic Uses

The anti-IL18-BP antibodies (*e.g*., anti-IL18-BP antibodies including those described in Figures 1, 2, and/or 3) find use in treating patients, such as human subjects, generally with a condition associated with IL18-BP or free IL18 levels. The term "treatment" as used herein, refers to both therapeutic treatment and prophylactic or preventative measures, which in this example relates to treatment of cancer. Those in need of treatment include those already with cancer as well as those in which the cancer is to be prevented. Hence, the mammal to be treated herein may have been diagnosed as having the cancer or may be predisposed or susceptible to the cancer. As used herein the term "treating" refers to preventing, delaying the onset of, curing, reversing, attenuating, alleviating, minimizing, suppressing, halting the deleterious effects or stabilizing of discernible symptoms of the above-described cancerous diseases, disorders or conditions. It also includes managing the cancer as described above. By "manage" it is meant reducing the severity of the disease, reducing the frequency of episodes of the disease, reducing the duration of such episodes, reducing the severity of such episodes, slowing/reducing cancer cell growth or proliferation, slowing progression of at least one symptom, amelioration of at least one measurable physical parameter and the like. For example, immunostimulatory anti-IL18-BP immune molecules should promote T cells, NK cells, NKT cells, Myeloid cells, Dendritic cells, MAIT T cells, γδ T cells, and/or innate lymphoid cells (ILCs), or cytokine immunity against target cells, *e.g.,* cancer, infected or pathogen cells and thereby treat cancer or infectious diseases by depleting the cells involved in the disease condition.

The IL18-BP antibodies of the invention are provided in therapeutically effective dosages. A "therapeutically effective dosage" of an anti-IL18-BP immune molecule according to at least some embodiments of the present invention preferably results in a decrease in severity of disease symptoms, an increase in frequency and duration of disease symptom-free periods, an increase in lifespan, disease remission, or a prevention or reduction of impairment or disability due to the disease affliction. For example, for the treatment of IL18-BP positive tumors, a "therapeutically effective dosage" preferably inhibits cell growth or tumor growth by at least about 20%, more preferably by at least about 40%, even more preferably by at least about 60%, and still more preferably by at least about 80% relative to untreated subjects. The ability of a compound to inhibit tumor growth can be evaluated in an animal model system predictive of efficacy in human tumors. Alternatively, this property of a composition can be evaluated by examining the ability of the compound to inhibit, such inhibition *in vitro* by assays known to the skilled practitioner. A therapeutically effective amount of a therapeutic compound can decrease tumor size, or otherwise ameliorate symptoms in a subject.

One of ordinary skill in the art would be able to determine a therapeutically effective amount based on such factors as the subject's size, the severity of the subject's symptoms, and the particular composition or route of administration selected.

### 1. Cancer Treatment

The IL18-BP antibodies of the invention, alone or in combination with other therapeutic agents, find particular use in the treatment of cancer. In general, the antibodies of the invention are immunomodulatory, in that rather than directly attack cancerous cells, the anti-IL18-BP antibodies of the invention stimulate the immune system, generally by inhibiting the action of IL18-BP. Thus, unlike tumor-targeted therapies, which are aimed at inhibiting molecular pathways that are crucial for tumor growth and development, and/or depleting tumor cells, cancer immunotherapy is aimed to stimulate the patient's own immune system to eliminate cancer cells, providing long-lived tumor destruction. Various approaches can be used in cancer immunotherapy, among them are therapeutic cancer vaccines to induce tumor-specific T cell responses, and immunostimulatory antibodies (*i.e.,* antagonists of inhibitory receptors = immune checkpoints) to remove immunosuppressive pathways.

Clinical responses with targeted therapy or conventional anti-cancer therapies tend to be transient as cancer cells develop resistance, and tumor recurrence takes place. However, the clinical use of cancer immunotherapy in the past few years has shown that this type of therapy can have durable clinical responses, showing dramatic impact on long term survival. However, although responses are long term, only a small number of patients respond (as opposed to conventional or targeted therapy, where a large number of patients respond, but responses are transient).

By the time a tumor is detected clinically, it has already evaded the immune-defense system by acquiring immunoresistant and immunosuppressive properties and creating an immunosuppressive tumor microenvironment through various mechanisms and a variety of immune cells.

Accordingly, the anti-IL18-BP antibodies of the invention are useful in treating cancer. Due to the nature of an immuno-oncology mechanism of action, IL18-BP does not necessarily need to be overexpressed on or correlated with a particular cancer type; that is, the goal is to have the anti-IL18-BP antibodies de-suppress T cells, NK cells, NKT cells, Myeloid cells, Dendritic cells, MAIT T cells, γδ T cells, and/or innate lymphoid cells (ILCs)activation, such that the immune system will go after the cancers.

"Cancer," as used herein, refers broadly to any neoplastic disease (whether invasive or metastatic) characterized by abnormal and uncontrolled cell division causing malignant growth or tumor (e.g., unregulated cell growth). The term "cancer" or "cancerous" as used herein should be understood to encompass any neoplastic disease (whether invasive, non-invasive or metastatic) which is characterized by abnormal and uncontrolled cell division causing malignant growth or tumor, non-limiting examples of which are described herein. This includes any physiological condition in mammals that is typically characterized by unregulated cell growth.

"Cancer therapy" herein refers to any method that prevents or treats cancer or ameliorates one or more of the symptoms of cancer. Typically, such therapies will comprise administration of immunostimulatory anti-IL18-BP antibodies (including antigen-binding fragments) either alone or in combination with chemotherapy or radiotherapy or other biologics and for enhancing the activity thereof, *i.e.,* in individuals wherein expression of IL18-BP suppresses antitumor responses and the efficacy of chemotherapy or radiotherapy or biologic efficacy.

The anti-IL18-BP antibodies of the present invention, as a monotherapy or as part of a combination therapy as described herein, can be used in the treatment of solid tumors (including, for example, cancers of the lung, liver, breast, brain, GI tract) and blood cancers (including for example, leukemia and preleukemic disorders, lymphoma, plasma cell disorders) carcinoma, lymphoma, blastoma, sarcoma, and leukemia or lymphoid malignancies. In some embodiments, the cancer is early. In some embodiments, the cancer is advanced (including metastatic). In some embodiments, the cancers amenable for treatment of the invention include cancers that express IL18-BP and further include non-metastatic or non-invasive, as well as invasive or metastatic cancers, including cancers where IL18-BP expression by immune, stromal, or diseased cells suppresses antitumor responses and antiinvasive immune responses. In some embodiments, the anti-IL18-BP antibodies can be used for the treatment of vascularized tumors. In some embodiments, the cancer for treatment using the anti-IL18-BP antibodies of the present invention includes carcinoma, lymphoma, sarcoma, and/or leukemia. In some embodiments, the cancer for treatment using the anti-IL18-BP antibodies of the present invention includes vascularized tumors, melanoma, nonmelanoma skin cancer (squamous and basal cell carcinoma), mesothelioma, squamous cell cancer, lung cancer, small-cell lung cancer, non-small cell lung cancer, neuroendocrine lung cancer (including pleural mesothelioma, neuroendocrine lung carcinoma), NSCL (large cell), NSCLC large cell adenocarcinoma, non-small cell lung carcinoma (NSCLC), NSCLC squamous cell, soft-tissue sarcoma, Kaposi's sarcoma, adenocarcinoma of the lung, squamous carcinoma of the lung, NSCLC with PDL1 >=50% TPS, neuroendocrine lung carcinoma, atypical carcinoid lung cancer, cancer of the peritoneum, esophageal cancer, hepatocellular cancer, liver cancer (including HCC), gastric cancer, stomach cancer (including gastrointestinal cancer), pancreatic cancer, glioblastoma, cervical cancer, ovarian cancer, urothelial cancer, bladder cancer, hepatoma, glioma, brain cancer (as well as edema, such as that associated with brain tumors), breast cancer (including, for example, triple negative breast cancer), testis cancer, testicular germ cell tumors, colon cancer, colorectal cancer (CRC), colorectal cancer MSS (MSS-CRC); refractory MSS colorectal; MSS (microsatellite stable status), primary peritoneal cancer, primary peritoneal ovarian carcinoma, microsatellite stable primary peritoneal cancer, platinum resistant microsatellite stable primary peritoneal cancer, CRC (MSS unknown), rectal cancer, endometrial cancer (including endometrial carcinoma), uterine carcinoma, salivary gland carcinoma, kidney cancer, renal cell cancer (RCC), renal cell carcinoma (RCC), gastro-esophageal junction cancer, prostate cancer, vulval cancer, thyroid cancer, hepatic carcinoma, carcinoid carcinoma, head and neck cancer, B-cell lymphoma (including non-Hodgkin's lymphoma, as well as low grade/follicular non-Hodgkin's lymphoma (NHL), small lymphocytic (SL) NHL, intermediate grade/follicular NHL, intermediate grade diffuse NHL, Diffuse Large B cell lymphoma, high grade immunoblastic NHL, high grade lymphoblastic NHL, high grade small non-cleaved cell NHL, bulky disease NHL, mantle cell lymphoma, AIDS-related lymphoma, and Waldenström's Macroglobulinemia, Hodgkin's lymphoma (HD), chronic lymphocytic leukemia (CLL), acute lymphoblastic leukemia (ALL), T cell Acute Lymphoblastic Leukemia (T-ALL), Acute myeloid leukemia (AML), Hairy cell leukemia, chronic myeloblastic leukemia, multiple myeloma, post-transplant lymphoproliferative disorder (PTLD), abnormal vascular proliferation associated with phakomatoses, Meigs' syndrome, Merkel Cell cancer, MSI-high cancer, KRAS mutant tumors, adult T-cell leukemia/lymphoma, adenoid cystic cancer (including adenoid cystic carcinoma), melanoma, malignant melanoma, metastatic melanoma, pancreatic cancer, pancreatic adenocarcinoma, ovarian cancer (including ovarian carcinoma), pleural mesothelioma, cervical squamous cell carcinoma (cervical SCC), anal squamous cell carcinoma (anal SCC), carcinoma of unknown primary, gallbladder cancer, pleural mesothelioma, chordoma, endometrial sarcoma, chondrosarcoma, uterine sarcoma, uveal melanoma, amyloidosis, AL-amyloidosis, astrocytoma, and/or Myelodysplastic syndromes (MDS).

In some embodiments, the cancer for treatment using the anti-IL18-BP antibodies of the present invention includes cancer selected from the group consisting of renal clear cell carcinoma (RCC), lung cancer, NSCLC, lung adenocarcinoma, lung squamous cell carcinoma, gastric adenocarcinoma, ovarian cancer, endometrial cancer, breast cancer, triple negative breast cancer (TNBC), head and neck tumor, colorectal adenocarcinoma, melanoma, and metastatic melanoma.

### 2. Anti-IL-18BP Antibody monotherapies

The IL-18BP antibodies of the invention find particular use in the treatment of cancer as a monotherapy. Due to the nature of an immuno-oncology mechanism of action, IL 18-BP does not necessarily need to be overexpressed on or correlated with a particular cancer type; that is, the goal is to have the anti-IL18-BP antibodies de-suppress T cell and NK cell activation, such that the immune system will go after the cancers.

Any anti-IL-18 antibody of Figure 1-3 finds use as a monotherapy.

### 3. Anti-IL18BP Antibody Combination Therapies

As is known in the art, combination therapies comprising a therapeutic antibody targeting an immunotherapy target and an additional therapeutic agent, specific for the disease condition, are showing great promise. For example, in the area of immunotherapy, there are a number of promising combination therapies using a chemotherapeutic agent (either a small molecule drug or an anti-tumor antibody) or with an immuno-oncology antibody.

The terms "in combination with" and "co-administration" are not limited to the administration of said prophylactic or therapeutic agents at exactly the same time. Instead, it is meant that the antibody and the other agent or agents are administered in a sequence and within a time interval such that they may act together to provide a benefit that is increased versus treatment with only either the antibody of the present invention or the other agent or agents. It is preferred that the antibody and the other agent or agents act additively, and especially preferred that they act synergistically.

Accordingly, the antibodies of the present invention may be administered concomitantly with one or more other therapeutic regimens or agents. In some embodiments, the antibodies of the present invention are administered in the same formulation with one or more other therapeutic regimens or agents. In some embodiments, the antibodies of the present invention are administered in a separate and/or different formulation from the one or more other therapeutic regimens or agents. The additional therapeutic regimes or agents may be used to improve the efficacy or safety of the antibody. Also, the additional therapeutic regimes or agents may be used to treat the same disease or a comorbidity rather than to alter the action of the antibody. For example, an antibody of the present invention may be administered to the patient along with chemotherapy, radiation therapy, or both chemotherapy and radiation therapy.

In some embodiment, the anti-IL18 BP antibodies of the invention can be combined with one of a number of checkpoint receptor antibodies. In some embodiments, a patient's tumor may be evaluated for expression of receptors and the results then used to inform a clinician as to which antibodies to administer. Any anti-IL-18 antibody of Figure 1-3 finds use as part of a combination therapy.

### a. Immune Checkpoint Inhibitor Combination Therapies

In some embodiments, the combination or composition further comprises an additional active agent, e.g., a second antigen binding protein. Optionally, the second antigen binding protein binds to a negative regulator of the immune system, an immune suppressor, or an immune checkpoint protein, including but not limited to PD-1, PD-L1, CTLA-4, PD-L2, B7-H3, B7-H4, CEACAM-1, TIGIT, PVR, LAG3, CD112, PVRIG, CD96, TIM3, and/or BTLA, or co-stimulatory receptor: ICOS, OX40, 41BB, CD27,and/or GITR.

In some embodiments, the anti-IL18-BP antibodies are used in combination with and antibody to an immune checkpoint inhibitor protein. In some embodiments, the immune checkpoint inhibitor protein is selected from the group consisting of an anti-PVRIG antibody, an anti-PD-1 antibody, an anti-PD-L1 antibody, an anti-TIGIT antibody, an anti-CTLA-4 antibody, an anti-PD-L2 antibody, an anti-B7-H3 antibody, an anti B7-H4 antibody, an anti-CEACAM-1 antibody, an anti-PVR antibody, an anti-LAG3 antibody, an anti-CD112 antibody, an anti-CD96 antibody, an anti-TIM3 antibody, an anti-BTLA antibody, an anti-ICOS antibody, an anti-OX40 antibody, or an anti-41BB antibody, an anti-CD27 antibody, or an anti-GITR antibody.

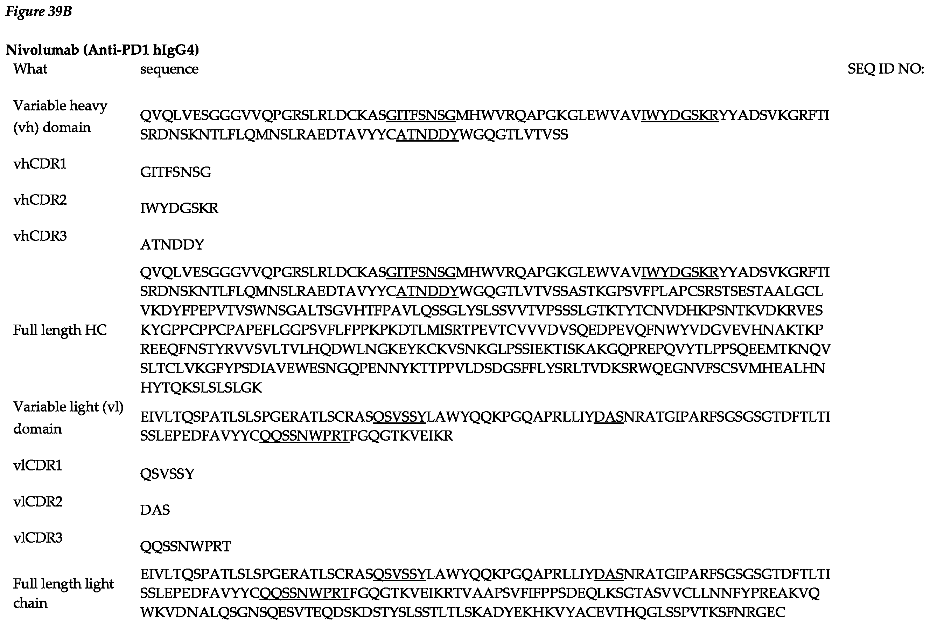

In some embodiments, the anti-IL18-BP antibodies are used in combination with one or more anti-PD-1 (e.g., anti-PD-1 targeting antibodies), including for example but not limited to nivolumab (Opdivo^{®}; BMS; CheckMate078), pembrolizumab ( KEYTRUDA^{®}; Merck), TSR-042 (Tesaro), cemiplimab (REGN2810; Regeneron Pharmaceuticals, see US20170174779), BMS-936559, Spartalizumab (PDR001, Novartis), pidilizumab (CT-011; Pfizer Inc), Tislelizumab (BGB-A317, BeiGene), Camrelizumab (SHR-1210, Incyte and Jiangsu HengRui), SHR-1210 (CTR20170299 and CTR20170322), SHR-1210 (CTR20160175 and CTR20170090), Sintilimab(Tyvyt^{®}; Eli lily and Innovent Biologics), Toripalimab (JS001, Shanghai Junshi Bioscience), JS-001 (CTR20160274), IBI308 (CTR20160735), BGB-A317 (CTR20160872), Penpulimab (AK105, Akeso Biopharma), Zimberelimab (Arcus), BAT1306 (Bio-Thera Solutions Ltd), Sasanlimab (PF-06801591, pfizer), Dostarlimab-gxly (GlaxoSmithKline LLC), Prolgolimab (Biocad), Cadonilimab (Akeso Inc), Geptanolimab (Genor BioPharma Co Ltd), Serplulimab (Shanghai Henlius Biotech Inc), Balstilimab (Agenus Inc), Retifanlimab (Incyte Corp), Cetrelimab (Johnson & Johnson), CS-1003 (EQRx Inc), IBI-318 (Innovent Biologics Inc), Ivonescimab (Akeso Inc), Pucotenlimab (Lepu Biopharma Co Ltd), QL-1604 (Qilu Pharmaceutical Co Ltd), SCTI-10A (SinoCelltech Group Ltd), Tebotelimab (MacroGenics Inc), AZD-7789 (AstraZeneca Plc), Budigalimab (AbbVie Inc), EMB-02 (EpimAb Biotherapeutics Inc), Ezabenlimab (Boehringer Ingelheim International GmbH), F-520 (Shandong New Time Pharmaceutical Co Ltd), HX-009 (Waterstone Hanxbio Pty Ltd), Zeluvalimab (Amgen), Peresolimab (Eli Lilly and Co), Rosnilimab (AnaptysBio Inc), Vudalimab (Xencor), Izuralimab (Xencor), Lorigerlimab (MacroGenics Inc), YBL-006 (Y-Biologics Inc), ONO-4685 (Ono Pharmaceutical Co Ltd), LY-3434172 (Eli Lilly and Co), and/or a PD-1 antibody as recited in US 2017/0081409 as well as others in development, which can be used in combination with the anti-IL18BP antibodies of the invention. Additional exemplary anti-PD-1 antibody sequences are shown in Figure 39.

In some embodiments, pembrolizumab is administered as a dosage of about 2 mg/kg to 10 mg/kg. In some embodiments, pembrolizumab is administered as a dosage of about 2 mg/kg. In some embodiments, pembrolizumab is administered as a dosage of about 2 mg/kg. In some embodiments, pembrolizumab is administered as a dosage of about 3 mg/kg. In some embodiments, pembrolizumab is administered as a dosage of about 4 mg/kg. In some embodiments, pembrolizumab is administered as a dosage of about 5 mg/kg. In some embodiments, pembrolizumab is administered as a dosage of about 6 mg/kg. In some embodiments, pembrolizumab is administered as a dosage of about 7 mg/kg. In some embodiments, pembrolizumab is administered as a dosage of about 8 mg/kg. In some embodiments, pembrolizumab is administered as a dosage of about 9 mg/kg. In some embodiments, pembrolizumab is administered as a dosage of about 10 mg/kg.

In some embodiments, pembrolizumab is administered as a dosage of about no more than 2 mg/kg. In some embodiments, pembrolizumab is administered as a dosage of about 1 mg/kg to 2 mg/kg. In some embodiments, pembrolizumab is administered as a dosage of about 0.1 mg/kg to 1 mg/kg. In some embodiments, pembrolizumab is administered as a dosage of about 0.01 mg/kg to 0.1 mg/kg.

In some embodiments, pembrolizumab is administered as a dosage of about at least 10 mg/kg. In some embodiments, pembrolizumab is administered as a dosage of about 10 mg/kg to 20 mg/kg. In some embodiments, pembrolizumab is administered as a dosage of about 20 mg/kg to 30 mg/kg. In some embodiments, pembrolizumab is administered as a dosage of about 30 mg/kg to 40 mg/kg. In some embodiments, pembrolizumab is administered as a dosage of about 40 mg/kg to 50 mg/kg.

In some embodiments, pembrolizumab is administered about every 1 week to every 6 weeks. In some embodiments, pembrolizumab is administered about every week. In some embodiments, pembrolizumab is administered about every 2 weeks. In some embodiments, pembrolizumab is administered about every 3 weeks. In some embodiments, pembrolizumab is administered about every 4 weeks. In some embodiments, pembrolizumab is administered about every 5 weeks. In some embodiments, pembrolizumab is administered about every 6 weeks.

In some embodiments, pembrolizumab is administered as a dosage of about 2 mg/kg every 3 weeks. In some embodiments, pembrolizumab is administered as a dosage of about 10 mg/kg every 3 weeks. In some embodiments, pembrolizumab is administered as a dosage of about 200 mg every 3 weeks. In some embodiments, pembrolizumab is administered as a dosage of about 400 mg every 6 weeks.

In some embodiments, the pembrolizumab is administered over about 10 minutes, over about 15 minutes, over about 20 minutes, over about 25 minutes, over about 30 minutes, over about 35 minutes, or over about 40 minutes. In some embodiments, the pembrolizumab is administered over about 30 minutes +/- 10 minutes.

Further disclosures of pembrolizumab are provided in https://www.accessdata.fda.gov/spl/data/157262d6-15e0-4b0a-968f-b99bab4aef50/157262d6-15e0-4b0a-968f-b99bab4aef50.xml.

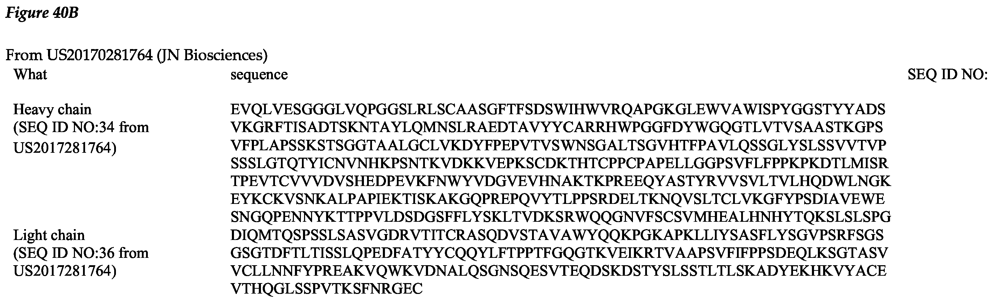

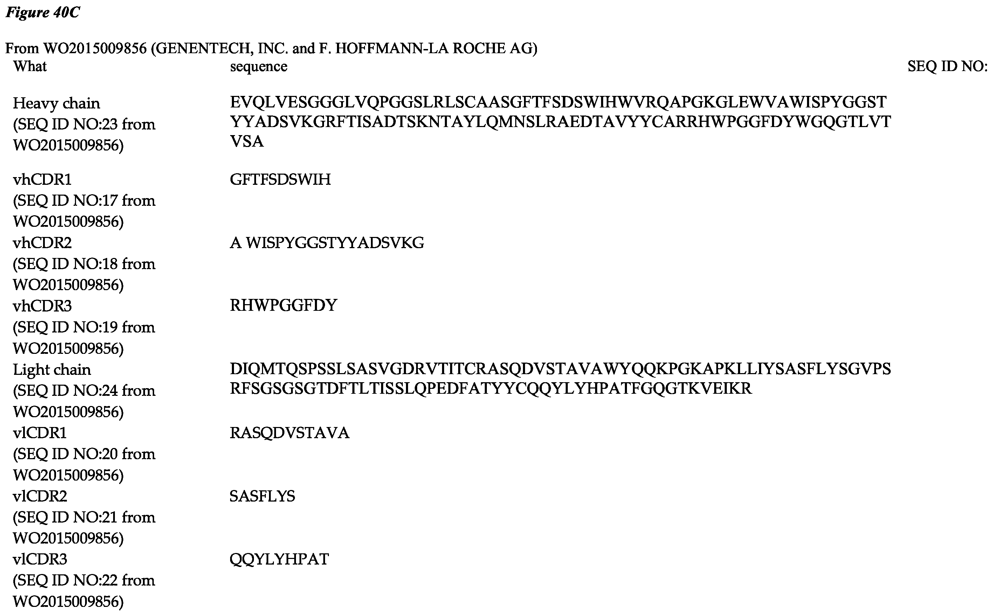

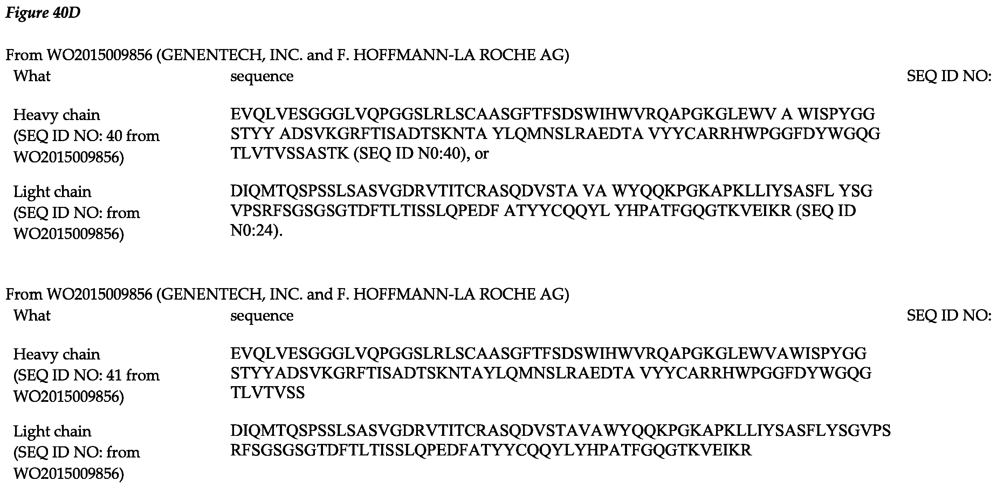

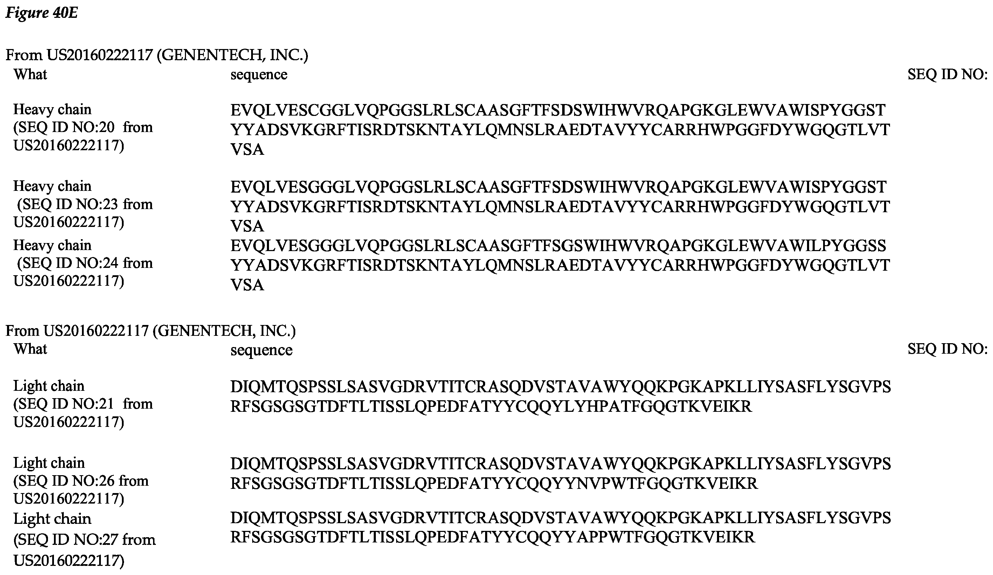

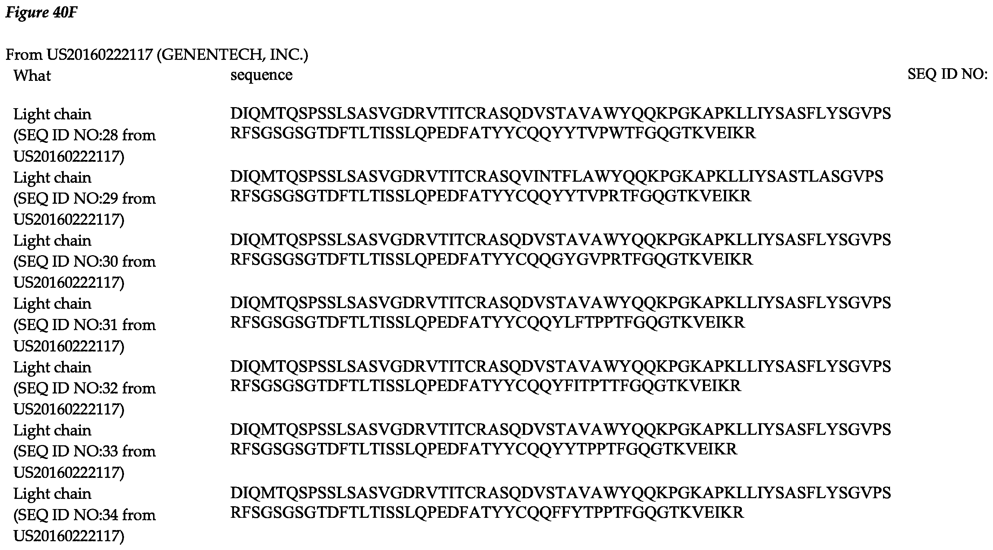

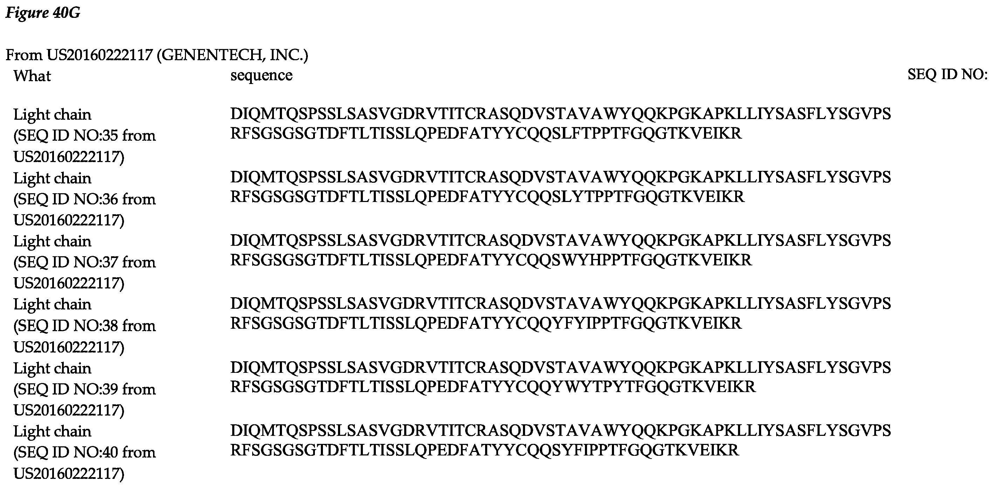

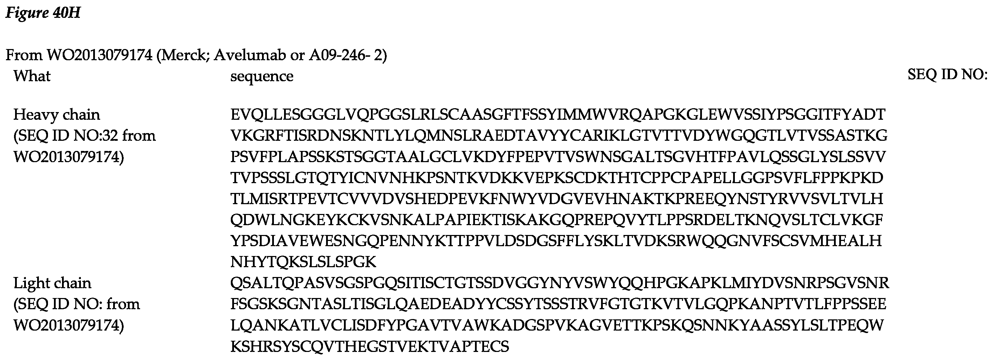

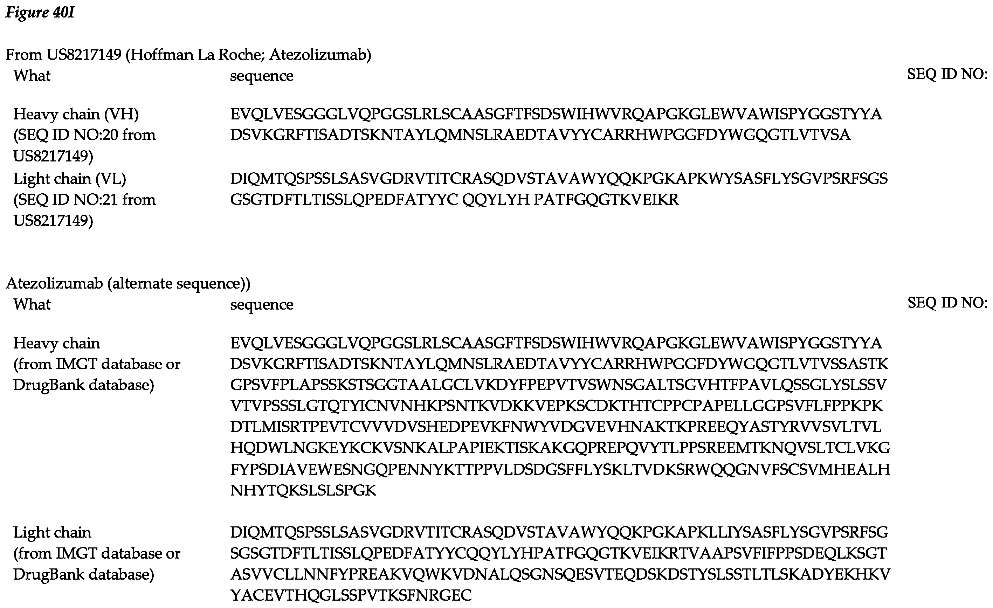

In some embodiments, the anti-IL18-BP antibodies are used in combination with one or more anti-PD-L1 antibody (*e.g*., anti-PD-L1 targeting antibodies). There are three approved anti-PD-L1 antibodies, atezolizumab (TECENTRIQ^{®}; MPDL3280A; IMpower110; Roche/Genentech), avelumab (BAVENCIO^{®}; MSB001071 8C; EMD Serono & Pfizer), and Durvalumab (MEDI4736; IMFINZI^{®};AstraZeneca). And other antibodies under development, for example, Lodapolimab (LY3300054, Eli Lily), Pimivalimab (Jounce Therapeutics Inc), SHR-1316 (Jiangsu Hengrui Medicine Co Ltd), Envafolimab (Jiangsu Simcere Pharmaceutical Co Ltd), sugemalimab (CStone Pharmaceuticals Co Ltd), cosibelimab (Checkpoint Therapeutics Inc), pacmilimab (CytomX Therapeutics Inc), IBI-318, IBI-322, IBI-323 (Innovent Biologics Inc), INBRX-105 (Inhibrx Inc), KN-046 (Alphamab Oncology), 6MW-3211 (Mabwell Shanghai Bioscience Co Ltd), BNT-311 (BioNTech SE), FS-118 (F-star Therapeutics Inc), GNC-038 (Systimmune Inc), GR-1405 (Genrix (Shanghai) Biopharmaceutical Co Ltd), HS-636 (Zhejiang Hisun Pharmaceutical Co Ltd), LP-002 (Lepu Biopharma Co Ltd), PM-1003 (Biotheus Inc), PM-8001 (Biotheus Inc), STIA-1015 (ImmuneOncia Therapeutics LLC), ATG-101 (Antengene Corp Ltd), BJ-005 (BJ Bioscience Inc), CDX-527 (Celldex Therapeutics Inc), GNC-035 (Systimmune Inc), GNC-039( Systimmune Inc), HLX-20 (Shanghai Henlius Biotech Inc), JS-003 (Shanghai Junshi Bioscience Co Ltd), LY-3434172 (Eli Lilly and Co), MCLA-145 (Merus NV), MSB-2311 (Transcenta Holding Ltd), PF-07257876 (Pfizer Inc), Q-1802 (QureBio Ltd), QL-301 (QLSF Biotherapeutics Inc), QLF-31907 (Qilu Pharmaceutical Co Ltd), RC-98 (RemeGen Co Ltd), TST-005 (Transcenta Holding Ltd), Atezolizumab (IMpower133), BMS-936559/MDX-1105, and/or RG-7446/MPDL3280A, and/or YW243.55.S70. In some embodiments, the PD-L1 antibody is one described in U.S. Patent Publication No. 2017/0281764 as well as WO 2013/079174 (avelumab) and WO 2010/077634 (or US 2016/0222117 or US 8,217,149; atezolizumab). In some embodiments, the PD-L1 antibody comprises a heavy chain sequence of SEQ ID NO: 34 and a light chain sequence of SEQ ID NO: 36 (from US 2017/281764), as well as others in development, which can be used in combination with the anti-IL18BP antibodies of the invention. Additional exemplary anti-PD-L1 antibody sequences are shown in Figure 40.

In some embodiments, the anti-IL18-BP antibodies are used in combination with one or more anti-PD-L2 antibodies (*e.g*., anti-PD-L2 targeting antibodies). Examples of anti-PD-L2 antibodies include for example but are not limited to anti-PD-L2 antibodies as described in WO 2010/036959, anti-PD-L2 antibodies as described in WO 20140/22758, as well as others in development, which can be used in combination with the anti-IL18-BP antibodies of the invention.

In some embodiments, the anti-IL18-BP antibodies are used in combination with one or more anti-CTLA-4 antibodies (*e.g*., anti-CTLA-4 targeting antibodies). Examples of anti-CTLA-4 antibody include for example but are not limited to the FDA approved antibody ipilimumab and tremelimumab. In some embodiments, an anti-CTLA-4 antibodies include for example but are not limited to Yervoy^{®} (ipilimumab or antibody 10D1, described in PCT Publication WO 01/14424), tremelimumab (formerly ticilimumab, CP-675,206), monoclonal or an anti-CTLA-4 antibody described in any of the following publications: WO 98/42752; WO 00/37504; U.S. Pat. No. 6,207,156; Hurwitz et al. (1998) Pro. Natl. Acad. Sci. USA 95(17): 10067-10071; Camacho et al. (2004) J. Clin. Oncology 22(145): antibodiestract No. 2505 (antibody CP-675206); and Mokyr et al. (1998) Cancer Res. 58:5301-5304. Any of the anti-CTLA-4 antibodies disclosed in WO2013/173223 can also be used, as well as others in development, which can be used in combination with the anti-IL18-BP antibodies of the invention.

In some embodiments, the anti-IL18-BP antibodies are used in combination with one or more anti-B7H3 antibodies (*e.g*., anti-B7H3 targeting antibodies). Examples of anti- B7H3 antibodies include the antibodies under clinical study, for example, Enoblituzumab (MGA271;MacroGenics), and anti-B7H3 antibodies as described WO 2016/033225, anti-B7H3 antibodies as outlined US 9,441,049, as well as others in development, which can be used in combination with the anti-IL18BP antibodies of the invention.

In some embodiments, the anti-IL18-BP antibodies are used in combination with one or more anti-B7H4 antibodies (*e.g*., anti-B7H4 targeting antibodies). Examples of anti-B7H4 antibodies include for example but are not limited to anti-B7H4 monoclonal antibody from FivePrime, FPA150, which is currently in clinical phase I, antibodies as described in WO 2022/002012, as well as others in development, which can be used in combination with the anti-IL18BP antibodies of the invention.

In some embodiments, the anti-IL18-BP antibodies are used in combination with one or more anti-Carcinoembryonic antigen-related cell adhesion molecule-1 antibodies, also known as anti-CEACAM1 antibody, or anti-CD66a antibody. Examples of anti-CEACAM-1 antibodies include for example but are not limited to the antibodies under clinical study, for example, Besilesomab (TheraPharm), AMG211(Amgen), and CM-24 (MK-6018, KitovPharma). Examples of anti-CEACAM-1 antibodies also include antibodies as outlined in US20200277398A1(CM-24 in development by Famewave Ltd), antibodies as outlined in US9072797B2(a CD66-binding component and radionuclide yttrium-90 (90Y)), as well as others in development, which can be used in combination with the anti-IL18-BP antibodies of the invention.

In some embodiments, the anti-IL18-BP antibodies are used in combination with one or more anti-PVR antibodies (*e.g*., anti-PVR targeting antibodies). Examples of anti-PVR antibodies include for example but are not limited to antibodies as described in WO 2017/149538, of anti-PVR antibodies include antibodies as described in WO 2021/070181. In some embodiments, the second agent is selected from one or more of an antagonist of PVRL1, PVRL2, PVRL3, PVRL4, and CD155, for example, ASG-22CE (Astellas Pharm/a Inc), Enfortumab (Astellas Pharma), as well as others in development, which can be used in combination with the anti-IL18-BP antibodies of the invention.

In some embodiments, the anti-IL18-BP antibodies are used in combination with one or more anti-LAG3 antibodies (*e.g*., anti-LAG3 targeting antibodies). Examples of anti-LAG3 antibodies include for example but are not limited to antibodies under clinical study, for example, LAG525(Novartis), TSR-033(Tesaro), Fianlimab (REGN3767, Regeneron), BI-754111(Boehringer Ingelheim), Sym-022 (Symphogen), RO7247669 (Roch), BMS-986016 (see, WO 2010/019570), GSK2831781 (see, US 2016/0017037), and Merck clones 22D2, 11C9, 4A10, and/or 19E8 (*see,* WO 2016/028672) and antibodies comprising the CDRs or variable regions of antibodies 25F7, 26H10, 25E3, 8B7, 11F2 or 17E5, which are described in US 2011/0150892, WO 2010/19570 and WO 2014/008218. Other art recognized anti-LAG-3 antibodies that can be used include IMP731 and IMP-321, described in US 2011/007023, WO 2008/132601, and WO 2009/44273. Anti-LAG-3 antibodies that compete with and/or bind to the same epitope as that of any of these antibodies can also be used in combination treatments. as well as others in development, which can be used in combination with the anti-IL18-BP antibodies of the invention.

In some embodiments, the anti-IL18-BP antibodies are used in combination with one or more anti-CD112 (also referred to as PVRL2; and including *e.g*., anti-CD112 targeting antibodies)) antibodies. Examples of anti-CD112 antibodies include for example but are not limited to anti-CD112 antibodies as outlined in US 2020/0040081, anti-CD112 antibodies as outlined in US 2019/0040154 or anti-CD112 antibodies as outlined in WO 2017/021526, as well as others in development, which can be used in combination with the anti-IL18-BP antibodies of the invention.

In some embodiments, the anti-IL18-BP antibodies are used in combination with one or more anti-CD96 antibodies (*e.g*., anti-CD96 targeting antibodies). Examples of anti-CD96 antibodies include for example but are not limited to anti-CD96 antibodies as outlined in WO 2019/091449, anti-CD96 antibodies as outlined in WO 2021042019, as well as others in development, which can be used in combination with the anti-IL18-BP antibodies of the invention.

In some embodiments, the anti-IL18-BP antibodies are used in combination with one or more anti-TIM3 antibodies (*e.g*., anti-TIM3 targeting antibodies). Examples of anti-TIM3 antibodies include antibodies under clinical study, for example, Sabatolimab (Novartis), TSR-022 (Tesaro), INCAGN02385 (Incyte Corporation), INCAGN02390 (Incyte Corporation), BGB-A425 (BeiGene), LY3321367 (Eli Lilly), BMS986258, as well as others in development, which can be used in combination with the anti-IL18-BP antibodies of the invention.

In some embodiments, the anti-IL18-BP antibodies are used in combination with one or more anti-BTLA antibodies (*e.g*., anti-BTLA targeting antibodies). Examples of anti-BTLA antibodies include for example but are not limited to JS004 (Shanghai Junshi Bioscience), anti-BTLA antibodies disclosed in WO 2011/014438, as well as others in development, which can be used in combination with the anti-IL18-BP antibodies of the invention.

In some embodiments, the anti-IL18-BP antibodies are used in combination with one or more anti-ICOS antibodies (*e.g*., anti-ICOS targeting antibodies). Examples of anti-ICOS antibodies include for example but are not limited to anti-ICOS antibodies under clinical study, for example, MEDI-570 (MedImmune), Vopratelimab (Jounce Therapeutics), KY1044 (Kymab Limited), Feladilimab (GlaxoSmithKline). Examples of anti-ICOS antibodies also include anti-ICOS antibodies as outlined in US 9,957,323, anti-ICOS antibodies as outlined in WO 2016/120789, anti-ICOS antibodies as outlined in WO 2016/154177, as well as others in development, which can be used in combination with the anti-IL18-BP antibodies of the invention.

In some embodiments, the anti-IL18-BP antibodies are used in combination with one or more anti-OX40 antibodies (*e.g*., anti-OX-40 targeting antibodies). Examples of anti-OX40 antibodies include for example but are not limited to anti-OX40 antibodies under clinical study, for example, PF-04518600 (Pfizer), BAT6026 (Bio-Thera Solutions), MEDI6469, MEDI-0562, MEDI6962 (MedImmune), BMS 986178, GSK3174998, ABBV-368 (AbbVie), ATOR-1015 (Alligator Bioscience). Examples of anti-OX40 antibodies also include anti-OX40 antibodies as outlined in US 10,730,951, anti-OX40 antibodies as outlined in US 10,851,173, as well as others in development, which can be used in combination with the anti-IL18-BP antibodies of the invention.

In some embodiments, the anti-IL18-BP antibodies are used in combination with one or more anti-41BB antibodies (*e.g*., anti-41BB targeting antibodies). Examples of anti- 41BB antibodies include for example but are not limited to utomilumab (Pfizer, PF-05082566), LVGN6051(Lyvgen Biopharma), ATOR-1017 (Alligator Bioscience), BMS-663513, anti-41BB antibodies as outlined in US 10,501,551, as well as others in development, which can be used in combination with the anti-IL18-BP antibodies of the invention.

In some embodiments, the anti-IL18-BP antibodies are used in combination with one or more anti-CD27 antibodies (*e.g*., anti-CD27 targeting antibodies). Examples of anti-CD27 antibodies include for example but are not limited to but are not limited to Varlilumab (CDX-1127, Leap Therapeutics), anti-CD27 antibodies as outlined in US 2020/0277393, anti-CD27 antibodies as outlined in WO 2019/195452, as well as others in development, which can be used in combination with the anti-IL18-BP antibodies of the invention.

In some embodiments, the anti-IL18-BP antibodies are used in combination with one or more anti-GITR antibodies (*e.g*., anti-GITR targeting antibodies). Clinical study examples of anti-GITR antibodies include for example but are not limited to but are not limited to MK-4166, MK-1248 (Merck Sharp & Dohme), BMS-986156, INCAGN01876 (Incyte Corporation), OMP-336B11 (OncoMed Pharmaceuticals), MEDI1873 (MedImmune). Examples of anti-GITR antibodies also include but are not limited to anti-GITR antibodies as described in n WO 2016/196792, anti-GITR antibody described in WO 2015/187835, e.g., antibodies having the heavy and light chain variable region CDRs, heavy and light chain variable regions, or heavy and light chains of antibodies 28F3, 19D3, 18E10, 3C3-1, 3C3-2, 2G6, 9G7-1, 9G7-2, 14E3, 19H8-1, 19H8-2, and/or 6G10, and variants thereof. The sequences of the antibodies described in WO 2015/187835 are provided in Table 2 (see SEQ ID NOs: 5-14 and 27-228). The patient may also be treated with any other anti-GITR antibodies, e.g., TRX518 (Leap Therapeutics), MK- 4166 (Merck), LKZ-145 (Novartis), GWN-323 (Novartis Pharmaceuticals Corp.), Medi 1873 (Medlmmune), INBRX-110 (Inhibrx), GITR-Fc protein (OncoMed) and antibodies described in WO 2006/105021, WO 2009/009116, WO 2011/028683, US 2014/0072565, US 2014/0072566, US 2014/0065152, WO 2015/031667, WO 2015/184099, WO 2015/184099, or WO 2016/054638.

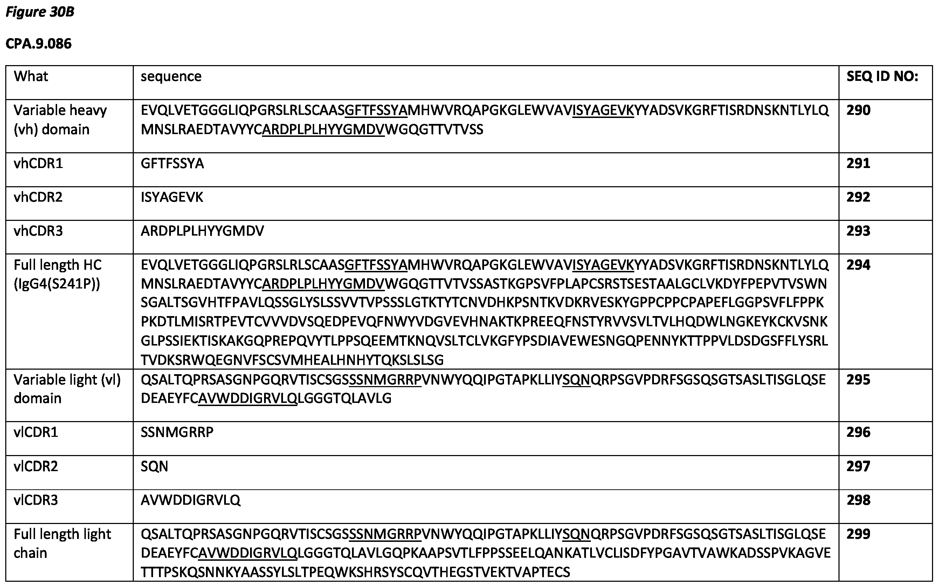

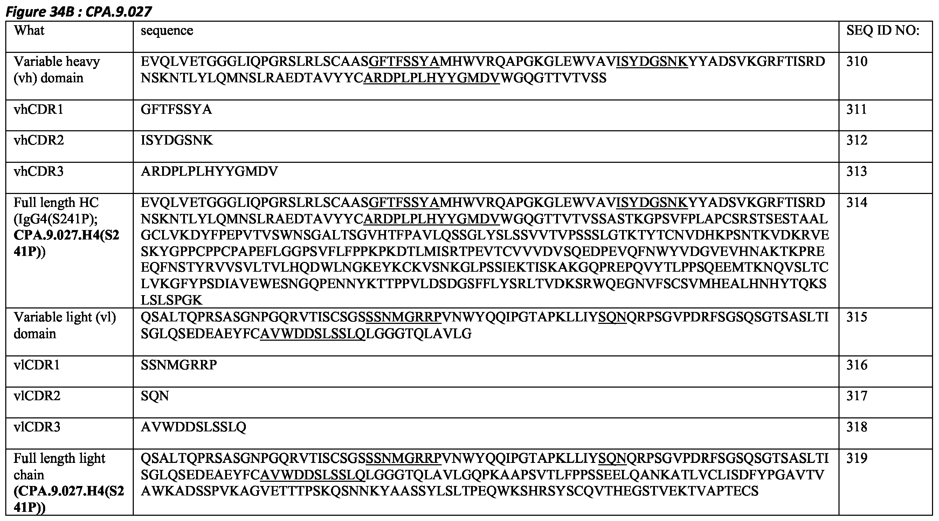

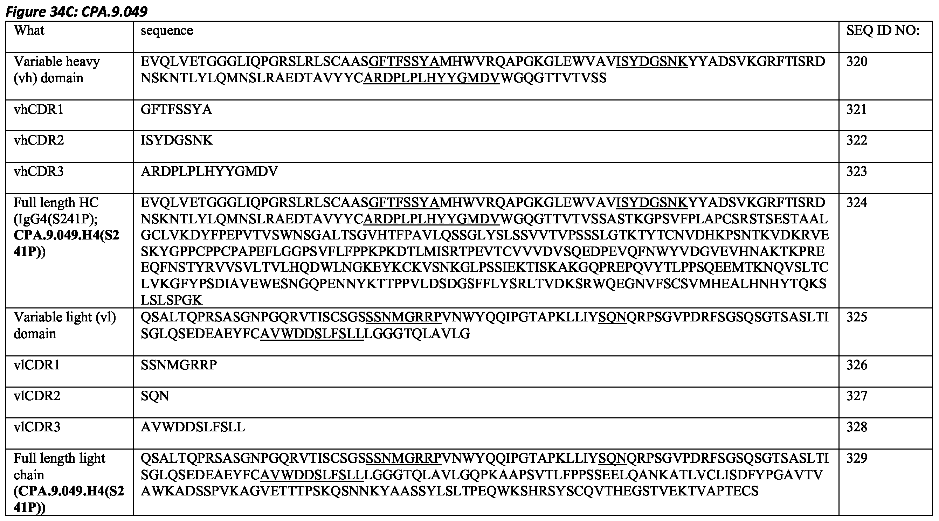

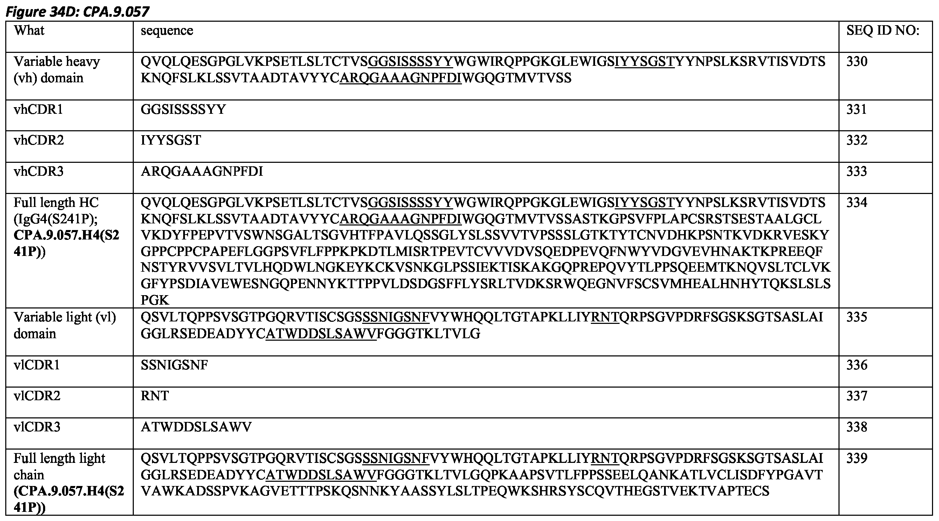

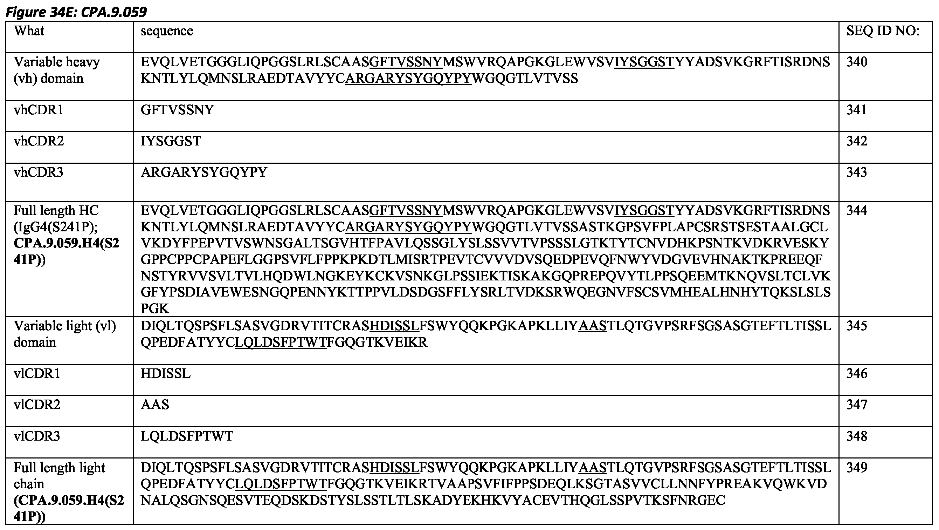

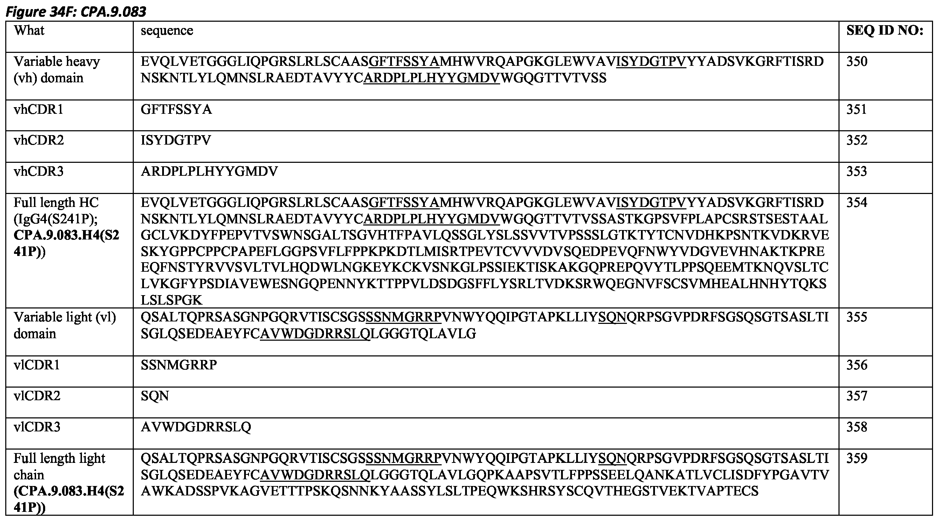

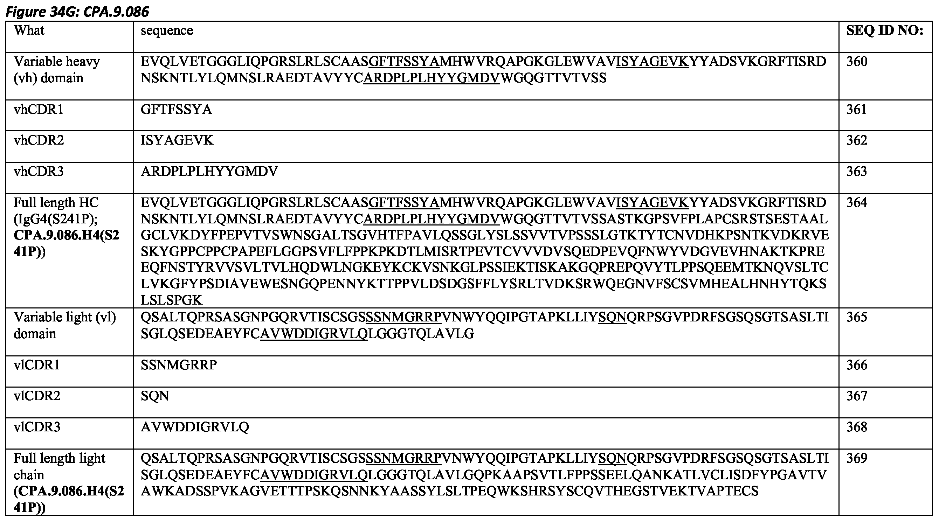

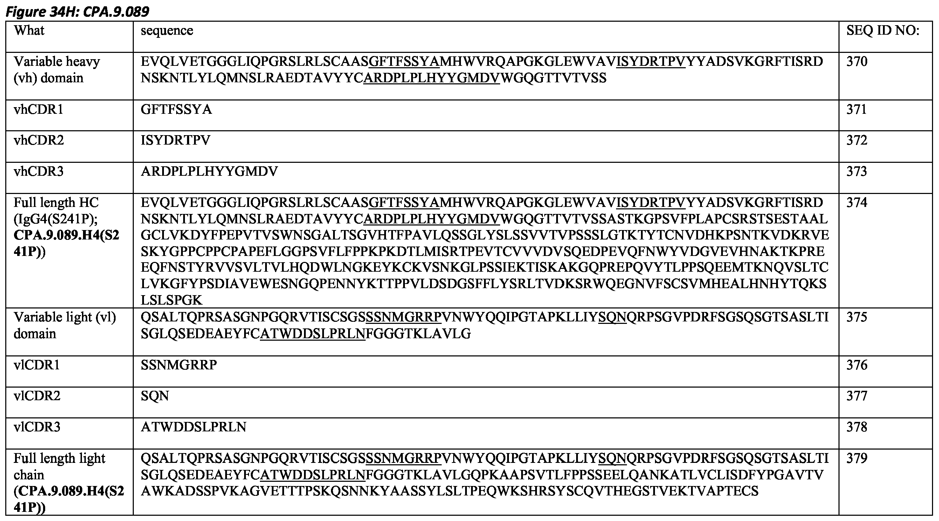

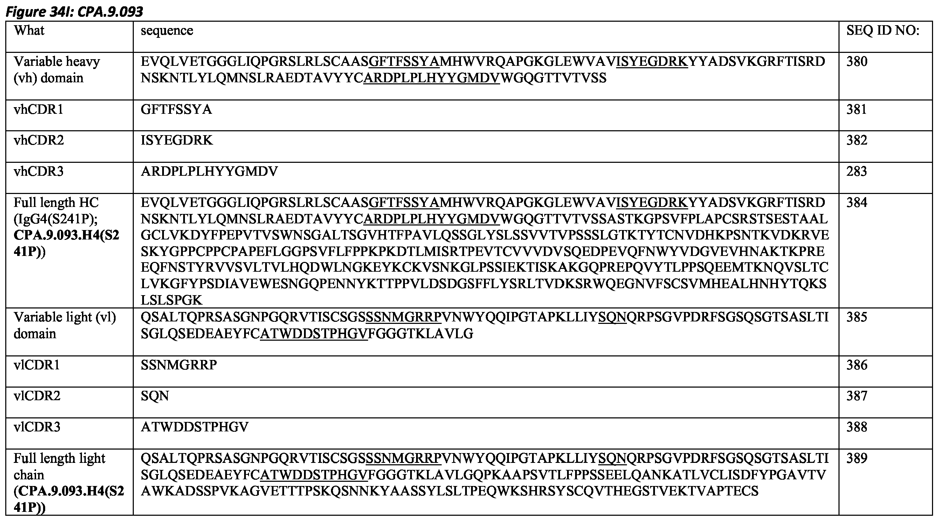

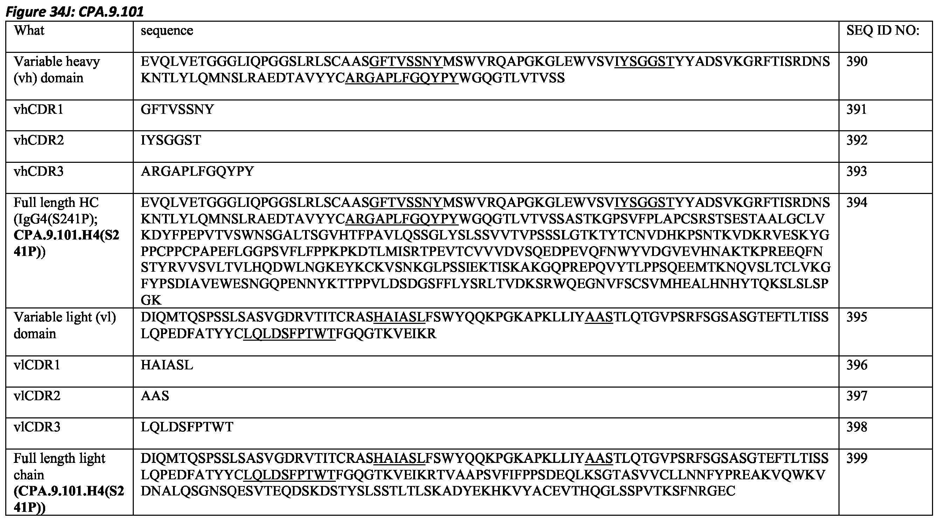

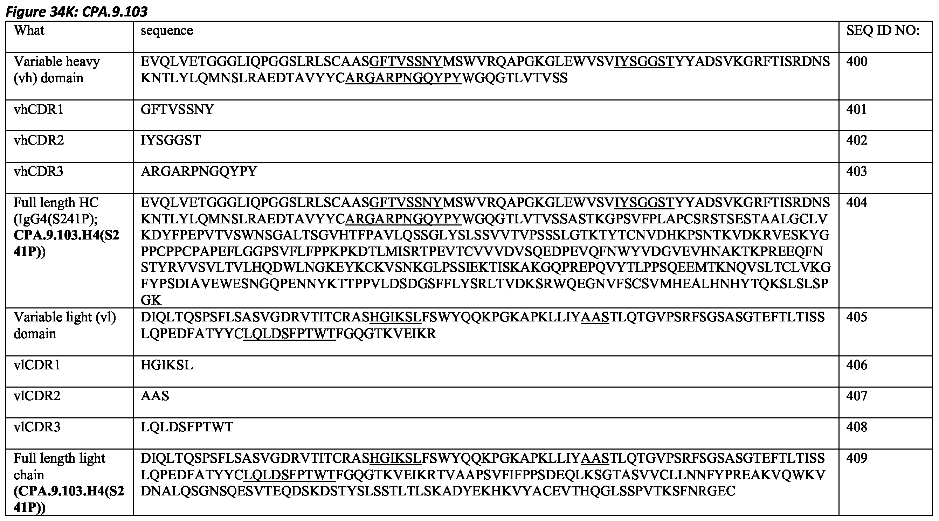

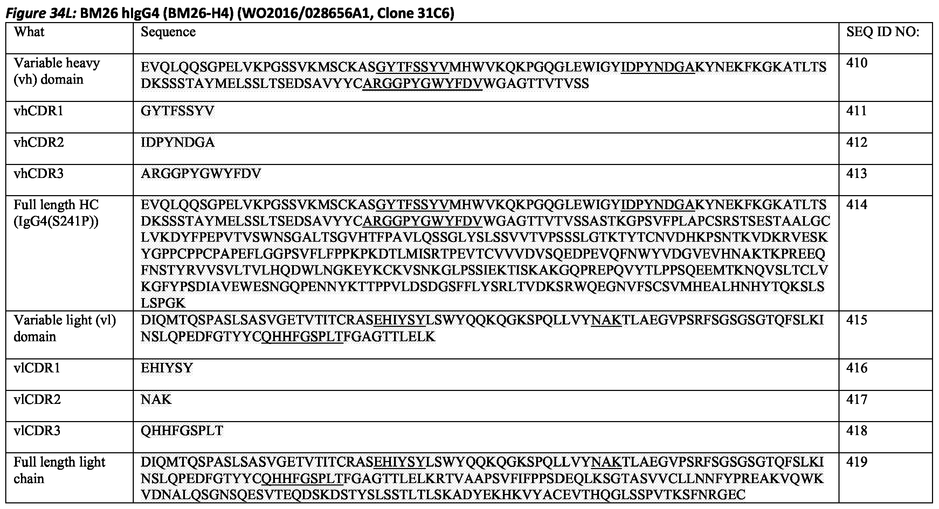

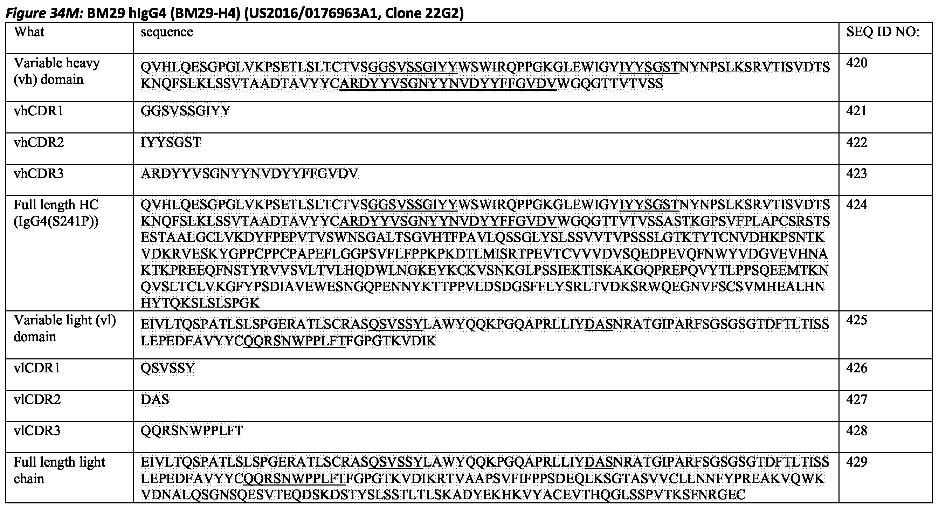

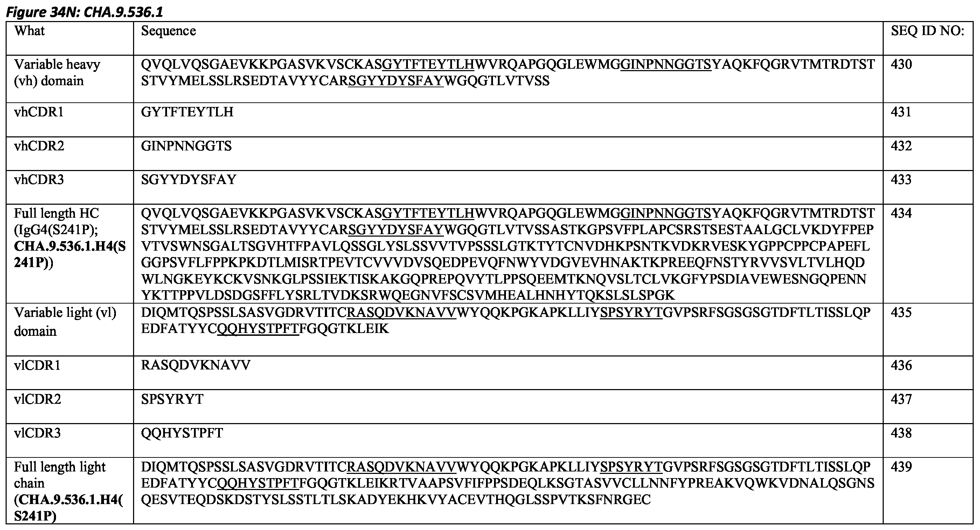

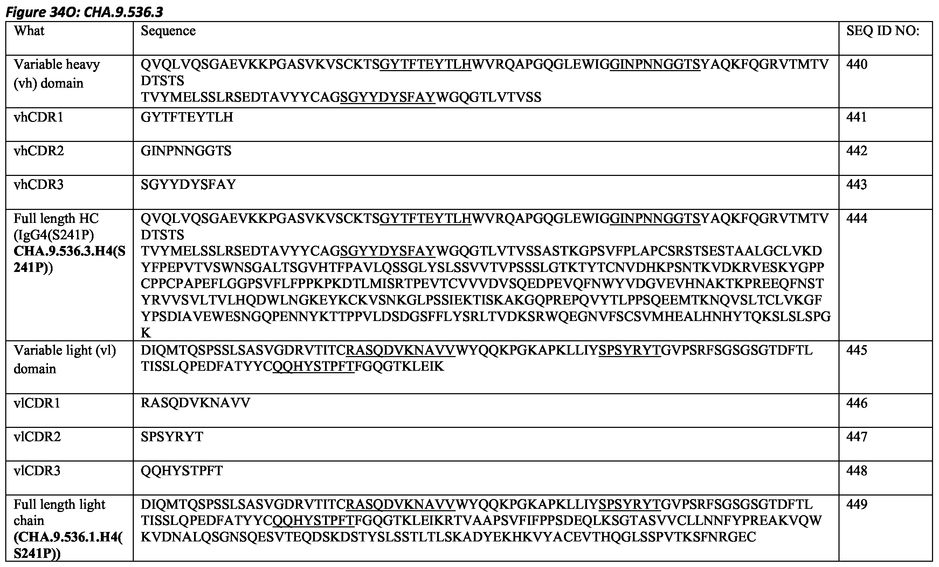

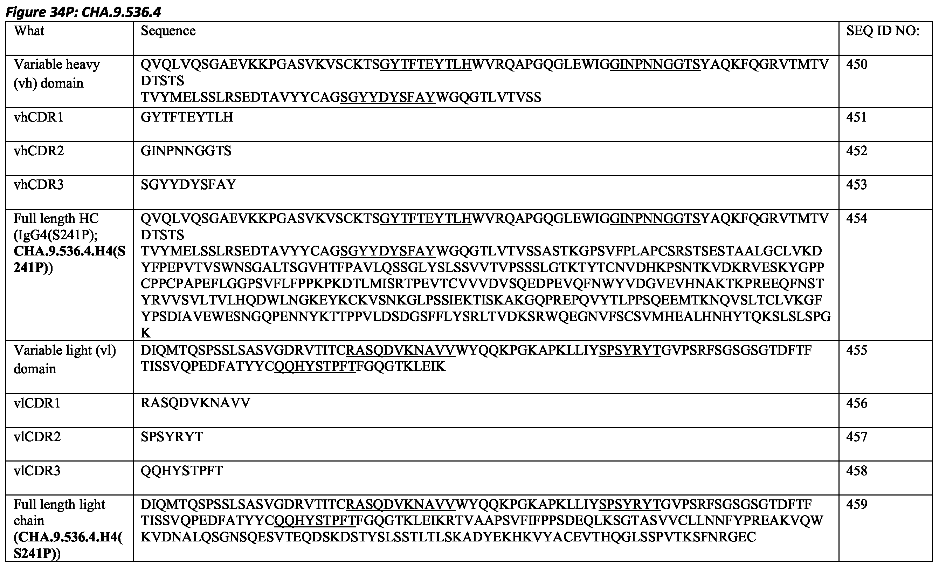

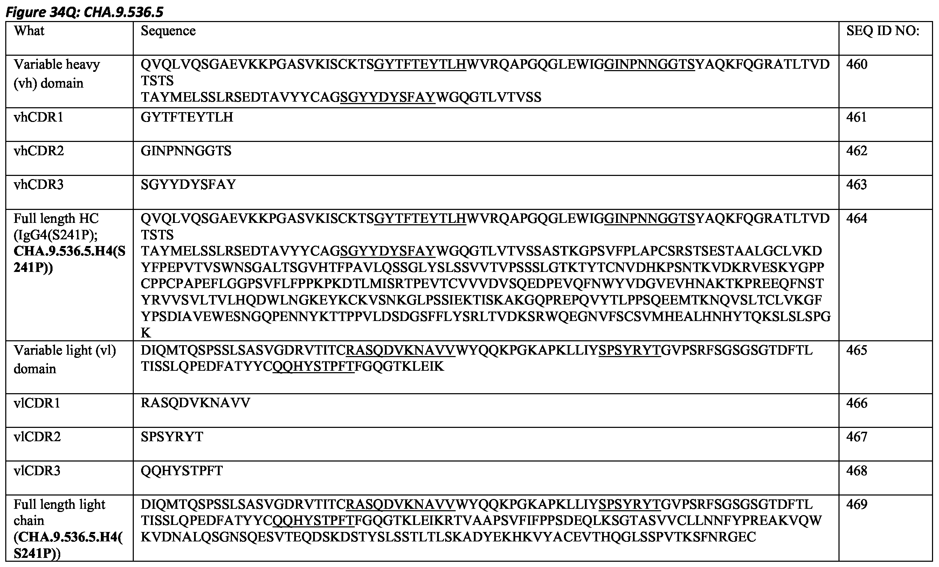

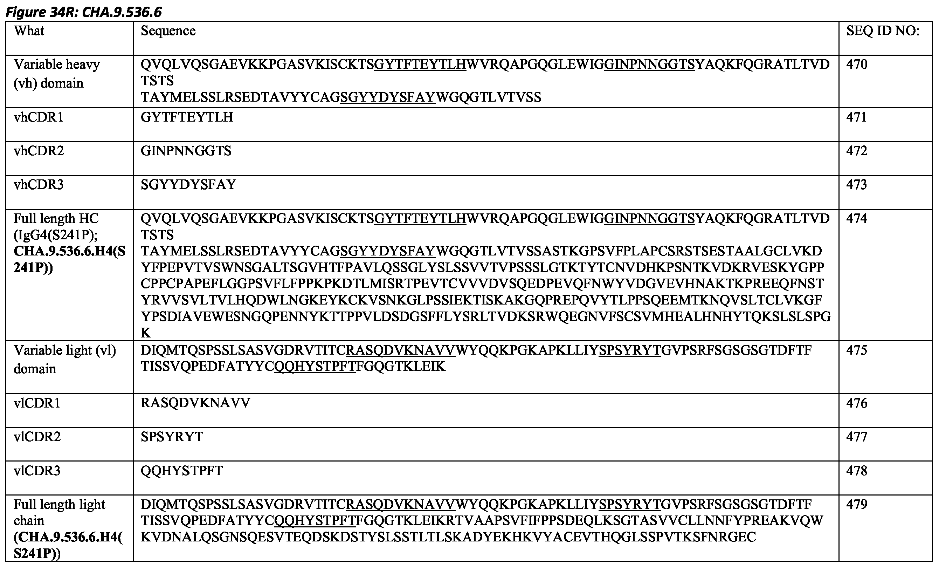

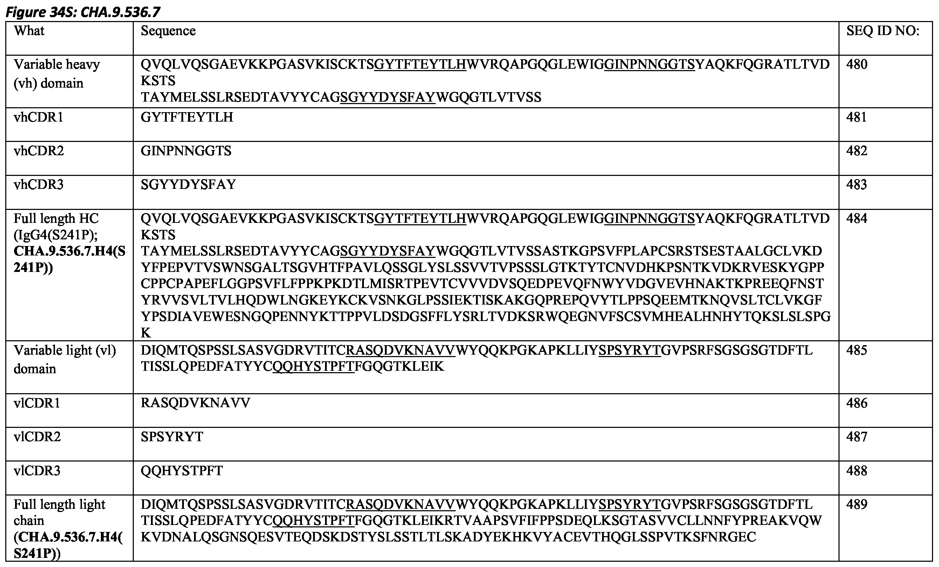

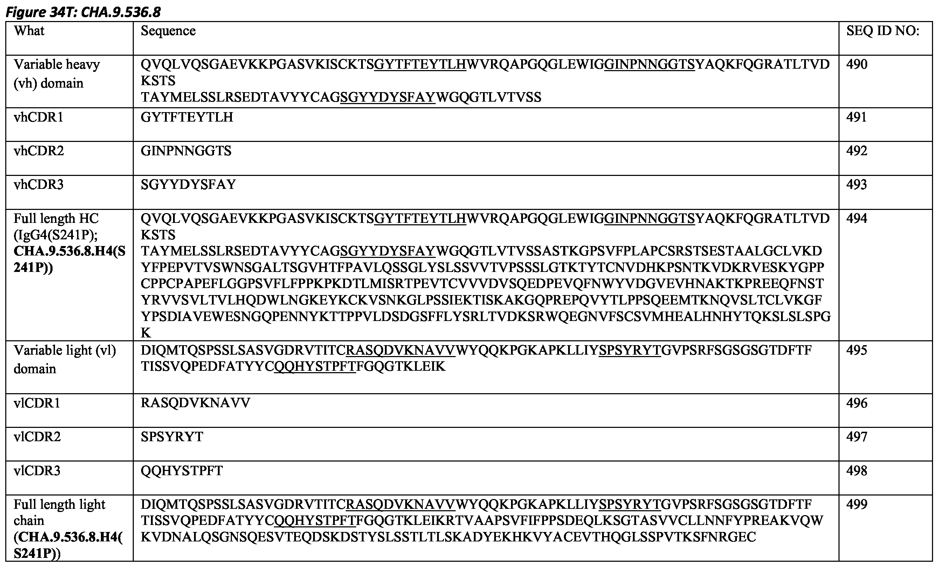

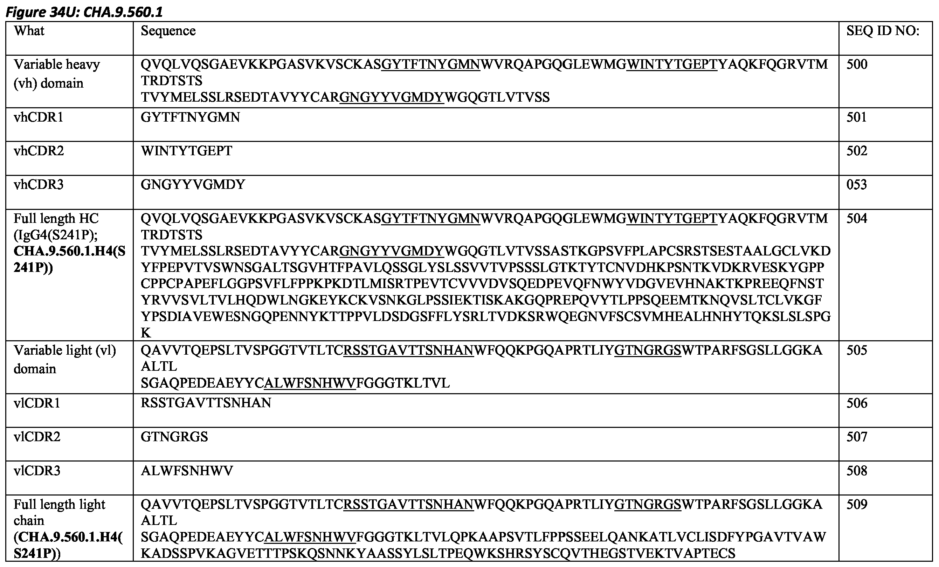

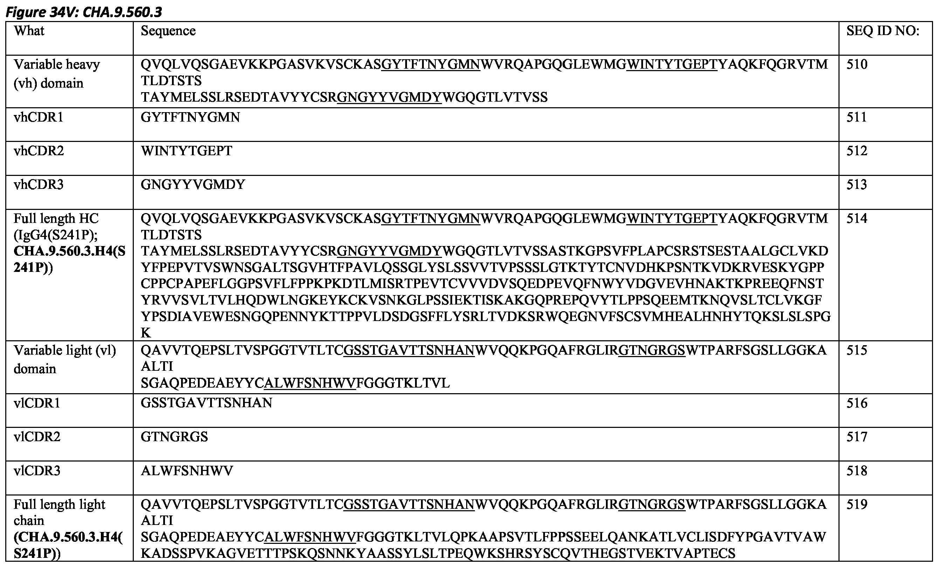

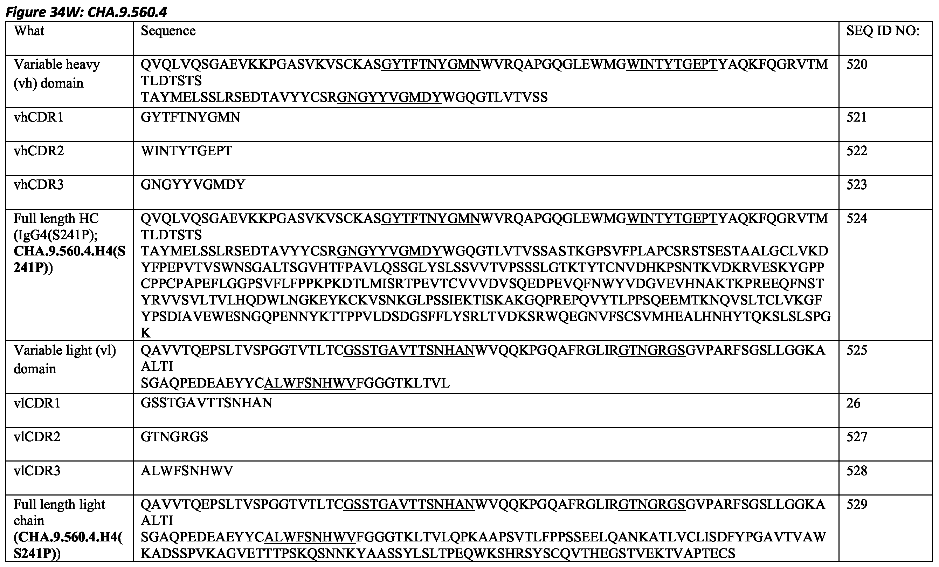

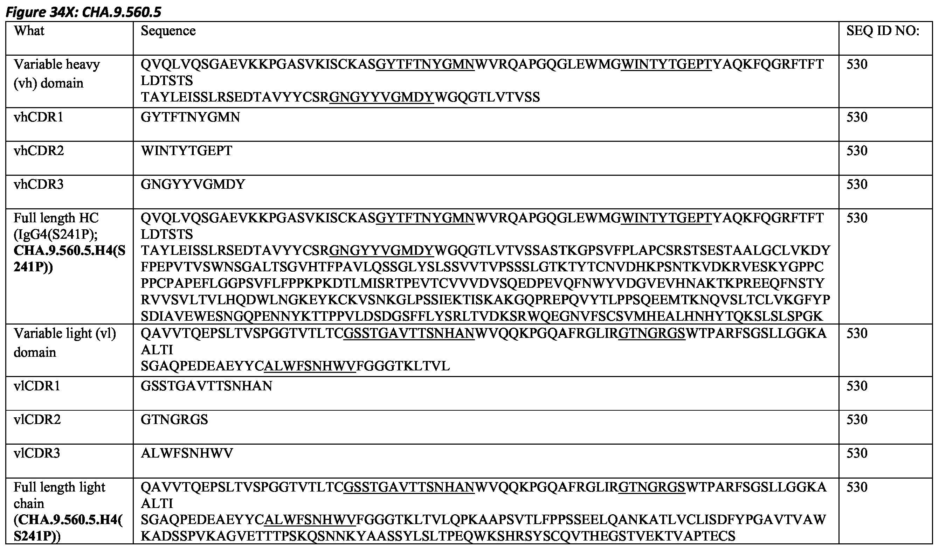

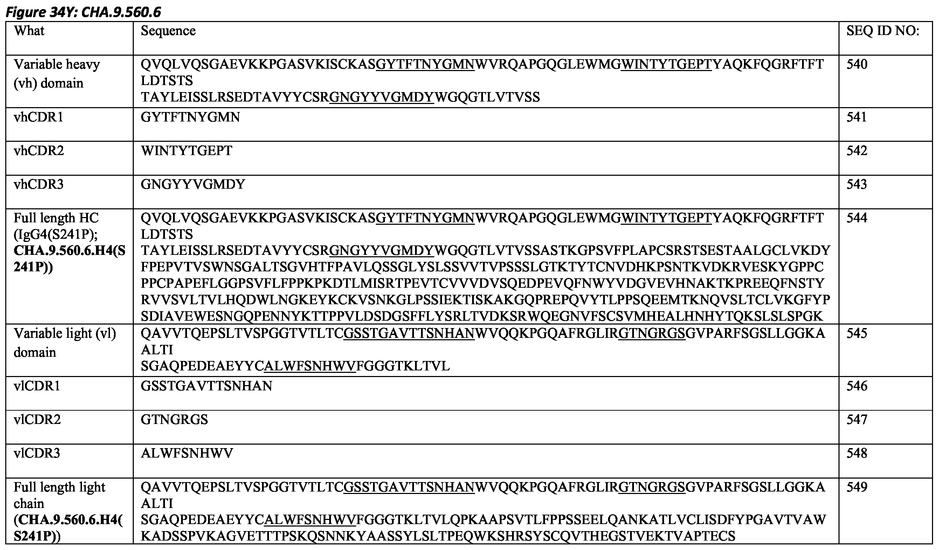

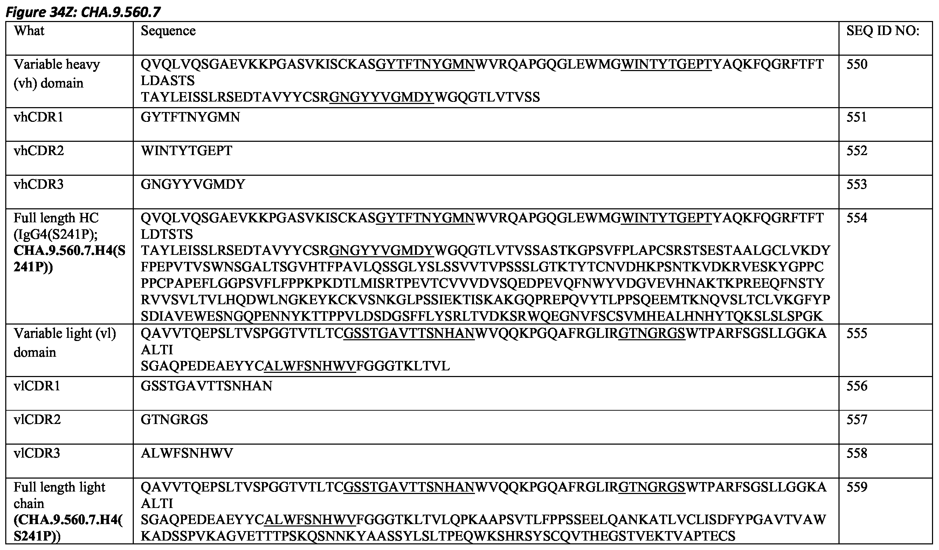

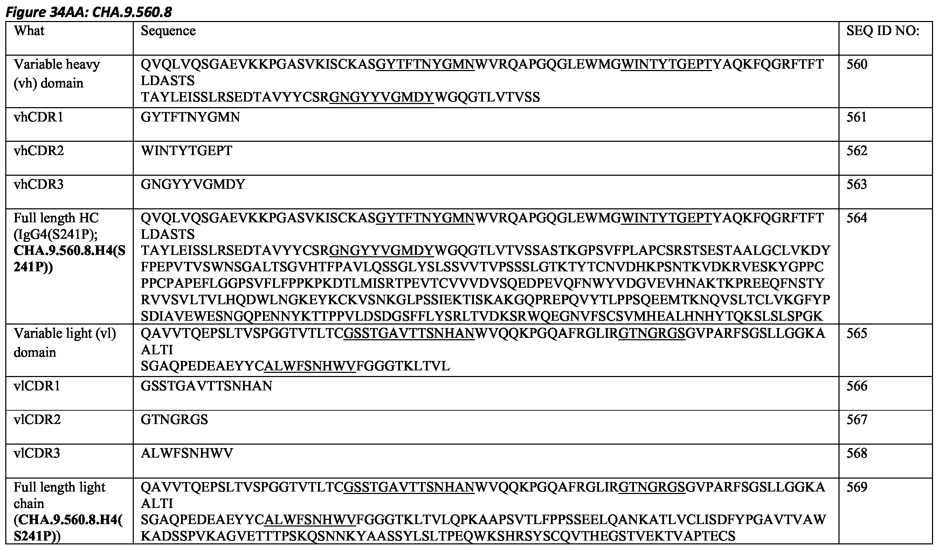

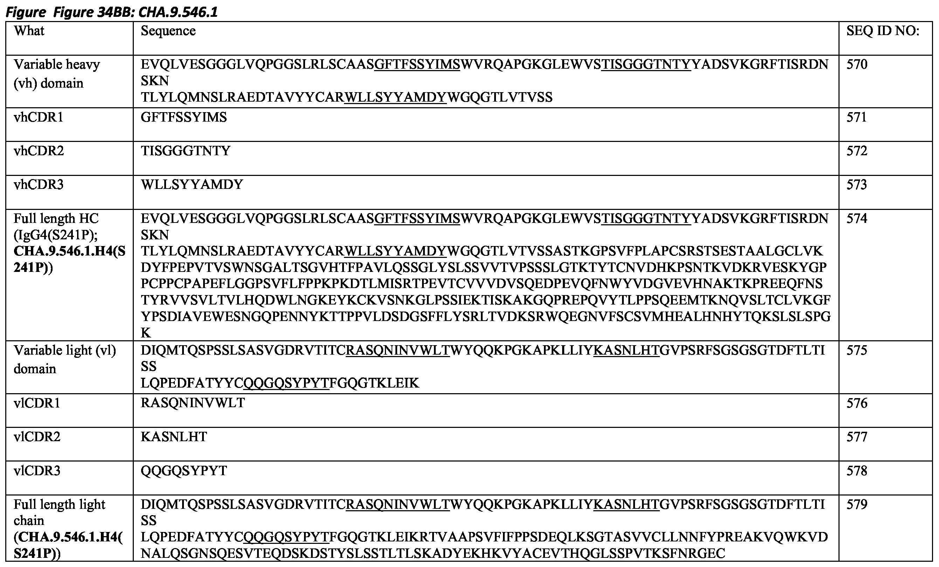

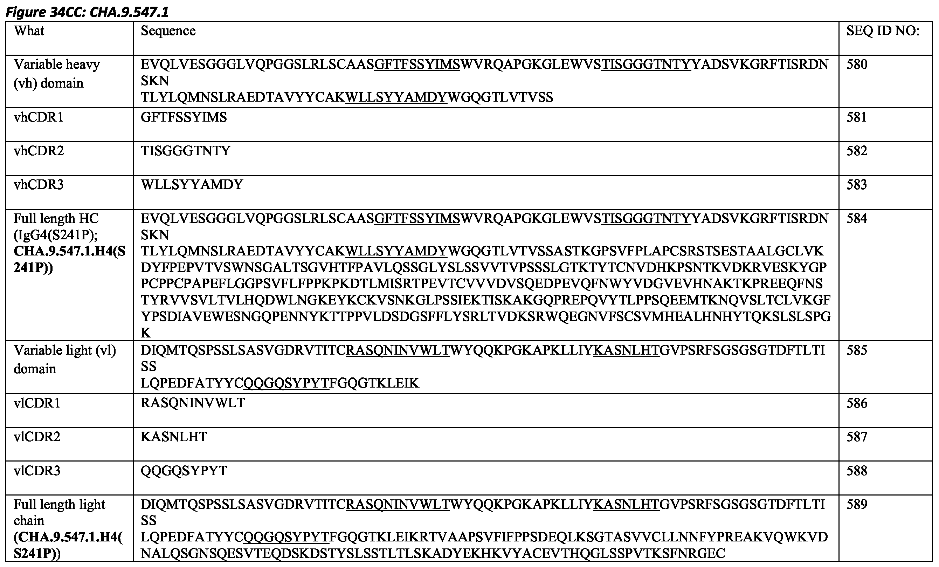

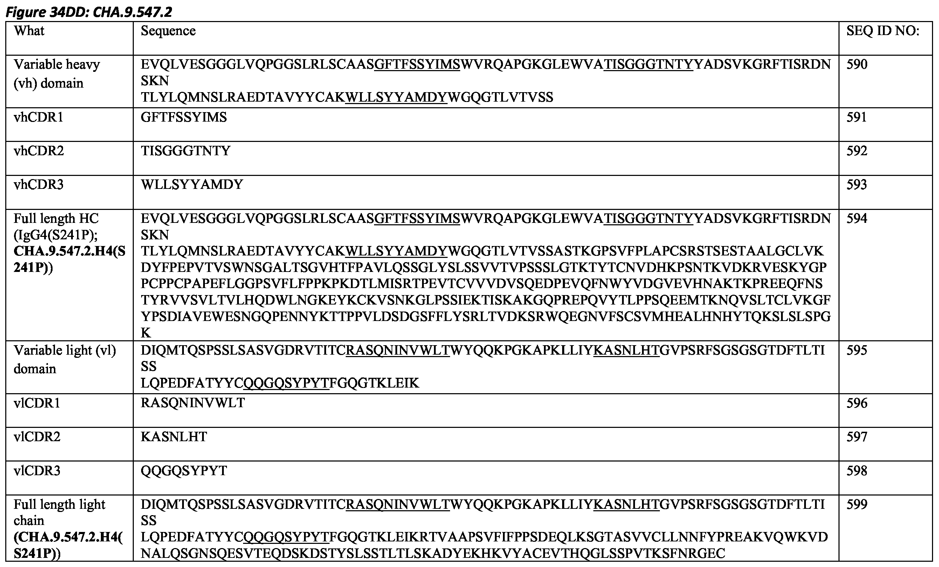

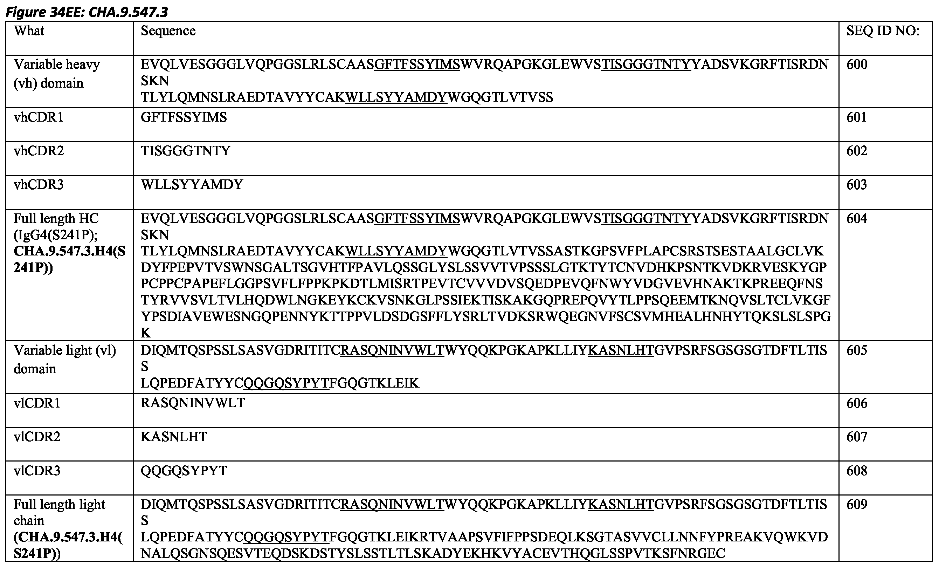

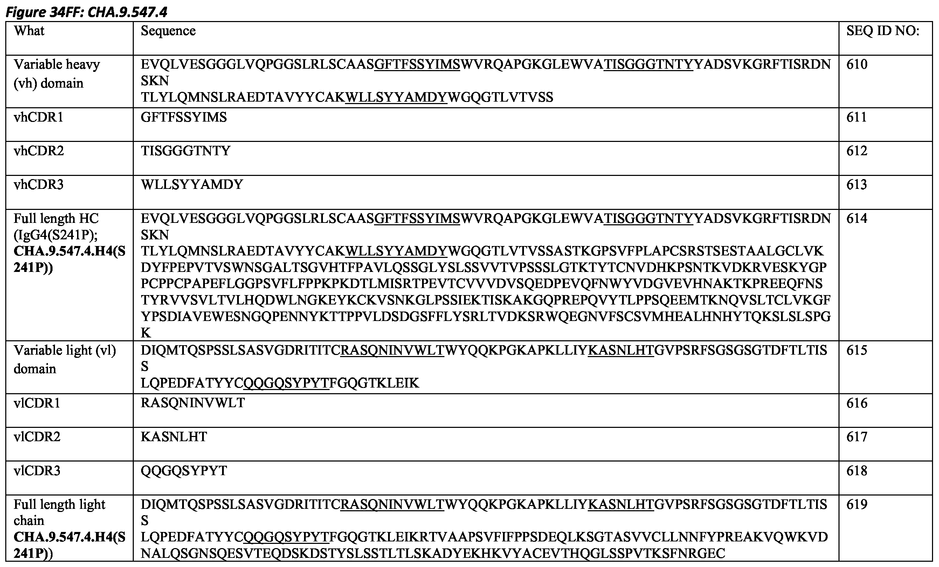

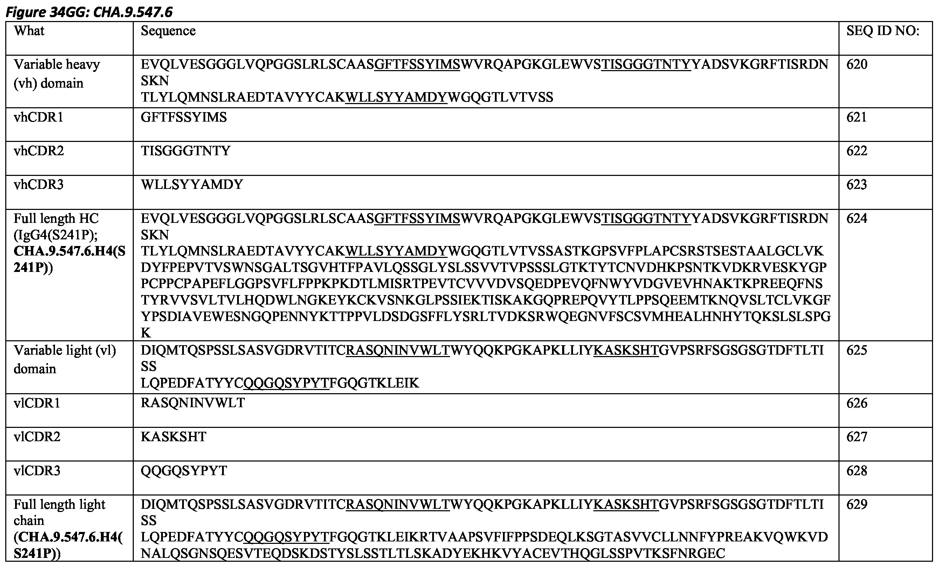

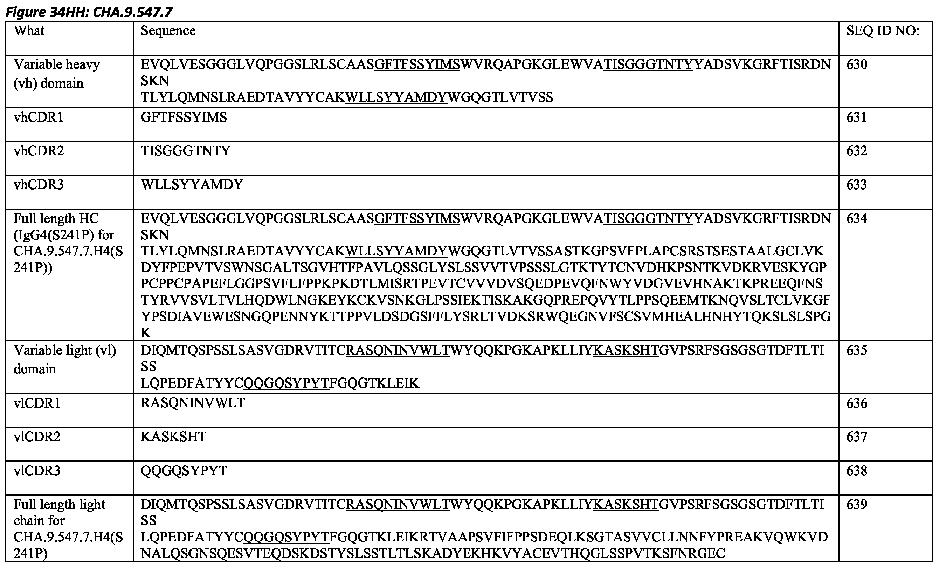

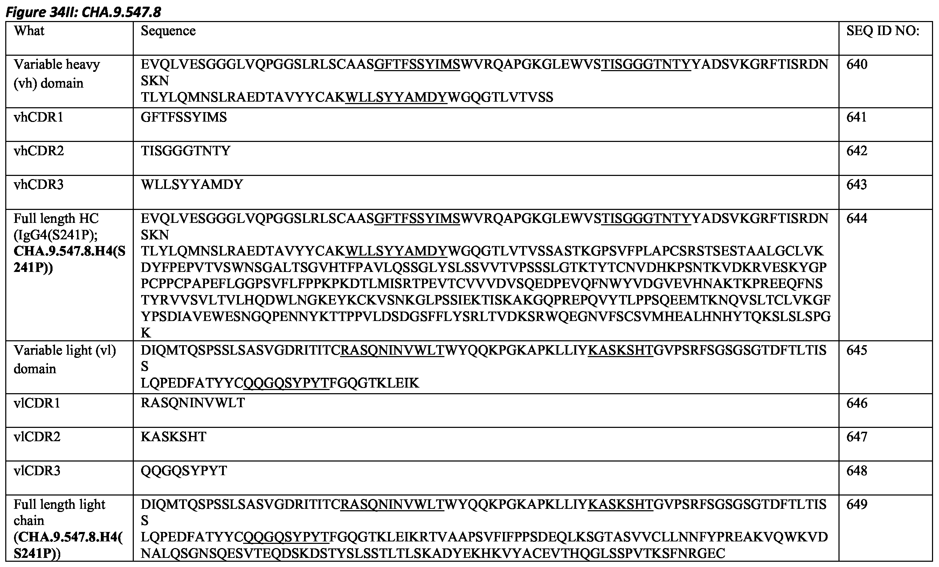

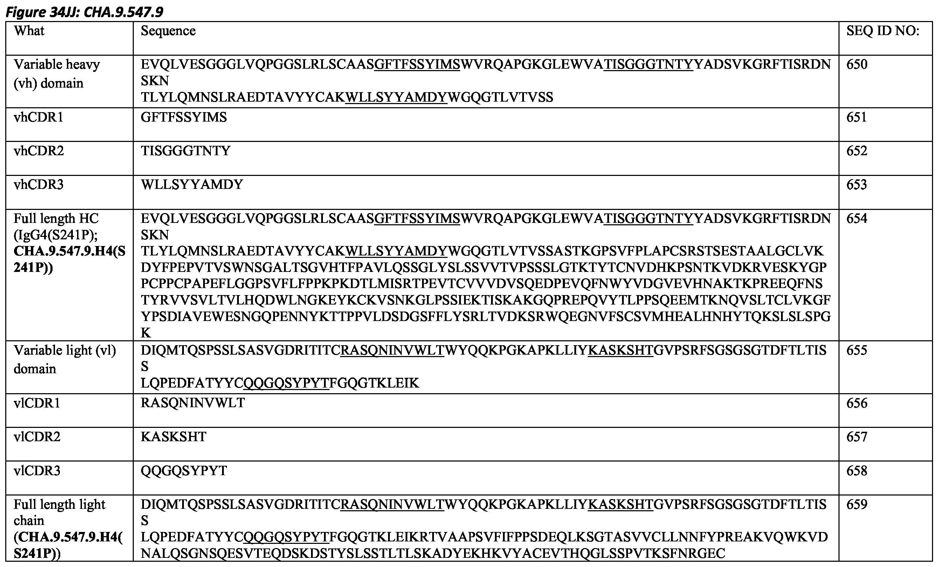

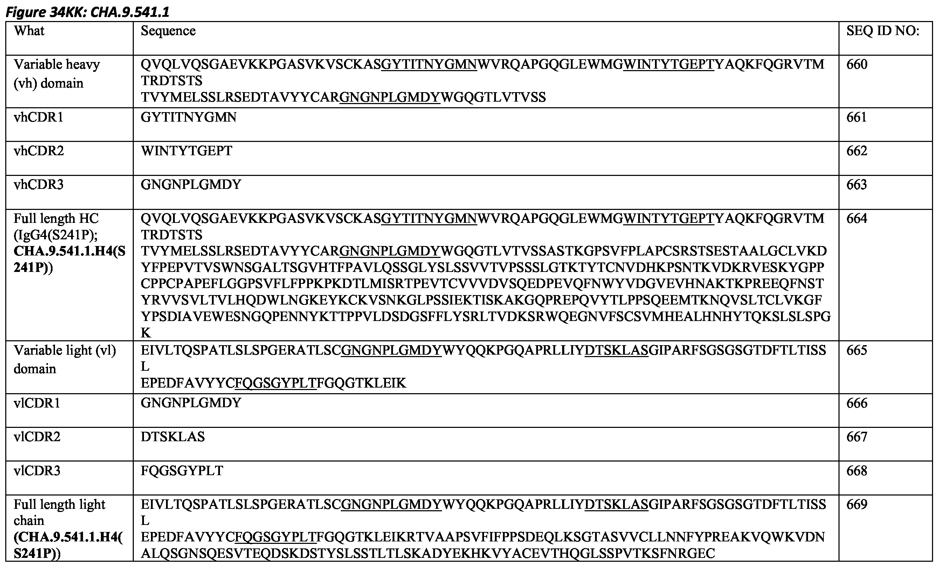

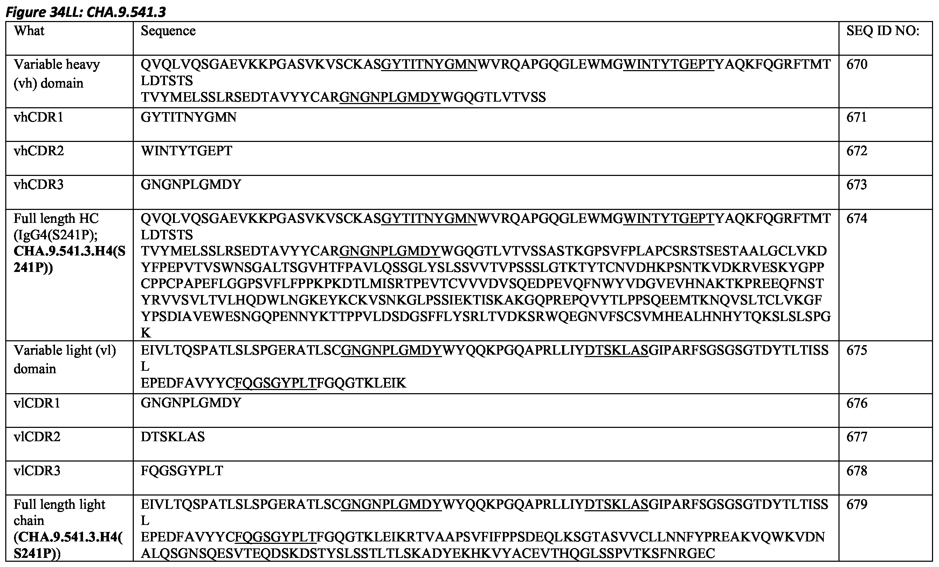

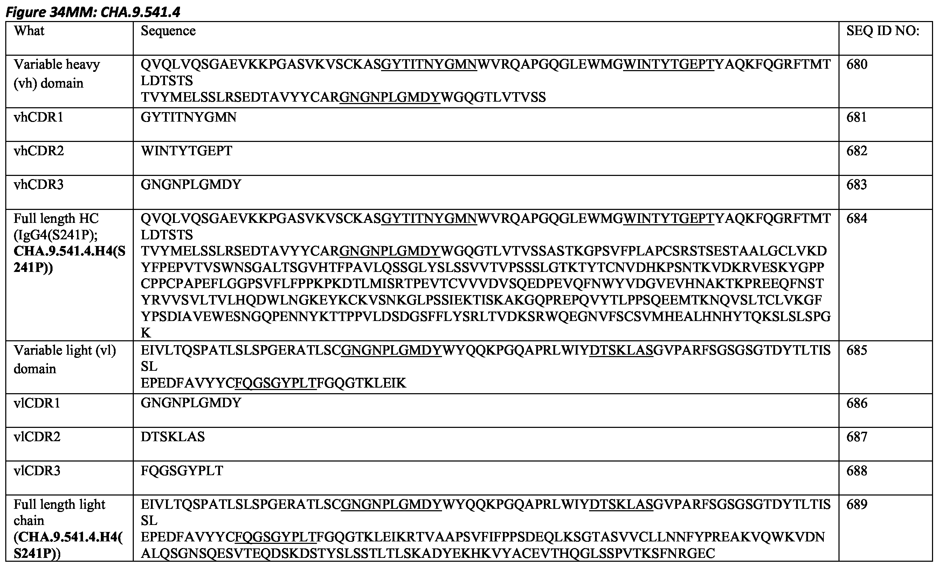

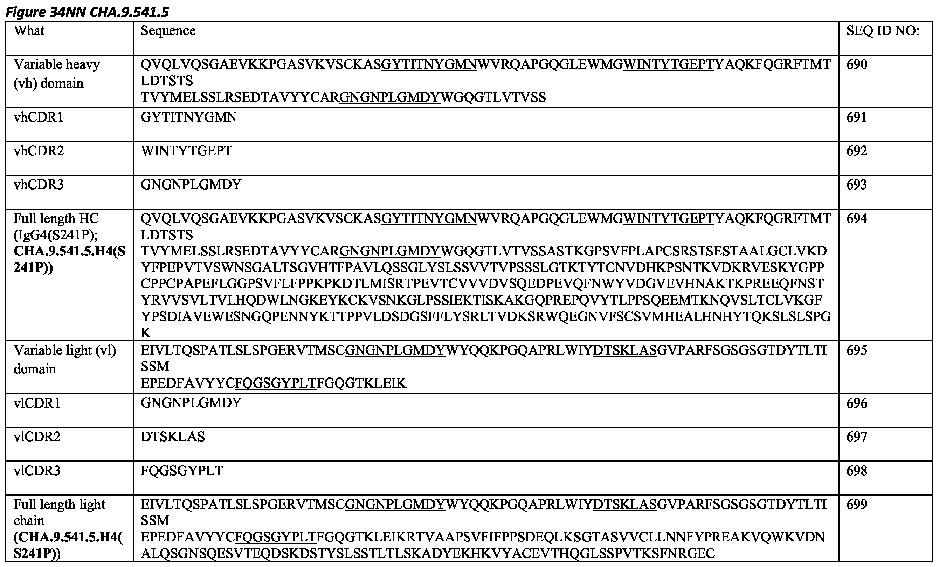

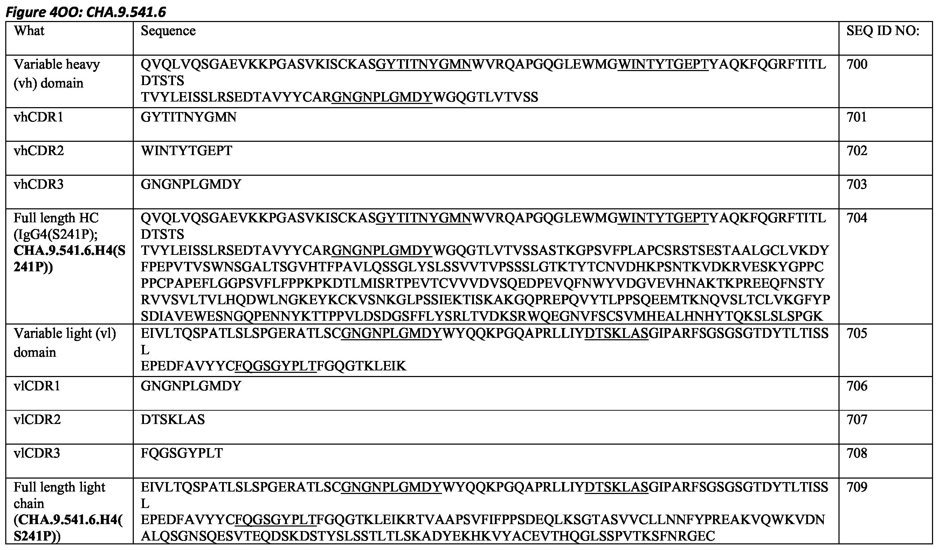

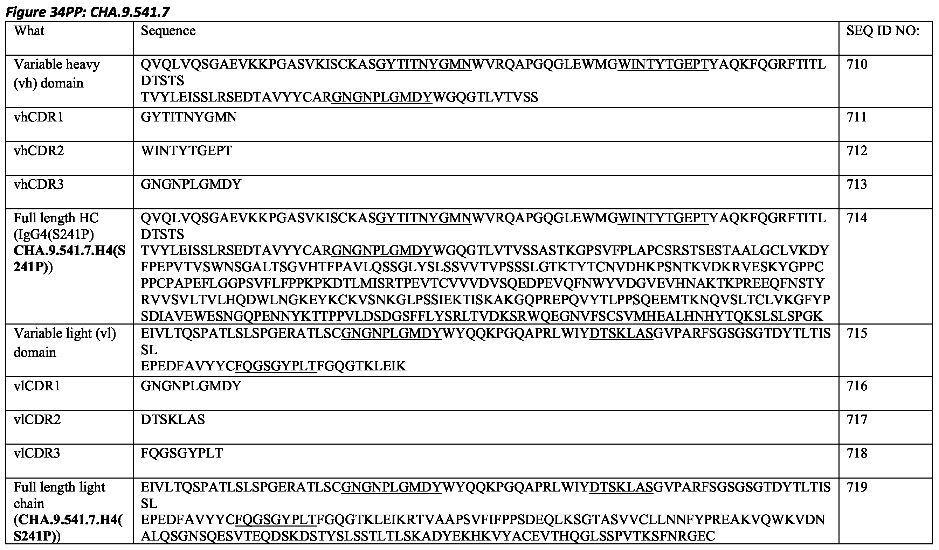

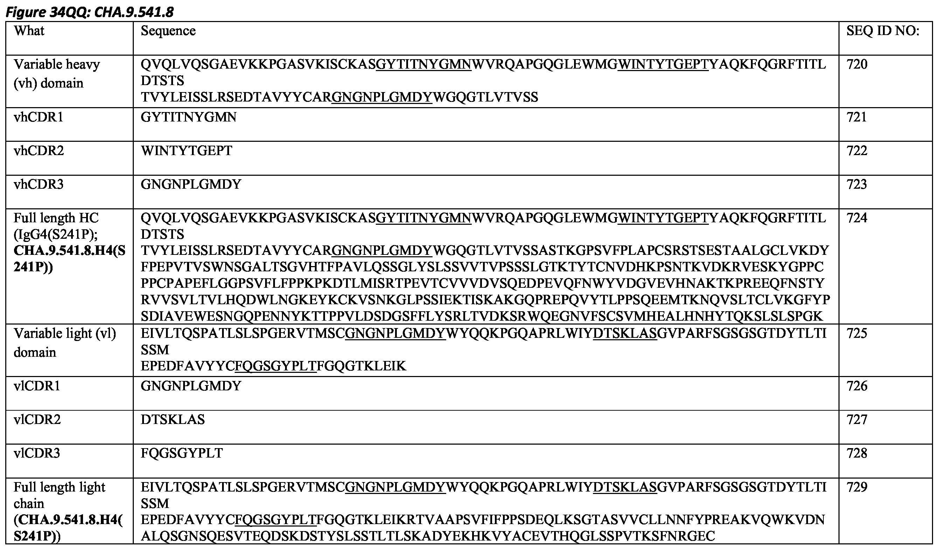

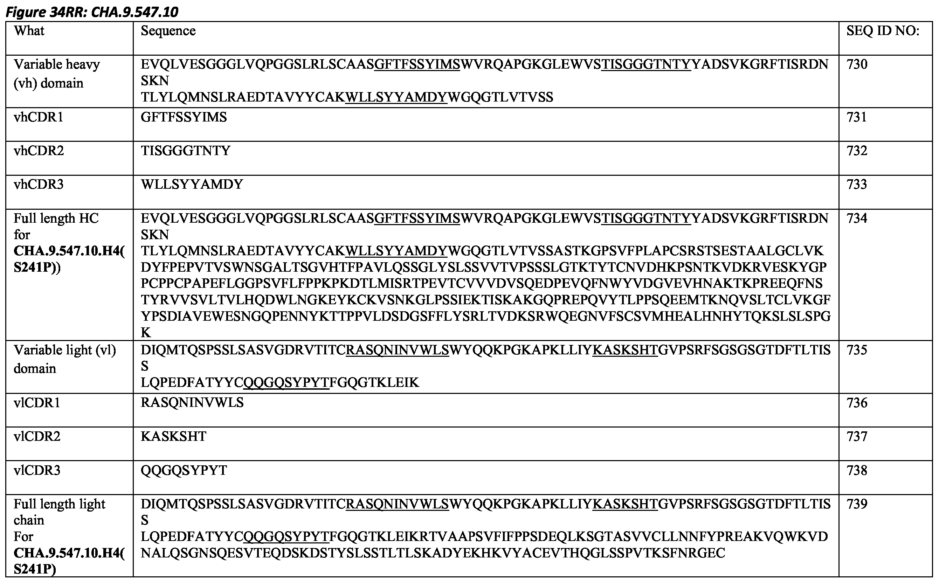

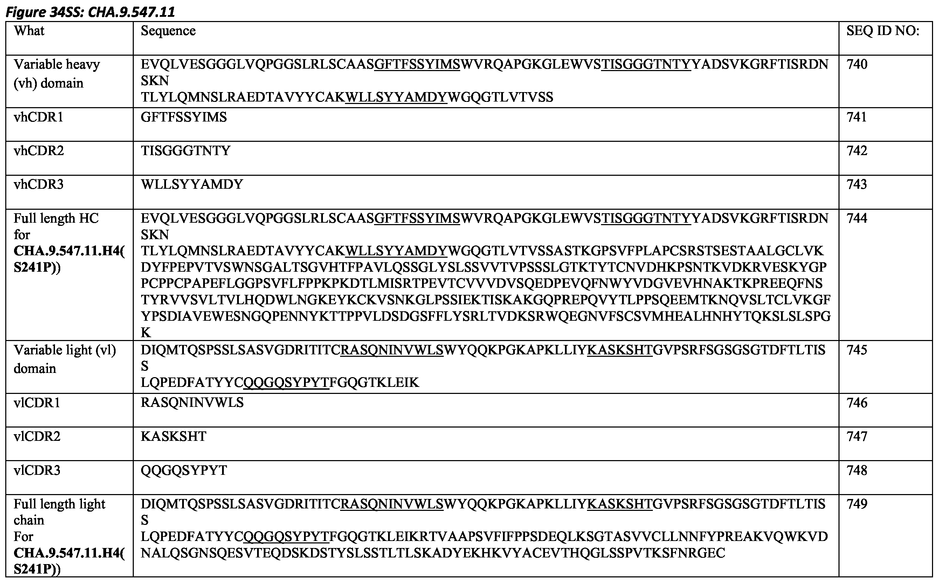

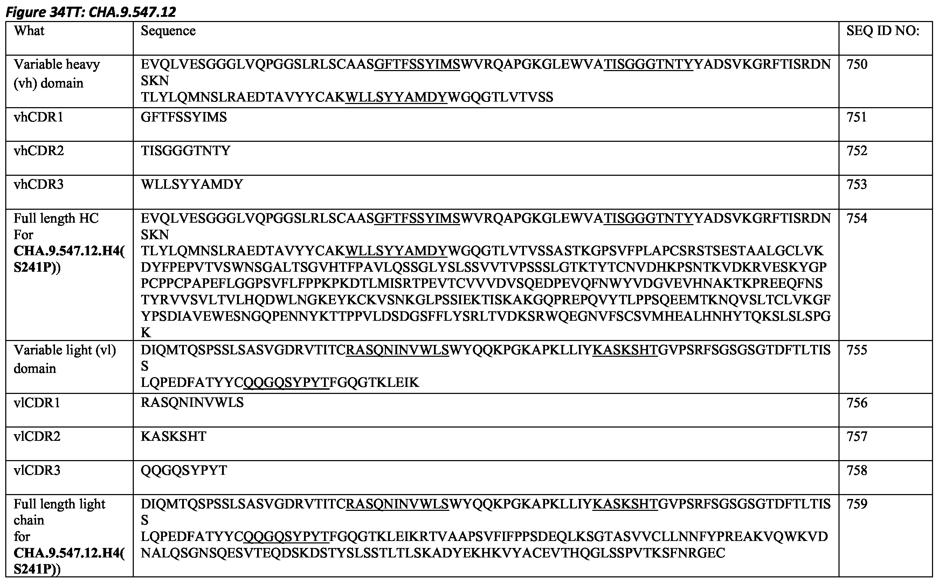

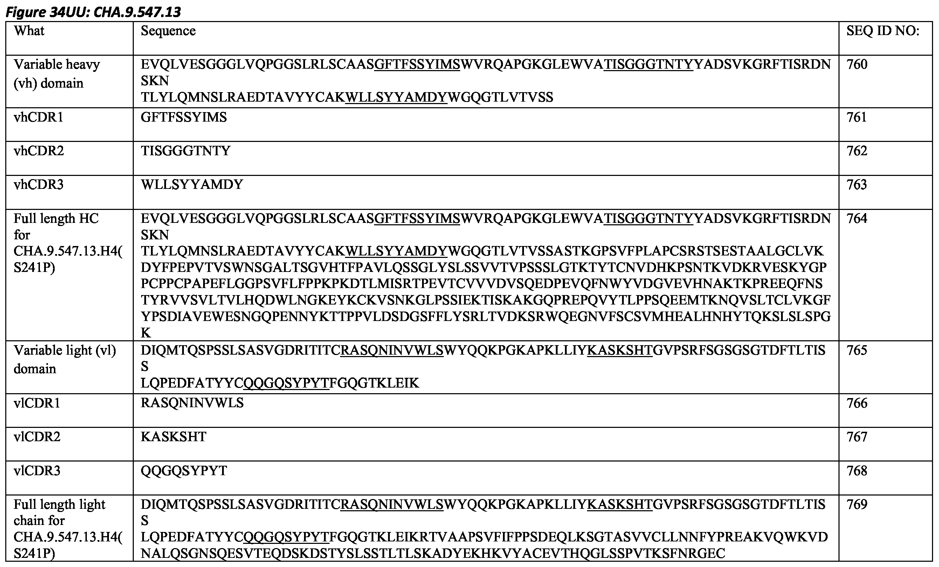

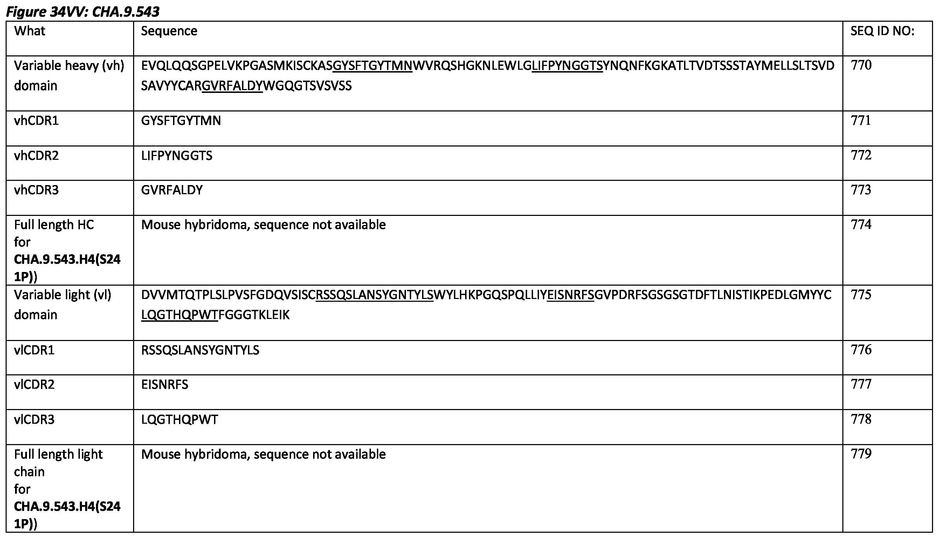

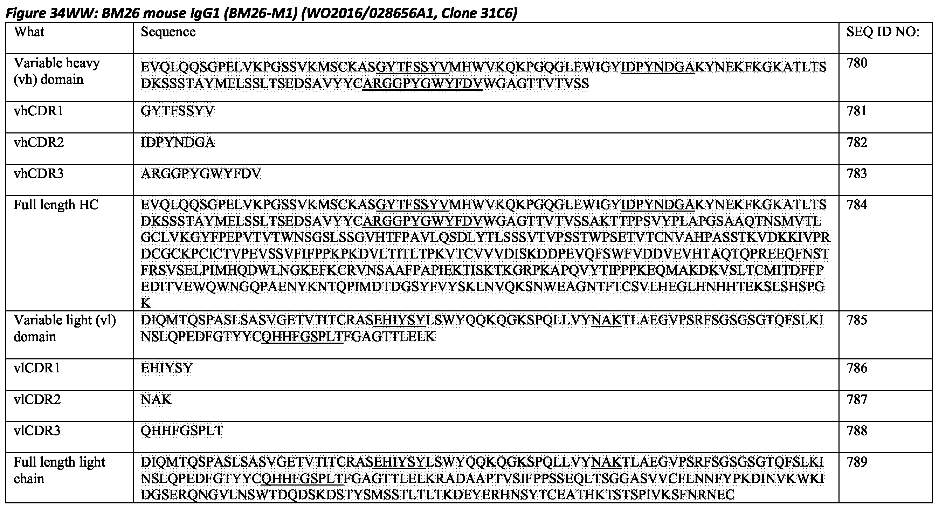

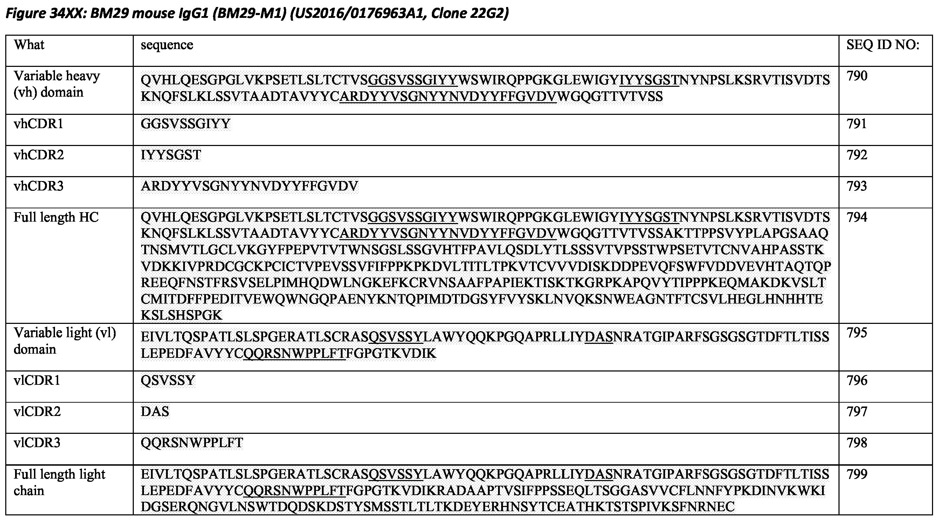

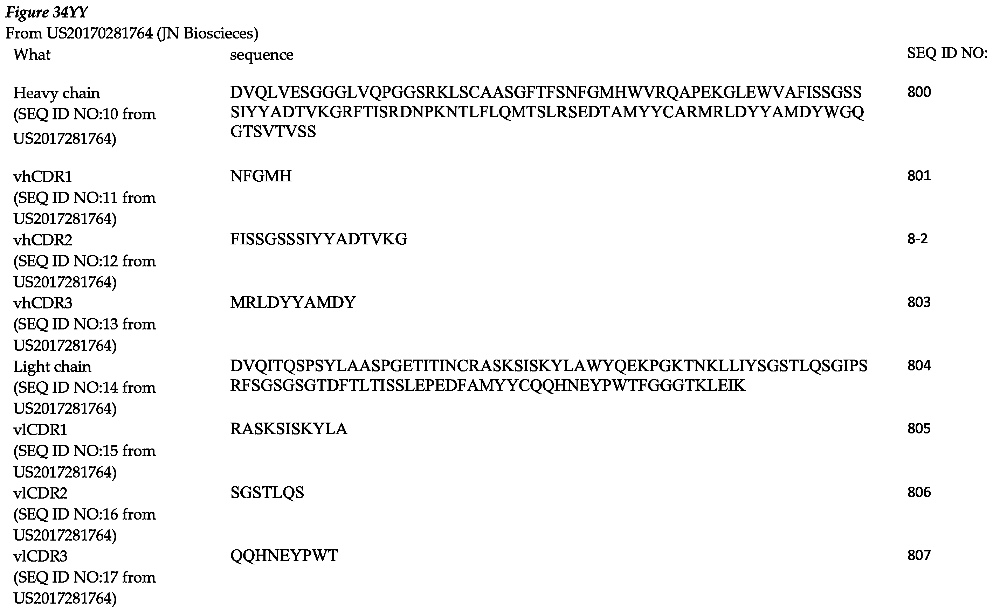

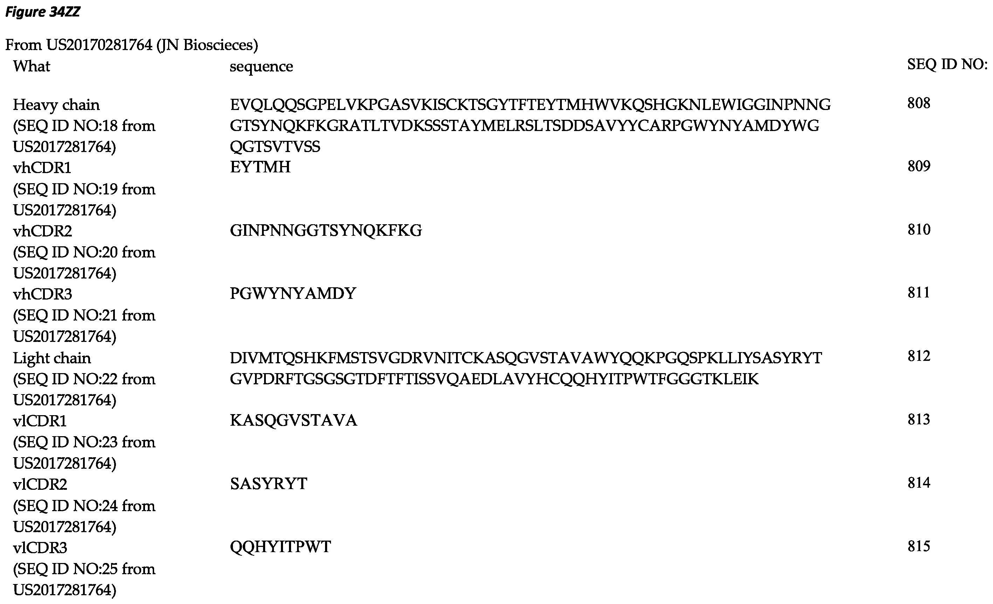

In some embodiments, the anti-IL18-BP antibodies are used in combination with one or more anti-TIGIT antibodies. Examples of anti-TIGIT antibodies include for example but are not limited to CPA.9.083.H4(S241P), CPA.9.086.H4(S241P), CHA.9.547.7.H4(S241P), CHA.9.547.13.H4(S241P), CPA.9.018, CPA.9.027, CPA.9.049, CPA.9.057, CPA.9.059, CPA.9.083, CPA.9.086, CPA.9.089, CPA.9.093, CPA.9.101, CPA.9.103, CHA.9.536.3.1, CHA.9.536.3, CHA.9.536.4, CHA.9.536.5, CHA.9.536.7, CHA.9.536.8, CHA.9.560.1, CHA.9.560.3, CHA.9.560.4, CHA.9.560.5, CHA.9.560.6, CHA.9.560.7, CHA.9.560.8, CHA.9.546.1, CHA.9.547.1, CHA.9.547.2, CHA.9.547.3, CHA.9.547.4, CHA.9.547.6, CHA.9.547.7, CHA.9.547.8, CHA.9.547.9, CHA.9.547.13, CHA.9.541.1, CHA.9.541.3, CHA.9.541.4, CHA.9.541.5, CHA.9.541.6, CHA.9.541.7 and CHA.9.541.8, CHA.9.547.18 as disclosed in WO 2018/220446 and other antibodies under clinical study, for example, EOS-448 (GlaxoSmithKline, iTeos Therapeutics), BMS-986207, domvanalimab (AB154, Arcus Biosciences, Inc.), AB308 (Arcus Bioscience), Ociperlimab (aBGB-A1217, BeiGene), Tiragolumab (MTIG7192A, Roche), BAT6021 (Bio-Thera Solutions), BAT6005 (Bio-Thera Solutions), IBI939 (Innovent Biologics, US2021/00040201), JS006 (Junshi Bioscience/COHERUS), ASP8374 (Astellas Pharma Inc), Vibostolimab (MK-7684, Merck Sharp & Dohme), M6332 (Merck KGAA), Etigilimab (OMP-313M32, Mereo BioPharma), SEA-TGT (Seagen)y, HB0030 (Huabo Biopharma), AK127 (AKESO), or anti-TIGIT antibodies include the Genentech antibody (MTIG7192A). In some embodiments, the anti-TIGIT antibodies are as described in U.S. 9,713,364 (including MAB1, MAB2, MAB3, MAB4, MAB5, MAB6, MAB7, MAB8, MAB9, MAB10, MAB11, MAB12, MAB13, MAB14, MAB15, MAB16, MAB17, MAB18, 40 MAB19, MAB20, and/or MAB21), anti-TIGIT antibodies are as described in US Patent No. 9,499,596, anti-TIGIT antibodies are as described in WO 2016/191643, anti-TIGIT antibodies are as described in WO 2017/053748, anti-TIGIT antibodies are as described in WO2016/191643, anti-TIGIT antibodies are as described in WO 2016/028656, anti-TIGIT antibodies are as described in WO 2017/030823, anti-TIGIT antibodies are as described in US 2016/0176963, anti-TIGIT antibodies are as described in WO 2017/037707, anti-TIGIT antibodies are as described in WO 2017/059095, anti-TIGIT antibodies are as described in U.S. 2017281764, anti-TIGIT antibodies as described in WO 2015/009856, the anti-TIGIT antibody is an antibody described in any of US 2017/0037133, the anti-TIGIT antibody as described in WO 2017/048824 (including 10A7, 1F4, 14A6, 28H5, 31C6, 15A6, 22G2, 11G11, and/or 10D7) as disclosed in, anti-TIGIT antibody is one of those described in International Patent Publication WO 2016/028656. In some embodiments, the anti-TIGIT antibodies, usually full length or scFv domains, that comprise the following CHA sets of CDRs, the sequences of which are shown in Figure 30A: CPA.9.083.H4(S241P)vhCDR1, CPA.9.083.H4(S241P)vhCDR2, CPA.9.083.H4(S241P)vhCDR3, CPA.9.083.H4(S241P)vlCDR1, CPA.9.083.H4(S241P)vlCDR2, and CPA.9.083.H4(S241P)vlCDR3. In some embodiments, the anti-TIGIT antibodies, usually full length or scFv domains, that comprise the following CHA sets of CDRs, the sequences of which are shown in Figure 30B: CPA.9.086.H4(S241P)vhCDR1, CPA.9.086.H4(S241P)vhCDR2, CPA.9.086.H4(S241P)vhCDR3, CPA.9.086.H4(S241P)vlCDR1, CPA.9.086.H4(S241P)vlCDR2, and CPA.9.086.H4(S241P)vlCDR3. Such anti-TIGIT antibodies can be used in combination with the anti-IL18-BP antibodies of the invention. Additional exemplary anti-TIGIT antibody sequences are shown in Figure 34.

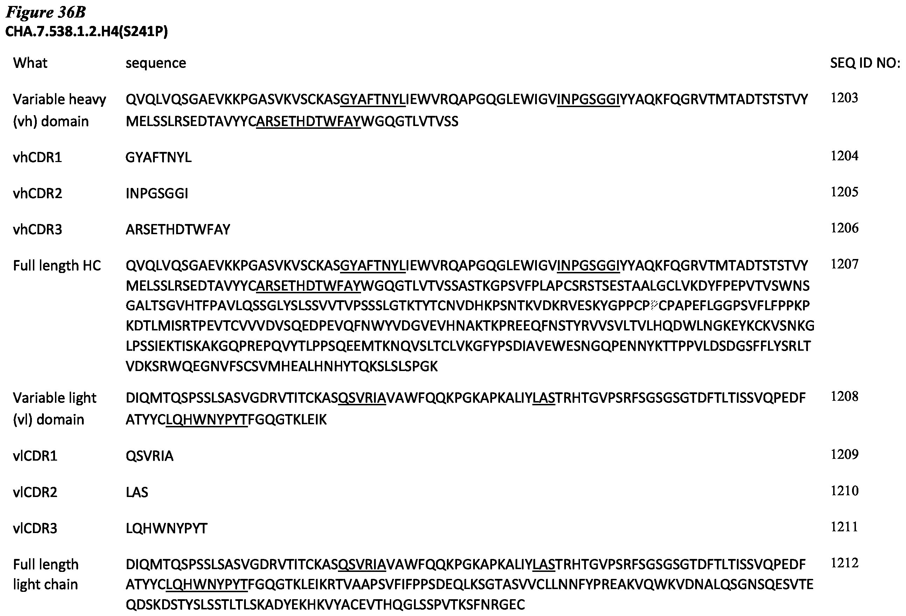

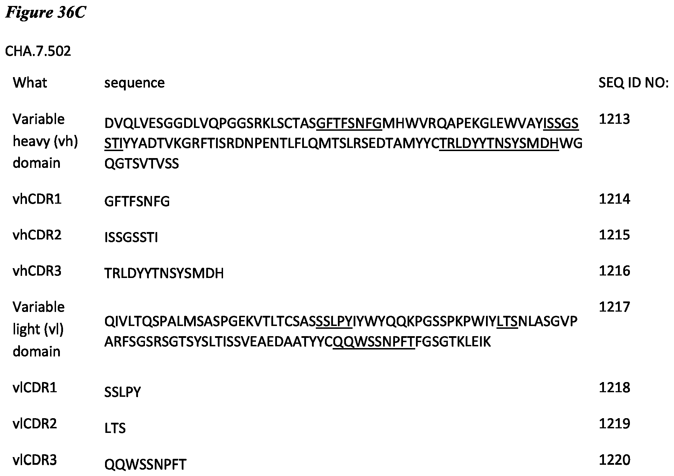

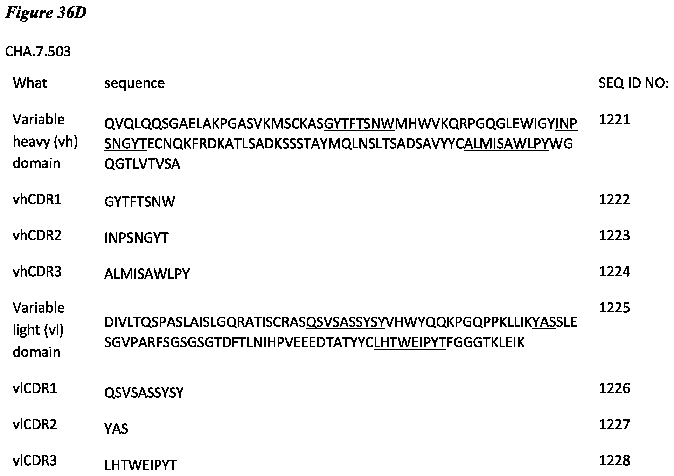

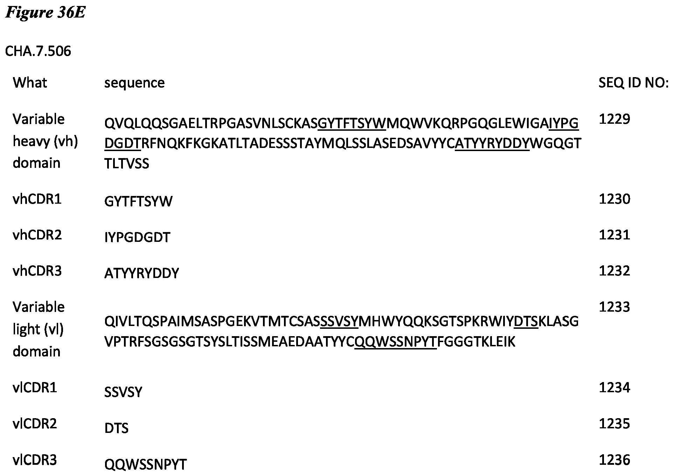

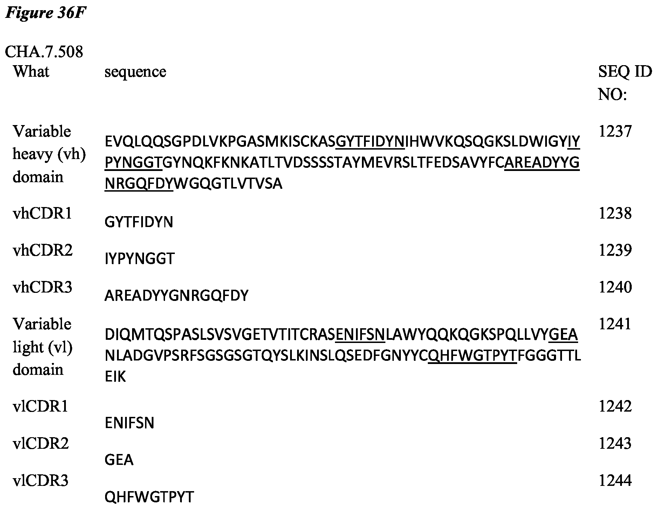

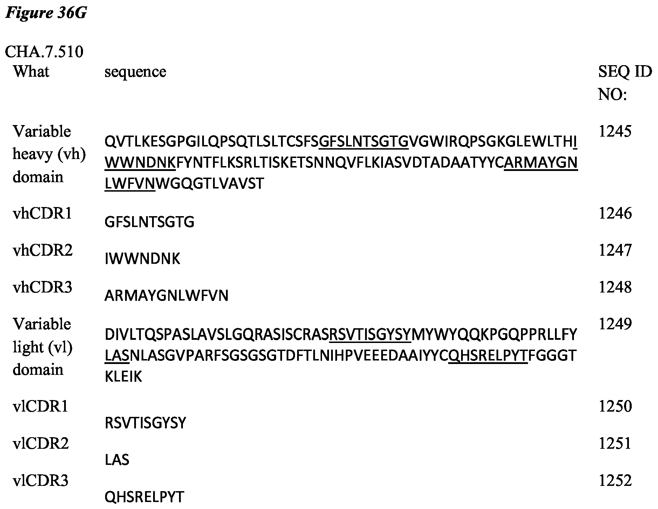

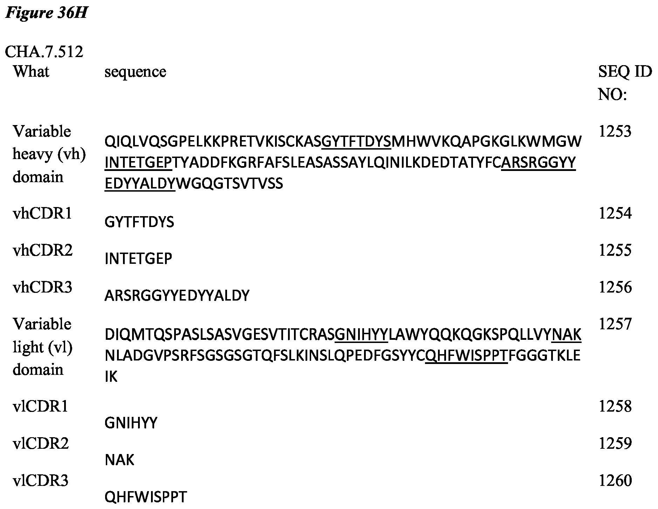

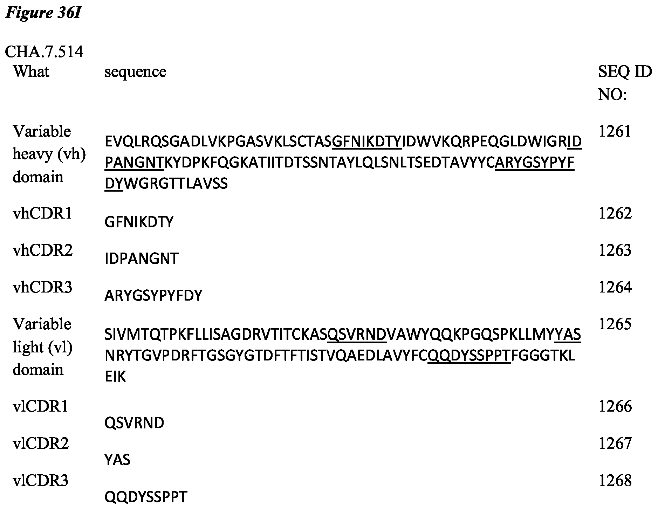

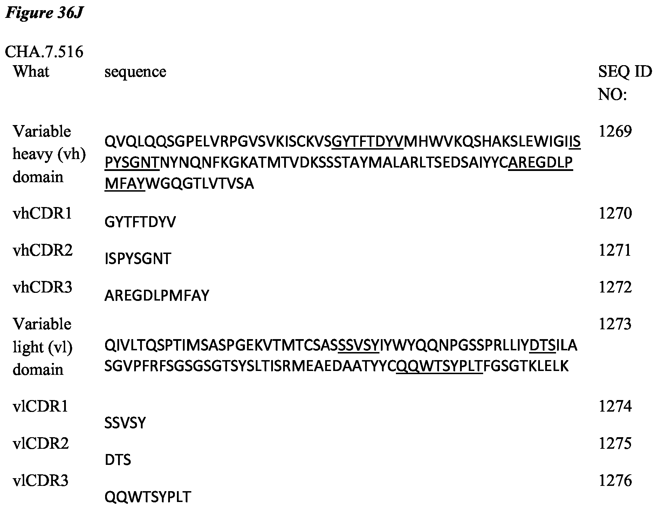

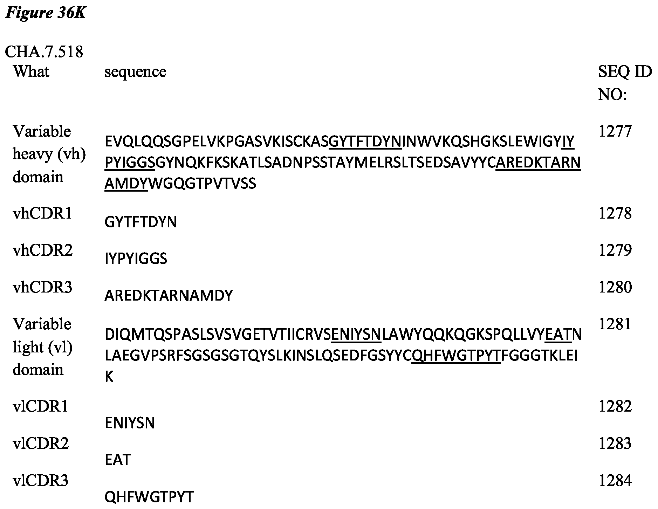

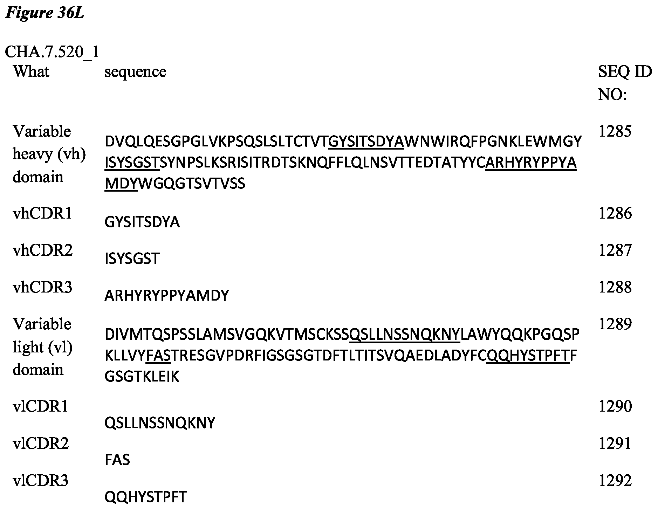

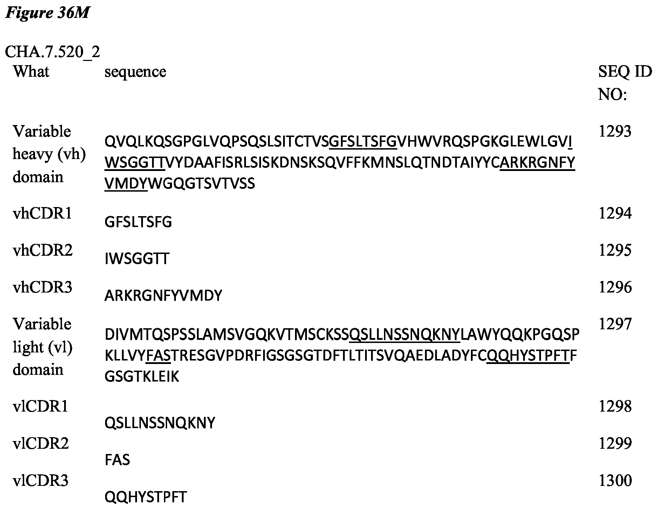

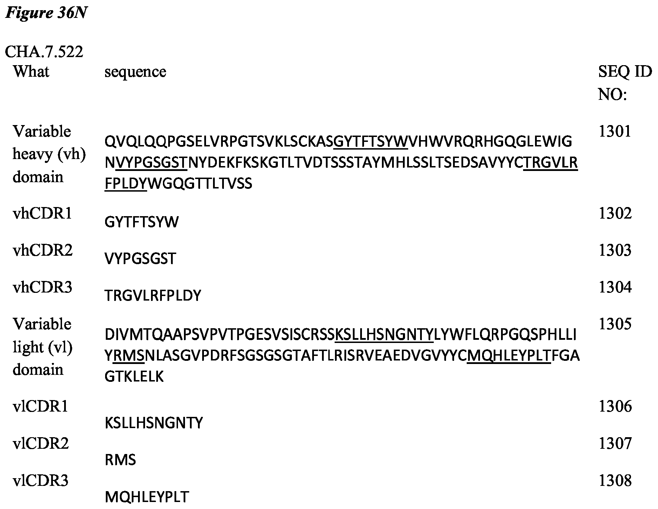

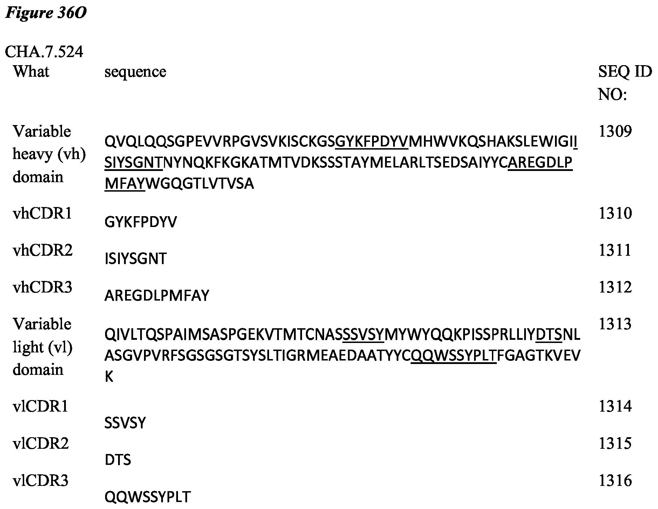

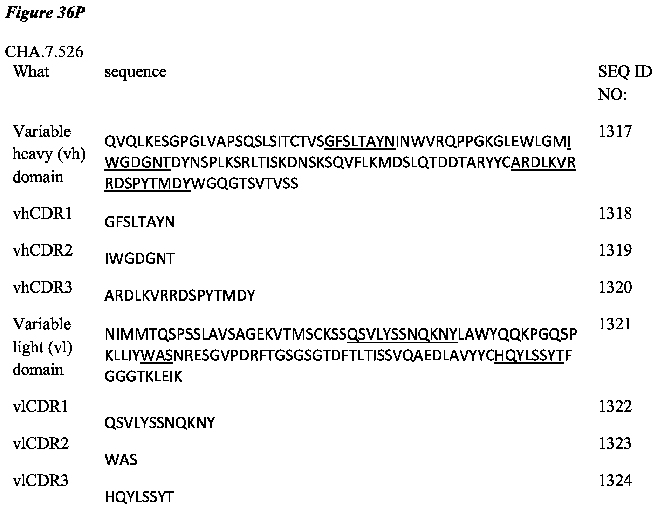

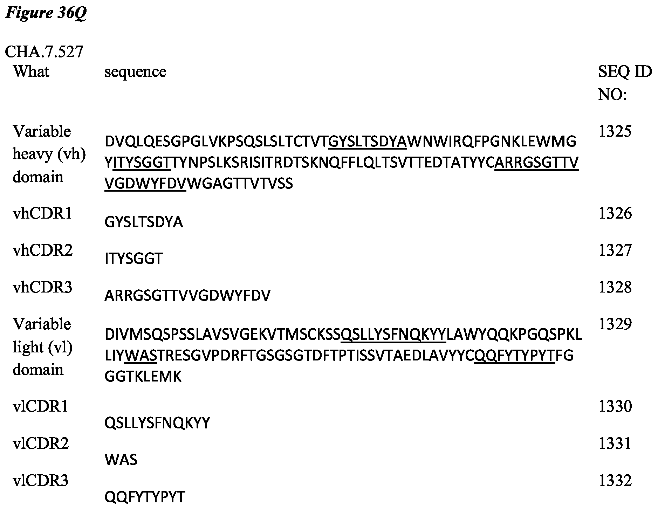

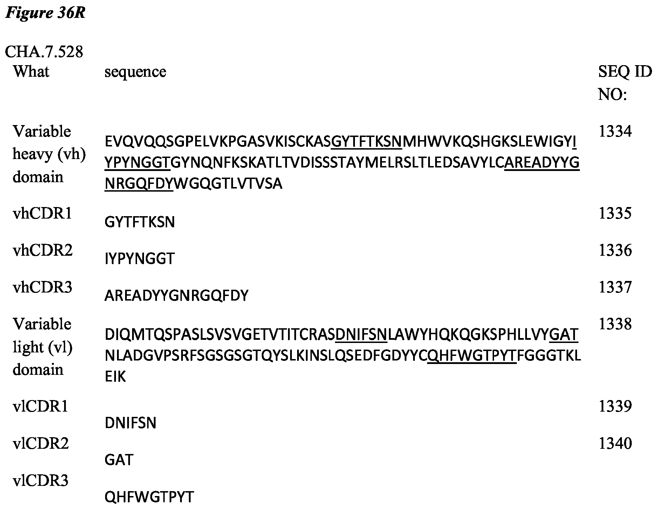

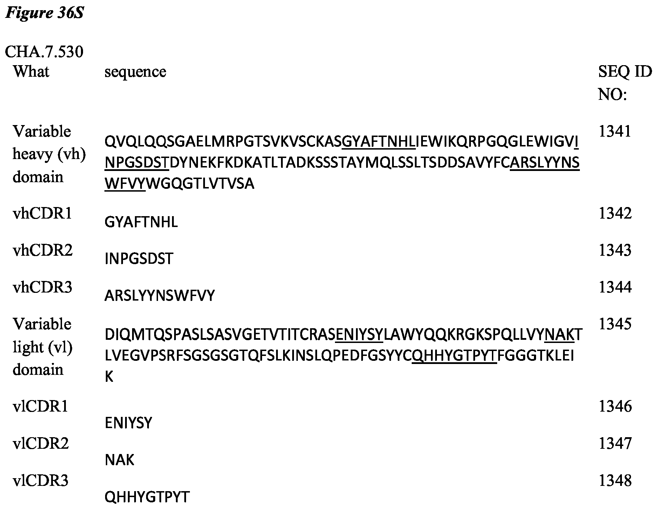

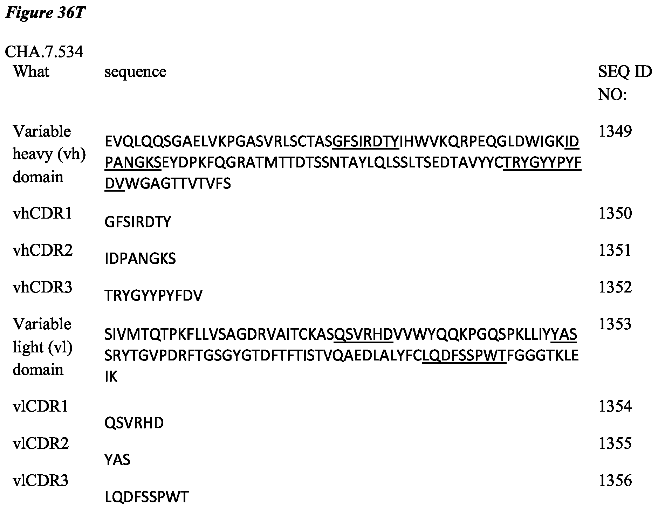

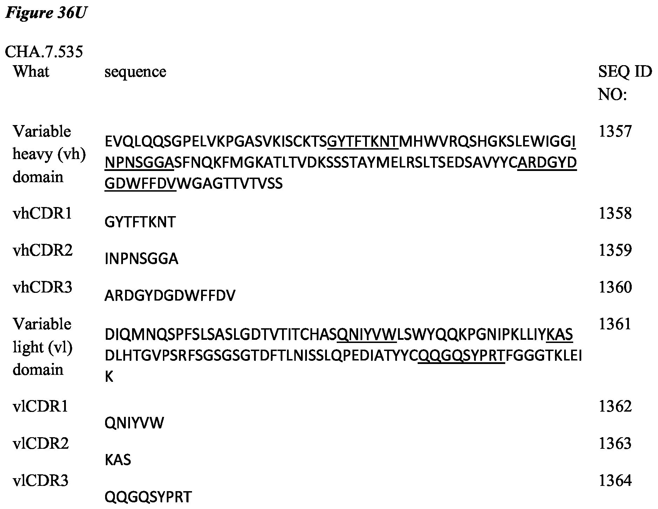

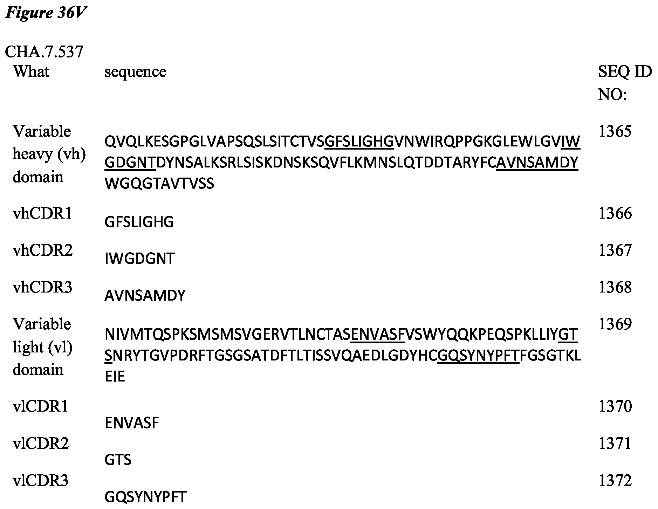

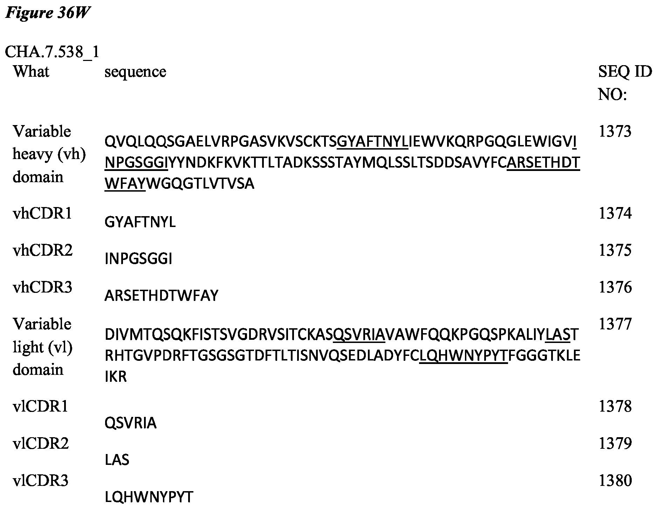

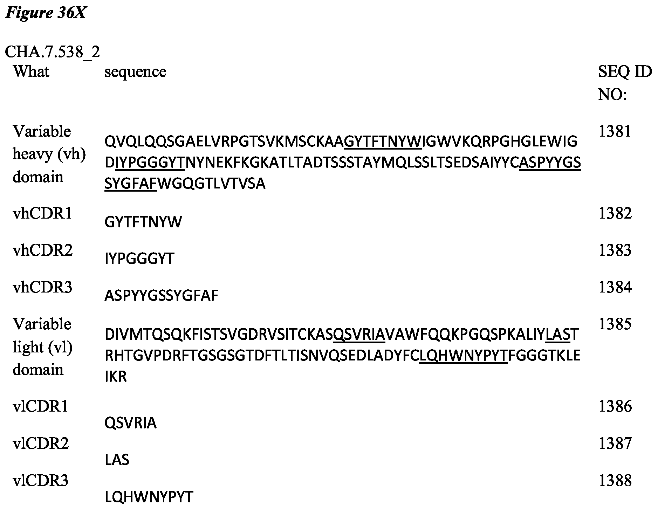

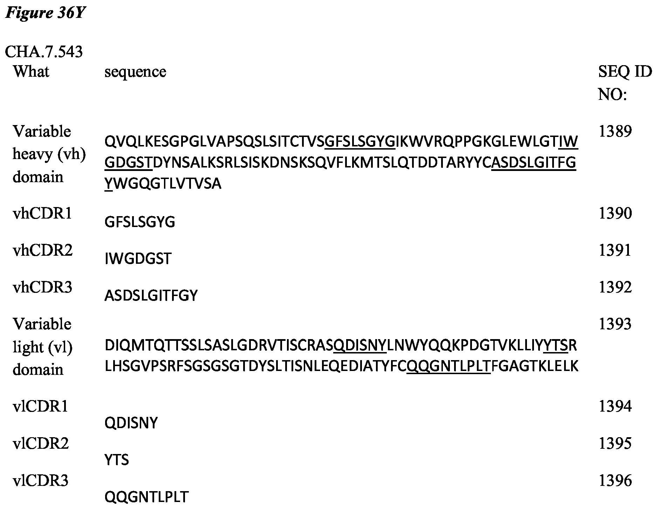

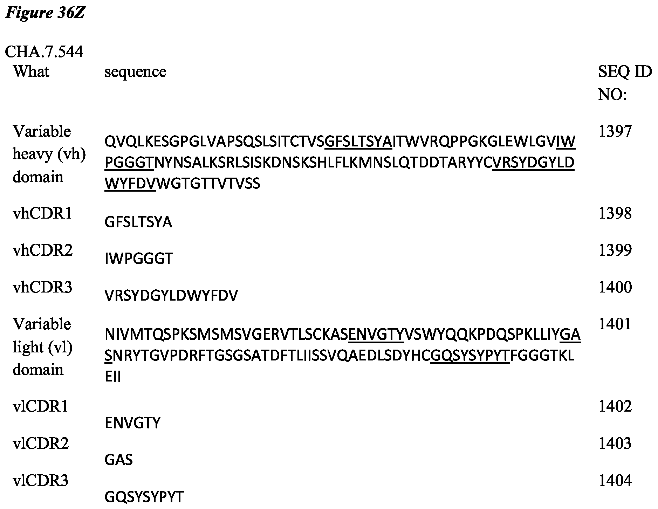

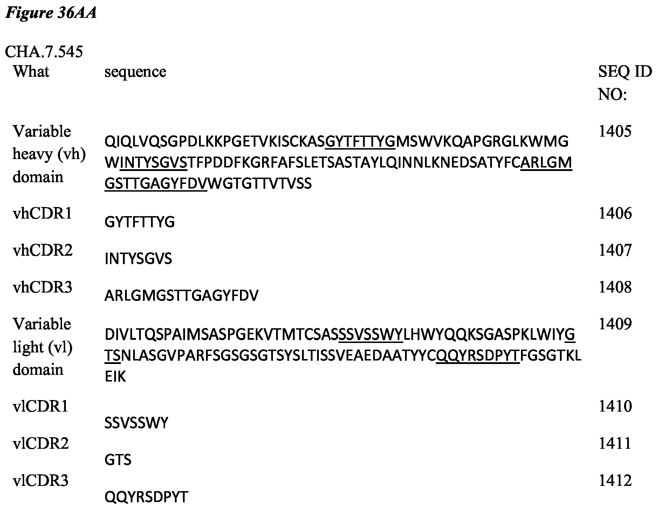

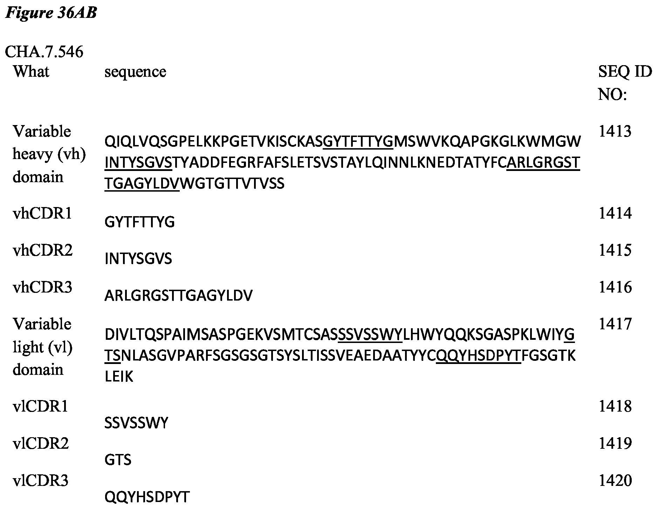

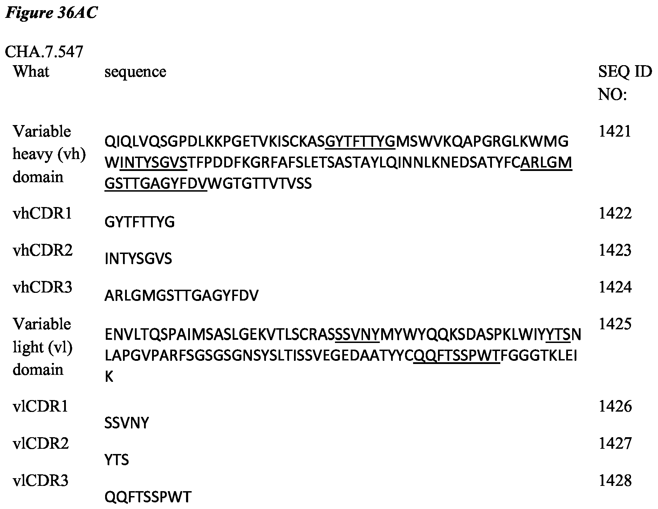

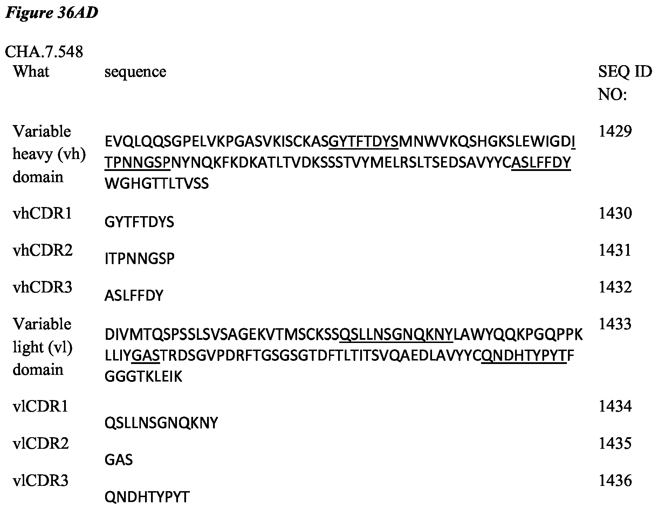

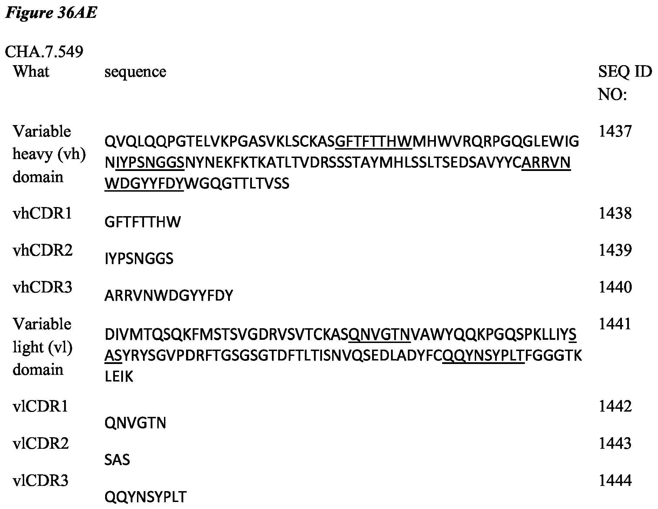

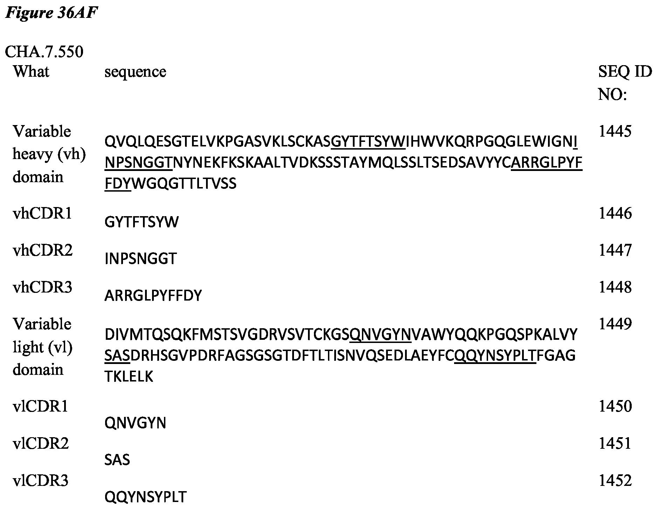

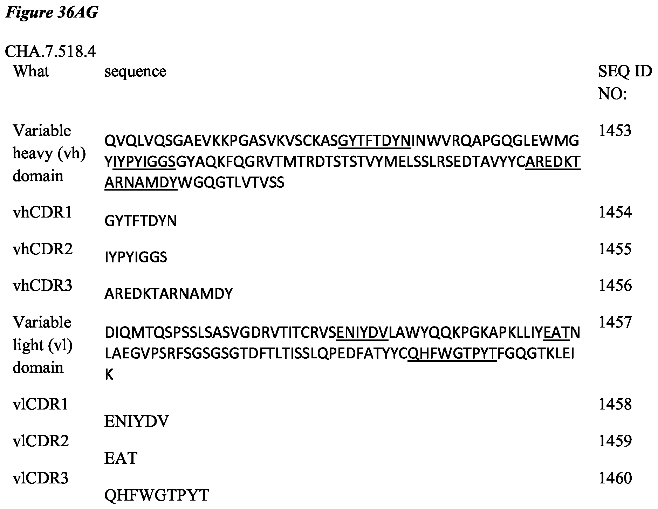

In some embodiments, the anti-IL18-BP antibodies are used in combination with one or more anti-PVRIG antibodies. Examples of anti-PVRIG antibodies include for example but are not limited to but are not limited to CHA.7.518.1.H4(S241P), CHA.7.538.1.2.H4(S241P), and CHA.7.502, CHA.7.503, CHA.7.506, CHA.7.508, CHA.7.510, CHA.7.512, CHA.7.514, CHA.7.516, CHA.7.518.1.H4(S241P), CHA.7.518, CHA.7.518.4, CHA.7.520.1, CHA.7.520.2, CHA.7.522, CHA.7.524, CHA.7.526, CHA.7.527, CHA.7.528, CHA.7.530, CHA.7.534, CHA.7.535, CHA.7.537, CHA.7.538.1.2.H4(S241P), CHA.7.538.1, CHA.7.538.2, CHA.7.543, CHA.7.544, CHA.7.545, CHA.7.546, CHA.7.547, CHA.7.548, CHA.7.549, CHA.7.550, CPA.7.001, CPA.7.003, CPA.7.004, CPA.7.006, CPA.7.008, CPA.7.009, CPA.7.010, CPA.7.011, CPA.7.012, CPA.7.013, CPA.7.014, CPA.7.015, CPA.7.017, CPA.7.018, CPA.7.019, CPA.7.021, CPA.7.022, CPA.7.023, CPA.7.024, CPA.7.033, CPA.7.034, CPA.7.036, CPA.7.040, CPA.7.046, CPA.7.047, CPA.7.049, and CPA.7.050, as disclosed in WO 2018/220446A9 and other antibodies under clinical study, for example, GSK4381562/SRF816 (GSK/Surface), NTX2R13(Nectin Therapeutics). In some embodiments, the antibody sequences is from WO 201/6134333. In some embodiments, the anti-PVRIG antibodies, usually full length or scFv domains, comprise the following CHA sets of CDRs, the sequences of which are shown in Figure 29A: CHA.7.518.1.H4(S241P)vhCDR1, CHA.7.518.1.H4(S241P)vhCDR2, CHA.7.518.1.H4(S241P)vhCDR3, CHA.7.518.1.H4(S241P)vlCDR1, CHA.7.518.1.H4(S241P)vlCDR2, and CHA.7.518.1.H4(S241P)vlCDR3. In some embodiments, the anti- PVRIG antibodies, usually full length or scFv domains, that comprise the following CHA sets of CDRs, the sequences of which are shown in Figure 30B: CHA.7.538.1.2.H4(S241P)vhCDR1, CHA.7.538.1.2.H4(S241P)vhCDR2, CHA.7.538.1.2.H4(S241P)vhCDR3, CHA.7.538.1.2.H4(S241P)vlCDR1, CHA.7.538.1.2.H4(S241P)vlCDR2, and CHA.7.538.1.2.H4(S241P)vlCDR3. Such anti-PVRIG antibodies can be used in combination with the anti-IL18-BP antibodies of the invention. Additional exemplary anti-PVRIG antibody sequences are shown in Figures 36, 37, and 38.

### b. Other cancer combination therapies

The anti-IL-18BP antibodies of the present invention may be administered in combination with one or more other prophylactic or therapeutic agents, including but not limited to cytotoxic agents, chemotherapeutic agents, cytokines, growth inhibitory agents, anti-hormonal agents, kinase inhibitors, anti-angiogenic agents, cardioprotectants, immunostimulatory agents, immunosuppressive agents, agents that promote proliferation of hematological cells, angiogenesis inhibitors, protein tyrosine kinase (PTK) inhibitors, or other therapeutic agents.

In this context, a "chemotherapeutic agent" is a chemical compound useful in the treatment of cancer. Examples of chemotherapeutic agents include alkylating agents such as thiotepa and cyclosphosphamide, alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylolomelamine; acetogenins (especially bullatacin and bullatacinone); delta-9-tetrahydrocannabinol (dronabinol, MARINOL'); beta-lapachone; lapachol; colchicines; betulinic acid; a camptothecin (including the synthetic analogue topotecan (HYCAMTN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (*e.g*., calicheamicin, especially calicheamicin gammall and calicheamicin omegall (*see, e.g.,* Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholinodoxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK.RTM. polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoids, e.g., paclitaxel (TAXOL^{®}; Bristol-Myers Squibb Oncology, Princeton, N.J.), ABRAXANE^{®}, cremophorfree, albumin-engineered nanoparticle formulation of paclitaxel (American Pharmaceutical Partners, Schaumberg, Ill.), and docetaxel (TAXOTERE^{®}; Rhone-Poulenc Rorer, Antony, France); chloranbucil; gemcitabine (GEMZARM^{®}); 6-thioguanine; mercaptopurine; methotrexate; platinum analogs such as cisplatin and carboplatin; vinblastine (VELBAN^{®}); platinum; etoposide (VP-16); ifosfamide; mitoxantrone; vincristine (ONCOVIN^{®}); oxaliplatin; leucovovin; vinorelbine (NAVELBINE^{®}); novantrone; edatrexate; daunomycin; aminopterin; ibandronate; topoisomerase inhibitor RFS 2000; difluoromethylornithine (DMFO); retinoids such as retinoic acid; capecitabine (XELODA^{®}); pharmaceutically acceptable salts, acids or derivatives of any of the above; as well as combinations of two or more of the above such as CHOP, an abbreviation for a combined therapy of cyclophosphamide, doxorubicin, vincristine, and prednisolone; CVP, an abbreviation for a combined therapy of cyclophosphamide, vincristine, and prednisolone; and FOLFOX, an abbreviation for a treatment regimen with oxaliplatin (ELOXATIN^{®}) combined with 5-FU and leucovorin.

In some embodiments, the chemotherapeutic agent is selected from the group consisting of Platinum, Oxaliplatin, Cisplatin, Paclitaxel (taxol), Sorafenib, Doxorubicin, Sorafenib, 5-FU, and Gemcitabine, Irinotecan (CPT-11).

In some embodiments, the other therapeutic is an agent used in radiation therapy for the treatment of cancer. Accordingly, in some embodiments, the active agents described herein are administered in combination with one or more of platinum coordination compounds, topoisomerase inhibitors, antibiotics, antimitotic alkaloids and difluoronucleosides.

In some embodiments, the anti-IL18BP antibody is in combination with one or more inflammasome activators. In some embodiments, the inflammasome activator is an CD39 inhibitor. In some embodiments, the CD39 inhibitor is an anti-CD39 antibody.

According to at least some embodiments, the anti IL 18BP antibodies could be used in combination with any of the known in the art standard of care cancer treatment (as can be found, for example, on the World Wide Web at cancer.gov/cancertopics).

### EXAMPLES

### EXAMPLE 1: EXPRESSION OF IL18 AND IL18BP IN THE TUMOR MICROENVIRONMENT

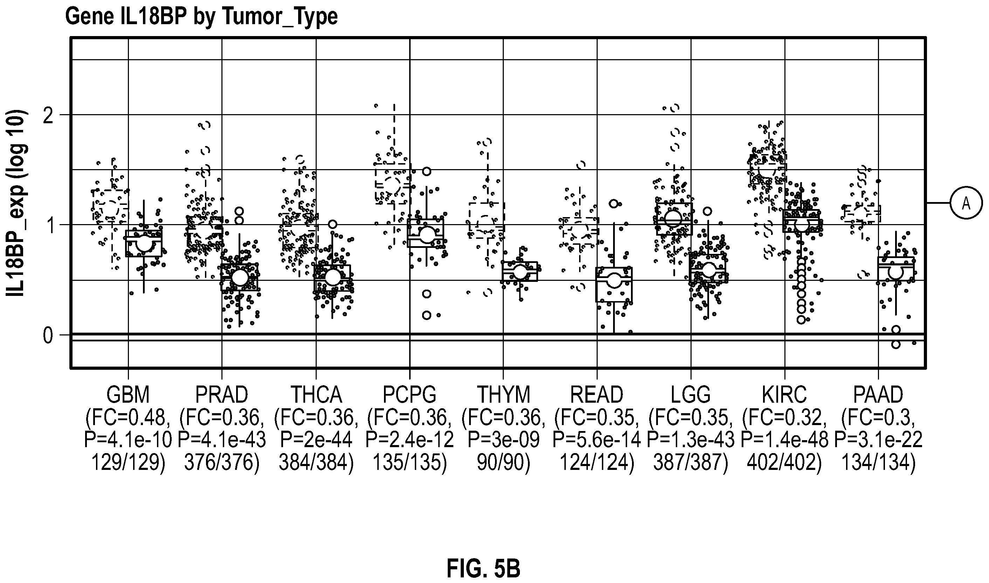

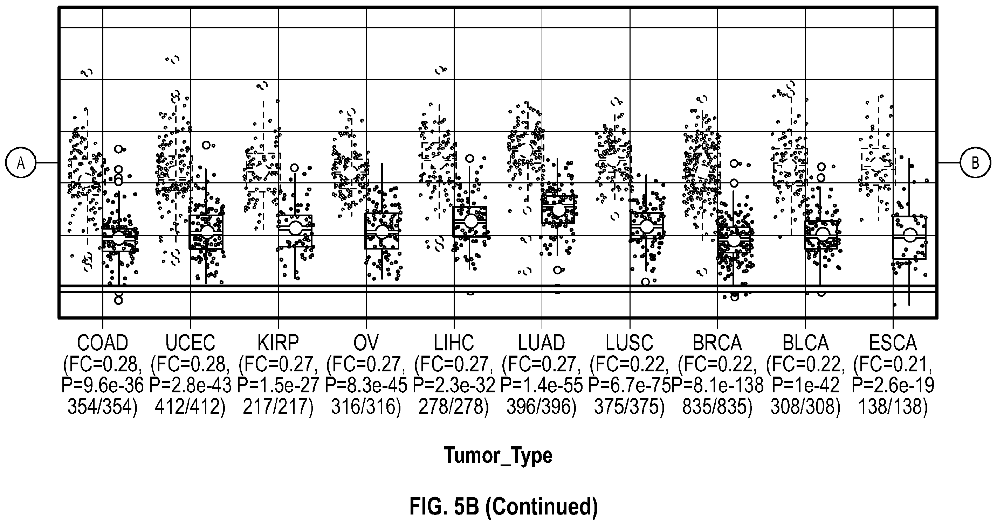

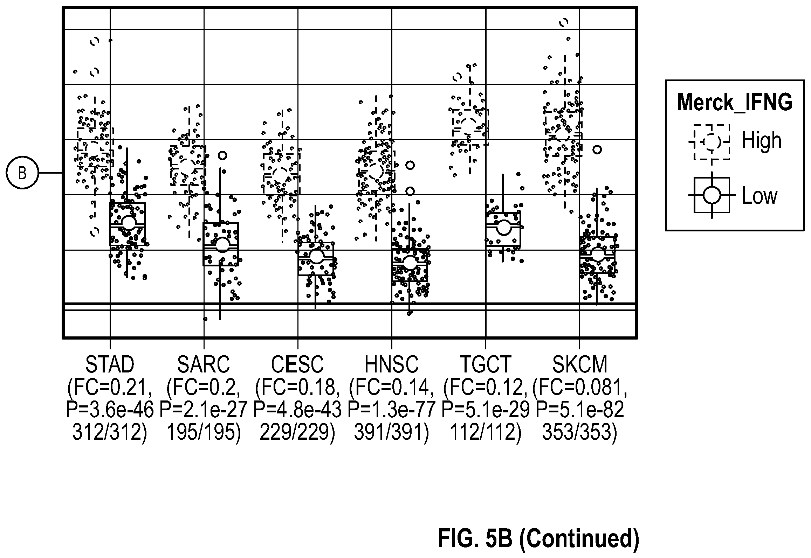

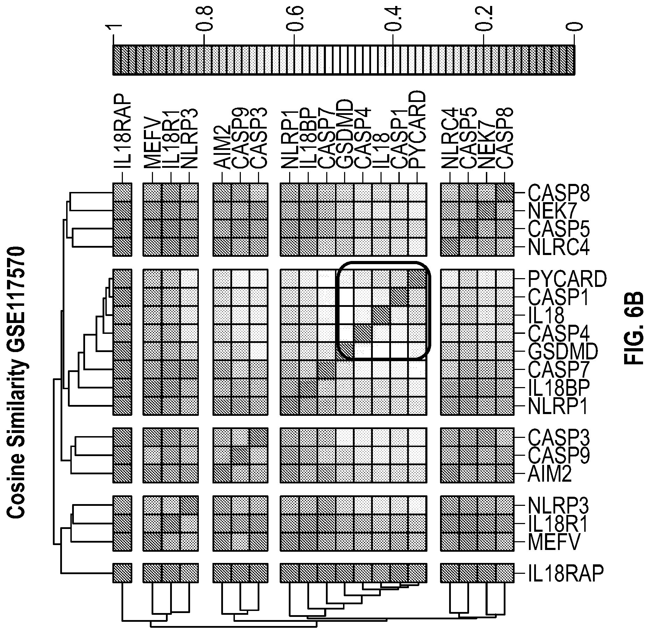

IL18-BP is a sequester for IL18 and results in inhibition of IL18 activity (Dinarello, et al., Front. Immunol., 1:1-10 (2013). Therefore, both IL18-BP, the target of antibody, as well as IL18 need to be present in the TME (Tumor Micro Environment) in order for the blocking of IL18-BP to be effective. Figure 4 shows the expression of both IL18 (Figure 4A) and IL18-BP (Figure 4B) and demonstrated that both proteins are expressed across all TCGA tumors, with only Pheochromocytoma and Paraganglioma (see Table 1 for TCGA tumor type abbreviations; exhibiting somewhat limited expression for IL18 in a subset of these tumor type (reference line at 1RPKM donates background expression levels below it). The eventual target cells for free IL18 are leukocyte/lymphocytes (Tominaga, K., et al., International Immunology, 12(2): 151-160 (2000) and Senju, H., et al., Int J Biol Sci., 14(3):331-340 (2018)), and these studies will target tumors which exhibit higher immune presence, as demonstrated by the IFNγ inflammation signature (see, for example, US 2016/0312295 A1). As can be seen in Figure 5, both IL18 (Figure 5A) and IL18-BP (Figure 5B) were prevalent across all tumor subsets with inflamed tumors (high IFNγ signature values). Even in lowest IFNγ subsets a significant expression of both proteins was detected. As IL18 relies on the inflammasome for its secretion, a signature of core inflammasome genes was generated. These genes are common to multiple types of inflammasome activation signals (Chauhan, D., et al., Immunological Reviews, 297:123-138 (2020)). The signature values were calculated as the mean of the log10 RPKM expression values of the genes listed in Table 2. As shown in Figure 6A, the core inflammasome signature was highly expressed in all TMEs, and in general had a higher expression in inflamed (high IFNγ signature) subsets. This pattern was very similar to the one presented for IL18 (Figure 5A) indicating that the machinery for IL18 was present in all inflamed (high IFNγ signature) tumors and most low IFNγ signature tumors, except for LGG and PCPG. In order to validate that indeed IL18 and the inflammasome core signature genes were expressed in the same cells in the TME single cell data was used and the cosine similarity between IL18, IL18-BP, and the core inflammasome genes was calculated, as well as additional upstream genes and the IL18 receptors. In Figure 6B, the cosine similarity matrix for macrophages in NSCLC are presented, indicating that indeed IL18 and the core inflammasome signature were present in the same cells. Similar results were obtained in additional tumor types (data not shown). Specifically, in breast cancer it was reported that Triple Negative breast cancer has more inflamed characteristics in about 30% of the cases, as compared to ~5-10% in hormone receptor positive types (Thomas, F., et al., Frontiers in Oncology, 10:1-17 (2021)). Therefor we checked specifically in the expression of IL18 and IL18-BP in breast cancer by single cell (raw data adopted from Bassez, A., et al., Nature Medicine, 27:820-832 (2021)). In Figure 7A, it is shown that both IL18 and IL18-BP are more abundant in TNBC, both by precent of expressing cells and average level of expression, flowed by HER2+ subset and with minimal expression in hormone receptor positive subsets, especially in the pre-treatment samples. In this specific dataset the patients were sampled pre-treatment then neoadjuvant treatment with aPD1 or aPD1 with chemotherapy was administered flowed by resection of the tumor (on treatment biopsies). The authors measured T-cell clonality expansion post treatment, T-cell clonal expansion could be regarded as a surrogate measurement for response to aPD1 treatment (Bassez et al., Nature Medicine, 27:820-832 (2021)). Figure 7B demonstrates that baseline levels of IL18 were lower in non-expanding patients while IL18-BP is higher in these patients. This could be an indicator for the potential role of IL18-BP to attenuate the IL18 activity and hamper the activity of immune checkpoint blockade (ICB) treatment. Both genes were up regulated post aPD1 treatment. These observations strengthen the selection of more inflamed indication in general and specifically TNBC.

### Materials and methods

### Preprocessing, filtering, and normalization

UMIs were quantified using Cellranger 3.0.2 (10x Genomics) with reference transcriptome GRCh38. Subsequent analyses were performed using "Seurat " (https://satijalab.org/seurat/), if not stated otherwise.

### Clustering and cell type annotation

Top 15 principal components were used to construct SNN graph and UMAP embedding. Cell annotation for Thomas et al. 2021 was based on authors submitted metadata.

Cells are clustered by a nested PCA. The clusters are annotated by their gene signatures expression.

Cosine similarity matrix for a desired set of genes is computed by computing for each pair of genes and their expression vectors the value of formulae (i) . $\frac{\left〈\vec{x}, \vec{y}\right〉}{\left‖\vec{x}\right‖ \left‖\vec{y}\right‖}$

The IFNγ inflammation signature (as described in, US 2016/0312295 A1) is calculated in three steps:
1. An IFNγ_up signature is calculated as the mean of the log10 RPKM expression values of the following genes:
   CCR5, HLA-DRA, CXCL13, CCL5, STAT1, KLRK1, NKG7, CXCL9, LAIR1, LAG3, CXCR6, KLRD1, GZMA, PRF1, SIGLEC14, PTPN22, CD86, SLA, SIRPG, CD72, HAVCR2, PSTPIP2, SLAMF6, CD84, CD300LF, CD3D, IFNG, CXCL11, CD2, CTSZ, GZMB, IL2RG, CXCL10, LILRB4, PDCD1, CCL8, CIITA, CCL4, IGSF6, PTPRC, CLEC9A, CST7, MYLIP, ITGAL, CDH1, PSTPIP1, GZMK, HLA-E, CD3E, TAGAP, TNFRSF9
2. The IFNγ_down signature is calculated as the mean of the log10 RPKM expression values of the following genes:
   CLEC3B, NR4A2, EEF1G, PIK3CA, TYRO3, CX3CL1, ING1, BST1, ACKR3, UBB, PPARG, PTEN, THY1, CLCA1, EFEMP1, GAS6, ITM2A, CD55, NFATC1, BCL6, RETNLB, PDCD4, TIMP3, CDO1, POLR1B, DDR1, F2R, CTSG, LILRA5, CX3CR1, TBP, CLEC1B, RGS16, PTPN13, IRF1, MON1B, CPD, PHACTR2, OAZ1, CASP3, IFI16, ITGA1, RPL19, CCR6, LTK, C10orf54, SLAMF1, and TNFAIP8L2
3. Finally, the IFNγ inflammation signature is calculated as the difference between the two signaures: *IFNy = IFN*γ*_up - IFN*γ*_down.*

### Clustering and cell type annotation

Top 15 principal components were used to construct SNN graph and UMAP embedding.

**Table 1 TCGA tumor abbreviation**

| **Study Abbreviation** | **Study Name** |
|---|---|
| LAML | Acute Myeloid Leukemia |
| ACC | Adrenocortical carcinoma |
| BLCA | Bladder Urothelial Carcinoma |
| LGG | Brain Lower Grade Glioma |
| BRCA | Breast invasive carcinoma |
| CESC | Cervical squamous cell carcinoma and endocervical adenocarcinoma |
| CHOL | Cholangiocarcinoma |
| LCML | Chronic Myelogenous Leukemia |
| COAD | Colon adenocarcinoma |
| CNTL | Controls |
| ESCA | Esophageal carcinoma |
| FPPP | FFPE Pilot Phase II |
| GBM | Glioblastoma multiforme |
| HNSC | Head and Neck squamous cell carcinoma |
| KICH | Kidney Chromophobe |
| KIRC | Kidney renal clear cell carcinoma |
| KIRP | Kidney renal papillary cell carcinoma |
| LIHC | Liver hepatocellular carcinoma |
| LUAD | Lung adenocarcinoma |
| LUSC | Lung squamous cell carcinoma |
| DLBC | Lymphoid Neoplasm Diffuse Large B-cell Lymphoma |
| MESO | Mesothelioma |
| MISC | Miscellaneous |
| OV | Ovarian serous cystadenocarcinoma |
| PAAD | Pancreatic adenocarcinoma |
| PCPG | Pheochromocytoma and Paraganglioma |
| PRAD | Prostate adenocarcinoma |
| READ | Rectum adenocarcinoma |
| SARC | Sarcoma |
| SKCM | Skin Cutaneous Melanoma |
| STAD | Stomach adenocarcinoma |
| TGCT | Testicular Germ Cell Tumors |
| THYM | Thymoma |
| THCA | Thyroid carcinoma |
| UCS | Uterine Carcinosarcoma |
| UCEC | Uterine Corpus Endometrial Carcinoma |
| UVM | Uveal Melanoma |

**Table 2: Inflammasome signature.**

| **Gene name** | **Synonyms** | **Enzyme Commission Number (EC)** |
|---|---|---|
| CASP1 | IL1BC, IL1BCE | 3.4.22.36 |
| CASP4 | ICH2 | 3.4.22.57 |
| CASP5 | ICH3 | 3.4.22.58 |
| PYCARD | ASC, CARD5, TMS1 | |
| GSDMD | DFNA5L, GSDMDC1, FKSG10 | |

### EXAMPLE 2: IL-18BP IS A SOLUBLE IMMUNE CHECKPOINT- RNA EXPRESSION DATA

### Upregulation of IL-18BP in the TME- TCGA vs GTEX

**Figure 47****:** expression of *IL18BP* transcripts in normal (green) or cancer (red) tissues from the TCGA and GTEX databases. GBM, glioblastoma multiforme; HSNC, head and neck squamous carcinoma; KIRC, kidney renal clear cell carcinoma; PAAD, pancreatic adenocarcinoma; SKCM, skin cutaneous melanoma; STAD, stomach adenocarcinoma (**P <* 0.01).

### IL-18BP is Expressed in Suppressive Myeloid Populations and correlate to PD-L1 in the TME Suggesting Resistance Mechanism

As shown in **figure 59A****:** IL-18BP correlates with PD-L1 at RNA level (TCGA) in colon and breast cancers suggesting a resistance mechanism to immune activation in the tumor microenvironment (TME)

As shown in **figure 59B** and **figure 48****:** Single-cell RNA analyses of tumor-infiltrating myeloid cells, including tumor associated macrophages (TAMs) and dendritic cells (DCs) in colon cancer patients showing that IL-18BP is up-regulated in myeloid population in the TME compared to the periphery (PBMCs), suggesting a resistance mechanism to immune activation in the TME.

As shown in **figure 59C****:** Single-cell RNA analyses of tumor-infiltrating myeloid cells, including tumor associated macrophages (TAMs) and dendritic cells (DCs) across indications showing that IL-18BP is up-regulated in myeloid population in the TME compared to the periphery (PBMCs), suggesting a resistance mechanism to immune activation in the TME

### Upregulation of IL-18BP in response to ICB treatment -scRNA/ bulk RNA data

**Figure 60A****-C:** IL-18BP is upregulated (RNA level) following immune checkpoint blockage (ICB) treatment IL-18BP levels are upregulated in the tumor microenvironment (RNA) following treatment with anti-PD-1 (breast and basal cell carcinoma) or anti-PD-1 plus anti CTLA-4 (melanoma) suggesting a potential resistance mechanism.

**Figure 60D****:** IL-18BP is elevated in NSCLC patient serum post aPD-(L)1 treatment Quantification of plasma IL-18BP protein level by ELISA for healthy donors (*n* = 22) and patients with NSCLC (*n* = 52) at baseline before treatment and at the time of the following CT scan after receiving treatment with anti-PD-(L)1 (*n* = 52).

### Association of IL-18BP levels with poor response to aPD-(L)1 blockage: 1) RCC (combo of Pembro+ Lenvatinib) and 2) IL18BP in melanoma responders/NR (Olink)

A supportive data for the role of IL-18BP as a soluble ICP and a potential resistance mechanism to PD1 blockage in Renal Cell Carcinoma patients receiving Pembrolizumab plus Lenvatinib. As can be seen in Figure 61A: High IL-18BP in patient serum pre-treated with Pembrolizumab plus Lenvatinib is associated with shorter progression free survival (PFS). As can be seen in **Figure 61B****:** High IL-18BP in patient serum is pre-treated with Pembrolizumab plus Lenvatinib associated with stable or progressive disease (SD/PD).

A supportive data for the role of IL-18BP as a soluble ICP and a potential resistance mechanism to PD1 blockage in melanoma cancer patients receiving anti PD-1 treatment. As can be seen in Figure 62, high IL-18BP in serum of melanoma cancer patients pre-treated with anti PD-1 is associated with poor response. Raw Olink data (NPX format) Student's T-test was performed for IL 18BP protein after intensity normalization for Target products.

### EXAMPLE 3: INFLAMMASOME INDUCED CYTOKINES SUCH AS IL-18 AND IL-1B ARE ABUNDANT IN THE TME.

Unlike other cytokines, inflammasome induced cytokines such as IL-18 and IL-1b are abundant in the TME.

### Methods:

Tumor were cut into small pieces with a scalpel and transferred to GentleMACs^{™} C tubes (Miltenyi Biotec) containing an enzyme mix using human tumor Dissociation Kit (Miltenyi Biotec), as per the manufacturer's protocol. After dissociation, samples were centrifuged at 300g for 5 minutes and supernatants were collected and recentrifuged at 3130g for 10 minutes. Following centrifugation, supernatants were recollected and distributed in aliquots for storage at -80c. At the day of the assay, samples were thawed at room temperature and subsequently centrifuged at 14,000 RPM for 10 min and supernatants were collected for immediate usage in ELISAs or CBA with the following kits:
- Human IL18 ELISA kit (MBL,7620)
- Human Th1/Th2/Th17 cytokine Cytometric Bead Array (CBA) (BD 560484)

Human Inflammatory Cytokine Cytometric Bead Array (CBA) (BD 551811)

### Results:

**Figure 71A****:** IL-18 and IL-1b are inflammasome derived cytokines with opposite effects in the TME. While IL-18 promotes T and NK cell activation and lead to anti tumorigenic activity, IL1b has a dual role and in sum of effects lead to pro-tumorigenic activity.

**Figure 71B****:** Dot plot graph shows levels of cytokines in tumor derived supernatants measured across various indications. Each dot represents one sample. The mean is depicted by the short black lines. All other cytokines beside IL-1b and IL-18 were below the lower limit of detection.

### EXAMPLE 4: IL18 AND IL18BP PROTEIN LEVEL IN PATIENT'S SERUM COMPARED WITH HEALTHY DONORS AND ACROSS INDICATIONS

### Methods:

Serum samples from healthy donors and cancer patients were thawed and levels of IL18 analytes (IL18 total, IL18BP) were measured by the following ELISA KITS according to manufacturer's protocol:
- Human IL18 ELISA kit (MBL,7620)
- Human IL18BP ELISA Kit (R&D DBP180)

### Results:

**Figure 56A****.** IL18 analytes levels in patient's serum across indication. **Figure 56B****.** Dot plot representing IL 18 analytes in serum samples from an individual patient or healthy donor. Statistical analysis was preformed using t test (two tailed), *P* < 0.001***. Expression of IL-18 is significantly increased in serum of cancer patients compared with healthy donors, confirming that IL-18 levels are enhanced in the periphery during malignancy.

### EXAMPLE 5: HIGHER LEVEL OF IL-18 PROTEIN IN SERUM OF HEAD &NECK CANCER PATIENTS WITH TUMOR'S SITE IN THE TONGUE

### Methods:

Serum samples from Head and Neck cancer patients were thawed and levels of IL18 analytes were measure by the following ELISA KITS according to manufacturer's protocol: Human IL18 ELISA kit (MBL,7620) and Human IL18BP ELISA Kit (R&D DBP180).

### Results:

**Figure 63A****-B:** Principal Component Analysis (PCA) shows that mainly tumor's sites separate between samples with high levels of IL-18 Vs. low levels. Location of tumor in tongue correlates with high levels of IL-18 and lower levels of IL18BP compared with other sites. **Figure 63C.** Individual patient's serum levels for IL-18 and IL18BP are shown in dot plots in different tumor's sites.

### EXAMPLE 6: LEVELS OF IL18BP AND IL18 PROTEINS IN THE PLASMA OF NSCLC PATIENTS TREATED WITH ANTI-PD1/ ANTI-PD1 PLUS CHEMO

### Methods:

Plasma samples of NSCLC patients were thawed and levels of IL18 and IL18BP were measured by the following ELISA kits according to manufacturer's protocol: Human IL18 ELISA kit (MBL,7620) and Human IL18BP ELISA Kit (R&D DBP180).

### Results:

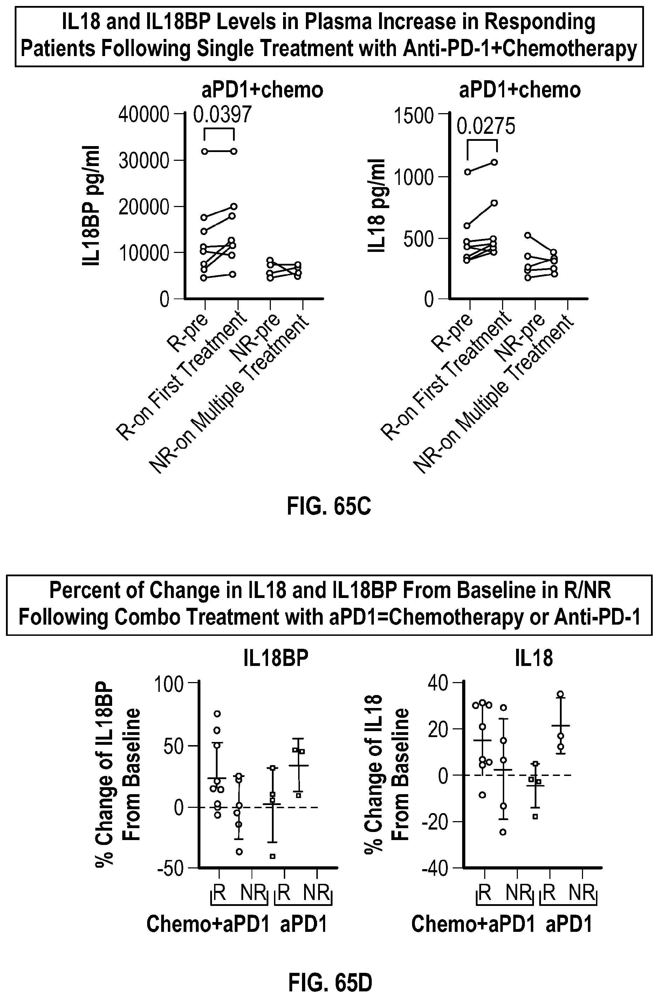

**Figure 65****:** Plasma of NSCLC patients was collected at baseline and following single dose of anti-PD-1 (Keytruda) (n=8) or following single dose of chemotherapy+ anti-PD-1 (n=14). Clinical assessment of patient's response (responding/non-responding, R/NR) was performed following PET-CT scan after several treatment cycles.

Average plasma levels of IL18BP and IL18 as measured at baseline, were higher in patients responding to therapies (anti-PD-1 monotherapy or combination of anti-PD-1 and chemotherapy) compared to non-responding patients (Figure 65A).

As shown in Figures 65B and 65D, patients clinically non-responding to anti-PD-1 monotherapy showed higher plasma levels of IL18 and IL18BP compared to baseline levels, whereas IL18 and IL18BP levels did not significantly change from baseline in patients responding to anti-PD-1 monotherapy. In contrast, only patients clinically responding to a combination of anti-PD-1 + chemotherapy showed higher levels of IL18 and IL18BP compared with baseline (Figure 65C, 65D).

### Discussion:

NSCLC patients treated with anti-PD1 (Keytruda) most likely express PDL1-CPS >50%, which potentially points to increased immune infiltrate and subsequent increased IFNg expression in the TME. Given that IL18BP is an IFNg-induced gene, this could suggest on a potential immune resistance mechanism in patients treated with anti-PD-1 and support the rational for a combined anti-IL18BP and anti-PD1 blockade to further increase patient's potential anti-tumor responses. Patients receiving combined treatment of chemotherapy + anti-PD-1, are PDL1-CPS <50%, and tend to have greater tumor masses. Patients responding clinically to anti-PD-1+chemotherapy combination, may have a potential increase in infiltration of immune cells which may secrete IL18, and a subsequent induction in IFNg levels which may potentially result in increase of IL18BP secretion. The clinical anti-tumor responses in these patients could be potentiated with anti-IL18BP antibodies.

### EXAMPLE 7: IL18 AND IL18BP PROTEIN LEVELS IN TUMOR DERIVED SUPERNATANTS (TDS)

### Methods:

Tumor were cut into small pieces with a scalpel and transferred to GentleMACs^{™} C tubes (Miltenyi Biotec) containing an enzyme mix using human tumor Dissociation Kit (Miltenyi Biotec), as per the manufacturer's protocol. After dissociation, samples were centrifuged at 300g for 5 minutes and supernatants were collected and recentrifuged at 3130g for 10 minutes. Following centrifugation, supernatants were recollected and distributed in aliquots for storage at -80°C. At the day of the assay, samples were thawed at room temperature and subsequently centrifuged at 14,000 RPM for 10 min and supernatants were collected for immediate usage in ELISAs with the following kits:
- Human IL18 ELISA kit (MBL,7620)
- Human IL18BP ELISA Kit (R&D DBP180)

### Results:

IL-18 and IL-18BP were detected in TDS across various indications.

**Figure 57****.** Dot plot representing IL18 and IL18BP in TDS samples from individual patients. **Figure 58****.** IL18 and IL18BP levels in patient's TDS across indication.

### EXAMPLE 8: IL18RA IS EXPRESSED ON TILS IN THE TME AND ITS EXPRESSION IS INDUCED ON CD4 TILS COMPARED WITH PERIPHERY

### Methods:

Tumor samples were cut into small pieces with a scalpel and transferred to GentleMACs^{™} C tubes (Miltenyi Biotec) containing an enzyme mix. After dissociation, cells were filtered through a 70 µm filter. Single-cell suspensions were seed into a 96-well V-bottomed plate and a cocktail of antibodies (Abs) to CD16 (BioLegend), CD32 (Thermo Fisher), and CD64 (BioLegend) were used to block Fc receptors. Immune populations were stained with anti-human IL18Ra or using its isotype control (BioLegend. After wash (1% BSA, 0.1% sodium azide, in PBS), cells were acquired on FACS Fortessa cytometer (BD Bioscience). Analysis was done using FlowJo.

### Results:

IL-18Ra expression is induced on tumor infiltrating T cells compared with matched PBMCs, with a statistical significance on CD4+ T cells, and a trend on CD8+ T cells.

**Figure 55A****.** Expression of IL18Ra on CD8⁺ and CD4⁺ and NK TILs from dissociated human tumors of various cancer types is shown. Each dot represents a distinct tumor from an individual patient. Fold expression value was calculated by dividing the MFI of a target by the MFI of the relevant isotype control. (FOI). Average and SEM is shown by the ticks. **Figure 55B****,** Expression of IL18Ra on CD4⁺ and CD8⁺ T and NK cells from donormatched PBMCs and TME. Statistical analysis was preformed using paired t test (two tailed), *P* < 0.05; **p=0.0064

### EXAMPLE 9: CO-EXPRESSION OF TIGIT AND IL18RA WITHIN THE TME

Tumor samples were mechanically disassociated and enzymatically digested using Milteny's human tumor disassociation kit (according to manufacturer's instructions). Single-cell suspensions were stained with Zombie-Nir to exclude dead cells and stained with the antibodies against CD45, CD3, CD4, CD8, CD56, TIGIT or IL18Ra. Cells were acquired on FACS Fortessa cytometer (BD Bioscience) and analyzed with FlowJo software (V10). Cell surface markers were used to detect the following immune populations: CD8 (CD3+CD8+), CD4 (CD3+CD4+), NK (CD3-CD56+) and NKT (CD3+CD56+).

The results are shown in Figure 33, presenting Flow cytometry dot plots showing co-expression of IL18Ra and TIGIT in the endometrium and colon TME, on CD8 T cells, CD4 T cells, NKs, and NKT cells. The co-expression of TIGIT and IL18Ra on same cells indicates that targeting both pathways by combined administration of inhibitory anti-IL18BP and anti-TIGIT antibodies might have a beneficial effect.

### EXAMPLE 10: GENERATION AND CHARACTERIZATION OF CUSTOM ABS AGAINST HUMAN IL18-BP PROTEIN BY ADIMAB LTD

### Generation of anti IL18-BP hIgG1-N297A Abs against human IL18-BP protein

### Antigen preparation

Antigens were biotinylated using the EZ-Link Sulfo-NHS-Biotinylation Kit (Thermo Scientific, Cat #21425).

The antigens were concentrated to ~1mg/mL and buffer exchanged into PBS before addition of 1:7.5 molar ratio biotinylation reagent. The mixture was held at 4C overnight prior to another buffer exchange to remove free biotin in the solution. Biotinylation was confirmed through streptavidin sensor binding of the labeled proteins on a ForteBio.

### Naive Library Selections

Eight naive human synthetic yeast libraries each of ~10⁹ diversity were propagated as previously described (see, e.g., Y. Xu et al, PEDS 26(10), 663-70 (2013); WO2009036379; WO2010105256; and WO2012009568.)

For the first two rounds of selection, a magnetic bead sorting technique utilizing the Miltenyi MACS system was performed, as previously described (see, e.g., Siegel et al, J Immunol Methods 286(1-2), 141-153 (2004).) Briefly, yeast cells (~10¹⁰ cells/library) were incubated with 10 nM biotinylated human IL18-BP-Fc fusion for 30 min at 30°C in wash buffer (phosphate-buffered saline (PBS)/0.1% bovine serum albumin (BSA)). After washing once with 40 mL ice-cold wash buffer, the cell pellet was resuspended in 20 mL wash buffer, and Streptavidin MicroBeads (500 µl) were added to the yeast and incubated for 15 min at 4°C. Next the yeast were pelleted, resuspended in 5 mL wash buffer, and loaded onto a Miltenyi LS column. After the 5 mL were loaded, the column was washed 3 times with 3 mL wash buffer. The column was then removed from the magnetic field, and the yeast were eluted with 5 mL of growth media and then grown overnight.

The following rounds of selection were performed using flow cytometry (FACS). Yeast were pelleted, washed three times with wash buffer, and incubated at 30°C with either 10 nM biotinylated human IL18-BP-Fc fusion, 10 nM biotinylated cyno IL18-BP-Fc fusion, 100 nM human IL18-BP-Fc monomer, 100 nM biotinylated cyno IL18-BP monomer, or with a polyspecificity reagent (PSR) to remove non-specific antibodies from the selection. Some selections were also performed to enrich for IL18 competitive antibodies by incubating with biotinylated human IL18-BP-Fc fusion precomplexed to human IL18. For the PSR depletion, the libraries were incubated with a 1:10 dilution of biotinylated PSR reagent as previously described (see, e.g., Y. Xu et al, PEDS 26(10), 663-70 (2013).) Yeast were then washed twice with wash buffer and stained with goat F(ab')₂ anti-human kappa-FITC (LC-FITC) diluted 1: 100 (Southern Biotech, Cat # 2062-02) and either Streptavidin-AF633 (SA-633) diluted 1:500 (Life Technologies, Cat # S21375) or Extravidin-_phycoerythrin (EA-PE) diluted 1:50 (Sigma-Aldrich, Cat # E4011), secondary reagents for 15 min at 4°C. After washing twice with ice-cold wash buffer, the cell pellets were resuspended in 0.3 mL wash buffer and transferred to strainer-capped sort tubes. Sorting was performed using a FACS ARIA sorter (BD Biosciences) and sort gates were determined to select for antibodies with desired characteristics. Selection rounds were repeated until a population with all of the desired characteristics was obtained. After the final round of sorting, yeast were plated and individual colonies were picked for characterization.

### Antibody Optimization

Optimization of antibodies was performed via a light chain batch shuffle, and then by introducing diversities into the heavy chain and light chain variable regions as described below. A combination of some of these approaches was used for each antibody.

Light chain batch shuffle: Heavy chains from the naive output were used to prepare light chain diversification libraries. Selections were performed on these libraries as described above, i.e., with one round of MACS and four rounds of FACS. In the different FACS selection rounds, the libraries were evaluated for, e.g., PSR binding and affinity pressure by antigen titration. Sorting was performed in order to obtain a population with the desired characteristics. Individual colonies from each terminal FACS selection round were picked for sequencing and characterization.

CDRH1 and CDRH2 selection: The CDRH3 of a single antibody was recombined into a premade library with CDRH1 and CDRH2 variants of a diversity of ~10⁸ and selections were performed with one round of MACS and four rounds of FACS as described in the naive selections. For each FACS round the libraries were looked at for PSR binding and affinity pressure, and sorting was performed in order to obtain a population with the desired characteristics.

CDRH3 and CDRL3 selection: Oligos were ordered from IDT which comprised the CDRH3 and the CDRL3 as well as a flanking region on either side of the CDR3. Each oligo variegated one or two amino acids in the CDR3 via NNK diversity. The CDRH3 oligos were recombined with heavy chain FR1-FR3 variable regions containing selected variants from the CDRH1 and CDRH2 selections, and the CDRL3 oligos were recombined with the light chain FR1-FR3 variable regions from the parental antibody, for a combined library diversity of ~10⁸. Selections were performed with one round of MACS and four rounds of FACS as described in the naïve selections. For each FACS round the libraries were looked at for PSR binding and affinity pressure, and sorting was performed in order to obtain a population with the desired characteristics. For these selections affinity pressures were applied by preincubating the antigen with parental IgG for 30 minutes and then applying that precomplexed mixture to the yeast library for a length of time which would allow the selection to reach an equilibrium. The higher affinity antibodies were then able to be sorted.

### Antibody production and purification

Yeast clones were grown to saturation and then induced for 48 h at 30°C with shaking. After induction, yeast cells were pelleted, and the supernatants were harvested for purification. IgGs were purified using a Protein A column and eluted with acetic acid, pH 3.5.

### Size Exclusion Chromatography

A TSKgel SuperSW mAb HTP column (22855) was used for fast SEC analysis of mammalian produced mAbs at 0.4 mL/min with a cycle time of 6 min/run. 200 mM Sodium Phosphate and 250 mM Sodium Chloride was used as the mobile phase.

### Dynamic Scanning Fluorimetry

10 uL of 20x Sypro Orange is added to 20 uL of 0.2-1mg/mL mAb or Fab solution. A RT-PCR instrument (BioRad CFX96 RT PCR) is used to ramp the sample plate temperature from 40 to 95 C at 0.5C increment, with 2min equilibrate at each temperature. The negative of first derivative for the raw data is used to extract Tm.

**Table 3: Summary of SEC-HPLC (%) and Fab Tm by DSF (^{O}C) for optimized antibodies**

| **Clone** | **Fab Tm (DSF, °C)** | **SEC-HPLC, % monomer** |
|---|---|---|
| ADI-71663 | 68.0 | 93.9 |
| ADI-71701 | 71.5 | 98.6 |
| ADI-71707 | 71.0 | 98.8 |
| ADI-71709 | 71.5 | 97.6 |
| ADI-71710 | 71.5 | 97.9 |
| ADI-71719 | 72.5 | 94.5 |
| ADI-71720 | 72.5 | 98.6 |
| ADI-71722 | 73.0 | 95.9 |
| ADI-71728 | 73.0 | 95.0 |
| ADI-71736 | 73.5 | 79.2 |
| ADI-71739 | 73.0 | 94.9 |
| ADI-71741 | 73.5 | 99.4 |
| ADI-71742 | 74.0 | 91.3 |
| ADI-71744 | 74.5 | 88.1 |
| ADI-71753 | 74.5 | 96.9 |
| ADI-71755 | 74.5 | 92.9 |

### Anti IL18-BP hIgG1 Abs analysis included the following steps:

### Affinity measurements of anti-human Abs to human IL18-BP-Fc protein and cynomolgus monkey IL 18-BP-Fc protein by ForteBio Octet - naïve output

Octet affinity measurements were performed on an Octet HTX generally as previously described (see, e.g., Estep et al, Mabs 5(2), 270-278 (2013)). Briefly, ForteBio affinity measurements were performed by loading IgGs on-line onto AHC sensors. Sensors were equilibrated off-line in assay buffer for 30 min and then monitored on-line for 60 seconds for baseline establishment. Sensors with loaded IgGs were exposed to 100 nM antigen for 3 minutes, and afterwards were transferred to assay buffer for 3 min for off-rate measurement. All kinetics were analyzed using the 1:1 binding model.

### SPR measurements

### Surface Plasmon Resonance K_{D} measurements

Kinetic analysis was conducted at 25 °C in a HBS-EP+ running buffer system (10 mM HEPES pH 7.4, 150 mM NaCl, 3 mM EDTA, 0.05% Surfactant P20) using a Biacore 8K optical biosensor (Global Life Sciences Solutions USA, Marlborough, MA). The sample compartment was maintained at 10°C for the duration of each experiment.

For antibody capture experiments, a goat anti-human Fc antibody (Jackson ImmunoResearch) was covalently coupled to flow cells 1 and 2 of a CM5 sensor chip surface via standard amine coupling (1:1 EDC:NHS) and then blocked with ethanolamine (1.0 M, pH 8.5). The antibodies (10.0 nM in running buffer) were injected (40 s at 10 µL/min) over flow cell 2. A series of concentrations of IL18-BP-Fc monomer ranging from 27.0 to 0.111 nM (3-fold dilutions in running buffer) were injected (300 s at 30 µL/min) over flow cells 1 and 2. Dissociation of II,18-BP-Fc monomer was monitored for 600 s or 5130 s. Several blank buffer samples were injected (300 s at 30 µL/min) over flow cells 1 and 2 and used for reference surface subtraction. All surfaces (flow cells 1 and 2) were regenerated via two injections (20 s at 30 µL/min) of 10 mM glycine, pH 1.5.

For biotinylated antigen capture-Fab or full Ab in solution experiments, each experiment cycle began with an injection (150 s at 2 µL/min) over flow cells 1 and 2 of a 1:20 solution of biotin CAPture reagent (Global Life Sciences Solutions USA) in running buffer. This was followed by an injection (120 s at 1.0 µL/min) of biotinylated IL18-BP-Fc fusion (10.0 nM) over flow cell 2. Upon capture of biotinylated IL18-BP-Fc fusion to the sensor surface, a series of Fab concentrations (24.3 - 0.1 nM, 3-fold dilution) of full Ab concentration (12.5nM - 0.8nM, 2 fold dilution) was injected (300 s at 30 µL/min) over flow cells 1 and 2. The dissociation of the Fabs or Abs were monitored for 600 s or 5130 s. Several blank buffer samples were injected (300 s at 30 µL/min) over flow cells 1 and 2 and used for reference surface subtraction. Finally, an injection (120 s at 10 µL/min) of regeneration solution (6 M Guanidine-HCl in 0.25 M NaOH) over flow cells 1 and 2 prepared the sensor surface for another cycle.

For data processing and fitting, the sensorgrams were cropped to include only the association and dissociation steps. This cropped data was subsequently aligned, double reference subtracted, and then non-linear least squares fit to a 1:1 binding model using Biacore Insight Evaluation software version 3.0.11.15423.

The results are shown in Figures 41 and 42.

Figure 41A: Biacore image of the anti-IL18BP Fab -human IL18BP interactions; 10min dissociation.

Figure 41B: Biacore image of the anti-IL18BP Fab -human IL18BP interactions; 85min dissociation.

Figure 41C: Biacore image of the anti-IL18BP Fab -cyno IL18BP interactions, 10min dissociation.

Figure 41D: Biacore image of the anti-IL18BP Fab -cyno IL18BP interactions, 85min dissociation.

Figure 42 presents a Table, showing KD values for human/cyno anti-IL18BP Fab - IL18BP interactions measured by Biacore.

### MSD-SET K_{D} measurements

Equilibrium affinity measurements performed as previously described (Estep et al., 2013). Solution equilibrium titrations (SET) were performed in PBS + 0.1% IgG-Free BSA (PBSF) with antigen (biotinylated IL18-BP-Fc fusion) held constant at 50 pM and incubated with 1.5-to 3-fold serial dilutions of Fab starting at 10 nM to 500 pM (experimental condition is sample dependent). Antibodies (20 nM in PBS) were coated onto standard bind MSD-ECL plates overnight at 4°C or at room temperature for 30 min. Plates were then blocked by BSA for 30 min with shaking at 700 rpm, followed by a wash with wash buffer (PBSF + 0.05% Tween 20). SET samples were applied and incubated on the plates for 150 sec with shaking at 700 rpm followed by one wash. Antigen captured on a plate was detected with 1000 ng/mL sulfotag-labeled streptavidin in PBSF by incubation on the plate for 3 min. The plates were washed once with wash buffer and then read on the MSD Meso Sector S 600 instrument using 1x Read Buffer T with surfactant. The percent free antigen was plotted as a function of titrated antibody in Prism and fit to a quadratic equation to extract the K_{D}.

The results are shown in Figures 43-45.

Figure 43A: Overlay of the Fab-IL18BP MSD Image (in Black) with the Human IL-18 - IL18BP MSD Image (in Green).

Figure 43B: Overlay of the Fab-IL18BP MSD Image (in Black) with the Cyno IL-18 - IL18BP MSD Image (in Green).

Figure 44 presents a Table, showing K_{D} values for human/cyno anti-IL18BP Fab - IL18BP interactions measured by MSD.

Figure 45 presents a Table, showing K_{D} values for human/cyno IL18-IL18BP interactions measured by MSD.

### ForteBio Octet Epitope Binning

Epitope binning was performed using a standard sandwich format cross-blocking assay. Control anti-target IgG was loaded onto AHQ sensors and unoccupied Fc-binding sites on the sensor were blocked with an irrelevant human IgG1 antibody. The sensors were then exposed to 100 nM human IL18-BP-Fc antigen followed by a second anti-IL18-BP antibody. Additional binding by the second antibody after antigen association indicates an unoccupied epitope (non-competitor), while no binding indicates epitope blocking (competitor).

### AlphaLISA competition assay

Anti-HIS tag acceptor beads (Perkin Elmer AL178C) were incubated with 2.5 nM human or cyno IL18-BP His, along with 2.5 nM biotinylated human or cyno IL18 and 150 nM IgG for 60 mins. Following this incubation Steptavidin donor beads (Perkin Elmer 6760002S) were added and incubated for an additional 30 mins at room temperature. The samples are then read using a Perkin Elmer EnSpire Alpha Multimode Plate Reader (Perkin Elmer 2390). The samples are read at 615 nm after an excitation at 680 nm. Competition is calculated using Photon (PBSF only)/Photon (Antibody) ratio. For top binders, assay was repeated using antibody dose-titration (150 nM, 3-fold dilution).

### Affinity measurements of anti-human IL18-BP Abs to human IL18-BP monomeric protein and cynomolgus monkey IL 18-BP-HIS tag protein by BiaCORE - optimized output

For biotinylated antigen capture-Fab in solution experiments, each experiment cycle began with an injection (150 s at 2 µL/min) over flow cells 1 and 2 of a 1:20 solution of biotin CAPture reagent (Global Life Sciences Solutions USA) in running buffer. This was followed by an injection (120 s at 1.0 µL/min) of biotinylated IL18-BP-Fc fusion (10.0 nM) over flow cell 2. Upon capture of biotinylated IL18-BP-Fc fusion to the sensor surface, a series of Fab concentrations (24.3 - 0.1 nM, 3-fold dilution) was injected (300 s at 30 µL/min) over flow cells 1 and 2. The dissociation of the Fabs were monitored for 600 s or 5130 s. Several blank buffer samples were injected (300 s at 30 µL/min) over flow cells 1 and 2 and used for reference surface subtraction. Finally, an injection (120 s at 10 µL/min) of regeneration solution (6 M Guanidine-HCl in 0.25 M NaOH) over flow cells 1 and 2 prepared the sensor surface for another cycle.

For data processing and fitting, the sensorgrams were cropped to include only the association and dissociation steps. This cropped data was subsequently aligned, double reference subtracted, and then non-linear least squares fit to a 1:1 binding model using Biacore Insight Evaluation software version 3.0.11.15423.

### Blocking of human IL18: IL18-BP interaction by ELISA

Anti-human IL18-BP Abs from Adimab were tested for inhibition of human IL18-BP -Fc fusion protein binding to IL-18 (R&D) by ELISA. Human anti-IL18-BP polyclonal antibody (R&D, cat. AF119) was coated on the wells of a high binding plate overnight at 4°C (2.5 µg/ml, 50 µl/well volume). Coated plate was rinsed once with PBS and incubated with 250 µL blocking buffer (2.5% skim milk in PBS) for 2 hr in room temperature (RT). Serial dilutions of anti-human IL18-BP Abs (1:2, 4-0.06 µg/ml, 50 µL/well) mixed with 1nM human IL18-BP-Fc and 4nM biotinylated human IL18 at RT were pre-incubated for 1h at RT. Blocking buffer was removed and plate was washed and, incubated with 100 µl/well protein mix for 2h at RT. Plate was washed and incubated with streptavidin-HRP solution (Jackson; 50 µL/well volume) at RT for 1h. Plate was washed 3 times with PBS-T, once with PBS, and incubated with TMB substrate solution (50 µL/well) at RT to allow signal development. The HRP reaction was stopped by addition of 1N HCl solution (50 µL/well), and absorbance signal was read at 450 nm on a luminescence Reader (EnSpire, Perkin Elmar). Data were exported to Excel (Microsoft) and plotted in GraphPad Prism (GraphPad Software, Inc.). Blocking was calculated as a decrease in the binding signal of biotinylated human IL-18 to IL18-BP-Fc protein in the presence of an Ab compared to the binding signal in the presence of an isotype control.

### Rescue of free human IL18 from pre-complexed human serum and recombinant human IL18 by ELISA

Anti-human IL18-BP Abs from Adimab were tested for rescue of human recombinant IL18 pre-bound by human IL18-BP in human serum by ELISA. Human anti-IL18 Mab (MOR09464_N30K antibody Novartis patent US 2014/O112915 A1) was coated on the wells of a high binding plate overnight at 4°C (2.5 µg/ml, 50 µl/well volume). Coated plate was rinsed once with PBS and incubated with 250 µL blocking buffer (2.5% skim milk in PBS) for 2 hr in room temperature (RT). Serial dilutions of anti-human IL18-BP Abs (1:2, 5-0.078 µg/ml, 50 µL/well) were mixed and incubated for 2h at 37^{O}C with human healthy donor serum (ISERS50 Almog) spiked with 4ng/ml of recombinant human IL18 (R&D) for 1h at RT. For standatd curve, serial dilutions of recombinant IL18 in blocking buffer were made (1:2, 3-0.05ng/ml). Blocking buffer was removed and plate was washed and, incubated with 100 µl/well protein mix for 2h at RT. Plate was washed and incubated with D045-6 -biotin (1:1000 in 1% BSA PBS, 100 µl/well, R&D) at RT for 1h. Plate was washed and incubated streptavidin-HRP solution (Jackson; 50 µL/well volume) at RT for 1h. Plate was washed 3 times with PBS-T, once with PBS, and incubated with TMB substrate solution (50 µL/well) at RT to allow signal development. The HRP reaction was stopped by addition of 1N HCl solution (50 µL/well), and absorbance signal was read at 450 nm on a luminescence Reader (EnSpire, Perkin Elmar). Data were exported to Excel (Microsoft) and plotted in GraphPad Prism (GraphPad Software, Inc.). % IL18 rescue was calculated as an addition of free IL18 detected over total IL18: IL18-BP complex amount in the presence of an Ab compared to the binding signal in the presence of an isotype control.

### Rescue of free cyno recombinant IL18 from pre-complexed cyno recombinant IL18-BP with recombinant cyno IL18 by ELISA

Anti-human IL18-BP Abs from Adimab were tested for rescue of cyno recombinant IL18 pre-bound by cyno recombinant IL18-BP by ELISA. Human anti-IL18 Mab (MOR09464_N30K antibody Novartis patent US 2014/O112915 A1) was coated on the wells of a high binding plate overnight at 4°C (2.5 µg/ml, 50 µl/well volume). Coated plate was rinsed once with PBS and incubated with 250 µL blocking buffer (2.5% skim milk in PBS) for 2 hr in room temperature (RT). Blocking buffer was removed and plate was washed and, incubated for 1h at 37°C with 100 µl/well of serial dilutions of anti-human IL18-BP Abs (1:3, 10-0.004 µg/ml, 50 µL/well) mixed with pre-formed cyno IL18:IL18-BP complex (1ng/ml rhesus IL18, R&D and 25ng/ml IL18BP-His, R&D; incubated 1h at 37°C). Plate was washed and incubated with D045-6 -biotin (1:2000 in 1% BSA PBS, 100 µl/well, R&D) at RT for 1h. Plate was washed and incubated streptavidin-HRP solution (Jackson; 50 µL/well volume) at RT for 1h. Plate was washed 3 times with PBS-T, once with PBS, and incubated with TMB substrate solution (50 µL/well) at RT to allow signal development. The HRP reaction was stopped by addition of 1N HCl solution (50 µL/well), and absorbance signal was read at 450 nm on a luminescence Reader (EnSpire, Perkin Elmar). Data were exported to Excel (Microsoft) and plotted in GraphPad Prism (GraphPad Software, Inc.). % IL18 rescue was calculated as an addition of free IL18 detected over total IL18:IL18-BP complex amount in the presence of an Ab compared to the binding signal in the presence of an isotype control.

### Rescue of free human IL18 from pre-complexed human IL18-IL18-BP by IL18 HEK293 reporter cells

0.1 ng/ml human IL18 (R&D) was pre-incubated with cell medium express high levels of IL18-BP from SUIT2 INF-gamma treated cell (24h, 1000 U/ml) in the presence of 3 µg/ml of Adimab Abs or isotype control. 50K /well of HEK293 reporter cells (Invivogen) were seeded in 96 well plate in Test medium (DMEM high glucose, 10%FBS, 1% pen-strep, 1% glutamax) and 20 ul of sample was added to each well. Cells were incubated for 20h at 37°C CO₂ incubator. Next day, 20 µl induced cells supernatant were added to 180ul prewarmed Quanti-Blue solution (Invivogen) in 96 well plate. Cells were incubated at 37°C for 1 hour and SEAP levels were measured by OD650 reading. All samples were measured in duplicates. Blocking was calculated as an increase of the free IL18 detection in the presence of an Ab compared to the free IL18 levels in the presence of an isotype control.

### Results:

### Anti-human IL18-BP hIgG1 generation

The yeast naïve libraries at Adimab were used in 5 rounds of selection using human IL18-BP fused to hIgG1 Fc protein or cynomolgus monkey IL18-BP -Fc protein (Adimab) and one round of counter selection against poly-specificity reagent for depletion of nonspecific antibodies. Human IL18 was added on top of human IL18-BP antigen is several rounds to enrich for blocking antibodies.

In the first screen, 740 clones were isolated, sequenced and screened for binding to human IL18-BP in Kd ranking using Bio-Layer Interferometry (BLI) technology on a label-free, dip-and-read biosensor platform (ForteBio Octet^{®} RED384) Octet instrument. Out of 740 clones, 341 were unique and identified as positive binders to human IL18-BP, 266 antibodies had the affinity below 100nM to human monomeric IL18BP. A secondary screen of top 341 Octet-positive antibodies included affinity measurements to cynomolgus monkey IL18-BP fused to hIgG1 Fc or to cynomolgus monkey IL18-BP-HIS and to human IL18-BP monomeric protein. 195 antibodies were human/cyno cross-reactive. Initial antibody binning was performed using sandwich approach in Octet instrument; however, the assay could not discriminate between likely IL 18-competitors and non-competitors. To overcome this, binning of the antibodies was performed using ligand competition in FACS. Individual clones were tested in the presence of 10nM Hu IL18BP Fc with or without 100nM IL18. All clones picked show competition with the IL18 for the binding to IL18BP (Antibodies represent only bin 1 and all are ligand competitive).

Next, variable heavy region from 341 unique clones from the naive selection were subcloned into pre-made light chain shuffled library. Selection of LCBS libraries were performed as described above, with 3 rounds of selection using either human or cynomolgus monkey IL18-BP antigen and one round of counter selection using PSR.

In the first screen, 1152 clones were isolated, sequenced and screened for binding to human IL18-BP in KD ranking using Bio-Layer Interferometry (BLI) technology on a label-free, dip-and-read biosensor platform (ForteBio Octet^{®} RED384) Octet instrument. Out of 1152 clones, 658 were unique and identified as positive binders to human IL18-BP. Antibodies were ranked based on binding affinity to human IL18-BP-Fc protein and top 87 clones were picked for further characterization and purified from the medium of the yeast expressing cells using affinity column. A secondary screen of top 87 Octet-positive antibodies included affinity measurements to cynomolgus monkey IL18-BP fused to hIgG1 Fc or to cynomolgus monkey IL18-BP-HIS and to human IL18-BP monomeric protein. Antibodies were binned according to IL18-BP-Fc binding and competition with human IL18. Competition for the binding of IL18-BP-Fc was performed in AlfaLISA assay with 150nM of purified hIgG1. Based on all above, antibodies were ranked, and top 16 antibodies were screened in AlfaLISA using dose-titration of the antibodies (150 nM, 3-fold dilution). Top 6 blocking human/cyno IL18-BP binders were selected for optimization.

Relevant CDR's from top 6 parental clones were shuffled into pre-made CDRH1 and CDRH2 libraries and 3 rounds of selections were performed at Adimab using human IL18-BP monomeric protein or cynomolgus monkey IL18-BP-HIS protein. 79 unique clones were identified and screened for the binding to monomeric human and cyno IL18-BP proteins in Octet.

Relevant CDR's from 79 unique clones were used to create CDRH3 and CDRL3 diversification libraries. CDRH3/L3 libraries were panned using precomplex of 10 nM of IL18-BP monomer with 100 nM of parental IgG to pressure for Koff enriched clones. 47 unique clones were identified and purified from the medium of the yeast expressing cells using affinity column. Analysis of top 47 antibodies included affinity measurements (Octet and Biacore) to cynomolgus monkey and to human IL18-BP monomeric protein. All 47 clones reached Koff limit of detection by Octet for 85-minute dissociation. Affinity of 47 clones to human and cynomolgus monkey IL18-BP was measured by Biacore, 24 out of 47 clones reached Koff limit of detection for 85 minute dissociation as measured by Biacore for human IL18-BP and 5 reached Koff limit of detection for 85 minute dissociation as measured by Biacore for cyno IL18-BP (Figure 8). Competition with human IL18 for the binding of IL18-BP-Fc was performed in AlfaLISA assay with 15nM of purified hIgG1 (Figure 9).

### Blocking of human and cyno IL18- IL18-BP interaction by ELISA

The blocking activity of the parental mAbs against human IL18-BP was analyzed by ELISA. As shown in Figure 10, anti-human IL18-BP Abs (1:2, 4-0.06 µg/ml), showed dose-dependent blocking effect as compared to isotype control. Anti-human IL18-BP Abs (1:3, 10-0.01 µg/ml), showed dose-dependent blocking effect for cyno IL18: IL18-BP interaction as compared to isotype control (Figure 11). IC50 values for the anti-human IL18-BP Abs are shown in Figure 12.

### Rescue of free human/cyno IL18 from pre-complexed human or cyno IL18-IL18BP by anti-IL-18BP Abs

The ability of anti-human IL-18BP affinity matured mAbs to release human IL18 bound by human IL18BP and cyno IL18 bound by cyno IL18BP protein was demonstrated using ELISA assay.

As shown in figure 31, anti-human IL18BP mAbs were able to release human IL18 from a pre-formed human IL-18:IL-18BP complex compared to isotype control.

As shown in figure 32, dose titration (10ug/ml, serial dilution 1:3) of anti-human IL18BP mAbs released cyno IL18 from a pre-formed cyno IL-18:IL-18BP complex compared to isotype control.

### Rescue of free human IL18 from pre-complexed human IL18-IL18-BP by IL18 HEK293 reporter cells

The ability of the mAbs against human IL18-BP to rescue human IL18 bound by IL18-BP protein was demonstrated using IL18 HEK293 reporter cells. Addition of 30 ng/ml anti-human IL18-BP Abs was able to restore free IL18 for all tested Abs (Figure 13).

Consensus sequence was generated using optimized sequences from each parental library using SnapGene MUSCLE alignment. High affinity IL18BP binders from each parental lineage were aligned to respective germline sequences. Consensus was generated with >90% threshold.

The 66650 lineage (VH1-03; VL-kappa-1-5) consensus sequence of CDRs (Figure 1A) was generated using ADI-71701, ADI-71709, ADI-71710, ADI-71707 and ADI-71717 antibodies. The respective sequence alignment is shown in Figure 3B.

The 66650 lineage (VH1-03; VL-kappa-1-5) consensus sequence comprises:
- CDR-H1 having the sequence Y-T-F-X-X2-Y-A-X3-H, wherein X is N, R, D, G or K; X2 is S, H, I or Q; X3 is M or V;
- CDR-H2 having the sequence W-I-H-A-G-T-G-X-T-X2-Y-S-Q-K-F-Q-G, wherein X is N, A or V; X2 is K or L;
- CDR-H3 having the sequence A-R-G-L-G-X-V-G-P-T-G-T-S-W-F-D-P, wherein X is S or E;
- CDR-L1 having the sequence R-A-S-Q-G-I-S-S-W-L-A;
- CDR-L2 having the sequence E-A-S-S-L-E-S; and
- CDR-L3 having the sequence Q-Q-Y-R-X-X2-P-F-T, wherein X is S, V, Y, L or Q; X2 is F, S, or G.

The 66670 lineage (VH1-69; VL-kappa-1-12) consensus sequence of CDRs (Figure 1B) was generated using ADI-71719, ADI-71720, ADI-71722 and ADI-71728 antibodies. The respective sequence alignment is shown in Figure 3C.

The 66670 lineage (VH1-69; VL-kappa-1-12) consensus sequence comprises
- CDR-H1 having the sequence G-T-F-X-X2-Y-X3-I-S, wherein X is S or N; X2 is E or S; X3 is V or P;
- CDR-H2 having the sequence G-I-I-P-G-X2-G-T-A-X3-Y-A-Q-K-F-Q-G, wherein X is G or Y, X2 is A or S; X3 is N, I or V
- CDR-H3 having the sequence A-R-G-R-H-X-H-E-T, wherein X is S, G, or F;
- CDR-L1 having the sequence R-A-S-Q-G-I-S-S-W-L-A;
- CDR-L2 having the sequence A-A-S-S-L-Q-S; and
- CDR-L3 having the sequence Q-Q-V-Y-X-X2-P-W-T, wherein X is S or R; X2 is L, I, or F.

The 66692 lineage (VH3-23, VL-kappa-1-12) consensus sequence of CDRs (Figure 1C) was generated using ADI-71662, ADI-71663 and ADI-66692 antibodies. The respective sequence alignment is shown in Figure 3A.

The 66692 lineage (VH3-23, VL-kappa-1-12) consensus sequence comprises:
- CDR-H1 having the sequence F-T-F-X-N-X2-A-M-S, wherein X is G or D or S; X2 is T or V or Y;
- CDR-H2 having the sequence A-I-S-X-X1-X2-G-S-T-Y-Y-A-D-S-V-K-G, wherein X is G or A; X2 is N or S; X3 is A or G;
- CDR-H3 having the sequence A-K-G-P-D-R-Q-V-F-D-Y;
- CDR-L1 having the sequence R-A-S-Q-G-I-X-S-W-L-A, wherein X is S or D;
- CDR-L2 having the sequence A-A-S-S-L-Q-S; and
- CDR-L3 having the sequence Q-H-A-X-X1-F-P-Y-T, wherein X is Y or L; X2 is S or F.

The 66716 lineage (VH1-39; VL-kappa-1-12) consensus sequence of CDRs (Figure 1D) was generated using ADI-71736, ADI-71739 and ADI-66716 antibodies. The respective sequence alignment is shown in Figure 3D.

The 66716 lineage (VH1-39; VL-kappa-1-12) consensus sequence comprises:
- CDR-H1 having the sequence G-S-I-S-S-X-X2-Y-X3-W-G, wherein X is S or P; X2 is E or D; X3 is G, P or Y;
- CDR-H2 having the sequence S-I-X-X2-X3-G-X4-T-Y-Y-N-P-S-L-K-S, wherein X is Y or V; X2 is Y or N; X3 is Q or S; X4 is S or A;
- CDR-H3 having the sequence A-R-G-P-X-R-Q-X2-F-D-Y, wherein X is Y or H, X2 is V or L;
- CDR-L1 having the sequence R-A-S-Q-G-I-S-S-W-L-A;
- CDR-L2 having the sequence A-A-S-S-L-Q-S; and
- CDR-L3 having the sequence Q-Q-G-X-X2-F-P-Y-T, wherein X is S or F; X2 is S or V.

### EXAMPLE 11: AFFINITY OF ANTI IL-18BP AB TO IL18BP COMPARED TO IL-18BP:IL-18 BY KINEXA AND BIACORE

Affinity of ADI-71739:IL-18BP vs IL-18:IL-18BP in human, cyno and clone W19089C (Biolegend): IL-18BP (KinExA) and mouse (Biacore) and characterization information.

The Kinetic Exclusion Assay (KinExA^{®}) measures the equilibrium binding affinity and kinetics between unmodified molecules in solution. For affinity analysis, the equilibrium dissociation constant, Kd, is experimentally determined and reflects the strength of the binding interaction. The rate of association, Kon, is also experimentally determined, while the rate of dissociation, koff, is usually calculated based on the following equation: koff = Kd x kon.

A Kd analysis requires immobilization of one interaction partner to a solid phase which is then used as a probe to capture the other interaction partner, the constant binding partner (CBP). For each experiment, one of the binding partners is titrated in a background of the CBP and allowed to reach equilibrium. The solutions are then briefly exposed to the solid phase and a portion of free CBP is captured. The captured CBP is then labeled with a fluorescent secondary molecule. The short contact time with the solid phase is less than the time needed for dissociation of the pre-formed complex in solution, thus competition between the solution and the solid phase titrated binding partner is "kinetically excluded." Since the solid phase is only used as a probe for the free CBP in each sample, the solution equilibrium is not altered during KinExA measurements. The signals generated from the captured CBP, which are directly proportional to the concentration of free CBP in the equilibrated samples, are used to determine the Kd value. The KinExA Pro software performs a least squares analysis on the measured data to fit optimal solutions for the Kd and the activity of the CBP to a curve representative of a 1:1 reversible bi-molecular interaction. For each data point along the curve, the x-axis reflects the molar concentration of the titrated binding partner, and the y-axis reflects the percentage of free CBP at that particular titrant concentration at equilibrium.

### SPR affinity measurements of anti-IL18BP binding to mouse IL18BP

Fab preparation: Fab fragments were prepared from 1mg of anti-IL18BP hIgG1 N97A yeast produced antibodies using Fab digestion kit (Pierce, cat.44985). SDS PAGE gel analysis of purified Fab fragments was performed in reduced and non-reduced conditions

All experiments were performed in Biacore T100 optical biosensor (Global Life Sciences Solutions USA, Marlborough, MA)

### Capture Chip Preparation

A human Fc capture reagent (Cytyva, BR1008-39) was covalently coupled to flow cells 3 and 4 of a CM5 sensor chip surface via standard amine coupling at 10µg/mL in pH 5 acetate buffer, followed by a six-minute blocking step with ethanolamine (1.0 M, pH 8.5).

### Mouse IL18BP Binding Kinetics to anti-IL18BP Fab

For antigen capture in solution experiments, each experiment cycle began with an injection (60 s at 5 µL/min) over flow cells 3 and 4 of a 10ug/ml solution of mouse IL18BP-Fc or hIgG1 isotype control, respectively. Upon capture of mouse IL18-BP-Fc fusion or isotype control to the sensor surface, a series of Fab concentrations (300 - 2.21 nM, 2-fold dilution) was injected (60 s at 30 µL/min) over flow cells 3 and 4. The dissociation of the Fabs were monitored for 900 s. Several blank buffer samples were injected (60 s at 30 uL/min) over flow cells 3 and 4 and used for reference surface subtraction. Finally, an injection (60 s at 10 µL/min) of regeneration solution (10mM Glycine pH=1.5) over flow cells 3 and 4 prepared the sensor surface for another cycle.

As seen in figure 52, ADI-71739 binds human and cyno IL-18BP (Kd~291fM, Kd~208fM respectively) at higher affinity than human and cyno IL-18 (Kd~441fM, Kd~345fM respectively). ADI-71739 binds mouse IL-18BP (Kd~4nM) at lower affinity than IL-18 (Kd~3.7pM).

### EXAMPLE 12: IL-18BP - BIOCHEMICAL COMPARISION BETWEEN COMMERCIAL ABS AND ADIMAB ANTI IL-18BP AB

### Methods:

### Blocking of hIL18BP- hIL-18 interaction using a-hIL18BP Abs by ELISA- IL18BP plate bound

This assay was utilized to identify anti-human IL 18BP Abs that inhibit the binding interaction between human IL18BP- and its counterpart, human IL-18. Commercial Ab anti human IL18BP (clone W19089C, cat. 947703, Biolegend), ADI-71739 and ADI-71722 were tested for inhibition of human IL18BP protein binding to IL-18 by ELISA. Human IL18BP-Fc protein was coated on the wells of a high binding plate overnight at 4°C (1 µg/ml, 100 µl/well volume). Plates were washed three times with PBS-T buffer (1X PBS pH 7.4, 0.05% Tween20) incubated with 250 µL blocking buffer (2.5% skim milk in PBS) at room temperature (RT) for 2 hr. Blocking buffer was removed and plates were washed three times with PBS-T buffer. Plate-bound ligands were incubated with anti-human IL18BP Abs in 1% BSA in PBS buffer two times serially diluted (2.5-0.019 µg/ml, 100 µL/well volume) at RT for 1h. Plates were washed one time with PBS. Plate-bound ligands were incubated with human IL-18 (cat. 9124-IL, R&D) in in 1% BSA in PBS buffer (1ng/ml, 100 µL/well volume) at RT for 1h. Plates were washed three times with PBS-T (0.05% Tween20 in PBS). Biotinylated anti-IL18 detection antibody, cat. D045-6, R&D 1:1000 in 1% BSA in PBS buffer was added (100 µL/well). This was incubated at RT for 1hr, and plates were washed again. Peroxidase Streptavidin, Jackson, cat. 016-030-084 1:1000 in 2.5% skim milk in PBS was added (100 µL/well) for 1hr at RT. Plates were washed three times with PBS-T buffer (1X PBS pH 7.4, 0.05% Tween20). ELISA signals were developed in all wells by adding 50 µL of TMB substrate and incubating for signal development, 1.45min. The HRP reaction was stopped by adding 50 µL 1N HCl and absorbance signals at 450 nm were read on a luminescence Reader- EnSpire (Perkin Elmar). The data were exported to Excel (Microsoft) and plotted in GraphPad Prism (GraphPad Software, Inc.).

### Competition ELISA using complex of soluble IL18-IL18BP and anti IL18BP Abs

This assay was utilized to identify anti-human IL18BP Abs that inhibit the binding interaction between human IL18BP- and its counterpart, human IL-18. Commercial Ab anti human IL18BP (Clone 136007, cat. MAB1191, R&D systems) and ADI-66716 were tested for inhibition of human IL18BP protein binding to IL-18 by ELISA. Human IL18BP antibody (cat.AF119, R&D) was coated on the wells of a high binding plate overnight at 4°C (1ug/ml, 100ul/well volume). Plates were washed three times with PBS-T buffer (1X PBS pH 7.4, 0.05% Tween20) incubated with 250 µL blocking buffer (2.5% skim milk in PBS) at room temperature (RT) for 2 hr. Blocking buffer was removed and plates were washed three times with PBS-T buffer. Complex pre-formed 1 hour at 37c with 0.25nM human IL-18-BP (R&D, cat. 119BP) and 3nM Human IL-18 Biotin (9124-IL, R&D) in 1% BSA in PBS buffer. Complex was incubated for 2 hr at RT together with anti-human IL18BP Abs (in 1% BSA in PBS buffer two times serially diluted. 4-0.06ug/ml, 100 µL/well volume). Peroxidase Streptavidin, Jackson, cat. 016-030-084 1:1000 in 2.5% skim milk in PBS was added (100µL/well) for 1hr at RT. Plates were washed three times with PBS-T buffer (1X PBS pH 7.4, 0.05% Tween20). ELISA signals were developed in all wells by adding 50 µL of TMB substrate and incubating for signal development. The HRP reaction was stopped by adding 50 µL 1N HCl and absorbance signals at 450 nm were read on a luminescence Reader- EnSpire (Perkin Elmar). The data were exported to Excel (Microsoft) and plotted in GraphPad Prism (GraphPad Software, Inc.).

### Results:

Blocking activity of AB-71739 and AB-71722 was compared to anti-IL18BP Ab commercial antibody (clone W19089C, Biolegand). As seen in Figure 53, the blocking effect of AB-71739 and AB-71722 was superior to Biolegend Ab.

Affinity of Biolegend antibody (cat. 947703, clone W19089C) to human IL18BP was measured by KinExA and was found to be 63.8pM (See method in Example 11).

Blocking activity of ADI-66716 was compared to anti-IL18BP Ab commercial antibody (Clone 136007, cat. MAB1191, R&D systems) in soluble blocking ELISA assay. Figure 54 showed that ADI-66716 (right hand bars) had superior blocking effect to R&D antibody.

Affinity of R&D antibody (cat.MAB1191) to human IL18BP was measured by Biacore and was found to be 2.73*10^-10M (Figure 76).

### EXAMPLE 13: FUNCTIONAL ASSESSMENT OF ANTI-IL18-BP ABS FROM ADIMAB CAMPAIGN

### Methods:

### NK-Based Assay for Functional Assessment of Anti-IL18-BP Antibodies

Human NK cells were thawed in RPMI 1640 with 20% FBS and washed once more with full RPMI (RPMI 1640, 10% FBS, 1% Glutamax, 1% Penicillin-Streptomycin Solution). The cells were then seeded at 50k cells/well in a 96 well plate and incubated 30 minutes in 37°C, 5% CO₂ incubator with a combination of rhIL-12 (10 ng/ml, R&D systems, 10018-1L/CF), rhIL-18 (3ng or 10ng/ml, R&D systems, 9124-IL/CF) and rhIL-18BP-Fc chimeric protein (1µg/ml, R&D systems 119-BP) to allow IL-18+IL-18BP complex formation. After 30 minutes, decreasing concentrations of anti-human IL-18BP mAbs or relevant isotype control were added to the culture to examine their capability to restore IL-18 activity. Cells and all added solutions were prepared in full RPMI media to a final volume of 150 µl/well. Plates were incubated for 24 hours in 37°C, 5% CO₂ incubator, after which the supernatant was collected for IFNγ secretion evaluation. On some occasions cell pellets were harvested and stained for membrane CD69 levels as a measurement of NK cell activation. All tests were done in triplicates and each repeated with four donors.

### PBMC-Based Assay for Blocking Endogenously Secreted IL-18BP

Human PBMCs were thawed in RPMI 1640 with 20% FBS, washed once more with full RPMI (RPMI 1640, 10%FBS, 1% Glutamax, 1% Penicillin-Streptomycin Solution) and incubated in a T-75 flask for 24 hours in 37°C, 5% CO₂ incubator to allow recovery. The cells were then seeded at 200k cells/well in a 96 well plate and cultured with a combination of rhIL-12 (10 ng/ml, R&D systems, 10018-1L/CF), rhIL-18 (33.3 ng/ml or 2 ng/ml, R&D systems, 9124-IL/CF) and decreasing concentrations of anti-human IL-18BP mAbs or isotype control. Cells and all added solutions were prepared in full RPMI media to a final volume of 150 µl/well. Plates were incubated for 24 hours in 37°C, 5% CO₂ incubator, after which supernatant was collected for IFNγ secretion evaluation. IL-18BP secretion was confirmed by IL-18/IL-18BP complex ELISA (R&D Systems, DY8936-05, not shown). All tests were done in triplicates and each repeated with two donors for ADI66716 and ADI66692, and five donors for affinity matured antibodies.

### CD69 expression

After 24 hours of incubation, NK cell pellets were collected, washed from residual medium with PBS and labeled with a viability dye (Zombie NIR) diluted 1: 1000 in PBS for 15min at RT, in the dark. The cells were then incubated with Fc receptor blocking solution (Trustain Fcx, Biolegend, 2.5 µl/reaction) for 10 min at room temperature. To detect cell surface expression of CD69, the cells were incubated with PE-anti-human CD69 Ab (BioLegend, 1 µg/ml) for 30 min on ice, in the dark. The cells were then washed once and analyzed using MACSquant analyzer.

### Cytokine secretion

To measure IFNγ secretion from the cells, supernatants were collected 24 hours post stimulation and tested by CBA human IFNγ kit (BD Biosciences, 558269) for NK cells or CBA Human Th1, Th2, Th17 cytokine kit (BD Biosciences, 560484) for PBMCs.

### Data analysis and statistics

All FACS files were analyzed by FlowJo software. Where applicable, EC50s were calculated using GraphPad Prism software.

### Results:

### Analysis of mAbs performance in blocking of mIL18-BP- mIL-18 interaction in an in-vitro NK based assay

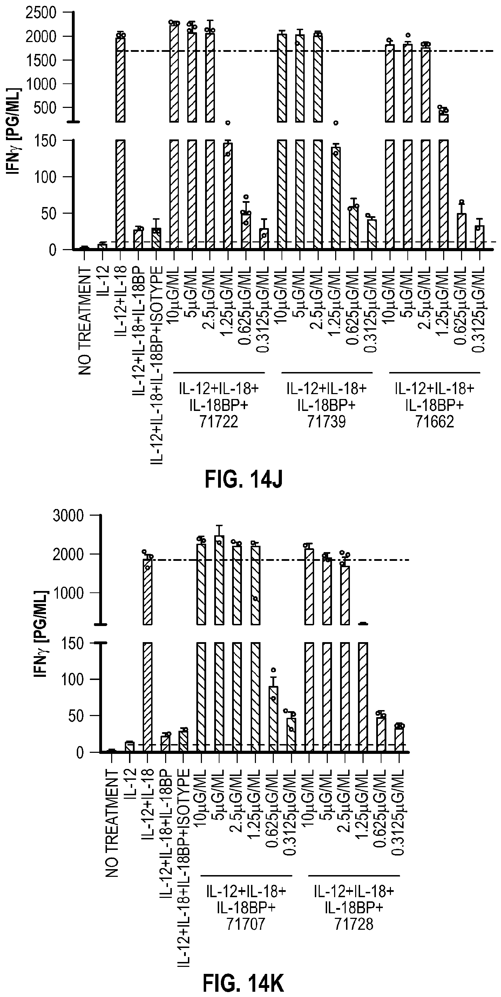

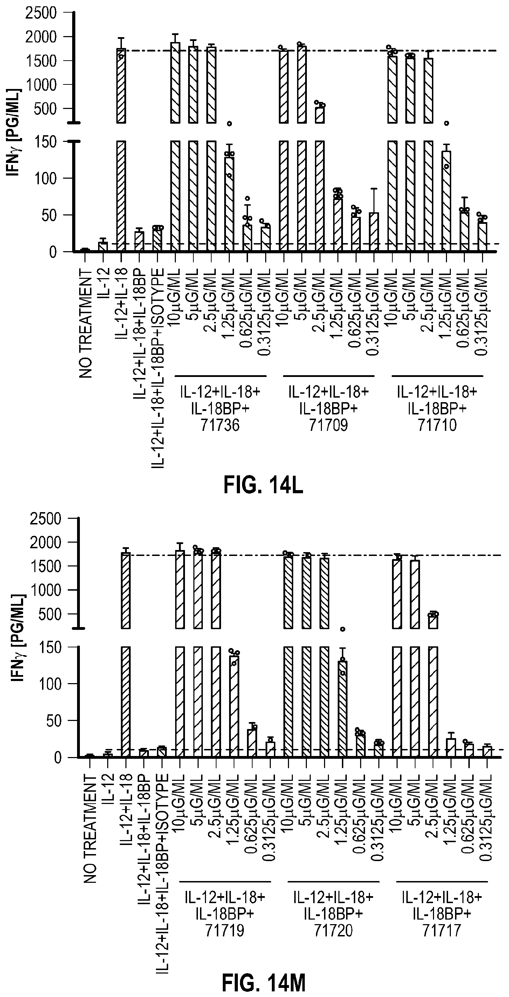

The functional blocking activity of mAbs against recombinant human IL18-BP was evaluated by an NK-based assay. As shown in Figure 14, anti-human IL18-BP Abs were able to block recombinant IL-18BP and fully restored IL-18 activity, depicted by IFNγ secretion and CD69 expression in a dose dependent manner as compared with the isotype control. EC50 value were in the single and double-digit nM range.

### Analysis of mAbs performance in blocking of mIL18-BP- mIL-18 interaction in an in-vitro PBMC based assay

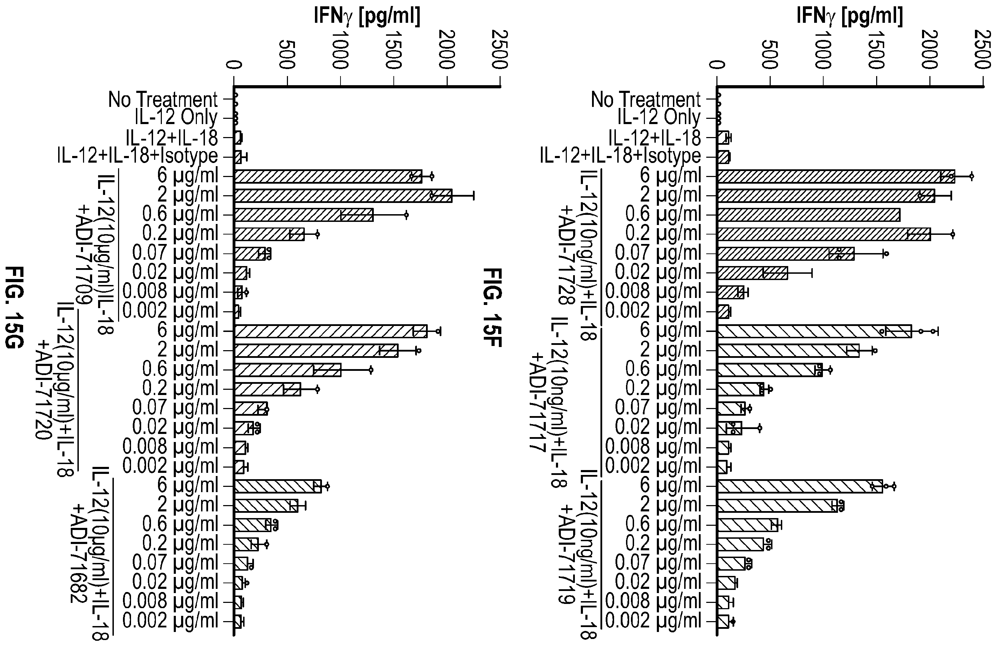

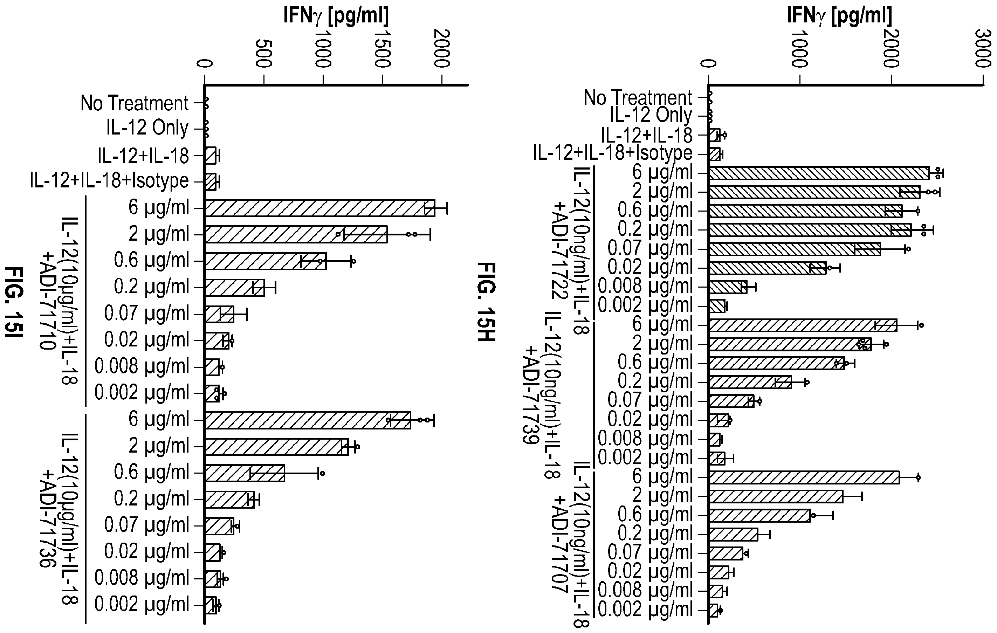

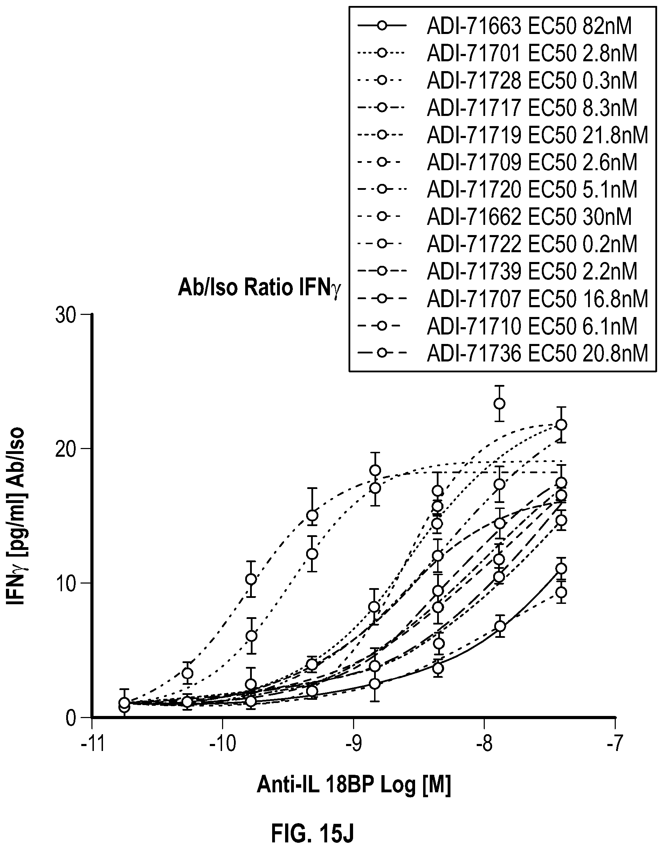

The functional blocking activity of mAbs against endogenous human IL18-BP was evaluated by an PBMC-based assay. As shown in Figure 15, anti-human IL18-BP Abs were able to block endogenous IL-18BP and to restore IFNγ secretion in a dose dependent manner as compared with the isotype control.

### EXAMPLE 14: FUNCTIONAL ASSESSMENT OF ANTI IL-18BP ABS IN T CELL BASED ASSAY AND IN COMBINATION WITH ICB

A soluble immune checkpoint up-regulated in the TME in response to IFNγ. αIL-18BP restores T and NK activity. This provides a proposed mechanism for anti-PD-1 resistance in IFNγ-high patients.

Activity: In vitro - αIL-18BP restores T and NK cells activity. In vivo activity with αIL-18BP Ab demonstrates tumor growth inhibition both as a monotherapy and in combination with ICB.

### MEL624:TIL Assay for Functional Assessment of Anti-IL18-BP Antibodies

### Methods:

Human MEL624 cells were thawed and grown in DMEM with 10% FBS, 1% Glutamax, 1% Penicillin-Streptomycin Solution and 1% HEPES buffer. The cells were then seeded at 75k cells/well in a 96 well plate in the assay medium (IMDM with 10% human serum, 1% Glutamax, 1% MEM eagle, 1% Sodium Pyruvate and 1% Penicillin-Streptomycin Solution) and incubated 1 hour in 37°C, 5% CO₂ incubator before co-culture with human tumor infiltrating lymphocytes that were previously expanded using known melanoma antigens (TILs). Human TILs were thawed in full TIL media and 75k cells/well were co-cultured with the MEL624 cells to create an effector: target ratio of 1:1. The co-cultured cells were then treated with rhIL-18 (30 ng/ml) and rhIL-18BP (1 µg/ml) for 30 min in 37°C, 5% CO₂ incubator to allow IL-18:IL-18BP complex formation. After 30 minutes, anti-human IL-18BP mAb (ADI-71722, dose titration, 30 µg/ml-0.01 µg/ml, dilution factor of 1:3) or relevant isotype control (hIgG1 30µg/ml) were added to the co-culture to examine its capability to restore IL-18 activity. Cells and all added solutions were prepared in full assay medium to a final volume of 200 µl/well. Plates were incubated for 24 hours in 37°C, 5% CO₂ incubator, after which the supernatant was collected for cytokine secretion evaluation. All tests were done in triplicates and each repeated with TIL four donors.

### Cytokine secretion

To measure cytokine secretion from the cells, supernatants were collected 24 hours post stimulation and tested by CBA Human Th1, Th2, Th17 cytokine kit (BD Biosciences, 560484).

Where applicable, the EC50 value was calculated using GraphPad Prism software.

### Results:

The functional blocking activity of mAbs against recombinant human IL18-BP was evaluated by a MEL624:TIL assay. As shown in Figures 49A-B and 50, the anti-human IL18-BP Ab (ADI-71722), was able to block recombinant IL-18BP and to fully restore IL-18 activity, depicted by IFNγ secretion, in a dose dependent manner as compared with the isotype control.

### CMV Recall Assay for functional assessment of anti-IL18-BP Abs as mono and in combination with anti-PVRIG/anti-TIGIT/Pembrolizumab

### Methods:

Human MEL-624 overexpressing PD-L1 cells were thawed and loaded with CMV pp65 peptide (0.03µg/ml). The cells were seeded at 100K/well in a 96 well plate and incubated for 30 minutes in 37°C, 5% CO₂ incubator with a combination rhIL-18 (30 ng/ml, R&D systems, 9124-IL/CF) and rhIL-18BP-Fc chimeric protein (2 µg/ml, R&D systems 119-BP) to allow IL-18+IL-18BP complex formation. After 30 minutes, anti-human IL-18BP (ADI-71722), anti-human PVRIG, anti-human TIGIT (anti-TIGIT), anti-human PD1 (Pembrolizumab) or relevant isotype control (hIgG4) were added to the culture to examine their capability to restore IL-18 activity. All antibodies were added to a final concentration of 10µg/ml. 30 minutes post incubation with antibodies, thawed CMV-reactive T-cells were added to the culture. Cells and all added solutions were prepared in full IMDM (IMDM media with 10% human serum, 1% Glutamax, 1% MEM eagle, 1% sodium pyruvate, and 1% Penicillin-Streptomycin Solution) to a final volume of 200 µl/well. Plates were incubated for 24 hours in 37°C, 5% CO₂ incubator, after which the supernatant was collected for IFNγ secretion evaluation. All tests were done in triplicates and each repeated with three donors.

### Results:

The functional blocking activity of mAbs against recombinant human IL18-BP was evaluated by CMV recall assay. As shown in Figures 49C-D and 51, the anti-human IL18-BP Ab was able to block recombinant IL-18BP and to fully restore IL-18 activity, as depicted by IFNγ secretion. Combination of the anti-IL-18BP Ab with anti-PVRIG/anti-TIGIT/Pembrolizumab resulted in greater IFNγ secretion indicating a beneficial effect on T-cell activation.

### EXAMPLE 15: FUNCTIONAL ASSESSMENT OF ANTI IL-18BP ABS IN T CELL BASED ASSAY AND IN COMBINATION WITH ICB

### Whole blood assay

As shown in Figure 66, anti-IL-18BP antibody Ab-71709, as mono or in combination with Nivolumab, did not show signs of systemic immune activation in ID. Flow, an ex vivo system that mimics the human blood circulation. Fresh whole blood was taken from six healthy volunteers and immediately transferred to a whole blood loop system. The test items were administered, and the blood was set to circulate at 37°C to prevent clotting. Blood samples collected at the 24hr time point were analyzed for hematology and flow cytometry parameters and then processed to plasma for cytokine analysis. The anti-CD52 antibody Alemtuzumab was included as a reference antibody with manageable cytokine release in the clinic. As opposed to Alemtuzumab, according to the various readouts employed, the anti-IL-18BP antibody did not induce any signs of systemic immune activation, as mono or in combination with the anti-PD1 antibody Nivolumab.

### In vitro studies testing the effects of ADI-71739 on killing of melanoma cells by human TILs

As shown in Figure 67, Anti-IL18-BP antibody ADI-71739 increased killing of melanoma cells by tumor infiltrating lymphocytes. Schematic representation of assay setup is shown in Figure 67A. MEL624 cells were co-cultured with human TILs that were previously enriched for MART1 or gp100 peptide-specific clones. rhIL-18 (R&D systems, 50 ng/ml) and rhIL-18BP (R&D systems, 1µg/ml) were added to the co-culture for 30 minutes to allow the formation of IL-18:IL-18BP complex prior to treatment with 10µg/ml ADI-71739 or isotype control. The co-culture was monitored for 72 hours using an IncuCyte live cell imaging instrument. As shown in Figure 67B, addition of IL-18 (grey) enhanced tumor cell killing as indicated by lower confluence (left) and increased apoptosis (right) over time of the MEL624 cells. In the presence of the isotype control antibody (black), IL-18BP abrogated the effects of IL-18, while the anti-IL-18BP antibody (turquoise) was able to completely restore these effects.

### In vitro studies testing the effects of combination of ADI-71739 Ab with other checkpoint blocking antibodies

As shown in Figure 68, ADI-71739 increased IFNg secretion by CMV-specific T cells as mono and in combination with aPVRIG/aTIGIT/Pembrolizumab. Schematic representation of assay setup is shown in Figure 68A. MEL624 cells that overexpress PD-L1 were loaded with CMV peptide pp65. The cells were cultured for 30 minutes with rhIL-18 (R&D systems, 30 ng/ml) and rhIL-18BP (R&D systems, 2µg/ml) to allow the formation of IL-18:IL-18BP complex, and the cells were then treated with 10µg/ml ADI-71739 or aPVRIG (anti-PVRIG) or aTIGIT (anti-TIGIT) or Pembrolizumab (anti-PD-L1) or isotype control, as mono or in various combinations. CMV-specific T-cells were then added to the culture and IFNg secretion was measured after an overnight incubation. As shown in Figure 68B, ADI-71739 alone was able to increase IFNγ secretion by the T cells, and this effect was augmented upon combination with Pembrolizumab/aPVRIG/aTIGIT.

### In vitro studies testing the effects of ADI-71739 on human TIL function in the presence of endogenous IL-18BP levels

As shown in Figure 69, Anti-IL18BP antibody ADI-71739 increased IFNg release by tumor infiltrating lymphocytes. A. Schematic representation of assay setup. MEL624 cells were co-cultured with human TILs that were previously enriched for MART1 or gp100 peptide-specific clones. IL-18 (3.7 ng/ml) was added to the co-culture along with 5µg/ml ADI-71739 or isotype control. The co-culture was set for 18 hours following which IFNg levels were measured in supernatants. B. IFNg levels were increased in co-cultures treated with ADI-71739 (turquoise) as compared with isotype-treated samples (black). Representative examples from two TIL donors are shown.

### Bound IL-18 levels in the TME are above required amount for T cell activation in vitro

### Methods:

Human MEL624 cells were thawed and grown in DMEM with 10% FBS, 1% Glutamax, 1% Penicillin-Streptomycin Solution and 1% HEPES buffer. The cells were then seeded at 75k cells/well in a 96 well plate in the assay medium (IMDM with 10% human serum, 1% Glutamax, 1% MEM eagle, 1% Sodium Pyruvate and 1% Penicillin-Streptomycin Solution) and incubated 1 hour in 37°C, 5% CO2 incubator before co-culture with human tumor infiltrating lymphocytes that were previously expanded using known melanoma antigens (TILs). Human TILs were thawed in full TIL media and 75k cells/well were co-cultured with the MEL624 cells to create an effector: target ratio of 1:1. The co-cultured cells were then treated with rhIL-18 (1.23-300ng/ml). Cells and all added solutions were prepared in full assay medium to a final volume of 200 µl/well. Plates were incubated for 24 hours in 37°C, 5% CO2 incubator, after which the supernatant was collected for cytokine secretion evaluation. All tests were done in triplicates.

Tumor were cut into small pieces with a scalpel and transferred to GentleMACs^{™} C tubes (Miltenyi Biotec) containing an enzyme mix using human tumor Dissociation Kit (Miltenyi Biotec), as per the manufacturer's protocol. After dissociation, samples were centrifuged at 300g for 5 minutes and supernatants were collected and recentrifuged at 3130g for 10 minutes. Following centrifugation, supernatants were recollected and distributed in aliquots for storage at -80c. At the day of the assay, samples were thawed at room temperature and subsequently centrifuged at 14,000 RPM for 10 min and supernatants were collected for immediate usage in ELISAs with the following kits:
- Human IL18 ELISA kit (MBL,7620)
- Human IL18 free detection kit (in house protocol)

### Human free IL18 ELISA protocol:

Anti-human IL18 hIgG1 clone 12GL (patent US 2014/0004128A1) was diluted to 1µg/ml in PBS and coated on ELISA plate overnight at 4°C (100ul/well). Coated plates were washed three times with PBST and incubated with 300µl blocking buffer (1% BSA in PBS) at room temperature (RT) for 2 hrs. Blocking buffer was removed and plates were washed three times with PBST. Human healthy donor serums were diluted 1:2 with 1% BSA in PBS. Standard curve was generated by incubating 2-fold serial dilutions of human IL18 (starting at 1ng/ml) in 1% BSA in PBS. Plates were washed three times with PBST buffer (1X PBS pH 7.4, 0.05% Tween20) and 100ul/well biotinylated anti-IL18 detection antibody, cat.D0456 R&D; 1:1000 diluted in 1% BSA in PBS was added. This was incubated for 1 hr and plates were washed again as described above after antibody binding. 100ul/well horse radish peroxidase HRP-conjugated streptavidin, Jackson, 1: 1000 was added, and plates were incubated for 1 hr at RT. plates were washed again as described above after antibody binding. ELISA signals were developed in all wells by adding 50 µL of TMB substrate (Scytek) and incubating for 5-20 mins. The HRP reaction was stopped by adding 50 µL 1N HCL and absorbance signals at 450 nm were read (EnSpire, Perkin Elmar). The assay was done in duplicate. Data was analyzed using GraphPad Prism software

### Results:

A schematic representation of assay setup is shown in Figure 70A, thawed tumor infiltrating lymphocytes (TILs), co-cultured with MEL624 cells in a 1:1 ratio, were treated with rhIL-18 (R&D systems, 1.23-300 ng/ml) for 24hr. As can be seen in Figure 70B, rhIL-18 increased IFNγ secretion in a dose-dependent manner. rhIL-18 activates TILs in concentration above ~1ng/ml and reached saturation at ~100ng/ml.

Figure 70C: Levels of bound IL-18 in TDS across indications are mostly above the level required for *in vitro* T cell activation. Bound IL 18 levels were calculated by deducting IL18 free from total IL-18 measured for each sample by two separate ELISA kits. Dashed red line represent the level required for functional activity (1.5ng/gr). Black lines represent the median level bound IL-18 for each tumor type.

### EXAMPLE 16: GENERATION AND CHARACTERIZATION OF CUSTOM ABS AGAINST MOUSE IL18-BP PROTEIN

### Methods:

### Generation of Fab's against mouse IL18-BP protein

Fab's were raised at AbD Serotec (Bio Rad, Germany) using Human Combinatorial Antibody Library (HuCAL^{®}) production service. The HuCAL^{®} library is based on the human IgG1 Fab format, which consists of the first two domains of the antibody heavy chain and the complete light chain.

### Study Design

Generation of Fab's against mouse IL18-BP was performed at AbD Serotec (Bio Rad, Germany). Antibodies against the mouse IL18-BP protein were raised using the HuCAL^{®} phage library, using 3 rounds of enrichment and counter selection against non-related human IgG1 fusion protein for the depletion of unspecific antibodies. Next, the enriched antibody pool from the phage display vector was subcloned into expression vector to determine the final Fab format. The selected Fab format is Fab- FH (Monovalent Fab mini Ab containing a Flag and 6 His tag) The antibodies were raised using the mouse IL18-BP Fc fusion protein, mouse IL18-BP fused to human IgG1.

### Anti-mouse IL18-BP Fab's generation

Fab's generation at AbD Serotec included the following steps:
1. Antigen immobilization - immobilization of the antigen on a solid support. The standard method uses covalent coupling to magnetic beads.
2. Phage display selection - panning - The HuCAL^{®} platinum library presented on phage particles is incubated with the immobilized antigen. Nonspecific antibodies are removed by extensive washing and specific antibody phage are eluted by adding a reducing agent. An E. coli culture is infected with eluted phage and helper phage to generate an enriched antibody phage library for the next panning round. Typically, three rounds of panning.
3. Subcloning into antibody expression vector - After panning, the enriched antibody DNA is isolated as a pool and subcloned into a Fab expression vector. E. coli are transformed with the ligation mixture and plated on agar plates. Each growing colony represents a monoclonal antibody at this stage.
4. Primary screening - Colonies are picked and grown in a 384-well microtiter plate. Antibody expression is induced, and the culture is lysed to release the antibody molecules. Cultures are screened for specific antigen binding by ELISA.
5. Secondary screening - K off ranking of top 95 ELISA-positive clones included using Bio-Layer Interferometry (BLI) technology on a label-free, dip-and-read biosensor platform (ForteBio Octet^{®} RED384) Sequencing - Hits from the primary and secondary screening experiment are sequenced to identify unique antibodies.
6. Expression and purification - The unique Fab's are expressed and purified using one-step affinity chromatography.
7. Antibody QC - Purified Fab's are tested by ELISA using recombinant protein.

### Analysis of the mAbs performance

### Binding measurement of anti-mouse IL18-BP Abs to mouse IL18-BP protein by ELISA

Mouse IL18-BP His fusion protein (Sino Biological) was coated on the wells of a high binding plate overnight at 4°C (2.5 µg/ml, 50 µl/well volume). Mouse anti histidine tag HRP was used to ensure mouse IL18-BP His coating (diluted 1:500 in blocking buffer). Coated plate was rinsed once with PBS and incubated with 250 µL blocking buffer (2.5% skim milk in PBS-indicated per experiment) at room temperature (RT) for 2 hr. Blocking buffer was removed, plate was rinsed once more with PBS, and incubated with anti-mouse IL18-BP Abs from Biorad (1:3, 5-0.002 µg/ml, 50 µL/well) for 2 hr at RT. Plate was washed 3 times with PBS-T (0.05% Tween20 in PBS), followed by one wash once with PBS, and incubated with HRP-conjugated secondary antibody (50 µL/well) for 1hr at RT. Plate was washed 3 times with PBS-T, once with PBS, and incubated with TMB substrate solution (50 µL/well) at RT to allow signal development. The HRP reaction was stopped by addition of 1N HCl solution (50 µL/well), and absorbance signal was read at 450 nm on a luminescence Reader (EnSpire, Perkin Elmar). Data were exported to Excel (Microsoft) and plotted in GraphPad Prism (GraphPad Software, Inc.).

### Affinity measurement to mouse IL18-BP protein by BiaCore

1. Immobilization of anti-human Fc: all SPR measurements were performed with BiaCore T-100 instrument in PBS 0.05% Tween 25 running buffer. Series S CM5 chip (cat. BR100530 Cytiva) was primed for 7 min in running buffer. Normalization of the chip was performed with 8 min injection of 70% glycerol. Mouse antibody capture kit (cat. BR100838 Cytiva) was used for the capture. 0.4 M 1-ethyl-3-(3-dimethylaminopropyl)-carbodiimide in water was mixed with 0.1 M N-hydroxysuccinimide in water in 1: 1 ratio and chip surface was activated for 420 sec at 10 µl/min. Next, 30 µg/ml of mouse Fc capture reagent diluted in immobilization buffer (10 mM sodium acetate pH 5.0, cat. BR100838 Cytiva) was injected at 5 µl/min over all 4 channels until ΔRU reached 12000RU. Chip was blocked with 1 M ethanolamine-HCl pH 8.5 at 10 µl/min for 7 min.
2. Capturing of anti-mouse IL18-BP antibodies: For capture, AB-837 mIgG1 D265A (AbD35328) was diluted to 10 µg/ml in running buffer and injected at 5 µl/min rate over specific channel. CH1 was used for capture of isotype control (synagis mIgG1 D265A). The injection was stopped when capture levels reached ~250 RU.
3. Kinetic measurements of anti-mouse IL18-BP Ab: 12 two-fold serial dilutions of mouse IL18-BP-Fc (cat. 122-BP, R&D) starting from 256 nM diluted in running buffer was injected over all channels at 30 µl/min for 180 sec. Dissociation of the bound protein from captured antibodies was monitored for 1000 sec. Chip surface was regenerated with 10 µl/min injection of glycine-HCl pH 1.7 for 60 sec after each cycle. The resulting sensorgrams were processed and double-referenced using a Biacore T100 evaluation software. Where appropriate, the sensorgrams were fit with a simple 1: 1 kinetic binding model.

### Blocking of mIL18-BP- mIL-18 interaction by ELISA

Anti-mouse IL18-BP Abs from Biorad were tested for inhibition of mouse IL18-BP His fusion protein binding to IL-18 (Sino Biological) by ELISA. IL18-BP His fusion protein was coated on the wells of a high binding plate overnight at 4°C (2.5 µg/ml, 50 µl/well volume). Coated plate was rinsed once with PBS and incubated with 250 µL blocking buffer (2.5% skim milk in PBS) for 2 hr in room temperature (RT). Buffer was removed and plate was washed and incubated with serial dilutions of anti-mouse IL18-BP Abs from Biorad, (1:2, 5-0.04 µg/ml, 50 µL/well) at RT for 30 min. Plate was washed and incubated with mouse IL-18 biotinylated in blocking buffer (1 µg/ml, 50 µL/well volume) at RT for 1h. Plate was washed, and HRP-conjugated secondary antibody was added (50 µL/well) for 1 hr at RT. ELISA signal was develop as describe above. Blocking was calculated as a decrease in the binding signal of biotinylated mouse IL-18 to IL18-BP-His protein in the presence of an Ab compared to the binding signal in the presence of an isotype control.

### Blocking of mIL18-BP- mIL-18 interaction in-vitro

Mouse CD3⁺ T cells were isolated from freshly harvested spleens of C57BL/6 mice using the EasySep^{™} Mouse T Cell Isolation Kit according to manufacturer's instructions and plated on anti-CD3-coated (10 µg/ml) T-75cm² flasks at 0.8*10^6 cells/ml. Anti-CD28 (1µg /ml) was supplemented and cells were cultured for 3 days at 37°C, 5% CO₂. Cells were subsequently harvested, washed and cultured in the presence of rmIL-12 (2ng/ml) for 24 additional hours. The next day, IL-18 and IL-18 BP were allowed to complex for 30 minutes at 37°C, 5% CO₂ in 96-well plates (25 µl from each/well) and anti-IL-18 BP mAbs (serial dilutions, 25 µl/well) were added for additional 30 minutes. Cells were harvested, washed, supplemented with rmIL-12 (0.1 ng/ml final) and added to the IL-18/IL-18 BP/anti-IL-18 BP containing wells (40K/25 µl/well) for 24h at 37°C, 5% CO₂. Following the 24h culture, supernatants were collected for IFNγ secretion analysis by the mouse Th1/Th2/Th17 Cytometric Bead Array (CBA, BD Biosciences).

### Results:

### Anti-mouse IL18-BP Fab's generation

The panning was performed at BioRad using mouse IL18-BP fused to Fc of hIgG1 protein in 3 rounds of selection and counter selection against non-relevant Fc tagged control protein (Recombinant Mouse IL-15R alpha Fc Chimera Protein R&D cat.551-MR-100) for depletion of unspecific antibodies.

In the first screen ~360 clones were examined for binding to mouse IL18-BP vs non-relevant protein by ELISA assay. Out of ~360 clones, 150 were identified as positive binders to mouse IL18-BP. A secondary screen of top 95 ELISA-positive clones included koff ranking using Bio-Layer Interferometry (BLI) technology on a label-free, dip-and-read biosensor platform (ForteBio Octet^{®} RED384), where Abs were ranked based on their slowest of rate. Confirmatory screen by ELISA resulted in 41 positives unique Fab's binders. Fab's were purified and their binding to mouse IL18-BP was confirmed by ELISA. The 41 Fab's were further analyzed by affinity measurement to mouse IL18-BP protein, blocking activity and binning (ELISA, data not shown). Eleven Fab's, which belonged to the same bin, showed high blocking and binding activity were identified.

Reformation of the Fab's into full length immunoglobulin was done by BioRad. The conversion to mouse IgG1 D256A was done to top 6 Fab's (AbD35357, AbD35327, AbD35346, AbD35328, AbD35350, AbD35344).

### Analysis of the mAbs performance

### Affinity measurement to mouse IL18-BP protein by ELISA

The affinity of the 6 BioRad purified mAbs against mouse IL18-BP was analyzed by ELISA. As shown in Figure 16, anti-mouse IL18-BP, AbD35328 (also called "AB-837", "837", "Ab837") binds mouse IL18-BP His fused protein (2.5 µg/ml) with Kd value of 0.4nM.

### SPR kinetic measurement of anti-mouse IL18-BP (AbD35328)

The binding of anti-mouse IL18-BP mAb, AbD35328, to mouse IL18-BP-Fc protein is demonstrated in Figure 17. For this interaction, k off constant was below the limit of detection of the instrument (<10^-9 1/S) and ka=4.93*10^4 1/M*s. KD value couldn't be uniquely determined, and it was estimated that the value is below 1*10^-12M.

### Analysis of mAbs performance in functional blocking of mIL18-BP- mIL-18 interaction

The blocking activity of the 6 BioRad purified mAbs against mouse IL18-BP was analyzed by ELISA. As shown in Figure 18, anti-mouse IL18-BP Ab (1:2, 5-0.04 µg/ml 2.5% skim milk in PBS), showed dose dependent blocking effect as compared to isotype control. IC50 value for anti-mouse IL18-BP (AbD35328) is 3.3nM (Figure 19).

### Analysis of mAbs performance in blocking of mIL18-BP- mIL-18 interaction in an in-vitro T cell activation assay

The functional blocking activity of the BioRad purified mAbs against mouse IL18-BP was evaluated in a T cell activation assay. As shown in Figure 20, anti-mouse IL18-BP Ab (AbD35328) showed a dose dependent blocking effect by enhancing the IFNγ secretion as compared to isotype control. EC50 value for anti-mouse IL18-BP is 7.9nM (Figure 21).

### EXAMPLE 17: EFFICACY OF ANTI IL-18BP AS MONOTHERAPY AND IN COMBINATION WITH IMMUNE CHECKPOINTS BLOCKERS

This example describes the efficacy of anti-mouse IL18-BP mAb treatment in CT26 murine colon carcinoma model, B16/Db-hmgp100 melanoma model, MC38OVA^{dim} CRC model and E0771 triple negative breast cancer (TNBC) model as monotherapy or in combination with immune checkpoints blockers.

### Materials and Methods

### Tumor Challenge Experiments:

CT26 colon carcinoma was purchased from ATCC (CRL-2638). Cells were cultured in RPMI 1640 (Biological Industries, 01-100-1A) with 10% FBS (Biological Industries, 04-127-1A), and 100 µg/mL penicillin/streptomycin (Biological Industries, 03-031-1B). For tumor implantation, cells were harvested and washed, counted, suspended in cold RPMI 1640 and placed on ice. BALB/c mice ((female, 8wk) Envigo), were anesthetized with 10% Ketamine (Clorketam; SAGARPA Q-7090-053) and 10% Xylazine (Sedaxylan ;BE-V254834) mixture injected intraperitoneal. Next, the back of the mice was shaved and disinfected with a 70% Ethanol solution. Tumor cells were injected as 50µl of 2.5×10⁵ CT26 cells subcutaneously into the back right flank of mice.

B16/Db-hmgp100 cells were kindly provided by Dr. Hanada et al. (HHS agency) and were licensed from NIH. B16/Db-hmgp100 cells were generated by double transduction of B16F10 with H-2Db and a retrovirus that encodes chimeric mouse gp100 that is comprised of the human gp100₂₅₋₃₃ and the rest of mouse gp100. Cells were cultured in RPMI 1640 (Biological Industries, 01-100-1A) with 10% FBS (Biological Industries, 04-127-1A), and 100 µg/mL penicillin/streptomycin (Biological Industries, 03-031-1B), 1% Glutamax (Life technologies, 35050-038), 1% Sodium pyruvate (Biological Industries, 03-042-1B), 0.01% 2-mercaptoethanol (Life technologies, 31350-010), 10 µg/ml Blasticidin (InvivoGen, ant-bl-05). 1×10⁵ B16/Db-hmgp100 cells subcutaneously into the back right flank of mice.

In both CT26 and B16/Db-hmgp100 models mAb administration started at day 4 (mono treatment) or day 7 post tumor inoculation when tumors were at volume of 30-50 mm³ (combo treatment); and was given intra-peritoneal (i.p.) in a final volume/injection of 200 µl, for 3wks for a total of 6 administrations. Tumor growth was measured with electronic caliper every 2-3 days and was reported as 0.5 X W2 X L mm³. Mice were sacrificed with CO₂ at either study termination or any of the following clinical endpoints: tumor volume ≥1800mm³, tumor ulceration, body weight loss ≥20%, or moribund appearance.

MC38OVA^{dim} cells (clone UC10 4H10) were received from the Peter MacCallum cancer center. Cells were grown in DMEM or RPMI media containing 10% FBS, 1% Glutamax, 1% Sodium pyruvate, 0.01% 2-mercaptoethanol, 1% Penicillin-Streptomycin, 1% HEPES, 1% NEAA. MC38OVA^{dim} cells (10⁶ or 1.2x10⁶) cells in 50ul/mouse were injected subcutaneously into the right flank of the mouse. At a tumor volume of 130-260 mm³ mice were randomly assigned into treatment groups. Mice were treated with 15mg/kg Synagis isotype control or with AB-837 mAbs injected twice a week for a total of 6 treatments. Tumor growth was measured with an electronic caliper every 2-3 days and was reported as 0.5 X W2 X L mm3. The experimental endpoint is defined at tumor volume of 1800mm³. Mice with body weight loss of above 10% between measurements, or 20% reduction from initial weight were excluded.

E0771 murine TNBC model was purchased from CH3 BioSystems (Product: #94A001). Cells were cultured in RPMI 1640 (Biological Industries, 01-100-1A) with 10% FBS (Biological Industries, 04-127-1A), and 100 µg/mL penicillin/streptomycin (Biological Industries, 03-031-1B). For tumor implantation, cells were harvested and washed, counted, and suspended to 10⁷ cells/ml in cold RPMI 1640 and placed on ice. C57BL/6 mice ((female, 8wk) Envigo), were anesthetized with 10% Ketamine (Clorketam; SAGARPA Q-7090-053) and 10% Xylazine (Sedaxylan ;BE-V254834) mixture injected intraperitoneally. Next, tumor cells were injected with mixture containing 50 µl of 5×10⁵ E0771 cells and 50 µl of Matrigel Matrix (Corning; 354234), orthotopically into the right third mammary fat pad of C57BL/6 mice. At tumor volume of 330 mm³, mice were randomly assigned into treatment groups of n=10. Mice were treated with 15mg/kg Synagis D265A isotype control, AB-837 mIgG1-D265A and combination with anti-PD-L1. The mAbs were injected twice a week for a total of 6 treatments. Tumor growth was measured with an electronic caliper every 2-3 days and was reported as 0.5 X W2 X L mm³. The experimental endpoint is defined at tumor volume of 1800mm³. Mice with body weight loss of above 10% between measurements, or 20% reduction from initial weight were excluded.

### Antibodies:

The phage display anti-mouse IL18-BP mAb (AbD35328) used in this study, engineered as a mouse IgG1 D265A isotype monoclonal antibody (mAb) was shown to bind to IL18-BP in ELISA assay and block binding of mIL-18 to IL18-BP. The anti-mouse PD-L1 inhibitor, on a mIgG1 backbone, used in this study was mAb YW243.55.S70 which was described in WO 2010/077634 (heavy and light chain variable region sequences shown in SEQ ID NOs. 20 and 21, respectively, of WO 2010/077634), having a sequence disclosed therein.

All mAbs were formulated in sterile PBS and were low in endotoxin (<0.05 EU/mg).

**Table 4. Tested mAbs.**

| | | |
|---|---|---|
| 1 | Mouse IgG1 | |
| 2 | Mouse IgG1 D265A | |
| 3 | Benchmark anti PD-L1(mIgG1) | YW243.55.S70 |
| 4 | Anti-IL18-BP mAb Mouse IgG1 D265A | AbD35328 |
| 5. | Anti PVRIG mAb 407 mAb mouse IgG1 | BOJ-5G4-F4 |
| 6. | Anti TIGIT mAb mouse IgG1 | CPA.9.086 M1 |

### Study Design for CT26 and B16/Db-hmgp100 models

### Mono Treatment

Six-eight weeks old female BALB/c (for CT26) or C57BL/6 (for B16/Db-hmgp100 and E0771) mice were purchased from Envigo and acclimated in SPF animal facility for 1 week prior to beginning the experiment. Mice were anesthetized, shaved and inoculated subcutaneously with 50 µl of 2.5x10⁵ CT26 or 1x10⁵ B16/Db-hmgp100 or 5x10⁵ E0771 cells tumor cells.

At day 4 post tumor inoculation mice were treated with mAbs (as detailed below) injected on day 4, 7, 11, 14, 18 and 21 post inoculation. Tumor growth was measured with caliper every 2-3 days.

**Table 5. Treatment groups.**

| **# Group** | **Treatment/mAb** | **Dose (mg/Kg)** | **# Dose** | **Vol/Dose (ul)** |
|---|---|---|---|---|
| 1 | mIgG1 iso Ctrl | 5 | 6 | 200 |
| 2 | mIgG1- D265A iso Ctrl | 15 | 6 | 200 |
| 3 | Anti-PD-L1 mIgG1 | 5 | 6 | 200 |
| 4 | Anti-IL18-BP mAb mIgG1 D265A | 15 | 6 | 200 |
| 5 | Anti-TIGIT mAb mIgG1 | 10 | 6 | 200 |
| 6 | Anti-PVRIG 407 mAb mIgG1 | 10 | 6 | 200 |

### Combo Treatment

For combination of anti-IL18-BP with anti-mPD-L1 mAbs treatments, at day 7 post tumor inoculation, mice were randomly assigned into treatment groups of n=10 as described below. Mice were treated with mAbs (as detailed below) injected on day 7, 11, 14, 18, 21 and 25 post tumor inoculation. For combination of anti-IL18-BP with anti-TIGIT or anti-PVRIG the administration of anti-IL18-BP, anti-PVRIG, anti-TIGIT and control Synagis mIgG1-D265A (anti-IL18-BP) and mIgG1 (anti-PVRIG, anti-TIGIT), initiated on day 4 post inoculation. Mice were treated with mAbs (as detailed below) injected on days 4, 7, 11, 14, 18 and 21 post inoculation.

**Table 6. Treatment dosages.**

| **# Group** | **Treatment/mAb 1** | **Dose (mg/Kg)** | **Treatment/mAb 2** | **Dose (mg/Kg)** | **# Dose** | **Vol/Dose (µl)** |
|---|---|---|---|---|---|---|
| 1 | mIgG1 iso Ctrl | 5 | mIgG1 D265A iso Ctrl | 15 | 6 | 200 |
| 2 | Anti-PD-L1 mIgG1 | 5 | mIgG1 D265Aiso Ctrl | 15 | 6 | 200 |
| 3 | Anti-PD-L1 mIgG1 | 5 | Anti-IL18-BP mAb mIgG1 D265A | 15 | 6 | 200 |
| 4 | Anti-TIGIT mAb mIgG1 | 10 | Anti-IL18-BP mAb mIgG1 D265A | 15 | 6 | 200 |
| 5 | Anti-PVRIG 407 mAb mIgG1 | 10 | Anti-IL18-BP mAb mIgG1 D265A | 15 | 6 | 200 |

### Study Design for E0771 model

At tumor volume of 330 mm³, mice were randomly assigned into treatment groups of n=10. Mice were treated with 15mg/kg Synagis D265A, 837 mIgG1-D265A and combination with anti-PD-L1, mAbs injected twice a week for a total of 6 treatments.

**Table 7. Treatment groups.**

| **# Group** | **Treatment/mAb** | **Dose (mg/Kg)** | **# Dose** | **Vol/Dose (ul)** |
|---|---|---|---|---|
| 1 | mIgG1 isotype control | 5 | 6 | 200 |
| 2 | mIgG1- D265A isotype control | 15 | 6 | 200 |
| 3 | Anti-PD-L1 mIgG1 | 5 | 6 | 200 |
| 4 | Anti-IL18-BP mAb mIgG1 D265A | 15 | 6 | 200 |

**Table 8. Treatment dosages.**

| **# Group** | **Treatment/mAb 1** | **Dose (mg/Kg)** | **Treatment/mAb 2** | **Dose (mg/Kg)** | **# Dose** | **Vol/Dose (µl)** |
|---|---|---|---|---|---|---|
| 1 | mIgG1 isotype control | 5 | mIgG1 D265A isotype control | 15 | 6 | 200 |
| 2 | Anti-PD-L1 mIgG1 | 5 | mIgG1 D265A isotype control | 15 | 6 | 200 |
| 3 | Anti-PD-L1 mIgG1 | 5 | Anti-IL18-BP mAb mIgG1 D265A | 15 | 6 | 200 |

### Statistical Analysis:

Two-way ANOVA with repeated measures, followed by two-way ANOVA with repeated measures for selected pairs of groups using JMP (Statistical Discoveries TM) software. Analyses of tumor growth measurements were performed by comparing tumor volumes measured on the last day on which all study animals were alive. Statistical differences in percentage of mice tumor free were determined by a Log Rank Mantel-Cox test. Values of P < 0.05 were considered significant. * p<0.05; ** p<0.01; *** p<0.001. For each experiment, the number of replicates performed and the number of animals per group are described in the corresponding figure legend(s).

### Results

### Monotherapy activity of anti-IL18-BP and anti-mPD-L1 in syngeneic CT26 Mouse Tumor Model

The effect of anti-IL18-BP monotherapy in mouse syngeneic CT26 tumor model was assessed and compared to monotherapy with anti-mPDL1. Mice were treated with isotype control antibody (mIgG1 or mIgG1 D265A), or with anti-PD-L1 mIgG1 antibody (YW243.55.S70) or mIgG1 D265A anti-IL18-BP (mAb AbD35328).

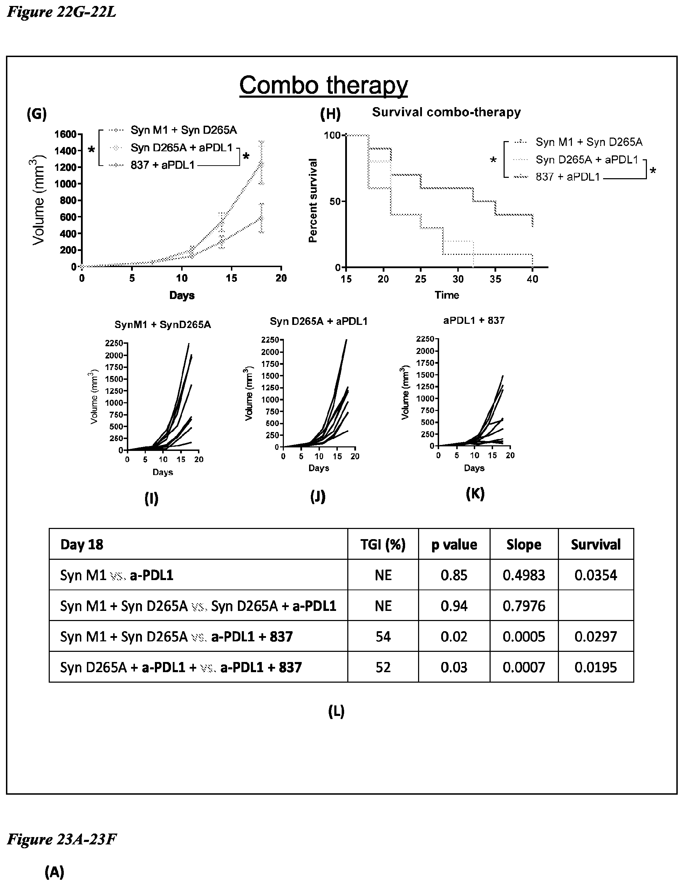

In a semi-therapeutic treatment model of CT26 colon carcinoma, monotherapy with anti-PD-L1 resulted in tumor growth rates similar to treatment with mIgG1 isotype control, albeit with a statistically significant benefit to mice survival. Mice in group treated with anti-IL18-BP mAb as a monotherapy, showed similar tumor growth rates to mice treated with mIgG1 D265A isotype control without survival benefit (Figure 22).

### Activity of anti-IL18-BP and anti-PD-L1 combination in syngeneic CT26 mouse tumor model

Next, the efficacy of anti-II,18-BP and anti-PD-L1 combination therapy in mouse syngeneic tumor model was assessed.

In a therapeutic treatment model of CT26 colon carcinoma, administration of anti-PD-L1 with control mIgG1 D265A isotype treatment, initiated on day 7 post inoculation, did not affect tumor growth, while combination of anti-IL18-BP mAb with anti-PD-L1 elicited significant TGI (52%, P=0.03, Figure 22), higher rates of response with 4 out of 10 individuals demonstrating tumor volumes below 200mm³ translated into a statistically significant benefit of mouse survival (P<0.05, Figure 22) and promoted increased and durable antitumor activity.

### Monotherapy activity of anti-IL18-BP in MC38OVAdim mouse tumor model

To validate the anti-tumor activity of anti-mouse IL18BP, AB-837, as a single agent, mice inoculated with MC38OVA^{dim} tumor cells were administered with 15mg/kg of AB-837 or isotype control. Monotherapy with AB-837 resulted in a 58% TGI (p<0.005, figure 78A).

### Monotherapy activity of anti-IL18-BP and anti-mPD-L1 in syngeneic B16/Db-hmgp100 mouse tumor model

We further explored the anti-tumor activity of AB-837 in a less immune infiltrated mouse melanoma tumor model B16/Db-hmgp100 either as monotherapy or in combination with anti PD-L1 Ab.

Mice were treated with isotype control antibody (mIgG1 or mIgG1 D265A), or with anti-PD-L1 mIgG1 antibody (YW243.55.S70) or mIgG1 D265A anti-IL18-BP (mAb AbD35328).

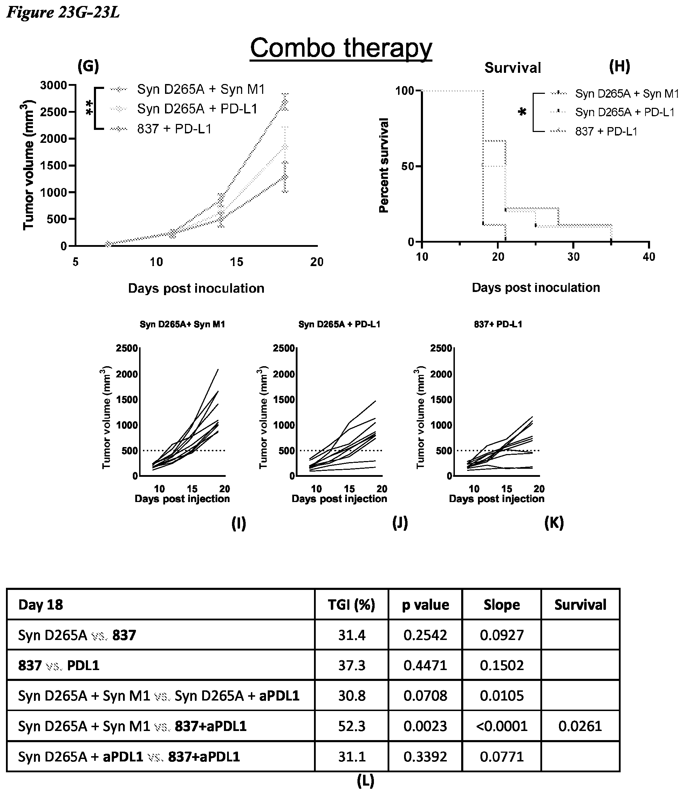

In a B16/Db-hmgp100 melanoma tumor model, monotherapy with anti-PD-L1 resulted in tumor growth rates similar to treatment with mIgG1 isotype control, without benefit to mice survival. Mice in group treated with anti-IL18-BP mAb as a monotherapy, showed 31.4% tumor growth inhibition compared to mIgG1 D265A isotype control, with a trend to statistical significance (p=0.09, Figure 23). In additional experiment treatment with anti-IL18-BP mAb resulted in 54% TGI ((p<0.005, figure 78B).

### Activity of anti-IL18-BP and anti-PD-L1 combination in syngeneic B16/Db-hmgp100 mouse tumor model

Next, the activity of anti-IL18-BP and anti-PD-L1 combination therapy in B16/Db-hmgp100 mouse syngeneic tumor model was assessed.

In of B16/Db-hmgp100 melanoma tumor model, administration of anti-PD-L1 with control mIgG1 D265A isotype control treatment, initiated on day 7 post inoculation, resulted in 30.8% tumor growth inhibition compared to mice treated with isotype control (P=0.07, Figure 23). Whereas a combination of anti-IL18-BP mAb with anti-PD-L1 elicited 31.1% of TGI compared to anti-PD-L1 monotherapy, and a significant TGI (52%, P=0.0023), compared to mice treated with isotype control treatment (Figure 23). Only mice treated with a combination of anti-IL18-BP mAb with anti-PD-L1 reached to statistically significant improvement in survival compared to control groups (P<0.05, Figure 23).

### Activity of anti-IL18-BP and anti-TIGIT combination in B16/Db-hmgp100 syngeneic mouse tumor model

Next, we assessed the activity of anti-IL18-BP and anti-TIGIT combination therapy in B16/Db-hmgp100 mouse syngeneic tumor model.

Mice treated with either anti-IL18-BP mAb or with ant-TIGIT mAb as monotherapies showed 34% (p=0.0594) and 43% TGI (p=0.0105) compared to mice treated with isotype control, respectively (Figure 24). However, when mice were treated with a combination of anti-IL18-BP and anti-TIGIT mAbs, 35% and 24% TGI was exhibited compared to anti-IL18-BP and anti-TIGIT monotherapies, respectively. Moreover, these mice exhibited 57% (p=0.02, Figure 24) TGI compared to group treated with isotype control mAbs. This effect was also translated into a significant improvement in mice survival (p=0.013, Figure 24).

### Activity of anti-IL18-BP and anti-PVRIG combination in B16/Db-hmgp100 syngeneic mouse tumor model

The activity of anti-IL18-BP and anti-PVRIG combination therapy in B16/Db-hmgp100 mouse syngeneic tumor model was also assessed.

While mice treated with anti-IL18-BP mAb monotherapy exhibited 34% (p=0.0594) TGI, mice treated with anti-PVRIG had a comparable tumor growth to mice treated with isotype control mAbs (Figure 25). None of the monotherapies significantly improved mice survival. However, when mice were treated with a combination of anti-IL18-BP and anti-PVRIG mAbs, a statistically significant TGI was shown (44%, p=0.0034) compared to mice treated with isotype control, which further resulted in a significantly improved mice survival (p=0.0034) (Figure 25).

### Anti IL18-BP activity as monotherapy and in combination with anti-mPD-L1 in orthotopic E0771 mouse tumor model

Monotherapy with anti-mouse IL18bp mAb, 837 mIgG1-D265A, in E0771 tumor bearing mice leads to 83% TGI (P<0.0001) compared to synagis D265A isotype control (Figure 72). A combination treatment of 837 mIgG1-D265A with aPD-L1 results in potentiated response, with 61% TGI compared to AB-837 administration as a monotherapy (p=0.029) and 91% TGI compared to isotype control (Figure 72, p<0.0001). These anti-tumor responses were translated into significant survival benefit: combination of 837 mIgG1-D265A with anti-PD-L1 significantly (P=0.004) increased the survival of mice compared to monotherapy with 837 mIgG1-D265A (P=0.01).

### Conclusions

The studies described in this example evaluated the *in vivo* anti-cancer efficacy of mAb directed against IL18-BP as a monotherapy or in combination with anti-PD-L1, anti-TIGIT or anti-PVRIG mAbs in 3 syngeneic mouse tumor models, CT26, B16/Db-hmgp100 and E0771.

In CT26 and B 16/Db-hmgp100 tumor models, treatment with 15 mg/kg (300 µg/mouse) of anti-IL18-BP as a monotherapy in a minimal disease set-up, *i.e.,* treatment initiation on day 4, resulted in increased TGI (0-35%) without a statistically significant survival advantage. However, when anti-IL18-BP mAbs were administered in combination with anti-PD-L1, anti-TIGIT or anti-PVRIG treatments, a synergism was exhibited by a statistically significant tumor growth inhibition and increased survival of mice.

In E0771 tumor model, treatment with 15 mg/kg (300 µg/mouse) of anti-IL18BP Ab as a monotherapy resulted in a significant anti-tumor activity (83% TGI) compared to control group. The activity of anti-IL-18BP Ab was further increased when it was administrated in combination with anti PD-L1 treatment.

In MC38OVAdim tumor model, treatment with 15 mg/kg (300 µg/mouse) of anti-IL18BP Ab as a monotherapy resulted in a significant anti-tumor activity (58% TGI) compared to control.

An *in vivo* effect of combining anti-IL18-BP with anti-mPD-L1 treatment was also demonstrated in MC38ova (data not shown).

### EXAMPLE 18: MONOTHERAPY WITH ANTI-IL18BP MAB INDUCES IMMUNOGENIC MEMORY IN E0771 TUMOR MODEL

This example describes the ability of anti IL18-BP Ab to induce immunogenic memory.

### Materials and Methods

### Tumor challenge experiments:

E0771 murine TNBC model was purchased from CH3 BioSystems (Product: #94A001). Cells were cultured in RPMI 1640 (Biological Industries, 01-100-1A) with 10% FBS (Biological Industries, 04-127-1A), and 100 µg/mL penicillin/streptomycin (Biological Industries, 03-031-1B). For tumor implantation, cells were harvested and washed, counted, and suspended to 10⁷ cells/ml in cold RPMI 1640 and placed in ice. C57BL/6 mice ((female, 8wk) Envigo), were anesthetized with 10% Ketamine (Clorketam; SAGARPA Q-7090-053) and 10% Xylazine (Sedaxylan; BE-V254834) mixture injected intraperitoneally. Next, tumor cells were injected in mixture containing 50 µl of 5×10⁵ E0771 cells and 50 µl of Matrigel Matrix (Corning; 354234), orthotopically into the right third mammary fat pad of C57BL/6 mice. The administration of mAbs started at day 11 post tumor inoculation when tumors were at volume of 270mm³; and was given intra-peritoneal (i.p.) in a final volume/injection of 200 µl, for 3 weeks for a total of 6 administrations. Tumor growth was measured with electronic caliper every 2-3 days and was reported as 0.5 X W2 X L mm³. Mice were sacrificed with CO₂ at either study termination or any of the following clinical endpoints: tumor volume ≥1800mm³, tumor ulceration, body weight loss ≥20%, or moribund appearance.

### Tumor re-challenge experiments:

For tumor re-challenge experiments, ninety days after primary E0771 inoculation, mice treated with anti-mouse IL18-BP mAbs and rejected primary tumors, and naive age-matched female C57BL/6 mice, were re-challenged with 5×10⁵ E0771 cells in the left third mammary fat pad (Figure 27A). Tumor growth was monitored as described above.

### Results:

### Monotherapy activity of anti-IL18-BP and anti-mPD-L1 in syngeneic E0771 orthotopic mouse tumor model

In E0771 tumor model mice were treated with a anti-PD-L1 mIgG1 antibody (YW243.55.S70) or with anti-IL18-BP mIgG1 D265A antibody (mAb AbD35328).

In orthotopic E0771 TNBC model, monotherapy with anti-PD-L1 resulted in tumor growth inhibition of 38.2% (p=0.72) compared to treatment with mIgG1 isotype control, with a benefit to mice survival (p=0.0362). Mice in group treated with anti-IL18-BP mAb as a monotherapy, showed 94.2% (p=0.0113) tumor growth inhibition compared to mIgG1 D265A isotype control (Figure 26). This anti-tumor response was translated into a statistically significant survival benefit (p=0.0011, Figure 26). A tumor growth inhibition of 81.6% was detected when comparing the therapeutic effects of anti-IL18-BP with the effects of anti-PD-L1.

### E0771 orthotopic tumor re-challenge model to assess generation of immune memory

Since monotherapy with anti IL18-BP mAb induced a complete rejection of E0771 tumors in mice, we examined whether the treatment induces generation of immunological memory by re-challenging mice without evident residual tumors (complete responders). Mice were treated with 15mg/kg anti IL18-BP Ab or isotype control. Two months after primary tumor inoculation, mice with no evident residual tumors and tumor-naïve aged-matched mice were re-inoculated with 5x10⁵ E0771 tumor cells. Five out of ten tumor-naive mice had tumor progression, while none of the mice completely rejecting primary tumors (5/5) developed tumors (Fig. 27A, B and C). In an additional experiment, 8/9 re-challenged mice rejected the tumors, in contrast to 1/9 tumor-naive mice. Spleen weight was significantly increased in rechallenged mice compared to tumor-naïve (P=0.004, Fig 27D). Moreover, we encountered a significant increase in percentage of CD44⁺CD62L⁻CD8⁺ effector T cells (22%, p= 0.02, Fig 5E) and CD19⁺ cells (20%, p=0.04, Fig. 27F) in rechallenged mice compared to tumor-naive mice. No other statistically significant differences were detected. Overall, these results show that a systemic memory was induced by anti- IL18bp monotherapy in mouse E0771 model.

### EXAMPLE 19: ADMINISTRATION OF ANTI-IL18BP IS EXPECTED TO HAVE A BETTER THERAPEUTIC POTENTIAL THAN ENGINEERED IL-18

### Material and Methods:

### Mouse antibodies and recombinant proteins

All mAbs and recombinant proteins were formulated in sterile PBS and were low in endotoxin (<0.05 EU/mg).

### Cell culture:

Nine days pre-inoculum, vials of MC38ovadim cells were thawed into RPMI media containing 10% FBS, 1% Glutamax, 1% Sodium pyruvate, 1% HEPES, 1% NEAA, 0.01% 2-mercaptoethanol, 1% Penicillin-Streptomycin. Following centrifugation at 300xg for 8min, cell pellet was resuspended, counted and seeded in a T175 flask. On days -7, -5 and -2, the cells were detached and re-seeded at 6-8*10⁶ cells/T175 flask. On the day of inoculum, the cells were detached, centrifuged at 300xg for 10min, filtered through 40µM cell strainer and resuspended in RPMI.

### Inoculation of mice:

Experiments were performed in C57Bl/6 (female, 6-8wk, Envigo). Mice were anesthetized with 10% Ketamine and 10% Xylazine mixture injected intraperitoneal. Next, mice were inoculated with MC38ova cells (1.2x10⁶) subcutaneously to the right flank in 50ul/mice. Tumor growth was measured with an electronic caliper every 2-3 days and was reported as 0.5 X W² X L mm³.

### Administration of anti-mIL18BP and engineered mIL-18 to tumor-bearing mice:

At tumor volume of ~120 mm³ (day 9), mice were randomly assigned into treatment groups. Mice were treated with Synagis mouse IgG1, k isotype control 15mg/kg (IP), anti-mIL18bp 837 mIgG1 15mg/kg (IP), PBS (SC), or engineered IL-18 (SC) 0.32mg/kg. Treatments were injected twice a week for a total of 6 treatments. Tumor growth was measured with caliper every 2-3 days. Mice were weighed every week. Mice were bled before the 4^{th} treatment, 4 hours after the 4^{th} treatment, and 24 hours after the 4^{th} treatment. Serum was analyzed for presence of IFNg, TNFa, MCP1, IL6 by Cytometric Bead Array (CBA) Mouse Inflammation Kit (BD Cat. No. 552364). Spleens were harvested from mice 24 hours after the 4^{th} treatment and weighed. For IL15 experiments, mice were treated with a single dose of 0.5ug, 1.5ug of IL15, or with a mix of 0.5ug IL15 and 2.33ug IL15R.

Engineered IL-18 (also referred to as DR-18; Sequence is from US Patent Publication 20190070262A1, listed therein as mCS2 (SEQ ID NO: 61)) was shown not to bind IL18-BP:

HFGRLHCTTAVIRNINDQVLFVDKRQPVFEDMTDIDQSASEPQTRLIIYAYGDSRARGKAVTLSVKDSKMST
LSCKNKIISFEEMDPPENIDDIQSDLIFFQKRVPGHNKMEFESSLYEGHFLACQKEDDAFKLILKKKDENGD
KSVMFTLTNLHQSHHHHHH

### Results:

### Analysis of MC38ovadom tumor-bearing mice treated with anti-mIL18bp and engineered mIL-18:

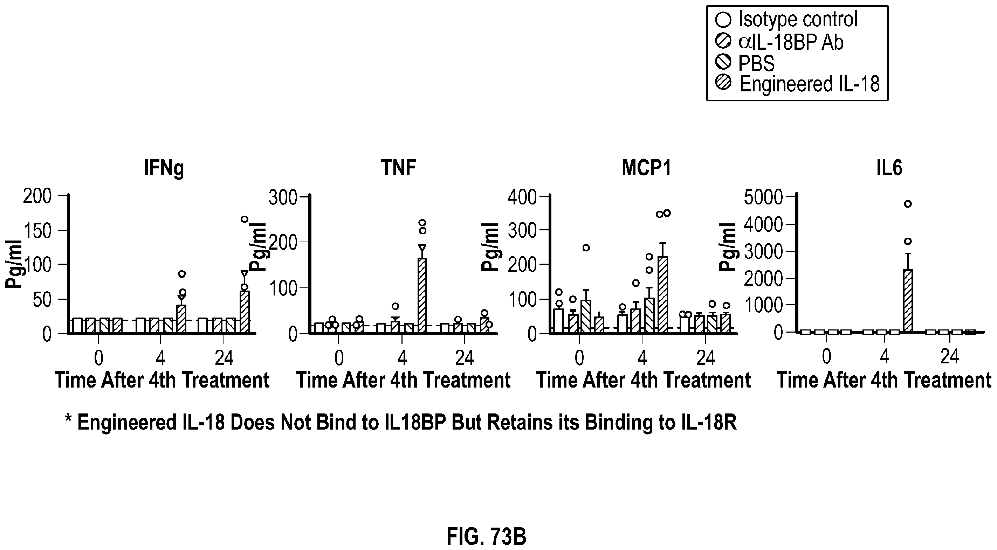

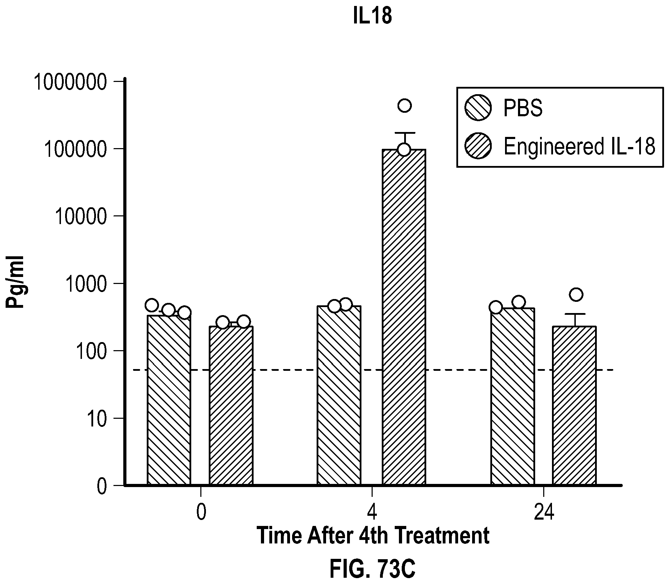

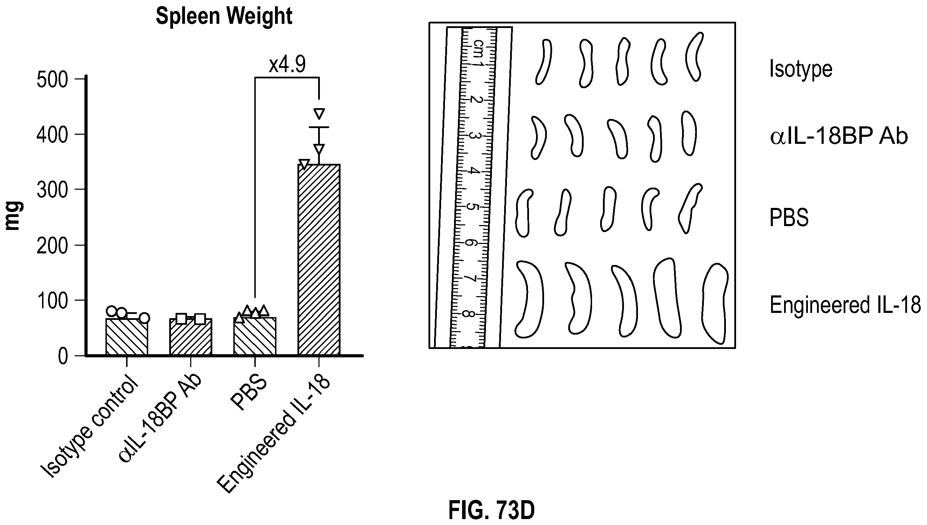

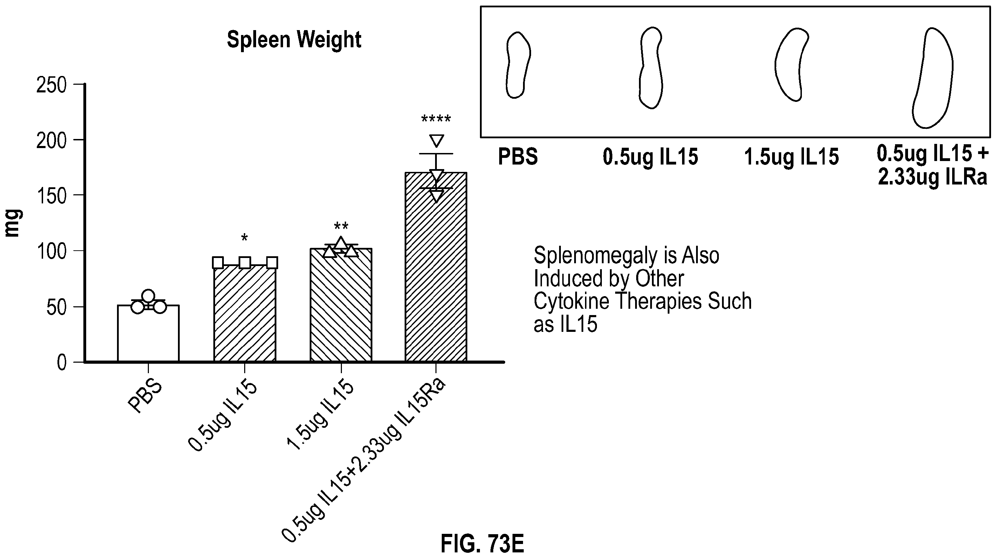

When treating mice with anti-mIL18bp, no loss of weight was observed, similar to control group (Figure 73A). When analyzing blood serum from mice treated with anti-mIL18bp, no increase in inflammatory cytokines IFNg, TNFa, MCP1 and IL6 was observed. In contrast, mice treated with engineered mIL-18 had elevated serum levels of IFNg, TNFa, MCP1 and IL6, 4 hours after the 4^{th} treatment, and elevated serum levels of IFNg 24 hours after the 4^{th} treatment (Figure 73B). Mice treated with engineered mIL-18 had very high serum levels of IL18 (method of IL18 detection identifies also engineered IL18), 4 hours after the 4^{th} treatment, which returned to baseline by 24 hours after the 4^{th} treatment (Figure 73C). Spleens harvested from mice 24 hours after the 4^{th} treatment of anti-mIL18bp were similar to spleens harvested from mice in control groups, while spleens harvested from mice treated with engineered mIL-18 were larger by an average of 4.9 folds compared to control (Figure 73D). Similarly, spleens harvested from mice treated with varying concentrations of IL-15 were larger than controls (Figure 73E).

### EXAMPLE 20: ANTI-IL-18BP ANTIBODY MODULATES TUMOR MICROENVIRONMENT WITHOUT EFFECTING PERIPHERY IN MURINE TUMOR MODEL

To further understand the effects of mouse anti-IL-18BP Ab on the tumor microenvironment and immune periphery, we studied the immune composition of tumors isolated from mice treated with AB-837 monotherapy, compared to control group treated with isotype control in MC38OVA^{dim} tumors.

### Material and Methods:

### Mouse antibodies and recombinant proteins

All mAbs and recombinant proteins were formulated in sterile PBS and were low in endotoxin (<0.05 EU/mg).

### Cell culture

Nine days pre-inoculum, vials of MC38OVA^{dim} cells were thawed into RPMI media containing 10% FBS, 1% Glutamax, 1% Sodium pyruvate, 1% HEPES, 1% NEAA, 0.01% 2-mercaptoethanol, 1% Penicillin-Streptomycin. Following centrifugation at 300xg for 8min, cell pellet was resuspended, counted, and seeded in a T175 flask. On days -7, -5 and -2, the cells were detached and re-seeded at 6-8*10⁶ cells/T175 flask. On the day of inoculum, the cells were detached, centrifuged at 300xg for 10min, filtered through 40uM cell strainer and resuspended in RPMI.

### Inoculation of mice

C57Bl/6 mice (female, 6-8wk, Envigo) were anesthetized with 10% Ketamine and 10% Xylazine mixture injected intraperitoneal. Next, mice were inoculated with MC38OVA^{dim} cells (1.2x10⁶) subcutaneously to the right flank in 50ul/mice. Tumor growth was measured with an electronic caliper every 2-3 days and was reported as 0.5 X W² X L mm³.

### Administration of anti-mIL18BP to tumor-bearing mice

At tumor volume of ~120 mm³ (day 9), mice were randomly assigned into treatment groups. Mice were treated with synagis mouse IgG1, k isotype control 15mg/kg (IP), anti-mIL18bp 837 mIgG1 15mg/kg (IP). Treatments were inoculated twice a week for a total of 4 treatments. Tumor growth was measured with caliper every 2-3 days. Mice were weighed every week. Mice were bled before the 4^{th} treatment, 4 hours after the 4^{th} treatment, 24 and 48 hours after the 4^{th} treatment. Serum was analyzed for levels of IL-18 by ELISA (MBL Cat. No. 7625) and IL18bp (in-house ELISA). Tumors were harvested from mice 24 hours after the 4^{th} treatment.

### Tumor immune phenotyping of MC38OVAdim tumor microenvironment

Mice were inoculated with MC38OVAdim and treated with anti-mouse IL-18BP Ab or isotype control twice a week. Tumors, spleens and serum were collected. Tumor samples were dissected into small pieces and transferred to GentleMACs^{™} C tubes (Miltenyi Biotec) containing an enzyme mix using human tumor Dissociation Kit (Miltenyi Biotec), as per the manufacturer's protocol. After dissociation, samples were centrifuged at 300g for 8 minutes and supernatants were collected. Cells were filtered through a 70µm filter. Single-cell suspensions were seeded into a 96-well V-bottomed plate. Cells were labeled for viability and dead cells were excluded using the Zombie Aqua viability dye (BioLegend). To block Fcγ receptors, cells were incubated with 10µg/mL of anti-CD16 and anti-CD32 antibodies (BD Bioscience) in cold 1xPBS buffer for 10 minutes. Various immune populations were stained with anti-mouse antibodies (see Table 9). For cytokine staining, cells were stimulated with 50ng/ml PMA, 1ug/ml ionomycin and BFA. Then, cells were stained extracellularly for membrane markers and intracellularly for cytokine expression. Cells were acquired on FACS Fortessa cytometer (BD Bioscience). Analysis was done using FlowJo. Collected supernatants were centrifuged at 3130g for 10 minutes. Following centrifugation, supernatants were recollected. Tumor supernatants and serum were analyzed for presence of IFNg by Cytometric Bead Array (CBA) Mouse Inflammation Kit (BD Cat. No. 552364).

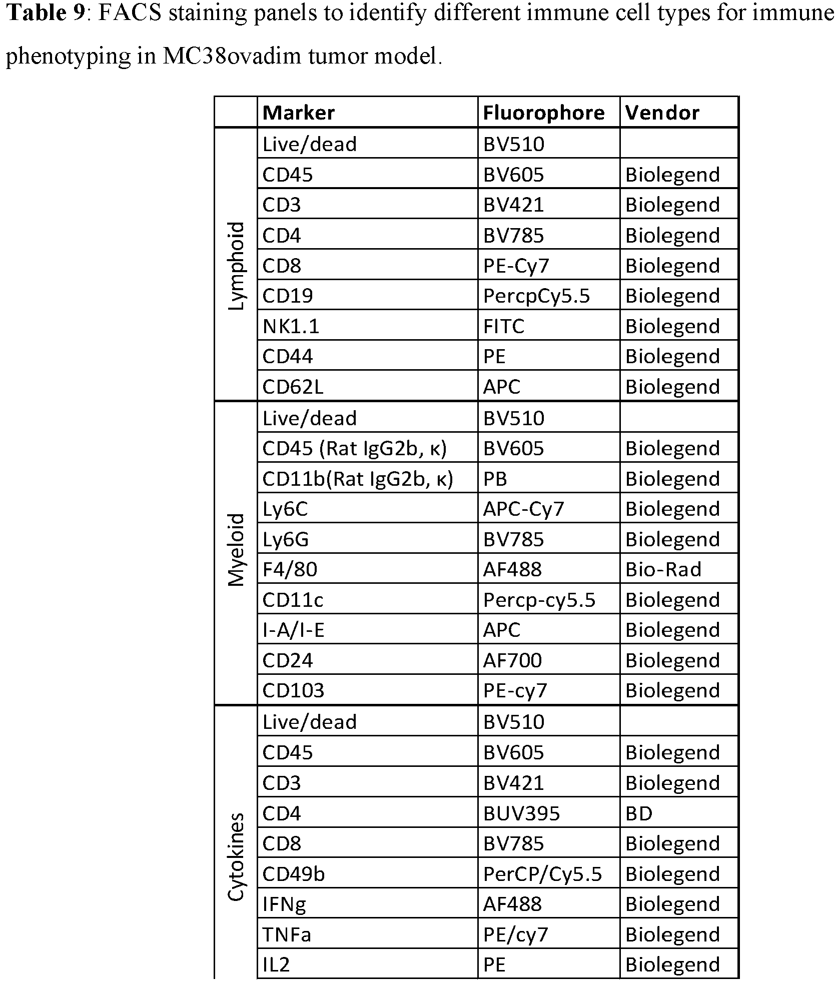

### Results:

When treating mice with anti-mouse IL18BP, a tumor growth inhibition of 41.1% was observed after 4 treatments (Figure 74). To assess the immune composition, tumors and spleens were harvested as described in materials and methods, single cells suspensions were generated, and cells were stained with panels of antibodies as described in Table 9. Tumor supernatants and blood serum were collected and analyzed for cytokine concentrations. Monotherapy of anti-IL-18BP Ab resulted in increased numbers of CD3⁺ (+100%, p=0.007) and CD8⁺ (+85%, p=0.0087) T cells in the TME (Fig. 75A-C). This therapy also induced increased concentrations of IFNg⁺ (+76%, p=0.0519) in tumor supernatants (Fig. 75D). When inspecting activation markers, there was a significant increase in CD8⁺CD69⁺CD107⁺ (+168%, p=0.0005) T cells (Fig. 75E), as well as CD107⁺ (+54%, p=0.0094) NK cells (Fig. 75F). In the myeloid compartment, there was a significant increase in numbers of DC cells (+136%, p=0.0017), as well as in MHC-II (+40%, p=0.0138) expression on them (Fig. 75G), indicating on potentially increased capacity to prime T cells. When inspecting similar parameters in spleens or serum of AB-837-treated animals, no immune activation was observed. Only minor effects were detected - a slight decrease in NK cells, macrophages and neutrophiles, an increase in CD69 expression on NK cells, and a decrease in mIL18Ra expression on NK cells (Fig. 75H). IFNg was not detected in the serum of mice treated with anti-IL-18BP Ab or with isotype control (Fig. 75I). In summary, monotherapy with AB-837 induced robust TME-constrained immune modulation, without peripheral immune activation.

### EXAMPLE 21: EFFICACY OF ANTI IL-18BP AB IN COMBINATION WITH CHEMOTHERAPY

### Methods

### Antibody and oxaliplatin administration

C57Bl/6 mice (female, 6-8wk, Envigo), were subcutaneously inoculated with 1.2x10⁶MC38OVA^{dim} mouse tumor cells in 50µl/mouse into the right flank. Tumor growth was measured with an electronic caliper every 2-3 days and was reported as 0.5 X W² X L mm³. The experimental endpoint is defined at tumor volume of 1800mm³. Mice with body weight loss of above 10% between measurements, or 20% reduction from initial weight were excluded.

At a tumor volume of 110mm³ mice were randomly assigned into two treatment groups: group administered with 5mg/kg oxaliplatin (Sigma-Aldrich, Cat. 09512) or control group administered with DDW. When tumors reached volume of 140mm³, mice in each group were assigned into two separate groups: group treated with 15mg/kg anti-mouse IL18bp antibody or isotype control. Antibodies were injected twice a week for a total of 6 treatments (see Table 10 for details).

**Table 10: Experimental treatment groups**

| **Group** | **Ab** | **Ab Dose (mg/kg)** | **Oxaliplatin dose (mg/kg)** | **N** |
|---|---|---|---|---|
| **1** | **DDW+Synagis mIgG1** | **15** | **0** | **10** |
| **2** | **DDW+anti-IL18bp mIgG1** | **15** | **0** | **10** |
| **3** | **Oxaliplatin+Synagis mIgG1** | **15** | **5** | **10** |
| **4** | **Oxaliplatin+anti-IL18bp mIgG1** | **15** | **5** | **10** |

### Results

### Combination of Oxaliplatin and anti-IL18BP antibody results in synergistic effects in MC38ova tumor model

To study the effects of combining anti-IL18bp mAb with oxaliplatin in MC38ovadim mouse tumor model mice were assigned to groups as described in table 10. As shown in figure 77, administration of combined therapy resulted in a synergistic anti-tumor responses compared to monotherapy with single agents. A combination therapy with oxaliplatin and anti-IL-18BP mAb resulted in 72% TGI (p<0.0001) compared to oxaliplatin monotherapy and 42% TGI (p=0.24) compared to anti-IL-18bp mAb monotherapy.

The examples set forth above are provided to give those of ordinary skill in the art a complete disclosure and description of how to make and use the embodiments of the compositions, systems and methods of the invention, and are not intended to limit the scope of what the inventors regard as their invention. All patents and publications mentioned in the specification are indicative of the levels of skill of those skilled in the art to which the invention pertains.

All headings and section designations are used for clarity and reference purposes only and are not to be considered limiting in any way. For example, those of skill in the art will appreciate the usefulness of combining various aspects from different headings and sections as appropriate.

## Claims

1. An anti-IL18-BP (interleukin-18 binding protein) antibody, wherein the anti-IL18-BP antibody specifically binds human IL18-BP and exhibits a binding affinity or KD equal to or lower than 1pM, as measured by solution equilibrium titration, and wherein said antibody comprises a vhCDR1, a vhCDR2, a vhCDR3, a vlCDR1, a vlCDR2 and a vlCDR3, wherein the vhCDR1, the vhCDR2, the vhCDR3, the vlCDR1, the vlCDR2 and the vlCDR3 are selected from the group consisting of:
i. a vhCDR1 having the amino acid sequence of SEQ ID NO: 155,
a vhCDR2 having the amino acid sequence of SEQ ID NO: 156,
a vhCDR3 having the amino acid sequence of SEQ ID NO: 157,
a vlCDR1 having the amino acid sequence of SEQ ID NO: 160,
a vlCDR2 having the amino acid sequence of SEQ ID NO: 161, and
a vlCDR3 having the amino acid sequence of SEQ ID NO: 162;
ii. a vhCDR1 having the amino acid sequence of SEQ ID NO: 75,
a vhCDR2 having the amino acid sequence of SEQ ID NO: 76,
a vhCDR3 having the amino acid sequence of SEQ ID NO: 77,
a vlCDR1 having the amino acid sequence of SEQ ID NO: 80,
a vlCDR2 having the amino acid sequence of SEQ ID NO: 81, and
a vlCDR3 having the amino acid sequence of SEQ ID NO: 82;
iii. a vhCDR1 having the amino acid sequence of SEQ ID NO: 85,
a vhCDR2 having the amino acid sequence of SEQ ID NO: 86,
a vhCDR3 having the amino acid sequence of SEQ ID NO: 87,
a vlCDR1 having the amino acid sequence of SEQ ID NO: 90,
a vlCDR2 having the amino acid sequence of SEQ ID NO: 91, and
a vlCDR3 having the amino acid sequence of SEQ ID NO: 92;
iv. a vhCDR1 having the amino acid sequence of SEQ ID NO: 105,
a vhCDR2 having the amino acid sequence of SEQ ID NO: 106,
a vhCDR3 having the amino acid sequence of SEQ ID NO: 107,
a vlCDR1 having the amino acid sequence of SEQ ID NO: 110,
a vlCDR2 having the amino acid sequence of SEQ ID NO: 111, and
a vlCDR3 having the amino acid sequence of SEQ ID NO: 112; and
v. a vhCDR1 having the amino acid sequence of SEQ ID NO: 195,
a vhCDR2 having the amino acid sequence of SEQ ID NO: 196,
a vhCDR3 having the amino acid sequence of SEQ ID NO: 197,
a vlCDR1 having the amino acid sequence of SEQ ID NO: 200,
a vlCDR2 having the amino acid sequence of SEQ ID NO: 201, and
a vlCDR3 having the amino acid sequence of SEQ ID NO: 202.

2. The anti-IL18-BP antibody of claim 1, wherein said antibody comprises
a vhCDR1 having an amino acid sequence of SEQ ID NO: 155,
a vhCDR2 having an amino acid sequence of SEQ ID NO: 156,
a vhCDR3 having an amino acid sequence of SEQ ID NO: 157,
a vlCDR1 having an amino acid sequence of SEQ ID NO: 160,
a vlCDR2 having an amino acid sequence of SEQ ID NO: 161, and
a vlCDR3 having an amino acid sequence of SEQ ID NO: 162.

3. The anti-IL18-BP antibody of claim 1, wherein said antibody comprises:
a vhCDR1 having an amino acid sequence of SEQ ID NO: 85,
a vhCDR2 having an amino acid sequence of SEQ ID NO: 86,
a vhCDR3 having an amino acid sequence of SEQ ID NO: 87,
a vlCDR1 having an amino acid sequence of SEQ ID NO: 90,
a vlCDR2 having an amino acid sequence of SEQ ID NO: 91, and
a vlCDR3 having an amino acid sequence of SEQ ID NO: 92.

4. The anti-IL18-BP antibody according to claim 1, wherein said antibody comprises a heavy chain variable domain and a light chain variable domain, wherein the heavy chain variable domain and the light chain variable domain are selected from the group consisting of:
i. a heavy chain variable domain having the amino acid sequence of SEQ ID NO: 154 and a light chain variable domain having the amino acid sequence of SEQ ID NO: 159;
ii a heavy chain variable domain having the amino acid sequence of SEQ ID NO: 74 and a light chain variable domain having the amino acid sequence of SEQ ID NO: 79;
iii. a heavy chain variable domain having the amino acid sequence of SEQ ID NO: 84 and a light chain variable domain having the amino acid sequence of SEQ ID NO: 89;
iv. a heavy chain variable domain having the amino acid sequence of SEQ ID NO: 104 and a light chain variable domain having the amino acid sequence of SEQ ID NO: 109; and
v. a heavy chain variable domain having the amino acid sequence of SEQ ID NO: 194 and a light chain variable domain having the amino acid sequence of SEQ ID NO: 199.

5. The anti-IL18-BP antibody according to claim 2, wherein the heavy chain variable domain comprises an amino acid sequence having at least 90% sequence identity to SEQ ID NO: 154, and the light chain variable domain comprises an amino acid sequence having at least 90% sequence identity to SEQ ID NO: 159.

6. The anti-IL18-BP antibody according to claim 5, wherein the heavy chain variable domain comprises an amino acid sequence having at least 95% sequence identity to SEQ ID NO: 154, and the light chain variable domain comprises an amino acid sequence having at least 95% sequence identity to SEQ ID NO: 159.

7. The anti-IL18-BP antibody according to claim 5 or 6, wherein the heavy chain variable domain comprises an amino acid sequence of SEQ ID NO: 154, and the light chain variable domain comprises an amino acid sequence of SEQ ID NO: 159.

8. The anti-IL18-BP antibody of claim 3, wherein the heavy chain variable domain comprises an amino acid sequence having at least 90% sequence identity to SEQ ID NO: 84, and the light chain variable domain comprises an amino acid sequence having at least 90% sequence identity to SEQ ID NO: 89.

9. The anti-IL18-BP antibody of claim 8, wherein the heavy chain variable domain comprises an amino acid sequence having at least 95% sequence identity to SEQ ID NO: 84, and the light chain variable domain comprises an amino acid sequence having at least 95% sequence identity to SEQ ID NO: 89.

10. The anti-IL18-BP antibody of claim 8 or 9, wherein the heavy chain variable domain comprises an amino acid sequence of SEQ ID NO: 84, and the light chain variable domain comprises an amino acid sequence of SEQ ID NO: 89.

11. The anti-IL18-BP antibody according to any one of claims 1-10, wherein said antibody comprises:
a) a CH1-hinge-CH2-CH3 region from human IgG1, IgG2, or IgG3;
b) a CH1-hinge-CH2-CH3 region from human IgG4;
c) a CL region of human kappa 2 light chain; and/or
d) a CL region of human lambda 2 light chain.

12. The anti-IL18-BP antibody according to any one of claims 1, 4 or 11, wherein said antibody comprises a heavy chain and a light chain, wherein the heavy chain and the light chain are selected from the group consisting of:
i. a heavy chain having the amino acid sequence of SEQ ID NO: 158 and a light chain having the amino acid sequence of SEQ ID NO: 163;
ii. a heavy chain having the amino acid sequence of SEQ ID NO: 78 and a light chain having the amino acid sequence of SEQ ID NO: 83;
iii. a heavy chain having the amino acid sequence of SEQ ID NO: 88 and a light chain having the amino acid sequence of SEQ ID NO: 93;
iv. a heavy chain having the amino acid sequence of SEQ ID NO: 108 and a light chain having the amino acid sequence of SEQ ID NO: 113; and
v. a heavy chain having the amino acid sequence of SEQ ID NO: 198 and a light chain having the amino acid sequence of SEQ ID NO: 203.

13. The anti-IL18-BP antibody according to claim 7, wherein the heavy chain comprises an amino acid sequence of SEQ ID NO: 158, and the light chain comprises an amino acid sequence of SEQ ID NO: 163.

14. The anti-IL18-BP antibody of claim 10, wherein the heavy chain comprises an amino acid sequence of SEQ ID NO: 88, and the light chain comprises an amino acid sequence of SEQ ID NO: 93.

15. The anti-IL18-BP antibody according to any of claims 1-14, for use in a method of treatment.

16. The anti-IL18-BP antibody according to any of claims 1-14, wherein the antibody activates T cells, NK cells, NKT cells, Dendritic cells, MAIT T cells, γδ T cells, and/or innate lymphoid cells (ILCs), and/or modulates Myeloid cells, and wherein the antibody is for use in a method for the treatment of cancer.

17. The anti-IL18-BP antibody for use according to claim 16, wherein the method comprises administering the anti-IL18-BP to a patient, and wherein:
a) the T-cells, NK-cells, NKT-cells, dendritic cells, MAIT T cells, γδ T cells, or ILCs of the patient are activated or the myeloid cells of the patient are modulated;
b) said anti-IL18-BP antibody increases IL-18 mediated immuno-stimulating activity in the tumor microenvironment (TME), and/or lymph nodes; or
c) said anti-IL18-BP antibody restores activity on T cells, NK cells, NKT cells, Myeloid cells, Dendritic cells, and/or ILCs.

18. The anti-IL18-BP antibody for use according to claim 16 or 17, wherein:
a) said T-cells are cytotoxic T-cells (CTLs), optionally wherein said T-cells are selected from the group consisting of CD4⁺ T-cells and CD8⁺ T-cells; and/or
b) the patient for treatment:
i) comprises an increase in tumor growth inhibition of at least about 10%, 20%, 30%, 40%, 50%, 60%, 70%, 80%, 90%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000%, as compared to a control or an untreated patient; or
ii) exhibits a decrease in tumor growth of at least about 10%, 20%, 30%, 40%, 50%, 60%, 70%, 80%, 90%, 100%, 125%, 150%, 175%, 200%, 225%, 250%, 275%, 300%, 325%, 350%, 375%, 400%, 425%, 450%, 475%, 500%, 525%, 550%, 575%, 600%, 625%, 650%, 675%, 700%, 725%, 750%, 775%, 800%, 825%, 850%, 875%, 900%, 925%, 950%, 975%, or 1000%, as compared to a control or an untreated patient.

19. The anti-IL18-BP antibody for use according to any one of claims 16-18, wherein:
a) said NK-cells are CD16+ lymphocytes;
b) said NK-cells are CD56+ NK cells; or
c) said activation is measured as an increase in expression of one or more activation makers.

20. The anti-IL18-BP antibody for use according to any one of claims 15-19, wherein the method further comprises administering a second antibody, optionally wherein:
a) said second antibody is an antibody that binds to and/or inhibits a human checkpoint receptor protein; and/or
b) said second antibody is selected from the group consisting of an anti-PVRIG antibody, an anti-PD-1 antibody, an anti-PD-L1 antibody, an anti-TIGIT antibody, an anti-CTLA-4 antibody, an anti-PD-L2 antibody, an anti-B7-H3 antibody, an anti B7-H4 antibody, an anti-CEACAM-1 antibody, an anti-PVR antibody, an anti-LAG3 antibody, an anti-CD112 antibody, an anti-CD96 antibody, an anti-TIM3 antibody, an anti-BTLA antibody, an anti-ICOS antibody, an anti-OX40 antibody, or an anti-41BB antibody, an anti-CD27 antibody, or an anti-GITR antibody.

21. The anti-IL18-BP antibody for use according to any one of claims 15-20, wherein:
a) said anti-IL18-BP antibody is for use in combination with an immunostimulatory antibody, a cytokine therapy, an immunomodulatory drug, cytotoxic agents, chemotherapeutic agents, growth inhibitory agents, anti-hormonal agents, kinase inhibitors, anti-angiogenic agents, cardioprotectants, immunosuppressive agents, agents that promote proliferation of hematological cells, angiogenesis inhibitors, protein tyrosine kinase (PTK) inhibitors, or other therapeutic agents;
b) the method further comprises administering one or more inflammasome activators, optionally wherein said inflammasome activator is a chemotherapy agent, further optionally wherein said chemotherapy agent is selected from the group consisting of Platinum (including Platinum chemotherapy agent), Paclitaxel (taxol), Sorafenib, Doxorubicin, Sorafenib, 5-FU, Gemcitabine, and Irinotecan (CPT-11), preferably wherein said Platinum chemotherapy agent is Oxaliplatin or Cisplatin; and/or
c) the anti-IL18-BP antibody and the immunostimulatory antibody, cytokine therapy, immunomodulatory drug, cytotoxic agents, chemotherapeutic agents, growth inhibitory agents, anti-hormonal agents, kinase inhibitors, anti-angiogenic agents, cardioprotectants, immunosuppressive agents, agents that promote proliferation of hematological cells, angiogenesis inhibitors, protein tyrosine kinase (PTK) inhibitors, or other therapeutic agents are administered sequentially or simultaneously, in any order, and in one or more formulations.

22. The anti-IL18-BP antibody for use according to any one of claims 15-21, wherein the anti-IL18-BP antibody exhibits a binding affinity or KD of less than 0.25 pM, 0.30 pM, 0.35 pM, 0.40 pM, 0.45 pM, 0.50 pM, 0.55 pM, 0.60 pM, 0.65 pM, 0.70 pM, 0.75 pM, 0.80 pM, 0.85 pM, 0.90 pM, 0.95 pM, or 1 pM.

23. A composition comprising the anti-IL18-BP antibody according to any one of claims 1-14.

24. A composition comprising the anti-IL18-BP antibody according to any one of claims 1-14 for use according to any one of claims 15-22.

## Patentansprüche

1. Anti-IL18-BP(Interleukin-18-Bindungsprotein)-Antikörper, wobei der Anti-IL18-BP-Antikörper spezifisch menschliches IL18-BP bindet und eine Bindungsaffinität oder KD gleich oder kleiner 1 pM zeigt, wie mit Lösungsgleichgewichtstitration gemessen, und wobei der Antikörper eine vhCDR1, eine vhCDR2, eine vhCDR3, eine vlCDR1, eine vlCDR2 und eine vlCDR3 umfasst, wobei die vhCDR1, die vhCDR2, die vhCDR3, die vlCDR1, die vlCDR2 und die vlCDR3 ausgewählt sind aus der Gruppe bestehend aus:
i. einer vhCDR1 mit der Aminosäuresequenz unter SEQ ID NO: 155,
einer vhCDR2 mit der Aminosäuresequenz unter SEQ ID NO: 156,
einer vhCDR3 mit der Aminosäuresequenz unter SEQ ID NO: 157,
einer v1CDR1 mit der Aminosäuresequenz unter SEQ ID NO: 160,
einer vlCDR2 mit der Aminosäuresequenz unter SEQ ID NO: 161 und
einer vlCDR3 mit der Aminosäuresequenz unter SEQ ID NO: 162;
ii. einer vhCDR1 mit der Aminosäuresequenz unter SEQ ID NO: 75,
einer vhCDR2 mit der Aminosäuresequenz unter SEQ ID NO: 76,
einer vhCDR3 mit der Aminosäuresequenz unter SEQ ID NO: 77,
einer v1CDR1 mit der Aminosäuresequenz unter SEQ ID NO: 80,
einer vlCDR2 mit der Aminosäuresequenz unter SEQ ID NO: 81 und
einer vlCDR3 mit der Aminosäuresequenz unter SEQ ID NO: 82;
iii. einer vhCDR1 mit der Aminosäuresequenz unter SEQ ID NO: 85,
einer vhCDR2 mit der Aminosäuresequenz unter SEQ ID NO: 86,
einer vhCDR3 mit der Aminosäuresequenz unter SEQ ID NO: 87,
einer v1CDR1 mit der Aminosäuresequenz unter SEQ ID NO: 90,
einer vlCDR2 mit der Aminosäuresequenz unter SEQ ID NO: 91 und
einer vlCDR3 mit der Aminosäuresequenz unter SEQ ID NO: 92;
iv. einer vhCDR1 mit der Aminosäuresequenz unter SEQ ID NO: 105,
einer vhCDR2 mit der Aminosäuresequenz unter SEQ ID NO: 106,
einer vhCDR3 mit der Aminosäuresequenz unter SEQ ID NO: 107,
einer v1CDR1 mit der Aminosäuresequenz unter SEQ ID NO: 110,
einer vlCDR2 mit der Aminosäuresequenz unter SEQ ID NO: 111 und
einer vlCDR3 mit der Aminosäuresequenz unter SEQ ID NO: 112; und
v. einer vhCDR1 mit der Aminosäuresequenz unter SEQ ID NO: 195,
einer vhCDR2 mit der Aminosäuresequenz unter SEQ ID NO: 196,
einer vhCDR3 mit der Aminosäuresequenz unter SEQ ID NO: 197,
einer vlCDR1 mit der Aminosäuresequenz unter SEQ ID NO: 200,
einer vlCDR2 mit der Aminosäuresequenz unter SEQ ID NO: 201 und
einer vlCDR3 mit der Aminosäuresequenz unter SEQ ID NO: 202.

2. Anti-IL18-BP-Antikörper nach Anspruch 1, wobei der Antikörper
eine vhCDR1 mit einer Aminosäuresequenz unter SEQ ID NO: 155,
eine vhCDR2 mit einer Aminosäuresequenz unter SEQ ID NO: 156,
eine vhCDR3 mit einer Aminosäuresequenz unter SEQ ID NO: 157,
eine vlCDR1 mit einer Aminosäuresequenz unter SEQ ID NO: 160,
eine vlCDR2 mit einer Aminosäuresequenz unter SEQ ID NO: 161 und
eine vlCDR3 mit einer Aminosäuresequenz unter SEQ ID NO: 162 umfasst.

3. Anti-IL18-BP-Antikörper nach Anspruch 1, wobei der Antikörper Folgendes umfasst:
eine vhCDR1 mit einer Aminosäuresequenz unter SEQ ID NO: 85,
eine vhCDR2 mit einer Aminosäuresequenz unter SEQ ID NO: 86,
eine vhCDR3 mit einer Aminosäuresequenz unter SEQ ID NO: 87,
eine vlCDR1 mit einer Aminosäuresequenz unter SEQ ID NO: 90,
eine vlCDR2 mit einer Aminosäuresequenz unter SEQ ID NO: 91 und
eine vlCDR3 mit einer Aminosäuresequenz unter SEQ ID NO: 92.

4. Anti-IL18-BP-Antikörper gemäß Anspruch 1, wobei der Antikörper eine variable Schwerkettendomäne und eine variable Leichtkettendomäne umfasst, wobei die variable Schwerkettendomäne und die variable Leichtkettendomäne ausgewählt sind aus der Gruppe bestehend aus:
i. einer variablen Schwerkettendomäne mit der Aminosäuresequenz unter SEQ ID NO: 154 und einer variablen Leichtkettendomäne mit der Aminosäuresequenz unter SEQ ID NO: 159;
ii. einer variablen Schwerkettendomäne mit der Aminosäuresequenz unter SEQ ID NO: 74 und einer variablen Leichtkettendomäne mit der Aminosäuresequenz unter SEQ ID NO: 79;
iii. einer variablen Schwerkettendomäne mit der Aminosäuresequenz unter SEQ ID NO: 84 und einer variablen Leichtkettendomäne mit der Aminosäuresequenz unter SEQ ID NO: 89;
iv. einer variablen Schwerkettendomäne mit der Aminosäuresequenz unter SEQ ID NO: 104 und einer variablen Leichtkettendomäne mit der Aminosäuresequenz unter SEQ ID NO: 109; und
v. einer variablen Schwerkettendomäne mit der Aminosäuresequenz unter SEQ ID NO: 194 und einer variablen Leichtkettendomäne mit der Aminosäuresequenz unter SEQ ID NO: 199.

5. Anti-IL18-BP-Antikörper gemäß Anspruch 2, wobei die variable Schwerkettendomäne eine Aminosäuresequenz mit einer Sequenzidentität von mindestens 90 % mit SEQ ID NO: 154 umfasst und die variable Leichtkettendomäne eine Aminosäuresequenz mit einer Sequenzidentität von mindestens 90 % mit SEQ ID NO: 159 umfasst.

6. Anti-IL18-BP-Antikörper gemäß Anspruch 5, wobei die variable Schwerkettendomäne eine Aminosäuresequenz mit einer Sequenzidentität von mindestens 95 % mit SEQ ID NO: 154 umfasst und die variable Leichtkettendomäne eine Aminosäuresequenz mit einer Sequenzidentität von mindestens 95 % mit SEQ ID NO: 159 umfasst.

7. Anti-IL18-BP-Antikörper gemäß Anspruch 5 oder 6, wobei die variable Schwerkettendomäne eine Aminosäuresequenz unter SEQ ID NO: 154 umfasst und die variable Leichtkettendomäne eine Aminosäuresequenz unter SEQ ID NO: 159 umfasst.

8. Anti-IL18-BP-Antikörper nach Anspruch 3, wobei die variable Schwerkettendomäne eine Aminosäuresequenz mit einer Sequenzidentität von mindestens 90 % mit SEQ ID NO: 84 umfasst und die variable Leichtkettendomäne eine Aminosäuresequenz mit einer Sequenzidentität von mindestens 90 % mit SEQ ID NO: 89 umfasst.

9. Anti-IL18-BP-Antikörper nach Anspruch 8, wobei die variable Schwerkettendomäne eine Aminosäuresequenz mit einer Sequenzidentität von mindestens 95 % mit SEQ ID NO: 84 umfasst und die variable Leichtkettendomäne eine Aminosäuresequenz mit einer Sequenzidentität von mindestens 95 % mit SEQ ID NO: 89 umfasst.

10. Anti-IL18-BP-Antikörper nach Anspruch 8 oder 9, wobei die variable Schwerkettendomäne eine Aminosäuresequenz unter SEQ ID NO: 84 umfasst und die variable Leichtkettendomäne eine Aminosäuresequenz unter SEQ ID NO: 89 umfasst.

11. Anti-IL18-BP-Antikörper gemäß einem der Ansprüche 1-10, wobei der Antikörper Folgendes umfasst:
a) eine CH1-Hinge-CH2-CH3-Region von Mensch-IgG1, -IgG2 oder -IgG3;
b) eine CH1-Hinge-CH2-CH3-Region von Mensch-IgG4;
c) eine CL-Region der Mensch-kappa-2-Leichtkette; und/oder
d) eine CL-Region der Mensch-lambda-2-Leichtkette.

12. Anti-IL18-BP-Antikörper gemäß einem der Ansprüche 1, 4 oder 11, wobei der Antikörper eine Schwerkette und eine Leichtkette umfasst, wobei die Schwerkette und die Leichtkette ausgewählt sind aus der Gruppe bestehend aus:
i. einer Schwerkette mit der Aminosäuresequenz unter SEQ ID NO: 158 und einer Leichtkette mit der Aminosäuresequenz unter SEQ ID NO: 163;
ii. einer Schwerkette mit der Aminosäuresequenz unter SEQ ID NO: 78 und einer Leichtkette mit der Aminosäuresequenz unter SEQ ID NO: 83;
iii. einer Schwerkette mit der Aminosäuresequenz unter SEQ ID NO: 88 und einer Leichtkette mit der Aminosäuresequenz unter SEQ ID NO: 93;
iv. einer Schwerkette mit der Aminosäuresequenz unter SEQ ID NO: 108 und einer Leichtkette mit der Aminosäuresequenz unter SEQ ID NO: 113; und
v. einer Schwerkette mit der Aminosäuresequenz unter SEQ ID NO: 198 und einer Leichtkette mit der Aminosäuresequenz unter SEQ ID NO: 203.

13. Anti-IL18-BP-Antikörper gemäß Anspruch 7, wobei die Schwerkette eine Aminosäuresequenz unter SEQ ID NO: 158 umfasst und die Leichtkette eine Aminosäuresequenz unter SEQ ID NO: 163 umfasst.

14. Anti-IL18-BP-Antikörper nach Anspruch 10, wobei die Schwerkette eine Aminosäuresequenz unter SEQ ID NO: 88 umfasst und die Leichtkette eine Aminosäuresequenz unter SEQ ID NO: 93 umfasst.

15. Anti-IL18-BP-Antikörper gemäß einem der Ansprüche 1-14 zur Verwendung bei einem Behandlungsverfahren.

16. Anti-IL18-BP-Antikörper gemäß einem der Ansprüche 1-14, wobei der Antikörper T-Zellen, NK-Zellen, NKT-Zellen, dendritische Zellen, MAIT-T-Zellen, γδ-T-Zellen und/oder ILC (Innate Lymphoid Cells) aktiviert und/oder myeloische Zellen moduliert und wobei der Antikörper zur Verwendung bei einem Verfahren zur Behandlung von Krebs bestimmt ist.

17. Anti-IL18-BP-Antikörper zur Verwendung gemäß Anspruch 16, wobei das Verfahren Verabreichen des Anti-IL18-BP an einen Patienten umfasst und wobei:
a) die T-Zellen, NK-Zellen, NKT-Zellen, dendritischen Zellen, MAIT-T-Zellen, γδ-T-Zellen oder ILC des Patienten aktiviert oder die myeloischen Zellen des Patienten moduliert werden;
b) der Anti-IL18-BP-Antikörper IL-18-vermittelte immunstimulierende Aktivität in der Tumormikroumgebung (TME) und/oder Lymphknoten erhöht; oder
c) der Anti-IL18-BP-Antikörper Aktivität auf T-Zellen, NK-Zellen, NKT-Zellen, myeloischen Zellen, dendritischen Zellen, und/oder ILC wiederherstellt.

18. Anti-IL18-BP-Antikörper zur Verwendung gemäß Anspruch 16 oder 17, wobei:
a) es sich bei den T-Zellen um zytotoxische T-Zellen (CTL) handelt, wobei die T-Zellen gegebenenfalls aus der Gruppe bestehend aus CD4⁺-T-Zellen und CD8⁺-T-Zellen ausgewählt sind; und/oder
b) der Patient bei Behandlung:
i) eine Zunahme der Tumorwachstumshemmung um mindestens etwa 10 %, 20 %, 30 %, 40 %, 50 %, 60 %, 70 %, 80 %, 90 %, 100 %, 125 %, 150 %, 175 %, 200 %, 225 %, 250 %, 275 %, 300 %, 325 %, 350 %, 375 %, 400 %, 425 %, 450 %, 475 %, 500 %, 525 %, 550 %, 575 %, 600 %, 625 %, 650 %, 675 %, 700 %, 725 %, 750 %, 775 %, 800 %, 825 %, 850 %, 875 %, 900 %, 925 %, 950 %, 975 % oder 1000% im Vergleich zu einer Kontrolle oder einem unbehandelten Patienten umfasst; oder
ii) eine Abnahme des Tumorwachstums um mindestens etwa 10 %, 20 %, 30 %, 40 %, 50 %, 60 %, 70 %, 80 %, 90 %, 100 %, 125 %, 150 %, 175 %, 200 %, 225 %, 250 %, 275 %, 300 %, 325 %, 350 %, 375 %, 400 %, 425 %, 450 %, 475 %, 500 %, 525 %, 550 %, 575 %, 600 %, 625 %, 650 %, 675 %, 700 %, 725 %, 750 %, 775 %, 800 %, 825 %, 850 %, 875 %, 900 %, 925 %, 950 %, 975 % oder 1000% im Vergleich zu einer Kontrolle oder einem unbehandelten Patienten zeigt.

19. Anti-IL18-BP-Antikörper zur Verwendung gemäß einem der Ansprüche 16-18, wobei:
a) es sich bei den NK-Zellen um CD16+ Lymphozyten handelt;
b) es sich bei den NK-Zellen um CD56+ NK-Zellen handelt; oder
c) die Aktivierung als Erhöhung der Expression eines oder mehrerer Aktivierungsmacher gemessen wird.

20. Anti-IL18-BP-Antikörper zur Verwendung gemäß einem der Ansprüche 15-19, wobei das Verfahren ferner Verabreichen eines zweiten Antikörpers umfasst, gegebenenfalls wobei:
a) es sich bei dem zweiten Antikörper um einen Antikörper handelt, der an ein menschliches Checkpoint-Rezeptorprotein bindet und/oder dieses hemmt; und/oder
b) der zweite Antikörper aus der Gruppe bestehend aus einem Anti-PVRIG-Antikörper, einem Anti-PD-1-Antikörper, einem Anti-PD-L1-Antikörper, einem Anti-TIGIT-Antikörper, einem Anti-CTLA-4-Antikörper, einem Anti-PD-L2-Antikörper, einem Anti-B7-H3-Antikörper, einem Anti-B7-H4-Antikörper, einem Anti-CEACAM-1-Antikörper, einem Anti-PVR-Antikörper, einem Anti-LAG3-Antikörper, einem Anti-CD112-Antikörper, einem Anti-CD96-Antikörper, einem Anti-TIM3-Antikörper, einem Anti-BTLA-Antikörper, einem Anti-ICOS-Antikörper, einem Anti-OX40-Antikörper oder einem Anti-41BB-Antikörper, einem Anti-CD27-Antikörper oder einem Anti-GITR-Antikörper ausgewählt ist.

21. Anti-IL18-BP-Antikörper zur Verwendung gemäß einem der Ansprüche 15-20, wobei:
a) der Anti-IL18-BP-Antikörper zur Verwendung in Kombination mit einem immunstimulierenden Antikörper, einer Zytokintherapie, einem Immunmodulator, Zytostatika, Chemotherapeutika, Wachstumshemmern, Antihormonmitteln, Kinase-Inhibitoren, Antiangiogenetika, Kardioprotektoren, Immunsuppressiva, die Proliferation hämatologischer Zellen fördernden Mitteln, Angiogenesehemmern, Proteintyrosinkinase(PTK)-Hemmern oder anderen Therapeutika bestimmt ist;
b) das Verfahren ferner Verabreichen eines oder mehrerer Inflammasom-Aktivatoren umfasst, gegebenenfalls wobei es sich bei dem Inflammasom-Aktivator um ein Chemotherapeutikum handelt, weiter gegebenenfalls wobei das Chemotherapeutikum aus der Gruppe bestehend aus Platin (einschließlich Platin-Chemotherapeutikum), Paclitaxel (Taxol), Sorafenib, Doxorubicin, Sorafenib, 5-FU, Gemcitabin und Irinotecan (CPT-11) ausgewählt ist, vorzugsweise wobei es sich bei dem Platin-Chemotherapeutikum um Oxaliplatin oder Cisplatin handelt; und/oder
c) der Anti-IL18-BP-Antikörper und der immunstimulierende Antikörper, die Zytokintherapie, der Immunmodulator, die Zytostatika, Chemotherapeutika, Wachstumshemmer, Antihormonmittel, Kinase-Inhibitoren, Antiangiogenetika, Kardioprotektoren, Immunsuppressiva, die Proliferation hämatologischer Zellen fördernden Mittel, Angiogenesehemmer, Proteintyrosinkinase(PTK)-Hemmer oder anderen Therapeutika nacheinander oder gleichzeitig in beliebiger Reihenfolge und in einer oder mehreren Formulierungen verabreicht werden.

22. Anti-IL18-BP-Antikörper zur Verwendung gemäß einem der Ansprüche 15-21, wobei der Anti-IL18-BP-Antikörper eine Bindungsaffinität oder KD kleiner 0,25 pM, 0,30 pM, 0,35 pM, 0,40 pM, 0,45 pM, 0,50 pM, 0,55 pM, 0,60 pM, 0,65 pM, 0,70 pM, 0,75 pM, 0,80 pM, 0,85 pM, 0,90 pM, 0,95 pM oder 1 pM zeigt.

23. Zusammensetzung, umfassend den Anti-IL18-BP-Antikörper gemäß einem der Ansprüche 1-14.

24. Zusammensetzung, umfassend den Anti-IL18-BP-Antikörper gemäß einem der Ansprüche 1-14 zur Verwendung gemäß einem der Ansprüche 15-22.

## Revendications

1. Anticorps anti-IL18-BP (protéine de liaison à l'interleukine 18), l'anticorps anti-IL18-BP se liant spécifiquement à IL18-BP humaine et présentant une affinité de liaison ou KD inférieure ou égale à 1 pM, mesurée par titrage à l'équilibre en solution, et ledit anticorps comprenant une vhCDR1, une vhCDR2, une vhCDR3, une vlCDR1, une vlCDR2 et une vlCDR3, la vhCDR1, la vhCDR2, la vhCDR3, la vlCDR1, la vlCDR2 et la vlCDR3 étant choisies dans le groupe constitué par :
i. une vhCDR1 ayant la séquence d'acides aminés de SEQ ID NO : 155,
une vhCDR2 ayant la séquence d'acides aminés de SEQ ID NO : 156,
une vhCDR3 ayant la séquence d'acides aminés de SEQ ID NO : 157,
une vlCDR1 ayant la séquence d'acides aminés de SEQ ID NO : 160,
une vlCDR2 ayant la séquence d'acides aminés de SEQ ID NO : 161, et
une vlCDR3 ayant la séquence d'acides aminés de SEQ ID NO : 162 ;
ii. une vhCDR1 ayant la séquence d'acides aminés de SEQ ID NO : 75,
une vhCDR2 ayant la séquence d'acides aminés de SEQ ID NO : 76,
une vhCDR3 ayant la séquence d'acides aminés de SEQ ID NO : 77,
une vlCDR1 ayant la séquence d'acides aminés de SEQ ID NO : 80,
une vlCDR2 ayant la séquence d'acides aminés de SEQ ID NO : 81, et
une vlCDR3 ayant la séquence d'acides aminés de SEQ ID NO : 82 ;
iii. une vhCDR1 ayant la séquence d'acides aminés de SEQ ID NO : 85,
une vhCDR2 ayant la séquence d'acides aminés de SEQ ID NO : 86,
une vhCDR3 ayant la séquence d'acides aminés de SEQ ID NO : 87,
une vlCDR1 ayant la séquence d'acides aminés de SEQ ID NO : 90,
une vlCDR2 ayant la séquence d'acides aminés de SEQ ID NO : 91, et
une vlCDR3 ayant la séquence d'acides aminés de SEQ ID NO : 92 ;
iv. une vhCDR1 ayant la séquence d'acides aminés de SEQ ID NO : 105,
une vhCDR2 ayant la séquence d'acides aminés de SEQ ID NO : 106,
une vhCDR3 ayant la séquence d'acides aminés de SEQ ID NO : 107,
une vlCDR1 ayant la séquence d'acides aminés de SEQ ID NO : 110,
une vlCDR2 ayant la séquence d'acides aminés de SEQ ID NO : 111, et
une vlCDR3 ayant la séquence d'acides aminés de SEQ ID NO : 112 ; et
v. une vhCDR1 ayant la séquence d'acides aminés de SEQ ID NO : 195,
une vhCDR2 ayant la séquence d'acides aminés de SEQ ID NO : 196,
une vhCDR3 ayant la séquence d'acides aminés de SEQ ID NO : 197,
une vlCDR1 ayant la séquence d'acides aminés de SEQ ID NO : 200,
une vlCDR2 ayant la séquence d'acides aminés de SEQ ID NO : 201, et
une vlCDR3 ayant la séquence d'acides aminés de SEQ ID NO : 202.

2. Anticorps anti-IL18-BP selon la revendication 1, ledit anticorps comprenant
une vhCDR1 ayant une séquence d'acides aminés de SEQ ID NO : 155,
une vhCDR2 ayant une séquence d'acides aminés de SEQ ID NO : 156,
une vhCDR3 ayant une séquence d'acides aminés de SEQ ID NO : 157,
une vlCDR1 ayant une séquence d'acides aminés de SEQ ID NO : 160,
une vlCDR2 ayant une séquence d'acides aminés de SEQ ID NO : 161, et
une vlCDR3 ayant une séquence d'acides aminés de SEQ ID NO : 162.

3. Anticorps anti-IL18-BP selon la revendication 1, ledit anticorps comprenant :
une vhCDR1 ayant une séquence d'acides aminés de SEQ ID NO : 85,
une vhCDR2 ayant une séquence d'acides aminés de SEQ ID NO : 86,
une vhCDR3 ayant une séquence d'acides aminés de SEQ ID NO : 87,
une vlCDR1 ayant une séquence d'acides aminés de SEQ ID NO : 90,
une vlCDR2 ayant une séquence d'acides aminés de SEQ ID NO : 91, et
une vlCDR3 ayant une séquence d'acides aminés de SEQ ID NO : 92.

4. Anticorps anti-IL18-BP selon la revendication 1, ledit anticorps comprenant un domaine variable de chaîne lourde et un domaine variable de chaîne légère, le domaine variable de chaîne lourde et le domaine variable de chaîne légère étant choisis dans le groupe constitué par :
i. un domaine variable de chaîne lourde ayant la séquence d'acides aminés de SEQ ID NO : 154 et un domaine variable de chaîne légère ayant la séquence d'acides aminés de SEQ ID NO : 159 ;
ii. Un domaine variable de chaîne lourde ayant la séquence d'acides aminés de SEQ ID NO : 74 et un domaine variable de chaîne légère ayant la séquence d'acides aminés de SEQ ID NO : 79 ;
iii. un domaine variable de chaîne lourde ayant la séquence d'acides aminés de SEQ ID NO : 84 et un domaine variable de chaîne légère ayant la séquence d'acides aminés de SEQ ID NO : 89 ;
iv. un domaine variable de chaîne lourde ayant la séquence d'acides aminés de SEQ ID NO : 104 et un domaine variable de chaîne légère ayant la séquence d'acides aminés de SEQ ID NO : 109 ; et
v. un domaine variable de chaîne lourde ayant la séquence d'acides aminés de SEQ ID NO : 194 et un domaine variable de chaîne légère ayant la séquence d'acides aminés de SEQ ID NO : 199.

5. Anticorps anti-IL18-BP selon la revendication 2, dans lequel le domaine variable de chaîne lourde comprend une séquence d'acides aminés présentant au moins 90 % d'identité de séquence avec SEQ ID NO : 154, et le domaine variable de chaîne légère comprend une séquence d'acides aminés présentant au moins 90 % d'identité de séquence avec SEQ ID NO : 159.

6. Anticorps anti-IL18-BP selon la revendication 5, dans lequel le domaine variable de chaîne lourde comprend une séquence d'acides aminés présentant au moins 95 % d'identité de séquence avec SEQ ID NO : 154, et le domaine variable de chaîne légère comprend une séquence d'acides aminés présentant au moins 95 % d'identité de séquence avec SEQ ID NO : 159.

7. Anticorps anti-IL18-BP selon la revendication 5 ou 6, dans lequel le domaine variable de chaîne lourde comprend une séquence d'acides aminés de SEQ ID NO : 154, et le domaine variable de chaîne légère comprend une séquence d'acides aminés de SEQ ID NO : 159.

8. Anticorps anti-IL18-BP selon la revendication 3, dans lequel le domaine variable de chaîne lourde comprend une séquence d'acides aminés présentant au moins 90 % d'identité de séquence avec SEQ ID NO : 84, et le domaine variable de chaîne légère comprend une séquence d'acides aminés présentant au moins 90 % d'identité de séquence avec SEQ ID NO : 89.

9. Anticorps anti-IL18-BP selon la revendication 8, dans lequel le domaine variable de chaîne lourde comprend une séquence d'acides aminés présentant au moins 95 % d'identité de séquence avec SEQ ID NO : 84, et le domaine variable de chaîne légère comprend une séquence d'acides aminés présentant au moins 95 % d'identité de séquence avec SEQ ID NO : 89.

10. Anticorps anti-IL18-BP selon la revendication 8 ou 9, dans lequel le domaine variable de chaîne lourde comprend une séquence d'acides aminés de SEQ ID NO : 84, et le domaine variable de chaîne légère comprend une séquence d'acides aminés de SEQ ID NO : 89.

11. Anticorps anti-IL18-BP selon l'une quelconque des revendications 1 à 10, ledit anticorps comprenant :
a) une région CH1-charnière-CH2-CH3 d'IgG1, IgG2 ou IgG3 humaine ;
b) une région CH1-charnière-CH2-CH3 d'IgG4 humaine ;
c) une région CL de la chaîne légère kappa 2 humaine ; et/ou
d) une région CL de la chaîne légère lambda 2 humaine.

12. Anticorps anti-IL18-BP selon l'une quelconque des revendications 1, 4 ou 11, ledit anticorps comprenant une chaîne lourde et une chaîne légère, la chaîne lourde et la chaîne légère étant choisies dans le groupe constitué par :
i. une chaîne lourde ayant la séquence d'acides aminés de SEQ ID NO : 158 et une chaîne légère ayant la séquence d'acides aminés de SEQ ID NO : 163 ;
ii. une chaîne lourde ayant la séquence d'acides aminés de SEQ ID NO : 78 et une chaîne légère ayant la séquence d'acides aminés de SEQ ID NO : 83 ;
iii. une chaîne lourde ayant la séquence d'acides aminés de SEQ ID NO : 88 et une chaîne légère ayant la séquence d'acides aminés de SEQ ID NO : 93 ;
iv. une chaîne lourde ayant la séquence d'acides aminés de SEQ ID NO : 108 et une chaîne légère ayant la séquence d'acides aminés de SEQ ID NO : 113 ; et
v. une chaîne lourde ayant la séquence d'acides aminés de SEQ ID NO : 198 et une chaîne légère ayant la séquence d'acides aminés de SEQ ID NO : 203.

13. Anticorps anti-IL18-BP selon la revendication 7, dans lequel la chaîne lourde comprend une séquence d'acides aminés de SEQ ID NO : 158, et la chaîne légère comprend une séquence d'acides aminés de SEQ ID NO : 163.

14. Anticorps anti-IL18-BP de la revendication 10, dans lequel la chaîne lourde comprend une séquence d'acides aminés de SEQ ID NO : 88, et la chaîne légère comprend une séquence d'acides aminés de SEQ ID NO : 93.

15. Anticorps anti-IL18-BP selon l'une quelconque des revendications 1 à 14, destiné à être utilisé dans une méthode de traitement.

16. Anticorps anti-IL18-BP selon l'une quelconque des revendications 1 à 14, l'anticorps activant les lymphocytes T, les cellules NK, les cellules NKT, les cellules dendritiques, les lymphocytes T MAIT, les lymphocytes T γδ, et/ou les cellules lymphoïdes innées (ILC), et/ou modulant les cellules myéloïdes, et l'anticorps étant destiné à être utilisé dans une méthode de traitement du cancer.

17. Anticorps anti-IL18-BP selon la revendication 16, la méthode comprenant l'administration de l'anticorps anti-IL18-BP à un patient, et **caractérisé en ce que** :
a) les lymphocytes T, les cellules NK, les cellules NKT, les cellules dendritiques, les lymphocytes T MAIT, les lymphocytes T γδ, ou les ILC du patient sont activés ou les cellules myéloïdes du patient sont modulées ;
b) ledit anticorps anti-IL18-BP augmente l'activité immunostimulante véhiculée par l'IL-18 dans le microenvironnement tumoral (TME) et/ou les ganglions lymphatiques ; ou
c) ledit anticorps anti-IL18-BP restaure l'activité sur les lymphocytes T, les cellules NK, les cellules NKT, les cellules myéloïdes, les cellules dendritiques, et/ou les ILC.

18. Anticorps anti-IL18-BP selon la revendication 16 ou 17, **caractérisé en ce que** :
a) lesdits lymphocytes T sont des lymphocytes T cytotoxiques (CTL), facultativement, lesdits lymphocytes T étant choisis dans le groupe constitué par les lymphocytes T CD4⁺ et les lymphocytes T CD8⁺ ; et/ou
b) le patient devant recevoir le traitement :
i) présente une augmentation de l'inhibition de croissance tumorale d'au moins environ 10 %, 20 %, 30 %, 40 %, 50 %, 60 %, 70 %, 80 %, 90 %, 100 %, 125 %, 150 %, 175 %, 200 %, 225 %, 250 %, 275 %, 300 %, 325 %, 350 %, 375 %, 400 %, 425 %, 450 %, 475 %, 500 %, 525 %, 550 %, 575 %, 600 %, 625 %, 650 %, 675 %, 700 %, 725 %, 750 %, 775 %, 800 %, 825 %, 850 %, 875 %, 900 %, 925 %, 950 %, 975 % ou 1 000 %, par rapport à un patient témoin ou non traité ; ou
ii) présente une diminution de la croissance tumorale d'au moins environ 10 %, 20 %, 30 %, 40 %, 50 %, 60 %, 70 %, 80 %, 90 %, 100 %, 125 %, 150 %, 175 %, 200 %, 225 %, 250 %, 275 %, 300 %, 325 %, 350 %, 375 %, 400 %, 425 %, 450 %, 475 %, 500 %, 525 %, 550 %, 575 %, 600 %, 625 %, 650 %, 675 %, 700 %, 725 %, 750 %, 775 %, 800 %, 825 %, 850 %, 875 %, 900 %, 925 %, 950 %, 975 % ou 1 000 %, par rapport à un patient témoin ou non traité.

19. Anticorps anti-IL18-BP selon l'une quelconque des revendications 16 à 18, **caractérisé en ce que** :
a) lesdites cellules NK sont des lymphocytes CD16+ ;
b) lesdites cellules NK sont des cellules NK CD56+ ; ou
c) ladite activation est mesurée comme une augmentation de l'expression d'un ou plusieurs agents d'activation.

20. Anticorps anti-IL18-BP selon l'une quelconque des revendications 15 à 19, le procédé comprenant en outre l'administration d'un deuxième anticorps, facultativement **caractérisé en ce que** :
a) ledit deuxième anticorps est un anticorps qui se lie à une protéine de récepteur de point de contrôle humain et/ou inhibe celle-ci ; et/ou
b) ledit deuxième anticorps est choisi dans le groupe constitué par un anticorps anti-PVRIG, un anticorps anti-PD-1, un anticorps anti-PD-L1, un anticorps anti-TIGIT, un anticorps anti-CTLA-4, un anticorps anti-PD-L2, un anticorps anti-B7-H3, un anticorps anti-B7-H4, un anticorps anti-CEACAM-1, un anticorps anti-PVR, un anticorps anti-LAG3, un anticorps anti-CD112, un anticorps anti-CD96, un anticorps anti-TIM3, un anticorps anti-BTLA, un anticorps anti-ICOS, un anticorps anti-OX40, ou un anticorps anti-41BB, un anticorps anti-CD27 ou un anticorps anti-GITR.

21. Anticorps anti-IL18-BP selon l'une quelconque des revendications 15 à 20, **caractérisé en ce que** :
a) ledit anticorps anti-IL18-BP est destiné à être utilisé en association avec un anticorps immunostimulant, un traitement par des cytokines, un médicament immunomodulateur, des agents cytotoxiques, des agents chimiothérapeutiques, des agents inhibiteurs de croissance, des agents antihormonaux, des inhibiteurs de kinases, des agents antiangiogéniques, des cardioprotecteurs, des agents immunosuppresseurs, des agents favorisant la prolifération des cellules hématologiques, des inhibiteurs de l'angiogenèse, des inhibiteurs de protéine tyrosine kinase (PTK) ou d'autres agents thérapeutiques ;
b) la méthode comprend en outre l'administration d'un ou plusieurs activateurs de l'inflammasome, facultativement, ledit activateur de l'inflammasome étant un agent chimiothérapeutique, en outre, facultativement, ledit agent chimiothérapie étant choisi dans le groupe constitué par le platine (notamment un agent chimiothérapeutique à base de platine), le paclitaxel (taxol), le sorafénib, la doxorubicine, le sorafénib, 5-FU, la gemcitabine et l'irinotécan (CPT-11), de préférence, ledit agent chimiothérapeutique à base de platine étant l'oxaliplatine ou le cisplatine ; et/ou
c) l'anticorps anti-IL18-BP et l'anticorps immunostimulant, le traitement par des cytokines, le médicament immunomodulateur, les agents cytotoxiques, les agents chimiothérapeutiques, les agents inhibiteurs de croissance, les agents antihormonaux, les inhibiteurs de kinase, les agents antiangiogéniques, les cardioprotecteurs, les agents immunosuppresseurs, les agents favorisant la prolifération de cellules hématologiques, les inhibiteurs d'angiogenèse, les inhibiteurs de protéine tyrosine kinase (PTK) ou les autres agents thérapeutiques sont administrés séquentiellement ou simultanément, dans un ordre quelconque, et dans une ou plusieurs formulations.

22. Anticorps anti-IL18-BP selon l'une quelconque des revendications 15 à 21, l'anticorps anti-IL18-BP présentant une affinité de liaison ou KD inférieure à 0,25 pM, 0,30 pM, 0,35 pM, 0,40 pM, 0,45 pM, 0,50 pM, 0,55 pM, 0,60 pM, 0,65 pM, 0,70 pM, 0,75 pM, 0,80 pM, 0,85 pM, 0,90 pM, 0,95 pM ou 1 pM.

23. Composition comprenant l'anticorps anti-IL18-BP selon l'une quelconque des revendications 1 à 14.

24. Composition comprenant l'anticorps anti-IL18-BP selon l'une quelconque des revendications 1 à 14 pour une utilisation selon l'une quelconque des revendications 15 à 22.
